# EUROPEAN PATENT SPECIFICATION

(11) **EP 0 871 737 B1**
(45) Date of publication and mention of the grant of the patent: **13.04.2005**
(21) Application number: 96944651.7
(22) Date of filing: 23.12.1996
(51) Int. Cl.: C12N 15/32, C12N 15/82, C07K 14/325, C07K 14/32, A01N 63/00, A01H 5/00

(54) **METHOD OF PROTECTING CROP PLANTS AGAINST INSECT PESTS**
VERFAHREN ZUM SCHUTZ VON KULTURPFLANZEN VOR SCHÄDLICHEN INSEKTEN
PROCEDE DE PROTECTION DE PLANTES CULTIVEES CONTRE LES INSECTES NUISIBLES

(30) Priority: 15.01.1996 GB 9600786
(43) Date of publication of application: 21.10.1998
(73) Proprietor: Syngenta Participations AG, 4058 Basel (CH)
(72) Inventor: GAY, Philippe, Bernard, F-68100 Mulhouse (FR)
(74) Representative: Gaal, Jozsef Christopher
(86) International application number: PCT/EP1996/005828
(87) International publication number: WO 1997/026339

(56) References cited:
- EP-A- 0 142 924
- WO-A-93/07278
- WO-A-94/21795
- WO-A-96/10083
- YADAVA C P: "TOXICITY OF BACILLUS - THURINGIENSIS TO THE LARVAE OF SESAMIA -INFERENS NOCTUIDAE LEPIDOPTERA THE PINK BORER OF RICE." ORYZA, vol. 15, no. 1, 1979, page 105 XP000671146
- KOZIEL, M. ET AL.: "Field performance of elite transgenic maize plants expressing an insecticidal protein derived from Bacillus thuringiensis" BIOTECHNOLOGY, vol. 11, February 1993 (1993-02), pages 194-200, XP002029715
- ESTRUCH J J ET AL: "VIP3A, A NOVEL BACILLUS THURINGIENSIS VEGETATIVE INSECTICIDAL PROTEIN WITH A WIDE SPECTRUM OF ACTIVITIES AGAINST LEPIDOPTERAN INSECTS" PROCEEDINGS OF THE NATIONAL ACADEMY OF SCIENCES OF USA, vol. 93, 1 May 1996 (1996-05-01), pages 5389-5394, XP002071759 ISSN: 0027-8424
- KOZIEL, M. ET AL.: "Transgenic maize for the control of European corn borer and other maize insect pests" ANN.N.Y.ACAD.SCI., vol. 792, 1996, pages 164-171, XP000673013

### SEQUENCE LISTING

(1) GENERAL INFORMATION:
   (A) NAME: CIBA-GEIGY AG
   (B) STREET: Klybeckstr. 141
   (C) CITY: Basel
   (E) COUNTRY: Switzerland
   (F) POSTAL CODE (ZIP): 4002
   (G) TELEPHONE: +41 61 69 11 11
   (H) TELEFAX: + 41 61 696 79 76
   (I) TELEX: 962 991

   (ii) TITLE OF INVENTION: Method of Controlling Insect Pests
      (iii) NUMBER OF SEQUENCES: 55
      (iv) COMPUTER READABLE FORM:
         (A) MEDIUM TYPE: Floppy disk
         (B) COMPUTER: IBM PC compatible
         (C) OPERATING SYSTEM: PC-DOS/MS-DOS
         (D) SOFTWARE: PatentIn Release #1.0, Version #1.30B
(2) INFORMATION FOR SEQ ID NO:1:
   (i) SEQUENCE CHARACTERISTICS:
      (A) LENGTH: 6049 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: Bacillus cereus
      (B) STRAIN: AB78
      (C) INDIVIDUAL ISOLATE: NRRL B-21058
   (ix) FEATURE:
      (A) NAME/KEY: CDS
      (B) LOCATION: 1082..2467
      (D) OTHER INFORMATION: /product= "VIP2A(a)"
   (ix) FEATURE:
      (A) NAME/KEY: misc_feature
      (B) LOCATION: 2475..5126
      (D) OTHER INFORMATION: /note= "Coding sequence for the 100 kd VIP1A(a) protein. This coding sequence is repeated in SEQ ID NO:4 and translated separately."
   (xi) SEQUENCE DESCRIPTION: SEQ ID NO:1:
   ATCGATACAA TGTTGTTTTA CTTAGACCGG TAGTCTCTGT AATTTGTTTA ATGCTATATT 60
   CTTTACTTTG ATACATTTTA ATAGCCATTT CAACCTTATC AGTATGTTTT TGTGGTCTTC 120
   CTCCTTTTTT TCCACGAGCT CTAGCTGCGT TTAATCCTGT TTTGGTACGT TCGCTAATAA 180
   TATCTCTTTC TAATTCTGCA ATACTTGCCA TCATTCGAAA GAAGAATTTC CCCATAGCAT 240
   TAGAGGTATC AATGTTGTCA TGAATAGAAA TAAAATCTAC ACCTAGCTCT TTGAATTTTT 300
   CACTTAACTC AATTAGGTGT TTTGTAGAGC GAGAAATTCG ATCAAGTTTG TAAACAACTA 360
   TCTTATCGCC TTTACGTAAT ACTTTTAGCA ACTCTTCGAG TTGAGGGCGC TCTTTTTTTA 420
   TTCCTGTTAT TTTCTCCTGA TATAGCCTTT CTACACCATA TTGTTGCAAA GCATCTATTT 480
   GCATATCGAG ATTTTGTTCT TCTGTGCTGA CACGAGCATA ACCAAAAATC AAATTGGTTT 540
   CACTTCCTAT CTAAATATAT CTATTAAAAT AGCACCAAAA ACCTTATTAA ATTAAAATAA 600
   GGAACTTTGT TTTTGGATAT GGATTTTGGT ACTCAATATG GATGAGTTTT TAACGCTTTT 660
   GTTAAAAAAC AAACAAGTGC CATAAACGGT CGTTTTTGGG ATGACATAAT AAATAATCTG 720
   TTTGATTAAC CTAACCTTGT ATCCTTACAG CCCAGTTTTA TTTGTACTTC AACTGACTGA 780
   ATATGAAAAC AACATGAAGG TTTCATAAAA TTTATATATT TTCCATAACG GATGCTCTAT 840
   CTTTAGGTTA TAGTTAAATT ATAAGAAAAA AACAAACGGA GGGAGTGAAA AAAAGCATCT 900
   TCTCTATAAT TTTACAGGCT CTTTAATAAG AAGGGGGGAG ATTAGATAAT AAATATGAAT 960
   ATCTATCTAT AATTGTTTGC TTCTACAATA ACTTATCTAA CTTTCATATA CAACAACAAA 1020
   ACAGACTAAA TCCAGATTGT ATATTCATTT TCAGTTGTTC CTTTATAAAA TAATTTCATA 1080
   A ATG AAA AGA ATG GAG GGA AAG TTG TTT ATG GTG TCA AAA AAA TTA 1126
   Met Lys Arg Met Glu Gly Lys Leu Phe Met Val Ser Lys Lys Leu
   1 5 10 15
   CAA GTA GTT ACT AAA ACT GTA TTG CTT AGT ACA GTT TTC TCT ATA TCT 1174
   Gln Val Val Thr Lys Thr Val Leu Leu Ser Thr Val Phe Ser Ile Ser
   20 25 30
   TTA TTA AAT AAT GAA GTG ATA AAA GCT GAA CAA TTA AAT ATA AAT TCT 1222
   Leu Leu Asn Asn Glu Val Ile Lys Ala Glu Gln Leu Asn Ile Asn Ser
   35 40 45
   CAA AGT AAA TAT ACT AAC TTG CAA AAT CTA AAA ATC ACT GAC AAG GTA 1270
   Gln Ser Lys Tyr Thr Asn Leu Gln Asn Leu Lys Ile Thr Asp Lys Val
   50 55 60
   GAG GAT TTT AAA GAA GAT AAG GAA AAA GCG AAA GAA TGG GGG AAA GAA 1318
   Glu Asp Phe Lys Glu Asp Lys Glu Lys Ala Lys Glu Trp Gly Lys Glu
   65 70 75
   AAA GAA AAA GAG TGG AAA CTA ACT GCT ACT GAA AAA GGA AAA ATG AAT 1366
   Lys Glu Lys Glu Trp Lys Leu Thr Ala Thr Glu Lys Gly Lys Met Asn
   80 85 90 95
   AAT TTT TTA GAT AAT AAA AAT GAT ATA AAG ACA AAT TAT AAA GAA ATT 1414
   Asn Phe Leu Asp Asn Lys Asn Asp Ile Lys Thr Asn Tyr Lys Glu Ile
   100 105 110
   ACT TTT TCT ATG GCA GGC TCA TTT GAA GAT GAA ATA AAA GAT TTA AAA 1462
   Thr Phe Ser Met Ala Gly Ser Phe Glu Asp Glu Ile Lys Asp Leu Lys
   115 120 125
   GAA ATT GAT AAG ATG TTT GAT AAA ACC AAT CTA TCA AAT TCT ATT ATC 1510
   Glu Ile Asp Lys Met Phe Asp Lys Thr Asn Leu Ser Asn Ser Ile Ile
   130 135 140
   ACC TAT AAA AAT GTG GAA CCG ACA ACA ATT GGA TTT AAT AAA TCT TTA 1558
   Thr Tyr Lys Asn Val Glu Pro Thr Thr Ile Gly Phe Asn Lys Ser Leu
   145 150 155
   ACA GAA GGT AAT ACG ATT AAT TCT GAT GCA ATG GCA CAG TTT AAA GAA 1606
   Thr Glu Gly Asn Thr Ile Asn Ser Asp Ala Met Ala Gln Phe Lys Glu
   160 165 170 175
   CAA TTT TTA GAT AGG GAT ATT AAG TTT GAT AGT TAT CTA GAT ACG CAT 1654
   Gln Phe Leu Asp Arg Asp Ile Lys Phe Asp Ser Tyr Leu Asp Thr His
   180 185 190
   TTA ACT GCT CAA CAA GTT TCC AGT AAA GAA AGA GTT ATT TTG AAG GTT 1702
   Leu Thr Ala Gln Gln Val Ser Ser Lys Glu Arg Val Ile Leu Lys Val
   195 200 205
   ACG GTT CCG AGT GGG AAA GGT TCT ACT ACT CCA ACA AAA GCA GGT GTC 1750
   Thr Val Pro Ser Gly Lys Gly Ser Thr Thr Pro Thr Lys Ala Gly Val
   210 215 220
   ATT TTA AAT AAT AGT GAA TAC AAA ATG CTC ATT GAT AAT GGG TAT ATG 1798
   Ile Leu Asn Asn Ser Glu Tyr Lys Met Leu Ile Asp Asn Gly Tyr Met
   225 230 235
   GTC CAT GTA GAT AAG GTA TCA AAA GTG GTG AAA AAA GGG GTG GAG TGC 1846
   Val His Val Asp Lys Val Ser Lys Val Val Lys Lys Gly Val Glu Cys
   240 245 250 255
   TTA CAA ATT GAA GGG ACT TTA AAA AAG AGT CTT GAC TTT AAA AAT GAT 1894
   Leu Gln Ile Glu Gly Thr Leu Lys Lys Ser Leu Asp Phe Lys Asn Asp
   260 265 270
   ATA AAT GCT GAA GCG CAT AGC TGG GGT ATG AAG AAT TAT GAA GAG TGG 1942
   Ile Asn Ala Glu Ala His Ser Trp Gly Met Lys Asn Tyr Glu Glu Trp
   275 280 285
   GCT AAA GAT TTA ACC GAT TCG CAA AGG GAA GCT TTA GAT GGG TAT GCT 1990
   Ala Lys Asp Leu Thr Asp Ser Gln Arg Glu Ala Leu Asp Gly Tyr Ala
   290 295 300
   AGG CAA GAT TAT AAA GAA ATC AAT AAT TAT TTA AGA AAT CAA GGC GGA 2038
   Arg Gln Asp Tyr Lys Glu Ile Asn Asn Tyr Leu Arg Asn Gln Gly Gly
   305 310 315
   AGT GGA AAT GAA AAA CTA GAT GCT CAA ATA AAA AAT ATT TCT GAT GCT 2086
   Ser Gly Asn Glu Lys Leu Asp Ala Gln Ile Lys Asn Ile Ser Asp Ala
   320 325 330 335
   TTA GGG AAG AAA CCA ATA CCG GAA AAT ATT ACT GTG TAT AGA TGG TGT 2134
   Leu Gly Lys Lys Pro Ile Pro Glu Asn Ile Thr Val Tyr Arg Trp Cys
   340 345 350
   GGC ATG CCG GAA TTT GGT TAT CAA ATT AGT GAT CCG TTA CCT TCT TTA 2182
   Gly Met Pro Glu Phe Gly Tyr Gln Ile Ser Asp Pro Leu Pro Ser Leu
   355 360 365
   AAA GAT TTT GAA GAA CAA TTT TTA AAT ACA ATC AAA GAA GAC AAA GGA 2230
   Lys Asp Phe Glu Glu Gln Phe Leu Asn Thr Ile Lys Glu Asp Lys Gly
   370 375 380
   TAT ATG AGT ACA AGC TTA TCG AGT GAA CGT CTT GCA GCT TTT GGA TCT 2278
   Tyr Met Ser Thr Ser Leu Ser Ser Glu Arg Leu Ala Ala Phe Gly Ser
   385 390 395
   AGA AAA ATT ATA TTA CGA TTA CAA GTT CCG AAA GGA AGT ACG GGT GCG 2326
   Arg Lys Ile Ile Leu Arg Leu Gln Val Pro Lys Gly Ser Thr Gly Ala
   400 405 410 415
   TAT TTA AGT GCC ATT GGT GGA TTT GCA AGT GAA AAA GAG ATC CTA CTT 2374
   Tyr Leu Ser Ala Ile Gly Gly Phe Ala Ser Glu Lys Glu Ile Leu Leu
   420 425 430
   GAT AAA GAT AGT AAA TAT CAT ATT GAT AAA GTA ACA GAG GTA ATT ATT 2422
   Asp Lys Asp Ser Lys Tyr His Ile Asp Lys Val Thr Glu Val Ile Ile
   435 440 445
   AAA GGT GTT AAG CGA TAT GTA GTG GAT GCA ACA TTA TTA ACA AAT 2467
   Lys Gly Val Lys Arg Tyr Val Val Asp Ala Thr Leu Leu Thr Asn
   450 455 460
   TAAGGAGATG AAAAATATGA AGAAAAAGTT AGCAAGTGTT GTAACGTGTA CGTTATTAGC 2527
   TCCTATGTTT TTGAATGGAA ATGTGAATGC TGTTTACGCA GACAGCAAAA CAAATCAAAT 2587
   TTCTACAACA CAGAAAAATC AACAGAAAGA GATGGACCGA AAAGGATTAC TTGGGTATTA 2647
   TTTCAAAGGA AAAGATTTTA GTAATCTTAC TATGTTTGCA CCGACACGTG ATAGTACTCT 2707
   TATTTATGAT CAACAAACAG CAAATAAACT ATTAGATAAA AAACAACAAG AATATCAGTC 2767
   TATTCGTTGG ATTGGTTTGA TTCAGAGTAA AGAAACGGGA GATTTCACAT TTAACTTATC 2827
   TGAGGATGAA CAGGCAATTA TAGAAATCAA TGGGAAAATT ATTTCTAATA AAGGGAAAGA 2887
   AAAGCAAGTT GTCCATTTAG AAAAAGGAAA ATTAGTTCCA ATCAAAATAG AGTATCAATC 2947
   AGATACAAAA TTTAATATTG ACAGTAAAAC ATTTAAAGAA CTTAAATTAT TTAAAATAGA 3007
   TAGTCAAAAC CAACCCCAGC AAGTCCAGCA AGATGAACTG AGAAATCCTG AATTTAACAA 3067
   GAAAGAATCA CAGGAATTCT TAGCGAAACC ATCGAAAATA AATCTTTTCA CTCAAAAAAT 3127
   GAAAAGGGAA ATTGATGAAG ACACGGATAC GGATGGGGAC TCTATTCCTG ACCTTTGGGA 3187
   AGAAAATGGG TATACGATTC ACAATAGAAT CGCTGTAAAG TGGGACGATT CTCTAGCAAG 3247
   TAAAGGGTAT ACGAAATTTG TTTCAAATCC ACTAGAAAGT CACACAGTTG GTGATCCTTA 3307
   TACAGATTAT GAAAAGGCAG CAAGAGATCT AGATTTGTCA AATGCAAAGG AAACGTTTAA 3367
   CCCATTGGTA GCTGCTTTTC CAAGTGTGAA TGTTAGTATG GAAAAGGTGA TATTATCACC 3427
   AAATGAAAAT TTATCCAATA GTGTAGAGTC TCATTCATCC ACGAATTGGT CTTATACAAA 3487
   TACAGAAGGT GCTTCTGTTG AAGCGGGGAT TGGACCAAAA GGTATTTCGT TCGGAGTTAG 3547
   CGTAAACTAT CAACACTCTG AAACAGTTGC ACAAGAATGG GGAACATCTA CAGGAAATAC 3607
   TTCGCAATTC AATACGGCTT CAGCGGGATA TTTAAATGCA AATGTTCGAT ATAACAATGT 3667
   AGGAACTGGT GCCATCTACG ATGTAAAACC TACAACAAGT TTTGTATTAA ATAACGATAC 3727
   TATCGCAACT ATTACGGCGA AATCTAATTC TACAGCCTTA AATATATCTC CTGGAGAAAG 3787
   TTACCCGAAA AAAGGACAAA ATGGAATCGC AATAACATCA ATGGATGATT TTAATTCCCA 3847
   TCCGATTACA TTAAATAAAA AACAAGTAGA TAATCTGCTA AATAATAAAC CTATGATGTT 3907
   GGAAACAAAC CAAACAGATG GTGTTTATAA GATAAAAGAT ACACATGGAA ATATAGTAAC 3967
   TGGCGGAGAA TGGAATGGTG TCATACAACA AATCAAGGCT AAAACAGCGT CTATTATTGT 4027
   GGATGATGGG GAACGTGTAG CAGAAAAACG TGTAGCGGCA AAAGATTATG AAAATCCAGA 4087
   AGATAAAACA CCGTCTTTAA CTTTAAAAGA TGCCCTGAAG CTTTCATATC CAGATGAAAT 4147
   AAAAGAAATA GAGGGATTAT TATATTATAA AAACAAACCG ATATACGAAT CGAGCGTTAT 4207
   GACTTACTTA GATGAAAATA CAGCAAAAGA AGTGACCAAA CAATTAAATG ATACCACTGG 4267
   GAAATTTAAA GATGTAAGTC ATTTATATGA TGTAAAACTG ACTCCAAAAA TGAATGTTAC 4327
   AATCAAATTG TCTATACTTT ATGATAATGC TGAGTCTAAT GATAACTCAA TTGGTAAATG 4387
   GACAAACACA AATATTGTTT CAGGTGGAAA TAACGGAAAA AAACAATATT CTTCTAATAA 4447
   TCCGGATGCT AATTTGACAT TAAATACAGA TGCTCAAGAA AAATTAAATA AAAATCGTGA 4507
   CTATTATATA AGTTTATATA TGAAGTCAGA AAAAAACACA CAATGTGAGA TTACTATAGA 4567
   TGGGGAGATT TATCCGATCA CTACAAAAAC AGTGAATGTG AATAAAGACA ATTACAAAAG 4627
   ATTAGATATT ATAGCTCATA ATATAAAAAG TAATCCAATT TCTTCACTTC ATATTAAAAC 4687
   GAATGATGAA ATAACTTTAT TTTGGGATGA TATTTCTATA ACAGATGTAG CATCAATAAA 4747
   ACCGGAAAAT TTAACAGATT CAGAAATTAA ACAGATTTAT AGTAGGTATG GTATTAAGTT 4807
   AGAAGATGGA ATCCTTATTG ATAAAAAAGG TGGGATTCAT TATGGTGAAT TTATTAATGA 4867
   AGCTAGTTTT AATATTGAAC CATTGCAAAA TTATGTGACC AAATATGAAG TTACTTATAG 4927
   TAGTGAGTTA GGACCAAACG TGAGTGACAC ACTTGAAAGT GATAAAATTT ACAAGGATGG 4987
   GACAATTAAA TTTGATTTTA CCAAATATAG TAAAAATGAA CAAGGATTAT TTTATGACAG 5047
   TGGATTAAAT TGGGACTTTA AAATTAATGC TATTACTTAT GATGGTAAAG AGATGAATGT 5107
   TTTTCATAGA TATAATAAAT AGTTATTATA TCTATGAAGC TGGTGCTAAA GATAGTGTAA 5167
   AAGTTAATAT ACTGTAGGAT TGTAATAAAA GTAATGGAAT TGATATCGTA CTTTGGAGTG 5227
   GGGGATACTT TGTAAATAGT TCTATCAGAA ACATTAGACT AAGAAAAGTT ACTACCCCCA 5287
   CTTGAAAATG AAGATTCAAC TGATTACAAA CAACCTGTTA AATATTATAA GGTTTTAACA 5347
   AAATATTAAA CTCTTTATGT TAATACTGTA ATATAAAGAG TTTAATTGTA TTCAAATGAA 5407
   GCTTTCCCAC AAAATTAGAC TGATTATCTA ATGAAATAAT CAGTCTAATT TTGTAGAACA 5467
   GGTCTGGTAT TATTGTACGT GGTCACTAAA AGATATCTAA TATTATTGGG CAAGGCGTTC 5527
   CATGATTGAA TCCTCGAATG TCTTGCCCTT TTCATTTATT TAAGAAGGAT TGTGGAGAAA 5587
   TTATGGTTTA GATAATGAAG AAAGACTTCA CTTCTAATTT TTGATGTTAA ATAAATCAAA 5647
   ATTTGGCGAT TCACATTGTT TAATCCACTG ATAAAACATA CTGGAGTGTT CTTAAAAAAT 5707
   CAGCTTTTTT CTTTATAAAA TTTTGCTTAG CGTACGAAAT TCGTGTTTTG TTGGTGGGAC 5767
   CCCATGCCCA TCAACTTAAG AGTAAATTAG TAATGAACTT TCGTTCATCT GGATTAAAAT 5827
   AACCTCAAAT TAGGACATGT TTTTAAAAAT AAGCAGACCA AATAAGCCTA GAATAGGTAT 5887
   CATTTTTAAA AATTATGCTG CTTTCTTTTG TTTTCCAAAT CCATTATACT CATAAGCAAC 5947
   ACCCATAATG TCAAAGACTG TTTTTGTCTC ATATCGATAA GCTTGATATC GAATTCCTGC 6007
   AGCCCGGGGG ATCCACTAGT TCTAGAGCGG CCGCCACCGC GG 6049
(2) INFORMATION FOR SEQ ID NO:2:
   (i) SEQUENCE CHARACTERISTICS:
      (A) LENGTH: 462 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Lys Arg Met Glu Gly Lys Leu Phe Met Val Ser Lys Lys Leu Gln
   1               5                   10                  15
   Val Val Thr Lys Thr Val Leu Leu Ser Thr Val Phe Ser Ile Ser Leu
               20                  25                  30
   Leu Asn Asn Glu Val Ile Lys Ala Glu Gln Leu Asn Ile Asn Ser Gln
           35                  40                  45
   Ser Lys Tyr Thr Asn Leu Gln Asn Leu Lys Ile Thr Asp Lys Val Glu
       50                  55                  60
   Asp Phe Lys Glu Asp Lys Glu Lys Ala Lys Glu Trp Gly Lys Glu Lys
   65                  70                  75                  80
   Glu Lys Glu Trp Lys Leu Thr Ala Thr Glu Lys Gly Lys Met Asn Asn
                   85                  90                  95
   Phe Leu Asp Asn Lys Asn Asp Ile Lys Thr Asn Tyr Lys Glu Ile Thr
               100                 105                 110
   Phe Ser Met Ala Gly Ser Phe Glu Asp Glu Ile Lys Asp Leu Lys Glu
           115                 120                 125
   Ile Asp Lys Met Phe Asp Lys Thr Asn Leu Ser Asn Ser Ile Ile Thr
       130                 135                 140
   Tyr Lys Asn Val Glu Pro Thr Thr Ile Gly Phe Asn Lys Ser Leu Thr
   145                 150                 155                 160
   Glu Gly Asn Thr Ile Asn Ser Asp Ala Met Ala Gln Phe Lys Glu Gln
                   165                 170                 175
   Phe Leu Asp Arg Asp Ile Lys Phe Asp Ser Tyr Leu Asp Thr His Leu
               180                 185                 190
   Thr Ala Gln Gln Val Ser Ser Lys Glu Arg Val Ile Leu Lys Val Thr
           195                 200                 205
   Val Pro Ser Gly Lys Gly Ser Thr Thr Pro Thr Lys Ala Gly Val Ile
       210                 215                 220
   Leu Asn Asn Ser Glu Tyr Lys Met Leu Ile Asp Asn Gly Tyr Met Val
   225                 230                 235                 240
   His Val Asp Lys Val Ser Lys Val Val Lys Lys Gly Val Glu Cys Leu
   245 250 255
   Gln Ile Glu Gly Thr Leu Lys Lys Ser Leu Asp Phe Lys Asn Asp Ile
   260 265 270
   Asn Ala Glu Ala His Ser Trp Gly Met Lys Asn Tyr Glu Glu Trp Ala
   275 280 285
   Lys Asp Leu Thr Asp Ser Gln Arg Glu Ala Leu Asp Gly Tyr Ala Arg
   290 295 300
   Gln Asp Tyr Lys Glu Ile Asn Asn Tyr Leu Arg Asn Gln Gly Gly Ser
   305 310 315 320
   Gly Asn Glu Lys Leu Asp Ala Gln Ile Lys Asn Ile Ser Asp Ala Leu
   325 330 335
   Gly Lys Lys Pro Ile Pro Glu Asn Ile Thr Val Tyr Arg Trp Cys Gly
   340 345 350
   Met Pro Glu Phe Gly Tyr Gln Ile Ser Asp Pro Leu Pro Ser Leu Lys
   355 360 365
   Asp Phe Glu Glu Gln Phe Leu Asn Thr Ile Lys Glu Asp Lys Gly Tyr
   370 375 380
   Met Ser Thr Ser Leu Ser Ser Glu Arg Leu Ala Ala Phe Gly Ser Arg
   385 390 395 400
   Lys Ile Ile Leu Arg Leu Gln Val Pro Lys Gly Ser Thr Gly Ala Tyr
   405 410 415
   Leu Ser Ala Ile Gly Gly Phe Ala Ser Glu Lys Glu Ile Leu Leu Asp
   420 425 430
   Lys Asp Ser Lys Tyr His Ile Asp Lys Val Thr Glu Val Ile Ile Lys
   435 440 445
   Gly Val Lys Arg Tyr Val Val Asp Ala Thr Leu Leu Thr Asn
   450 455 460
(2) INFORMATION FOR SEQ ID NO:3:
   (i) SEQUENCE CHARACTERISTICS:
      (A) LENGTH: 20 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Peptide
      (B) LOCATION: 1..20
      (D) OTHER INFORMATION: /note= "Signal peptide for vacuolar targetting"
   (xi) SEQUENCE DESCRIPTION: SEQ ID NO:3:
   Ser Ser Ser Ser Phe Ala Asp Ser Asn Pro Ile Arg Val Thr Asp Arg
   1               5                   10                  15
   Ala Ala Ser Thr
               20
(2) INFORMATION FOR SEQ ID NO:4:
   (i) SEQUENCE CHARACTERISTICS:
      (A) LENGTH: 2655 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Bacillus cereus
      (B) STRAIN: AB78
      (C) INDIVIDUAL ISOLATE: NRRL B-21058
   (ix) FEATURE:
      (A) NAME/KEY: CDS
      (B) LOCATION: 1..2652
      (D) OTHER INFORMATION: /product= "100 kDa protein VEP1A(a)" /note= "This sequence is identical to the portion of SEQ ID NO:1 between and including nucleotide 2475 to 5126."
   (xi) SEQUENCE DESCRIPTION: SEQ ID NO:4:
   ATG AAA AAT ATG AAG AAA AAG TTA GCA AGT GTT GTA ACG TGT ACG TTA      48
   Met Lys Asn Met Lys Lys Lys Leu Ala Ser Val Val Thr Cys Thr Leu
       465                 470                 475
   TTA GCT CCT ATG TTT TTG AAT GGA AAT GTG AAT GCT GTT TAC GCA GAC      96
   Leu Ala Pro Met Phe Leu Asn Gly Asn Val Asn Ala Val Tyr Ala Asp
   480                 485                 490
   AGC AAA ACA AAT CAA ATT TCT ACA ACA CAG AAA AAT CAA CAG AAA GAG     144
   Ser Lys Thr Asn Gln Ile Ser Thr Thr Gln Lys Asn Gln Gln Lys Glu
   495                 500                 505                 510
   ATG GAC CGA AAA GGA TTA CTT GGG TAT TAT TTC AAA GGA AAA GAT TTT      192
   Met Asp Arg Lys Gly Leu Leu Gly Tyr Tyr Phe Lys Gly Lys Asp Phe
                   515                 520                 525
   AGT AAT CTT ACT ATG TTT GCA CCG ACA CGT GAT AGT ACT CTT ATT TAT      240
   Ser Asn Leu Thr Met Phe Ala Pro Thr Arg Asp Ser Thr Leu Ile Tyr
               530                 535                 540
   GAT CAA CAA ACA GCA AAT AAA CTA TTA GAT AAA AAA CAA CAA GAA TAT      288
   Asp Gln Gln Thr Ala Asn Lys Leu Leu Asp Lys Lys Gln Gln Glu Tyr
           545                 550                 555
   CAG TCT ATT CGT TGG ATT GGT TTG ATT CAG AGT AAA GAA ACG GGA GAT      336
   Gln Ser Ile Arg Trp Ile Gly Leu Ile Gln Ser Lys Glu Thr Gly Asp
       560                 565                 570
   TTC ACA TTT AAC TTA TCT GAG GAT GAA CAG GCA ATT ATA GAA ATC AAT      384
   Phe Thr Phe Asn Leu Ser Glu Asp Glu Gln Ala Ile Ile Glu Ile Asn
   575                 580                 585                 590
   GGG AAA ATT ATT TCT AAT AAA GGG AAA GAA AAG CAA GTT GTC CAT TTA      432
   Gly Lys Ile Ile Ser Asn Lys Gly Lys Glu Lys Gln Val Val His Leu
                   595                 600                 605
   GAA AAA GGA AAA TTA GTT CCA ATC AAA ATA GAG TAT CAA TCA GAT ACA      480
   Glu Lys Gly Lys Leu Val Pro Ile Lys Ile Glu Tyr Gln Ser Asp Thr
               610                 615                 620
   AAA TTT AAT ATT GAC AGT AAA ACA TTT AAA GAA CTT AAA TTA TTT AAA      528
   Lys Phe Asn Ile Asp Ser Lys Thr Phe Lys Glu Leu Lys Leu Phe Lys
           625                 630                 635
   ATA GAT AGT CAA AAC CAA CCC CAG CAA GTC CAG CAA GAT GAA CTG AGA      576
   Ile Asp Ser Gln Asn Gln Pro Gln Gln Val Gln Gln Asp Glu Leu Arg
       640                 645                 650
   AAT CCT GAA TTT AAC AAG AAA GAA TCA CAG GAA TTC TTA GCG AAA CCA      624
   Asn Pro Glu Phe Asn Lys Lys Glu Ser Gln Glu Phe Leu Ala Lys Pro
   655                 660                 665                 670
   TCG AAA ATA AAT CTT TTC ACT CAA AAA ATG AAA AGG GAA ATT GAT GAA      672
   Ser Lys Ile Asn Leu Phe Thr Gln Lys Met Lys Arg Glu Ile Asp Glu
                   675                 680                 685
   GAC ACG GAT ACG GAT GGG GAC TCT ATT CCT GAC CTT TGG GAA GAA AAT      720
   Asp Thr Asp Thr Asp Gly Asp Ser Ile Pro Asp Leu Trp Glu Glu Asn
               690                 695                 700
   GGG TAT ACG ATT CAA AAT AGA ATC GCT GTA AAG TGG GAC GAT TCT CTA      768
   Gly Tyr Thr Ile Gln Asn Arg Ile Ala Val Lys Trp Asp Asp Ser Leu
           705                 710                 715
   GCA AGT AAA GGG TAT ACG AAA TTT GTT TCA AAT CCA CTA GAA AGT CAC      816
   Ala Ser Lys Gly Tyr Thr Lys Phe Val Ser Asn Pro Leu Glu Ser His
       720                 725                 730
   ACA GTT GGT GAT CCT TAT ACA GAT TAT GAA AAG GCA GCA AGA GAT CTA 864
   Thr Val Gly Asp Pro Tyr Thr Asp Tyr Glu Lys Ala Ala Arg Asp Leu
   735 740 745 750
   GAT TTG TCA AAT GCA AAG GAA ACG TTT AAC CCA TTG GTA GCT GCT TTT 912
   Asp Leu Ser Asn Ala Lys Glu Thr Phe Asn Pro Leu Val Ala Ala Phe
   755 760 765
   CCA AGT GTG AAT GTT AGT ATG GAA AAG GTG ATA TTA TCA CCA AAT GAA 960
   Pro Ser Val Asn Val Ser Met Glu Lys Val Ile Leu Ser Pro Asn Glu
   770 775 780
   AAT TTA TCC AAT AGT GTA GAG TCT CAT TCA TCC ACG AAT TGG TCT TAT 1008
   Asn Leu Ser Asn Ser Val Glu Ser His Ser Ser Thr Asn Trp Ser Tyr
   785 790 795
   ACA AAT ACA GAA GGT GCT TCT GTT GAA GCG GGG ATT GGA CCA AAA GGT 1056
   Thr Asn Thr Glu Gly Ala Ser Val Glu Ala Gly Ile Gly Pro Lys Gly
   800 805 810
   ATT TCG TTC GGA GTT AGC GTA AAC TAT CAA CAC TCT GAA ACA GTT GCA 1104
   Ile Ser Phe Gly Val Ser Val Asn Tyr Gln His Ser Glu Thr Val Ala
   815 820 825 830
   CAA GAA TGG GGA ACA TCT ACA GGA AAT ACT TCG CAA TTC AAT ACG GCT 1152
   Gln Glu Trp Gly Thr Ser Thr Gly Asn Thr Ser Gln Phe Asn Thr Ala
   835 840 845
   TCA GCG GGA TAT TTA AAT GCA AAT GTT CGA TAT AAC AAT GTA GGA ACT 1200
   Ser Ala Gly Tyr Leu Asn Ala Asn Val Arg Tyr Asn Asn Val Gly Thr
   850 855 860
   GGT GCC ATC TAC GAT GTA AAA CCT ACA ACA AGT TTT GTA TTA AAT AAC 1248
   Gly Ala Ile Tyr Asp Val Lys Pro Thr Thr Ser Phe Val Leu Asn Asn
   865 870 875
   GAT ACT ATC GCA ACT ATT ACG GCG AAA TCT AAT TCT ACA GCC TTA AAT 1296
   Asp Thr Ile Ala Thr Ile Thr Ala Lys Ser Asn Ser Thr Ala Leu Asn
   880 885 890
   ATA TCT CCT GGA GAA AGT TAC CCG AAA AAA GGA CAA AAT GGA ATC GCA 1344
   Ile Ser Pro Gly Glu Ser Tyr Pro Lys Lys Gly Gln Asn Gly Ile Ala
   895 900 905 910
   ATA ACA TCA ATG GAT GAT TTT AAT TCC CAT CCG ATT ACA TTA AAT AAA 1392
   Ile Thr Ser Met Asp Asp Phe Asn Ser His Pro Ile Thr Leu Asn Lys
   915 920 925
   AAA CAA GTA GAT AAT CTG CTA AAT AAT AAA CCT ATG ATG TTG GAA ACA 1440
   Lys Gln Val Asp Asn Leu Leu Asn Asn Lys Pro Met Met Leu Glu Thr
   930 935 940
   AAC CAA ACA GAT GGT GTT TAT AAG ATA AAA GAT ACA CAT GGA AAT ATA 1488
   Asn Gln Thr Asp Gly Val Tyr Lys Ile Lys Asp Thr His Gly Asn Ile
         945                   950                   955
   GTA ACT GGC GGA GAA TGG AAT GGT GTC ATA CAA CAA ATC AAG GCT AAA       1536
   Val Thr Gly Gly Glu Trp Asn Gly Val Ile Gln Gln Ile Lys Ala Lys
       960                 965                 970
   ACA GCG TCT ATT ATT GTG GAT GAT GGG GAA CGT GTA GCA GAA AAA CGT       1584
   Thr Ala Ser Ile Ile Val Asp Asp Gly Glu Arg Val Ala Glu Lys Arg
   975                 980                 985                 990
   GTA GCG GCA AAA GAT TAT GAA AAT CCA GAA GAT AAA ACA CCG TCT TTA       1632
   Val Ala Ala Lys Asp Tyr Glu Asn Pro Glu Asp Lys Thr Pro Ser Leu
                   995                 1000                1005
   ACT TTA AAA GAT GCC CTG AAG CTT TCA TAT CCA GAT GAA ATA AAA GAA       1680
   Thr Leu Lys Asp Ala Leu Lys Leu Ser Tyr Pro Asp Glu Ile Lys Glu
               1010                1015                1020
   ATA GAG GGA TTA TTA TAT TAT AAA AAC AAA CCG ATA TAC GAA TCG AGC       1728
   Ile Glu Gly Leu Leu Tyr Tyr Lys Asn Lys Pro Ile Tyr Glu Ser Ser
           1025                1030                1035
   GTT ATG ACT TAC TTA GAT GAA AAT ACA GCA AAA GAA GTG ACC AAA CAA       1776
   Val Met Thr Tyr Leu Asp Glu Asn Thr Ala Lys Glu Val Thr Lys Gln
       1040                1045                1050
   TTA AAT GAT ACC ACT GGG AAA TTT AAA GAT GTA AGT CAT TTA TAT GAT       1824
   Leu Asn Asp Thr Thr Gly Lys Phe Lys Asp Val Ser His Leu Tyr Asp
   1055                1060                1065                1070
   GTA AAA CTG ACT CCA AAA ATG AAT GTT ACA ATC AAA TTG TCT ATA CTT       1872
   Val Lys Leu Thr Pro Lys Met Asn Val Thr Ile Lys Leu Ser Ile Leu
                   1075                1080                1085
   TAT GAT AAT GCT GAG TCT AAT GAT AAC TCA ATT GGT AAA TGG ACA AAC       1920
   Tyr Asp Asn Ala Glu Ser Asn Asp Asn Ser Ile Gly Lys Trp Thr Asn
               1090                1095                1100
   ACA AAT ATT GTT TCA GGT GGA AAT AAC GGA AAA AAA CAA TAT TCT TCT       1968
   Thr Asn Ile Val Ser Gly Gly Asn Asn Gly Lys Lys Gln Tyr Ser Ser
           1105                1110                1115
   AAT AAT CCG GAT GCT AAT TTG ACA TTA AAT ACA GAT GCT CAA GAA AAA       2016
   Asn Asn Pro Asp Ala Asn Leu Thr Leu Asn Thr Asp Ala Gln Glu Lys
       1120                1125                1130
   TTA AAT AAA AAT CGT GAC TAT TAT ATA AGT TTA TAT ATG AAG TCA GAA       2064
   Leu Asn Lys Asn Arg Asp Tyr Tyr Ile Ser Leu Tyr Met Lys Ser Glu
   1135                1140                1145                1150
   AAA AAC ACA CAA TGT GAG ATT ACT ATA GAT GGG GAG ATT TAT CCG ATC       2112
   Lys Asn Thr Gln Cys Glu Ile Thr Ile Asp Gly Glu Ile Tyr Pro Ile
                   1155                1160                1165
   ACT ACA AAA ACA GTG AAT GTG AAT AAA GAC AAT TAC AAA AGA TTA GAT       2160
   Thr Thr Lys Thr Val Asn Val Asn Lys Asp Asn Tyr Lys Arg Leu Asp
               1170                1175                1180
   ATT ATA GCT CAT AAT ATA AAA AGT AAT CCA ATT TCT TCA CTT CAT ATT      2208
   Ile Ile Ala His Asn Ile Lys Ser Asn Pro Ile Ser Ser Leu His Ile
           1185                1190                1195
   AAA ACG AAT GAT GAA ATA ACT TTA TTT TGG GAT GAT ATT TCT ATA ACA      2256
   Lys Thr Asn Asp Glu Ile Thr Leu Phe Trp Asp Asp Ile Ser Ile Thr
       1200                1205                1210
   GAT GTA GCA TCA ATA AAA CCG GAA AAT TTA ACA GAT TCA GAA ATT AAA      2304
   Asp Val Ala Ser Ile Lys Pro Glu Asn Leu Thr Asp Ser Glu Ile Lys
   1215                1220                1225                1230
   CAG ATT TAT AGT AGG TAT GGT ATT AAG TTA GAA GAT GGA ATC CTT ATT      2352
   Gln Ile Tyr Ser Arg Tyr Gly Ile Lys Leu Glu Asp Gly Ile Leu Ile
                   1235                1240                1245
   GAT AAA AAA GGT GGG ATT CAT TAT GGT GAA TTT ATT AAT GAA GCT AGT      2400
   Asp Lys Lys Gly Gly Ile His Tyr Gly Glu Phe Ile Asn Glu Ala Ser
               1250                1255                1260
   TTT AAT ATT GAA CCA TTG CAA AAT TAT GTG ACC AAA TAT GAA GTT ACT      2448
   Phe Asn Ile Glu Pro Leu Gln Asn Tyr Val Thr Lys Tyr Glu Val Thr
           1265                1270                1275
   TAT AGT AGT GAG TTA GGA CCA AAC GTG AGT GAC ACA CTT GAA AGT GAT      2496
   Tyr Ser Ser Glu Leu Gly Pro Asn Val Ser Asp Thr Leu Glu Ser Asp
       1280                1285                1290
   AAA ATT TAC AAG GAT GGG ACA ATT AAA TTT GAT TTT ACC AAA TAT AGT      2544
   Lys Ile Tyr Lys Asp Gly Thr Ile Lys Phe Asp Phe Thr Lys Tyr Ser
   1295                1300                1305                1310
   AAA AAT GAA CAA GGA TTA TTT TAT GAC AGT GGA TTA AAT TGG GAC TTT      2592
   Lys Asn Glu Gln Gly Leu Phe Tyr Asp Ser Gly Leu Asn Trp Asp Phe
                   1315                1320                1325
   AAA ATT AAT GCT ATT ACT TAT GAT GGT AAA GAG ATG AAT GTT TTT CAT      2640
   Lys Ile Asn Ala Ile Thr Tyr Asp Gly Lys Glu Met Asn Val Phe His
               1330                1335                1340
   AGA TAT AAT AAA TAG                                                  2655
   Arg Tyr Asn Lys
           1345
(2) INFORMATION FOR SEQ ID NO:5:
   (i) SEQUENCE CHARACTERISTICS:
      (A) LENGTH: 884 amino acids
      (B) TYPE: amino acid
      (D) TOPOLOGY: linear
   (ii) MOLECULE TYPE: protein
   (xi) SEQUENCE DESCRIPTION: SEQ ID NO:5:
   Met Lys Asn Met Lys Lys Lys Leu Ala Ser Val Val Thr Cys Thr Leu
   1 5 10 15
   Leu Ala Pro Met Phe Leu Asn Gly Asn Val Asn Ala Val Tyr Ala Asp
   20 25 30
   Ser Lys Thr Asn Gln Ile Ser Thr Thr Gln Lys Asn Gln Gln Lys Glu
   35 40 45
   Met Asp Arg Lys Gly Leu Leu Gly Tyr Tyr Phe Lys Gly Lys Asp Phe
   50 55 60
   Ser Asn Leu Thr Met Phe Ala Pro Thr Arg Asp Ser Thr Leu Ile Tyr
   65 70 75 80
   Asp Gln Gln Thr Ala Asn Lys Leu Leu Asp Lys Lys Gln Gln Glu Tyr
   85 90 95
   Gln Ser Ile Arg Trp Ile Gly Leu Ile Gln Ser Lys Glu Thr Gly Asp
   100 105 110
   Phe Thr Phe Asn Leu Ser Glu Asp Glu Gln Ala Ile Ile Glu Ile Asn
   115 120 125
   Gly Lys Ile Ile Ser Asn Lys Gly Lys Glu Lys Gln Val Val His Leu
   130 135 140
   Glu Lys Gly Lys Leu Val Pro Ile Lys Ile Glu Tyr Gln Ser Asp Thr
   145 150 155 160
   Lys Phe Asn Ile Asp Ser Lys Thr Phe Lys Glu Leu Lys Leu Phe Lys
   165 170 175
   Ile Asp Ser Gln Asn Gln Pro Gln Gln Val Gln Gln Asp Glu Leu Arg
   180 185 190
   Asn Pro Glu Phe Asn Lys Lys Glu Ser Gln Glu Phe Leu Ala Lys Pro
   195 200 205
   Ser Lys Ile Asn Leu Phe Thr Gln Lys Met Lys Arg Glu Ile Asp Glu
   210 215 220
   Asp Thr Asp Thr Asp Gly Asp Ser Ile Pro Asp Leu Trp Glu Glu Asn
   225 230 235 240
   Gly Tyr Thr Ile Gln Asn Arg Ile Ala Val Lys Trp Asp Asp Ser Leu
   245 250 255
   Ala Ser Lys Gly Tyr Thr Lys Phe Val Ser Asn Pro Leu Glu Ser His
   260 265 270
   Thr Val Gly Asp Pro Tyr Thr Asp Tyr Glu Lys Ala Ala Arg Asp Leu
   275 280 285
   Asp Leu Ser Asn Ala Lys Glu Thr Phe Asn Pro Leu Val Ala Ala Phe
   290 295 300
   Pro Ser Val Asn Val Ser Met Glu Lys Val Ile Leu Ser Pro Asn Glu
   305 310 315 320
   Asn Leu Ser Asn Ser Val Glu Ser His Ser Ser Thr Asn Trp Ser Tyr
   325 330 335
   Thr Asn Thr Glu Gly Ala Ser Val Glu Ala Gly Ile Gly Pro Lys Gly
   340 345 350
   Ile Ser Phe Gly Val Ser Val Asn Tyr Gln His Ser Glu Thr Val Ala
   355 360 365
   Gln Glu Trp Gly Thr Ser Thr Gly Asn Thr Ser Gln Phe Asn Thr Ala
   370 375 380
   Ser Ala Gly Tyr Leu Asn Ala Asn Val Arg Tyr Asn Asn Val Gly Thr
   385 390 395 400
   Gly Ala Ile Tyr Asp Val Lys Pro Thr Thr Ser Phe Val Leu Asn Asn
   405 410 415
   Asp Thr Ile Ala Thr Ile Thr Ala Lys Ser Asn Ser Thr Ala Leu Asn
   420 425 430
   Ile Ser Pro Gly Glu Ser Tyr Pro Lys Lys Gly Gln Asn Gly Ile Ala
   435 440 445
   Ile Thr Ser Met Asp Asp Phe Asn Ser His Pro Ile Thr Leu Asn Lys
   450 455 460
   Lys Gln Val Asp Asn Leu Leu Asn Asn Lys Pro Met Met Leu Glu Thr
   465 470 475 480
   Asn Gln Thr Asp Gly Val Tyr Lys Ile Lys Asp Thr His Gly Asn Ile
   485 490 495
   Val Thr Gly Gly Glu Trp Asn Gly Val Ile Gln Gln Ile Lys Ala Lys
   500 505 510
   Thr Ala Ser Ile Ile Val Asp Asp Gly Glu Arg Val Ala Glu Lys Arg
   515 520 525
   Val Ala Ala Lys Asp Tyr Glu Asn Pro Glu Asp Lys Thr Pro Ser Leu
   530 535 540
   Thr Leu Lys Asp Ala Leu Lys Leu Ser Tyr Pro Asp Glu Ile Lys Glu
   545 550 555 560
   Ile Glu Gly Leu Leu Tyr Tyr Lys Asn Lys Pro Ile Tyr Glu Ser Ser
   565 570 575
   Val Met Thr Tyr Leu Asp Glu Asn Thr Ala Lys Glu Val Thr Lys Gln
   580 585 590
   Leu Asn Asp Thr Thr Gly Lys Phe Lys Asp Val Ser His Leu Tyr Asp
   595 600 605
   Val Lys Leu Thr Pro Lys Met Asn Val Thr Ile Lys Leu Ser Ile Leu
   610 615 620
   Tyr Asp Asn Ala Glu Ser Asn Asp Asn Ser Ile Gly Lys Trp Thr Asn
   625 630 635 640
   Thr Asn Ile Val Ser Gly Gly Asn Asn Gly Lys Lys Gln Tyr Ser Ser
   645 650 655
   Asn Asn Pro Asp Ala Asn Leu Thr Leu Asn Thr Asp Ala Gln Glu Lys
   660 665 670
   Leu Asn Lys Asn Arg Asp Tyr Tyr Ile Ser Leu Tyr Met Lys Ser Glu
   675 680 685
   Lys Asn Thr Gln Cys Glu Ile Thr Ile Asp Gly Glu Ile Tyr Pro Ile
   690 695 700
   Thr Thr Lys Thr Val Asn Val Asn Lys Asp Asn Tyr Lys Arg Leu Asp
   705 710 715 720
   Ile Ile Ala His Asn Ile Lys Ser Asn Pro Ile Ser Ser Leu His Ile
   725 730 735
   Lys Thr Asn Asp Glu Ile Thr Leu Phe Trp Asp Asp Ile Ser Ile Thr
   740 745 750
   Asp Val Ala Ser Ile Lys Pro Glu Asn Leu Thr Asp Ser Glu Ile Lys
   755 760 765
   Gln Ile Tyr Ser Arg Tyr Gly Ile Lys Leu Glu Asp Gly Ile Leu Ile
   770 775 780
   Asp Lys Lys Gly Gly Ile His Tyr Gly Glu Phe Ile Asn Glu Ala Ser
   785 790 795 800
   Phe Asn Ile Glu Pro Leu Gln Asn Tyr Val Thr Lys Tyr Glu Val Thr
   805 810 815
   Tyr Ser Ser Glu Leu Gly Pro Asn Val Ser Asp Thr Leu Glu Ser Asp
   820 825 830
   Lys Ile Tyr Lys Asp Gly Thr Ile Lys Phe Asp Phe Thr Lys Tyr Ser
   835 840 845
   Lys Asn Glu Gln Gly Leu Phe Tyr Asp Ser Gly Leu Asn Trp Asp Phe
   850 855 860
   Lys Ile Asn Ala Ile Thr Tyr Asp Gly Lys Glu Met Asn Val Phe His
   865 870 875 880
   Arg Tyr Asn Lys
(2) INFORMATION FOR SEQ ID NO:6:
   (i) SEQUENCE CHARACTERISTICS:
      (A) LENGTH: 2004 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Bacillus cereus
      (B) STRAIN: AB78
      (C) INDIVIDUAL ISOLATE: NRRL B-21058
   (ix) FEATURE:
      (A) NAME/KEY: CDS
      (B) LOCATION: 1..2001
      (D) OTHER INFORMATION: /product= "80 kDa protein VIP1A(a)" /note= "This sequence is identical to that found in SEQ ID NO:1 between and including nucleotide positions 3126 and 5126"
   (xi) SEQUENCE DESCRIPTION: SEQ ID NO:6:
   ATG AAA AGG GAA ATT GAT GAA GAC ACG GAT ACG GAT GGG GAC TCT ATT        48
   Met Lys Arg Glu Ile Asp Glu Asp Thr Asp Thr Asp Gly Asp Ser Ile
   885                 890                 895                 900
   CCT GAC CTT TGG GAA GAA AAT GGG TAT ACG ATT CAA AAT AGA ATC GCT        96
   Pro Asp Leu Trp Glu Glu Asn Gly Tyr Thr Ile Gln Asn Arg Ile Ala
                   905                 910                 915
   GTA AAG TGG GAC GAT TCT CTA GCA AGT AAA GGG TAT ACG AAA TTT GTT       144
   Val Lys Trp Asp Asp Ser Leu Ala Ser Lys Gly Tyr Thr Lys Phe Val
               920                 925                 930
   TCA AAT CCA CTA GAA AGT CAC ACA GTT GGT GAT CCT TAT ACA GAT TAT       192
   Ser Asn Pro Leu Glu Ser His Thr Val Gly Asp Pro Tyr Thr Asp Tyr
           935                 940                 945
   GAA AAG GCA GCA AGA GAT CTA GAT TTG TCA AAT GCA AAG GAA ACG TTT       240
   Glu Lys Ala Ala Arg Asp Leu Asp Leu Ser Asn Ala Lys Glu Thr Phe
       950                 955                 960
   AAC CCA TTG GTA GCT GCT TTT CCA AGT GTG AAT GTT AGT ATG GAA AAG      288
   Asn Pro Leu Val Ala Ala Phe Pro Ser Val Asn Val Ser Met Glu Lys
   965                 970                 975                 980
   GTG ATA TTA TCA CCA AAT GAA AAT TTA TCC AAT AGT GTA GAG TCT CAT      336
   Val Ile Leu Ser Pro Asn Glu Asn Leu Ser Asn Ser Val Glu Ser His
                   985                 990                 995
   TCA TCC ACG AAT TGG TCT TAT ACA AAT ACA GAA GGT GCT TCT GTT GAA      384
   Ser Ser Thr Asn Trp Ser Tyr Thr Asn Thr Glu Gly Ala Ser Val Glu
               1000                1005                1010
   GCG GGG ATT GGA CCA AAA GGT ATT TCG TTC GGA GTT AGC GTA AAC TAT      432
   Ala Gly Ile Gly Pro Lys Gly Ile Ser Phe Gly Val Ser Val Asn Tyr
           1015                1020                1025
   CAA CAC TCT GAA ACA GTT GCA CAA GAA TGG GGA ACA TCT ACA GGA AAT      480
   Gln His Ser Glu Thr Val Ala Gln Glu Trp Gly Thr Ser Thr Gly Asn
       1030                1035                1040
   ACT TCG CAA TTC AAT ACG GCT TCA GCG GGA TAT TTA AAT GCA AAT GTT      528
   Thr Ser Gln Phe Asn Thr Ala Ser Ala Gly Tyr Leu Asn Ala Asn Val
   1045                1050                1055                1060
   CGA TAT AAC AAT GTA GGA ACT GGT GCC ATC TAC GAT GTA AAA CCT ACA      576
   Arg Tyr Asn Asn Val Gly Thr Gly Ala Ile Tyr Asp Val Lys Pro Thr
                   1065                1070                1075
   ACA AGT TTT GTA TTA AAT AAC GAT ACT ATC GCA ACT ATT ACG GCG AAA      624
   Thr Ser Phe Val Leu Asn Asn Asp Thr Ile Ala Thr Ile Thr Ala Lys
               1080                1085                1090
   TCT AAT TCT ACA GCC TTA AAT ATA TCT CCT GGA GAA AGT TAC CCG AAA      672
   Ser Asn Ser Thr Ala Leu Asn Ile Ser Pro Gly Glu Ser Tyr Pro Lys
           1095                1100                1105
   AAA GGA CAA AAT GGA ATC GCA ATA ACA TCA ATG GAT GAT TTT AAT TCC      720
   Lys Gly Gln Asn Gly Ile Ala Ile Thr Ser Met Asp Asp Phe Asn Ser
       1110                1115                1120
   CAT CCG ATT ACA TTA AAT AAA AAA CAA GTA GAT AAT CTG CTA AAT AAT      768
   His Pro Ile Thr Leu Asn Lys Lys Gln Val Asp Asn Leu Leu Asn Asn
   1125                1130                1135                1140
   AAA CCT ATG ATG TTG GAA ACA AAC CAA ACA GAT GGT GTT TAT AAG ATA      816
   Lys Pro Met Met Leu Glu Thr Asn Gln Thr Asp Gly Val Tyr Lys Ile
                   1145                1150                1155
   AAA GAT ACA CAT GGA AAT ATA GTA ACT GGC GGA GAA TGG AAT GGT GTC      864
   Lys Asp Thr His Gly Asn Ile Val Thr Gly Gly Glu Trp Asn Gly Val
               1160                1165                1170
   ATA CAA CAA ATC AAG GCT AAA ACA GCG TCT ATT ATT GTG GAT GAT GGG      912
   Ile Gln Gln Ile Lys Ala Lys Thr Ala Ser Ile Ile Val Asp Asp Gly
           1175                1180                1185
   GAA CGT GTA GCA GAA AAA CGT GTA GCG GCA AAA GAT TAT GAA AAT CCA       960
   Glu Arg Val Ala Glu Lys Arg Val Ala Ala Lys Asp Tyr Glu Asn Pro
       1190                1195                1200
   GAA GAT AAA ACA CCG TCT TTA ACT TTA AAA GAT GCC CTG AAG CTT TCA      1008
   Glu Asp Lys Thr Pro Ser Leu Thr Leu Lys Asp Ala Leu Lys Leu Ser
   1205                1210                1215                1220
   TAT CCA GAT GAA ATA AAA GAA ATA GAG GGA TTA TTA TAT TAT AAA AAC      1056
   Tyr Pro Asp Glu Ile Lys Glu Ile Glu Gly Leu Leu Tyr Tyr Lys Asn
                   1225                1230                1235
   AAA CCG ATA TAC GAA TCG AGC GTT ATG ACT TAC TTA GAT GAA AAT ACA      1104
   Lys Pro Ile Tyr Glu Ser Ser Val Met Thr Tyr Leu Asp Glu Asn Thr
               1240                1245                1250
   GCA AAA GAA GTG ACC AAA CAA TTA AAT GAT ACC ACT GGG AAA TTT AAA      1152
   Ala Lys Glu Val Thr Lys Gln Leu Asn Asp Thr Thr Gly Lys Phe Lys
           1255                1260                1265
   GAT GTA AGT CAT TTA TAT GAT GTA AAA CTG ACT CCA AAA ATG AAT GTT      1200
   Asp Val Ser His Leu Tyr Asp Val Lys Leu Thr Pro Lys Met Asn Val
       1270                1275                1280
   ACA ATC AAA TTG TCT ATA CTT TAT GAT AAT GCT GAG TCT AAT GAT AAC      1248
   Thr Ile Lys Leu Ser Ile Leu Tyr Asp Asn Ala Glu Ser Asn Asp Asn
   1285                1290                1295                1300
   TCA ATT GGT AAA TGG ACA AAC ACA AAT ATT GTT TCA GGT GGA AAT AAC      1296
   Ser Ile Gly Lys Trp Thr Asn Thr Asn Ile Val Ser Gly Gly Asn Asn
                   1305                1310                1315
   GGA AAA AAA CAA TAT TCT TCT AAT AAT CCG GAT GCT AAT TTG ACA TTA      1344
   Gly Lys Lys Gln Tyr Ser Ser Asn Asn Pro Asp Ala Asn Leu Thr Leu
               1320                1325                1330
   AAT ACA GAT GCT CAA GAA AAA TTA AAT AAA AAT CGT GAC TAT TAT ATA      1392
   Asn Thr Asp Ala Gln Glu Lys Leu Asn Lys Asn Arg Asp Tyr Tyr Ile
           1335                1340                1345
   AGT TTA TAT ATG AAG TCA GAA AAA AAC ACA CAA TGT GAG ATT ACT ATA      1440
   Ser Leu Tyr Met Lys Ser Glu Lys Asn Thr Gln Cys Glu Ile Thr Ile
       1350                1355                1360
   GAT GGG GAG ATT TAT CCG ATC ACT ACA AAA ACA GTG AAT GTG AAT AAA      1488
   Asp Gly Glu Ile Tyr Pro Ile Thr Thr Lys Thr Val Asn Val Asn Lys
   1365                1370                1375                1380
   GAC AAT TAC AAA AGA TTA GAT ATT ATA GCT CAT AAT ATA AAA AGT AAT      1536
   Asp Asn Tyr Lys Arg Leu Asp Ile Ile Ala His Asn Ile Lys Ser Asn
                   1385                1390                1395
   CCA ATT TCT TCA CTT CAT ATT AAA ACG AAT GAT GAA ATA ACT TTA TTT      1584
   Pro Ile Ser Ser Leu His Ile Lys Thr Asn Asp Glu Ile Thr Leu Phe
                   1400                1405                1410
   TGG GAT GAT ATT TCT ATA ACA GAT GTA GCA TCA ATA AAA CCG GAA AAT      1632
   Trp Asp Asp Ile Ser Ile Thr Asp Val Ala Ser Ile Lys Pro Glu Asn
               1415                1420                1425
   TTA ACA GAT TCA GAA ATT AAA CAG ATT TAT AGT AGG TAT GGT ATT AAG      1680
   Leu Thr Asp Ser Glu Ile Lys Gln Ile Tyr Ser Arg Tyr Gly Ile Lys
           1430                1435                1440
   TTA GAA GAT GGA ATC CTT ATT GAT AAA AAA GGT GGG ATT CAT TAT GGT      1728
   Leu Glu Asp Gly Ile Leu Ile Asp Lys Lys Gly Gly Ile His Tyr Gly
   1445                1450                1455                1460
   GAA TTT ATT AAT GAA GCT AGT TTT AAT ATT GAA CCA TTG CCA AAT TAT      1776
   Glu Phe Ile Asn Glu Ala Ser Phe Asn Ile Glu Pro Leu Pro Asn Tyr
                   1465                1470                1475
   GTG ACC AAA TAT GAA GTT ACT TAT AGT AGT GAG TTA GGA CCA AAC GTG      1824
   Val Thr Lys Tyr Glu Val Thr Tyr Ser Ser Glu Leu Gly Pro Asn Val
               1480                1485                1490
   AGT GAC ACA CTT GAA AGT GAT AAA ATT TAC AAG GAT GGG ACA ATT AAA      1872
   Ser Asp Thr Leu Glu Ser Asp Lys Ile Tyr Lys Asp Gly Thr Ile Lys
           1495                1500                1505
   TTT GAT TTT ACC AAA TAT AGT AAA AAT GAA CAA GGA TTA TTT TAT GAC      1920
   Phe Asp Phe Thr Lys Tyr Ser Lys Asn Glu Gln Gly Leu Phe Tyr Asp
       1510                1515                1520
   AGT GGA TTA AAT TGG GAC TTT AAA ATT AAT GCT ATT ACT TAT GAT GGT      1968
   Ser Gly Leu Asn Trp Asp Phe Lys Ile Asn Ala Ile Thr Tyr Asp Gly
   1525                1530                1535                1540
   AAA GAG ATG AAT GTT TTT CAT AGA TAT AAT AAA TAG                      2004
   Lys Glu Met Asn Val Phe His Arg Tyr Asn Lys
                   1545                1550
(2) INFORMATION FOR SEQ ID NO:7:
   (i) SEQUENCE CHARACTERISTICS:
      (A) LENGTH: 667 amino acids
      (B) TYPE: amino acid
      (D) TOPOLOGY: linear
   (ii) MOLECULE TYPE: protein
   (xi) SEQUENCE DESCRIPTION: SEQ ID NO:7:
   Met Lys Arg Glu Ile Asp Glu Asp Thr Asp Thr Asp Gly Asp Ser Ile
   1 5 10 15
   Pro Asp Leu Trp Glu Glu Asn Gly Tyr Thr Ile Gln Asn Arg Ile Ala
   20 25 30
   Val Lys Trp Asp Asp Ser Leu Ala Ser Lys Gly Tyr Thr Lys Phe Val
   35 40 45
   Ser Asn Pro Leu Glu Ser His Thr Val Gly Asp Pro Tyr Thr Asp Tyr
   50 55 60
   Glu Lys Ala Ala Arg Asp Leu Asp Leu Ser Asn Ala Lys Glu Thr Phe
   65 70 75 80
   Asn Pro Leu Val Ala Ala Phe Pro Ser Val Asn Val Ser Met Glu Lys
   85 90 95
   Val Ile Leu Ser Pro Asn Glu Asn Leu Ser Asn Ser Val Glu Ser His
   100 105 110
   Ser Ser Thr Asn Trp Ser Tyr Thr Asn Thr Glu Gly Ala Ser Val Glu
   115 120 125
   Ala Gly Ile Gly Pro Lys Gly Ile Ser Phe Gly Val Ser Val Asn Tyr
   130 135 140
   Gln His Ser Glu Thr Val Ala Gln Glu Trp Gly Thr Ser Thr Gly Asn
   145 150 155 160
   Thr Ser Gln Phe Asn Thr Ala Ser Ala Gly Tyr Leu Asn Ala Asn Val
   165 170 175
   Arg Tyr Asn Asn Val Gly Thr Gly Ala Ile Tyr Asp Val Lys Pro Thr
   180 185 190
   Thr Ser Phe Val Leu Asn Asn Asp Thr Ile Ala Thr Ile Thr Ala Lys
   195 200 205
   Ser Asn Ser Thr Ala Leu Asn Ile Ser Pro Gly Glu Ser Tyr Pro Lys
   210 215 220
   Lys Gly Gln Asn Gly Ile Ala Ile Thr Ser Met Asp Asp Phe Asn Ser
   225 230 235 240
   His Pro Ile Thr Leu Asn Lys Lys Gln Val Asp Asn Leu Leu Asn Asn
   245 250 255
   Lys Pro Met Met Leu Glu Thr Asn Gln Thr Asp Gly Val Tyr Lys Ile
   260 265 270
   Lys Asp Thr His Gly Asn Ile Val Thr Gly Gly Glu Trp Asn Gly Val
   275 280 285
   Ile Gln Gln Ile Lys Ala Lys Thr Ala Ser Ile Ile Val Asp Asp Gly
   290 295 300
   Glu Arg Val Ala Glu Lys Arg Val Ala Ala Lys Asp Tyr Glu Asn Pro
   305 310 315 320
   Glu Asp Lys Thr Pro Ser Leu Thr Leu Lys Asp Ala Leu Lys Leu Ser
   325 330 335
   Tyr Pro Asp Glu Ile Lys Glu Ile Glu Gly Leu Leu Tyr Tyr Lys Asn
   340 345 350
   Lys Pro Ile Tyr Glu Ser Ser Val Met Thr Tyr Leu Asp Glu Asn Thr
   355 360 365
   Ala Lys Glu Val Thr Lys Gln Leu Asn Asp Thr Thr Gly Lys Phe Lys
   370 375 380
   Asp Val Ser His Leu Tyr Asp Val Lys Leu Thr Pro Lys Met Asn Val
   385 390 395 400
   Thr Ile Lys Leu Ser Ile Leu Tyr Asp Asn Ala Glu Ser Asn Asp Asn
   405 410 415
   Ser Ile Gly Lys Trp Thr Asn Thr Asn Ile Val Ser Gly Gly Asn Asn
   420 425 430
   Gly Lys Lys Gln Tyr Ser Ser Asn Asn Pro Asp Ala Asn Leu Thr Leu
   435 440 445
   Asn Thr Asp Ala Gln Glu Lys Leu Asn Lys Asn Arg Asp Tyr Tyr Ile
   450 455 460
   Ser Leu Tyr Met Lys Ser Glu Lys Asn Thr Gln Cys Glu Ile Thr Ile
   465 470 475 480
   Asp Gly Glu Ile Tyr Pro Ile Thr Thr Lys Thr Val Asn Val Asn Lys
   485 490 495
   Asp Asn Tyr Lys Arg Leu Asp Ile Ile Ala His Asn Ile Lys Ser Asn
   500 505 510
   Pro Ile Ser Ser Leu His Ile Lys Thr Asn Asp Glu Ile Thr Leu Phe
   515 520 525
   Trp Asp Asp Ile Ser Ile Thr Asp Val Ala Ser Ile Lys Pro Glu Asn
   530 535 540
   Leu Thr Asp Ser Glu Ile Lys Gln Ile Tyr Ser Arg Tyr Gly Ile Lys
   545 550 555 560
   Leu Glu Asp Gly Ile Leu Ile Asp Lys Lys Gly Gly Ile His Tyr Gly
   565 570 575
   Glu Phe Ile Asn Glu Ala Ser Phe Asn Ile Glu Pro Leu Pro Asn Tyr
   580 585 590
   Val Thr Lys Tyr Glu Val Thr Tyr Ser Ser Glu Leu Gly Pro Asn Val
   595 600 605
   Ser Asp Thr Leu Glu Ser Asp Lys Ile Tyr Lys Asp Gly Thr Ile Lys
   610 615 620
   Phe Asp Phe Thr Lys Tyr Ser Lys Asn Glu Gln Gly Leu Phe Tyr Asp
   625 630 635 640
   Ser Gly Leu Asn Trp Asp Phe Lys Ile Asn Ala Ile Thr Tyr Asp Gly
   645 650 655
   Lys Glu Met Asn Val Phe His Arg Tyr Asn Lys
   660 665
(2) INFORMATION FOR SEQ ID NO:8:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (vi) ORIGINAL SOURCE:
      (A) ORGANISM: Bacillus cereus
      (B) STRAIN: AB78
      (C) INDIVIDUAL ISOLATE: NRRL B-21058
   (ix) FEATURE:
      (A) NAME/KEY: Peptide
      (B) LOCATION: 1..16
      (D) OTHER INFORMATION: /note= "N-terminal sequence of protein purified from strain AB78°
   (xi) SEQUENCE EESCRIPTICN: SEQ ID NO:8:
(2) INFORMATION FOR SEQ ID NO: 9:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: misc_feature
      (B) LOCATION: 1..21
      (D) OTHER INFORMATION: /note= "Oligonucleotide probe based , on amino acids 3 to 9 of SEQ ID NO:8, using codon usage of Bacillus thuringiensis"
   (xi) SEQUENCE DESCRIPTION: SEQ ID NO:9:
   GAAATTGATC AAGATACNGA T 21
(2) INFORMATION FOR SEQ ID NO:10
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (vi) ORIGINAL SOURCE:
      (A) ORGANISM: Bacillus thuringiensis
      (B) STRAIN: AB88
   (ix) FEATURE:
      (A) NAME/KEY: Peptide
      (B) LOCATION: 1..14
      (D) OTHER INFORMATION: /note= "N-terminal amino acid sequence of protein known as anion exchange fraction 23 (smaller)"
   (xi) SEQUENCE DESCRIPTION: SEQ ID NO:10:
   Xaa Glu Pro Phe Val Ser Ala Xaa Xaa Xaa Gln Xaa Xaa Xaa
   1 5 10
(2) INFORMATION FOR SEQ ID NO:11:
   (i) SEQUENCE CHARACTERISTICS:
      (A) LENGTH: 13 amino acids
      (B) TYPE: amino acid
      (C) STRANDECNESS: single
      (D) TOPOLOGY: N-terminal
   (vi) ORIGINAL SOURCE:
      (A) ORGANISM: Bacillus thuringiensis
   (xi) SEQUENCE DESCRIPTION: SEQ ID NO:11:
   Xaa Glu Tyr Glu Asn Val Glu Pro Phe Val Ser Ala Xaa
   1               5                   10
(2) INFORMATION FOR SEQ ID NO:12:
   (i) SEQUENCE CHARACTERISTICS:
      (A) LENGTH: 14 amino acids
      (B) TYPE: amino acid
      (C) STRANDEDNESS: single
      (D) TOPOLOGY: N-terminal
   (vi) ORIGINAL SOURCE:
      (A) ORGANISM: Bacillus thurigiensis
   (xi) SEQUENCE DESCRIPTION: SEQ ID NO:12:
   Met Asn Lys Asn Asn Thr Lys Leu Pro Thr Arg Ala Leu Pro
   1               5                   10
(2) INFORMATION FOR SEQ ID NO:13:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: H-terminal
   (vi) ORIGINAL SOURCE:
      (A) ORGANISM: Bacillus thuringiensis
      (B) STRAIN: AB88
   (ix) FEATURE:
      (A) NAME/KEY: Peptide
      (B) LOCATION: 1..15
      (D) OTHER INFORMATION: /note= ''N-teoainal amino acid sequence of 35 kDa VIP active against Agrotis ipsilon"
   (xi) SEQUENCE DESCRIPTION: SEQ ID NO:13:
   Ala Leu Ser Glu Asn Thr Gly Lys Asp Gly Gly Tyr Ile Val Pro
   1 5 10 15
(2) INFORMATION FOR SEQ ID NO:14:
   (i) SEQUENCE CHARACTERISTICS:
      (A) LENGTH: 9 amino acids
      (B) TYPE: amino acid
      (C) STRANDEDNESS: single
      (D) TOPOLOGY: N-terminal
   (vi) ORIGINAL SOURCE:
      (A) ORGANISM: Bacillus thuringiensis
   (xi) SEQUENCE DESCRIPTION: SEQ ID NO:14:
   Met Asp Asn Asn Pro Asn Ile Asn Glu
   1 5
(2) INFORMATION FOR SEQ ID NO:15:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (ix) FEATURE:
      (A) NAME/KEY: Peptide
      (B) LOCATION: 1..9
      (D) OTHER INFORMATION: /note= "N-terminal sequence of 80 kDa delta-endotoxin"
   (xi) SEQUENCE DESCRIPTION: SEQ ID NO:15:
   Met Asp Asn Asn Pro Asn Ile Asn Glu
   1 5
(2) INFORMATION FOR SEQ ID NO:16:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (vi) ORIGINAL SOURCE:
      (A) ORGANISM: Bacillus thuringiensis
   (ix) FEATURE:
      (A) NAME/KEY: Peptide
      (B) LOCATION: 1..11
      (D) OTHER INFORMATION: /note= "N-terminal sequence from 60 kDa delta-endotoxin"
   (xi) SEQUENCE DESCRIPTION: SEQ ID NO:16:
   Met Asn Val Leu Asn Ser Gly Arg Thr Thr Ile
   1               5                   10
(2) INFORMATION FOR SEQ ID NO:17:
   (i) SEQUENCE CHARACTERISTICS:
      (A) LENGTH: 2655 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: misc_feature
      (B) LOCATION: 1..2652
      (D) OTHER INFORMATION: /note= "Maize optimized DNA sequence for 100 kd VIP1A(a) protein from AB78"
   (xi) SEQUENCE DESCRIPTION: SEQ ID NO:17:
   ATGAAGAACA TGAAGAAGAA GCTGGCCAGC GTGGTGACCT GCACCCTGCT GGCCCCCATG    60
   TTCCTGAACG GCAACGTGAA CGCCGTGTAC GCCGACAGCA AGACCAACCA GATCAGCACC   120
   ACCCAGAAGA ACCAGCAGAA GGAGATGGAC CGCAAGGGCC TGCTGGGCTA CTACTTCAAG   180
   GGCAAGGACT TCAGCAACCT GACCATGTTC GCCCCCACGC GTGACAGCAC CCTGATCTAC 240
   GACCAGCAGA CCGCCAACAA GCTGCTGGAC AAGAAGCAGC AGGAGTACCA GAGCATCCGC 300
   TGGATCGGCC TGATCCAGAG CAAGGAGACC GGCGACTTCA CCTTCAACCT GAGCGAGGAC 360
   GAGCAGGCCA TCATCGAGAT CAACGGCAAG ATCATCAGCA ACAAGGGCAA GGAGAAGCAG 420
   GTGGTGCACC TGGAGAAGGG CAAGCTGGTG CCCATCAAGA TCGAGTACCA GAGCGACACC 480
   AAGTTCAACA TCGACAGCAA GACCTTCAAG GAGCTGAAGC TTTTCAAGAT CGACAGCCAG 540
   AACCAGCCCC AGCAGGTGCA GCAGGACGAG CTGCGCAACC CCGAGTTCAA CAAGAAGGAG 600
   AGCCAGGAGT TCCTGGCCAA GCCCAGCAAG ATCAACCTGT TCACCCAGCA GATGAAGCGC 660
   GAGATCGACG AGGACACCGA CACCGACGGC GACAGCATCC CCGACCTGTG GGAGGAGAAC 720
   GGCTACACCA TCCAGAACCG CATCGCCGTG AAGTGGGACG ACAGCCTGGC TAGCAAGGGC 780
   TACACCAAGT TCGTGAGCAA CCCCCTGGAG AGCCACACCG TGGGCGACCC CTACACCGAC 840
   TACGAGAAGG CCGCCCGCGA CCTGGACCTG AGCAACGCCA AGGAGACCTT CAACCCCCTG 900
   GTGGCCGCCT TCCCCAGCGT GAACGTGAGC ATGGAGAAGG TGATCCTGAG CCCCAACGAG 960
   AACCTGAGCA ACAGCGTGGA GAGCCACTCG AGCACCAACT GGAGCTACAC CAACACCGAG 1020
   GGCGCCAGCG TGGAGGCCGG CATCGGTCCC AAGGGCATCA GCTTCGGCGT GAGCGTGAAC 1080
   TACCAGCACA GCGAGACCGT GGCCCAGGAG TGGGGCACCA GCACCGGCAA CACCAGCCAG 1140
   TTCAACACCG CCAGCGCCGG CTACCTGAAC GCCAACGTGC GCTACAACAA CGTGGGCACC 1200
   GGCGCCATCT ACGACGTGAA GCCCACCACC AGCTTCGTGC TGAACAACGA CACCATCGCC 1260
   ACCATCACCG CCAAGTCGAA TTCCACCGCC CTGAACATCA GCCCCGGCGA GAGCTACCCC 1320
   AAGAAGGGCC AGAACGGCAT CGCCATCACC AGCATGGACG ACTTCAACAG CCACCCCATC 1380
   ACCCTGAACA AGAAGCAGGT GGACAACCTG CTGAACAACA AGCCCATGAT GCTGGAGACC 1440
   AACCAGACCG ACGGCGTCTA CAAGATCAAG GACACCCACG GCAACATCGT GACCGGCGGC 1500
   GAGTGGAACG GCGTGATCCA GCAGATCAAG GCCAAGACCG CCAGCATCAT CGTCGACGAC 1560
   GGCGAGCGCG TGGCCGAGAA GCGCGTGGCC GCCAAGGACT ACGAGAACCC CGAGGACAAG 1620
   ACCCCCAGCC TGACCCTGAA GGACGCCCTG AAGCTGAGCT ACCCCGACGA GATCAAGGAG 1680
   ATCGAGGGCC TGCTGTACTA CAAGAACAAG CCCATCTACG AGAGCAGCGT GATGACCTAT 1740
   CTAGACGAGA ACACCGCCAA GGAGGTGACC AAGCAGCTGA ACGACACCAC CGGCAAGTTC 1800
   AAGGACGTGA GCCACCTGTA CGACGTGAAG CTGACCCCCA AGATGAACGT GACCATCAAG 1860
   CTGAGCATCC TGTACGACAA CGCCGAGAGC AACGACAACA GCATCGGCAA GTGGACCAAC   1920
   ACCAACATCG TGAGCGGCGG CAACAACGGC AAGAAGCAGT ACAGCAGCAA CAACCCCGAC   1980
   GCCAACCTGA CCCTGAACAC CGACGCCCAG GAGAAGCTGA ACAAGAACCG CGACTACTAC   2040
   ATCAGCCTGT ACATGAAGAG CGAGAAGAAC ACCCAGTGCG AGATCACCAT CGACGGCGAG   2100
   ATATACCCCA TCACCACCAA GACCGTGAAC GTGAACAAGG ACAACTACAA GCGCCTGGAC   2160
   ATCATCGCCC ACAACATCAA GAGCAACCCC ATCAGCAGCC TGCACATCAA GACCAACGAC   2220
   GAGATCACCC TGTTCTGGGA CGACATATCG ATTACCGACG TCGCCAGCAT CAAGCCCGAG   2280
   AACCTGACCG ACAGCGAGAT CAAGCAGATA TACAGTCGCT ACGGCATCAA GCTGGAGGAC   2340
   GGCATCCTGA TCGACAAGAA GGGCGGCATC CACTACGGCG AGTTCATCAA CGAGGCCAGC   2400
   TTCAACATCG AGCCCCTGCA GAACTACGTG ACCAAGTACG AGGTGACCTA CAGCAGCGAG   2460
   CTGGGCCCCA ACGTGAGCGA CACCCTGGAG AGCGACAAGA TTTACAAGGA CGGCACCATC   2520
   AAGTTCGACT TCACCAAGTA CAGCAAGAAC GAGCAGGGCC TGTTCTACGA CAGCGGCCTG   2580
   AACTGGGACT TCAAGATCAA CGCCATCACC TACGACGGCA AGGAGATGAA CGTGTTCCAC   2640
   CGCTACAACA AGTAG                                                    2655
(2) INFORMATION FOR SEQ ID NO:18:
   (i) SEQUENCE CHARACTERISTICS:
      (A) LENGTH: 2004 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: misc_feature
      (B) LOCATION: 1..2004
      (D) OTHER INFORMATION: /note= "Maize optimized DNA sequence for VIP1A(a) 80 kd protein from AB78°
   (xi) SEQUENCE DESCRIPTION: SEQ ID NO:18:
   ATGAAGCGCG AGATCGACGA GGACACCGAC ACCGACGGCG ACAGCATCCC CGACCTGTGG 60
   GAGGAGAACG GCTACACCAT CCAGAACCGC ATCGCCGTGA AGTGGGACGA CAGCCTGGCT 120
   AGCAAGGGCT ACACCAAGTT CGTGAGCAAC CCCCTGGAGA GCCACACCGT GGGCGACCCC 180
   TACACCGACT ACGAGAAGGC CGCCCGCGAC CTGGACCTGA GCAACGCCAA GGAGACCTTC 240
   AACCCCCTGG TGGCCGCCTT CCCCAGCGTG AACGTGAGCA TGGAGAAGGT GATCCTGAGC 300
   CCCAACGAGA ACCTGAGCAA CAGCGTGGAG AGCCACTCGA GCACCAACTG GAGCTACACC 360
   AACACCGAGG GCGCCAGCGT GGAGGCCGGC ATCGGTCCCA AGGGCATCAG CTTCGGCGTG 420
   AGCGTGAACT ACCAGCACAG CGAGACCGTG GCCCAGGAGT GGGGCACCAG CACCGGCAAC 480
   ACCAGCCAGT TCAACACCGC CAGCGCCGGC TACCTGAACG CCAACGTGCG CTACAACAAC 540
   GTGGGCACCG GCGCCATCTA CGACGTGAAG CCCACCACCA GCTTCGTGCT GAACAACGAC 600
   ACCATCGCCA CCATCACCGC CAAGTCGAAT TCCACCGCCC TGAACATCAG CCCCGGCGAG 660
   AGCTACCCCA AGAAGGGCCA GAACGGCATC GCCATCACCA GCATGGACGA CTTCAACAGC 720
   CACCCCATCA CCCTGAACAA GAAGCAGGTG GACAACCTGC TGAACAACAA GCCCATGATG 780
   CTGGAGACCA ACCAGACCGA CGGCGTCTAC AAGATCAAGG ACACCCACGG CAACATCGTG 840
   ACCGGCGGCG AGTGGAACGG CGTGATCCAG CAGATCAAGG CCAAGACCGC CAGCATCATC 900
   GTCGACGACG GCGAGCGCGT GGCCGAGAAG CGCGTGGCCG CCAAGGACTA CGAGAACCCC 960
   GAGGACAAGA CCCCCAGCCT GACCCTGAAG GACGCCCTGA AGCTGAGCTA CCCCGACGAG 1020
   ATCAAGGAGA TCGAGGGCCT GCTGTACTAC AAGAACAAGC CCATCTACGA GAGCAGCGTG 1080
   ATGACCTATC TAGACGAGAA CACCGCCAAG GAGGTGACCA AGCAGCTGAA CGACACCACC 1140
   GGCAAGTTCA AGGACGTGAG CCACCTGTAC GACGTGAAGC TGACCCCCAA GATGAACGTG 1200
   ACCATCAAGC TGAGCATCCT GTACGACAAC GCCGAGAGCA ACGACAACAG CATCGGCAAG 1260
   TGGACCAACA CCAACATCGT GAGCGGCGGC AACAACGGCA AGAAGCAGTA CAGCAGCAAC 1320
   AACCCCGACG CCAACCTGAC CCTGAACACC GACGCCCAGG AGAAGCTGAA CAAGAACCGC 1380
   GACTACTACA TCAGCCTGTA CATGAAGAGC GAGAAGAACA CCCAGTGCGA GATCACCATC 1440
   GACGGCGAGA TATACCCCAT CACCACCAAG ACCGTGAACG TGAACAAGGA CAACTACAAG 1500
   CGCCTGGACA TCATCGCCCA CAACATCAAG AGCAACCCCA TCAGCAGCCT GCACATCAAG 1560
   ACCAACGACG AGATCACCCT GTTCTGGGAC GACATATCGA TTACCGACGT CGCCAGCATC 1620
   AAGCCCGAGA ACCTGACCGA CAGCGAGATC AAGCAGATAT ACAGTCGCTA CGGCATCAAG 1680
   CTGGAGGACG GCATCCTGAT CGACAAGAAG GGCGGCATCC ACTACGGCGA GTTCATCAAC 1740
   GAGGCCAGCT TCAACATCGA GCCCCTGCAG AACTACGTGA CCAAGTACGA GGTGACCTAC 1800
   AGCAGCGAGC TGGGCCCCAA CGTGAGCGAC ACCCTGGAGA GCGACAAGAT TTACAAGGAC 1860
   GGCACCATCA AGTTCGACTT CACCAAGTAC AGCAAGAACG AGCAGGGCCT GTTCTACGAC 1920
   AGCGGCCTGA ACTGGGACTT CAAGATCAAC GCCATCACCT ACGACGGCAA GGAGATGAAC 1980
   GTGTTCCACC GCTACAACAA GTAG 2004
(2) INFORMATION FOR SEQ ID NO:19:
   (i) SEQUENCE CHARACTERISTICS:
      (A) LENGTH: 4074 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..1386
      (D) OTHER INFORMATION: /product= "VIP2A(b) from Btt"
   (ix) FEATURE:
      (A) NAME/KEY: CDS
      (B) LOCATION: 1394..3895
      (D) OTHER INFORMATION: /product= "VIP1A(b) from Btt"
   (ix) FEATURE:
      (A) NAME/KEY: misc_feature
      (B) LOCATION: 1..4074
      (D) OTHER INFORMATION: /note= ''Cloned DNA sequence from Btt which contains the genes for both VIP1A(b) and VIP2A(b)"
   (xi) SEQUENCE DESCRIPTION: SEQ ID NO:19:
   ATG CAA AGA ATG GAG GGA AAG TTG TTT GTG GTG TCA AAA ACA TTA CAA        48
   Met Gln Arg Met Glu Gly Lys Leu Phe Val Val Ser Lys Thr Leu Gln
           670                 675                 680
   GTA GTT ACT AGA ACT GTA TTG CTT AGT ACA GTT TAC TCT ATA ACT TTA        96
   Val Val Thr Arg Thr Val Leu Leu Ser Thr Val Tyr Ser Ile Thr Leu
       685                 690                 695
   TTA AAT AAT GTA GTG ATA AAA GCT GAC CAA TTA AAT ATA AAT TCT CAA       144
   Leu Asn Asn Val Val Ile Lys Ala Asp Gln Leu Asn Ile Asn Ser Gln
   700                 705                 710                 715
   AGT AAA TAT ACT AAC TTG CAA AAT CTA AAA ATC CCT GAT AAT GCA GAG       192
   Ser Lys Tyr Thr Asn Leu Gln Asn Leu Lys Ile Pro Asp Asn Ala Glu
   720 725 730
   GAT TTT AAA GAA GAT AAG GGG AAA GCG AAA GAA TGG GGG AAA GAG AAA 240
   Asp Phe Lys Glu Asp Lys Gly Lys Ala Lys Glu Trp Gly Lys Glu Lys
   735 740 745
   GGG GAA GAG TGG AGG CCT CCT GCT ACT GAG AAA GGA GAA ATG AAT AAT 288
   Gly Glu Glu Trp Arg Pro Pro Ala Thr Glu Lys Gly Glu Met Asn Asn
   750 755 760
   TTT TTA GAT AAT AAA AAT GAT ATA AAG ACC AAT TAT AAA GAA ATT ACT 336
   Phe Leu Asp Asn Lys Asn Asp Ile Lys Thr Asn Tyr Lys Glu Ile Thr
   765 770 775
   TTT TCT ATG GCA GGT TCA TGT GAA GAT GAA ATA AAA GAT TTA GAA GAA 384
   Phe Ser Met Ala Gly Ser Cys Glu Asp Glu Ile Lys Asp Leu Glu Glu
   780 785 790 795
   ATT GAT AAG ATC TTT GAT AAA GCC AAT CTC TCG AGT TCT ATT ATC ACC 432
   Ile Asp Lys Ile Phe Asp Lys Ala Asn Leu Ser Ser Ser Ile Ile Thr
   800 805 810
   TAT AAA AAT GTG GAA CCA GCA ACA ATT GGA TTT AAT AAA TCT TTA ACA 480
   Tyr Lys Asn Val Glu Pro Ala Thr Ile Gly Phe Asn Lys Ser Leu Thr
   815 820 825
   GAA GGT AAT ACG ATT AAT TCT GAT GCA ATG GCA CAG TTT AAA GAA CAA 528
   Glu Gly Asn Thr Ile Asn Ser Asp Ala Met Ala Gln Phe Lys Glu Gln
   830 835 840
   TTT TTA GGT AAG GAT ATG AAG TTT GAT AGT TAT CTA GAT ACT CAT TTA 576
   Phe Leu Gly Lys Asp Met Lys Phe Asp Ser Tyr Leu Asp Thr His Leu
   845 850 855
   ACT GCT CAA CAA GTT TCC AGT AAA AAA AGA GTT ATT TTG AAG GTT ACG 624
   Thr Ala Gln Gln Val Ser Ser Lys Lys Arg Val Ile Leu Lys Val Thr
   860 865 870 875
   GTT CCG AGT GGG AAA GGT TCT ACT ACT CCA ACA AAA GCA GGT GTC ATT 672
   Val Pro Ser Gly Lys Gly Ser Thr Thr Pro Thr Lys Ala Gly Val Ile
   880 885 890
   TTA AAC AAT AAT GAA TAC AAA ATG CTC ATT GAT AAT GGG TAT GTG CTC 720
   Leu Asn Asn Asn Glu Tyr Lys Met Leu Ile Asp Asn Gly Tyr Val Leu
   895 900 905
   CAT GTA GAT AAG GTA TCA AAA GTA GTA AAA AAA GGG ATG GAG TGC TTA 768
   His Val Asp Lys Val Ser Lys Val Val Lys Lys Gly Met Glu Cys Leu
   910 915 920
   CAA GTT GAA GGG ACT TTA AAA AAG AGT CTC GAC TTT AAA AAT GAT ATA 816
   Gln Val Glu Gly Thr Leu Lys Lys Ser Leu Asp Phe Lys Asn Asp Ile
   925 930 935
   AAT GCT GAA GCG CAT AGC TGG GGG ATG AAA ATT TAT GAA GAC TGG GCT 864
   Asn Ala Glu Ala His Ser Trp Gly Met Lys Ile Tyr Glu Asp Trp Ala
   940 945 950 955
   AAA AAT TTA ACC GCT TCG CAA AGG GAA GCT TTA GAT GGG TAT GCT AGG 912
   Lys Asn Leu Thr Ala Ser Gln Arg Glu Ala Leu Asp Gly Tyr Ala Arg
   960 965 970
   CAA GAT TAT AAA GAA ATC AAT AAT TAT TTG CGC AAT CAA GGC GGG AGT 960
   Gln Asp Tyr Lys Glu Ile Asn Asn Tyr Leu Arg Asn Gln Gly Gly Ser
   975 980 985
   GGA AAT GAA AAG CTG GAT GCC CAA TTA AAA AAT ATT TCT GAT GCT TTA 1008
   Gly Asn Glu Lys Leu Asp Ala Gln Leu Lys Asn Ile Ser Asp Ala Leu
   990 995 1000
   GGG AAG AAA CCC ATA CCA GAA AAT ATT ACC GTG TAT AGA TGG TGT GGC 1056
   Gly Lys Lys Pro Ile Pro Glu Asn Ile Thr Val Tyr Arg Trp Cys Gly
   1005 1010 1015
   ATG CCG GAA TTT GGT TAT CAA ATT AGT GAT CCG TTA CCT TCT TTA AAA 1104
   Met Pro Glu Phe Gly Tyr Gln Ile Ser Asp Pro Leu Pro Ser Leu Lys
   1020 1025 1030 1035
   GAT TTT GAA GAA CAA TTT TTA AAT ACA ATT AAA GAA GAC AAA GGG TAT 1152
   Asp Phe Glu Glu Gln Phe Leu Asn Thr Ile Lys Glu Asp Lys Gly Tyr
   1040 1045 1050
   ATG AGT ACA AGC TTA TCG AGT GAA CGT CTT GCA GCT TTT GGA TCT AGA 1200
   Met Ser Thr Ser Leu Ser Ser Glu Arg Leu Ala Ala Phe Gly Ser Arg
   1055 1060 1065
   AAA ATT ATA TTA CGC TTA CAA GTT CCG AAA GGA AGT ACG GGG GCG TAT 1248
   Lys Ile Ile Leu Arg Leu Gln Val Pro Lys Gly Ser Thr Gly Ala Tyr
   1070 1075 1080
   TTA AGT GCC ATT GGT GGA TTT GCA AGT GAA AAA GAG ATC CTA CTT GAT 1296
   Leu Ser Ala Ile Gly Gly Phe Ala Ser Glu Lys Glu Ile Leu Leu Asp
   1085 1090 1095
   AAA GAT AGT AAA TAT CAT ATT GAT AAA GCA ACA GAG GTA ATC ATT AAA 1344
   Lys Asp Ser Lys Tyr His Ile Asp Lys Ala Thr Glu Val Ile Ile Lys
   1100 1105 1110 1115
   GGT GTT AAG CGA TAT GTA GTG GAT GCA ACA TTA TTA ACA AAT 1386
   Gly Val Lys Arg Tyr Val Val Asp Ala Thr Leu Leu Thr Asn
   1120 1125
   TAAGGAG ATG AAA AAT ATG AAG AAA AAG TTA GCA AGT GTT GTA ACC TGT 1435
   Met Lys Asn Met Lys Lys Lys Leu Ala Ser Val Val Thr Cys
   1 5 10
   ATG TTA TTA GCT CCT ATG TTT TTG AAT GGA AAT GTG AAT GCT GTT AAC 1483
   Met Leu Leu Ala Pro Met Phe Leu Asn Gly Asn Val Asn Ala Val Asn
   15 20 25 30
   GCG GAT AGT AAA ATA AAT CAG ATT TCT ACA ACG CAG GAA AAC CAA CAG      1531
   Ala Asp Ser Lys Ile Asn Gln Ile Ser Thr Thr Gln Glu Asn Gln Gln
                   35                  40                  45
   AAA GAG ATG GAC CGA AAG GGA TTA TTG GGA TAT TAT TTC AAA GGA AAA      1579
   Lys Glu Met Asp Arg Lys Gly Leu Leu Gly Tyr Tyr Phe Lys Gly Lys
               50                  55                  60
   GAT TTT AAT AAT CTT ACT ATG TTT GCA CCG ACA CGT GAT AAT ACC CTT      1627
   Asp Phe Asn Asn Leu Thr Met Phe Ala Pro Thr Arg Asp Asn Thr Leu
           65                  70                  75
   ATG TAT GAC CAA CAA ACA GCG AAT GCA TTA TTA GAT AAA AAA CAA CAA      1675
   Met Tyr Asp Gln Gln Thr Ala Asn Ala Leu Leu Asp Lys Lys Gln Gln
       80                  85                  90
   GAA TAT CAG TCC ATT CGT TGG ATT GGT TTG ATT CAG CGT AAA GAA ACG      1723
   Glu Tyr Gln Ser Ile Arg Trp Ile Gly Leu Ile Gln Arg Lys Glu Thr
    95                 100                 105                 110
   GGC GAT TTC ACA TTT AAC TTA TCA AAG GAT GAA CAG GCA ATT ATA GAA      1771
   Gly Asp Phe Thr Phe Asn Leu Ser Lys Asp Glu Gln Ala Ile Ile Glu
                   115                 120                 125
   ATC GAT GGG AAA ATC ATT TCT AAT AAA GGG AAA GAA AAG CAA GTT GTC      1819
   Ile Asp Gly Lys Ile Ile Ser Asn Lys Gly Lys Glu Lys Gln Val Val
               130                 135                 140
   CAT TTA GAA AAA GAA AAA TTA GTT CCA ATC AAA ATA GAG TAT CAA TCA      1867
   His Leu Glu Lys Glu Lys Leu Val Pro Ile Lys Ile Glu Tyr Gln Ser
           145                 150                 155
   GAT ACG AAA TTT AAT ATT GAT AGT AAA ACA TTT AAA GAA CTT AAA TTA      1915
   Asp Thr Lys Phe Asn Ile Asp Ser Lys Thr Phe Lys Glu Leu Lys Leu
       160                 165                 170
   TTT AAA ATA GAT AGT CAA AAC CAA TCT CAA CAA GTT CAA CTG AGA AAC      1963
   Phe Lys Ile Asp Ser Gln Asn Gln Ser Gln Gln Val Gln Leu Arg Asn
   175                 180                 185                 190
   CCT GAA TTT AAC AAA AAA GAA TCA CAG GAA TTT TTA GCA AAA GCA TCA      2011
   Pro Glu Phe Asn Lys Lys Glu Ser Gln Glu Phe Leu Ala Lys Ala Ser
                   195                 200                 205
   AAA ACA AAC CTT TTT AAG CAA AAA ATG AAA AGA GAT ATT GAT GAA GAT      2059
   Lys Thr Asn Leu Phe Lys Gln Lys Met Lys Arg Asp Ile Asp Glu Asp
               210                 215                 220
   ACG GAT ACA GAT GGA GAC TCC ATT CCT GAT CTT TGG GAA GAA AAT GGG      2107
   Thr Asp Thr Asp Gly Asp Ser Ile Pro Asp Leu Trp Glu Glu Asn Gly
           225                 230                 235
   TAC ACG ATT CAA AAT AAA GTT GCT GTC AAA TGG GAT GAT TCG CTA GCA      2155
   Tyr Thr Ile Gln Asn Lys Val Ala Val Lys Trp Asp Asp Ser Leu Ala
       240                 245                 250
   AGT AAG GGA TAT ACA AAA TTT GTT TCG AAT CCA TTA GAC AGC CAC ACA      2203
   Ser Lys Gly Tyr Thr Lys Phe Val Ser Asn Pro Leu Asp Ser His Thr
   255                 260                 265                 270
   GTT GGC GAT CCC TAT ACT GAT TAT GAA AAG GCC GCA AGG GAT TTA GAT      2251
   Val Gly Asp Pro Tyr Thr Asp Tyr Glu Lys Ala Ala Arg Asp Leu Asp
                   275                 280                 285
   TTA TCA AAT GCA AAG GAA ACG TTC AAC CCA TTG GTA GCT GCT TTT CCA      2299
   Leu Ser Asn Ala Lys Glu Thr Phe Asn Pro Leu Val Ala Ala Phe Pro
               290                 295                 300
   AGT GTG AAT GTT AGT ATG GAA AAG GTG ATA TTA TCA CCA AAT GAA AAT      2347
   Ser Val Asn Val Ser Met Glu Lys Val Ile Leu Ser Pro Asn Glu Asn
           305                 310                 315
   TTA TCC AAT AGT GTA GAG TCT CAT TCA TCC ACG AAT TGG TCT TAT ACG      2395
   Leu Ser Asn Ser Val Glu Ser His Ser Ser Thr Asn Trp Ser Tyr Thr
       320                 325                 330
   AAT ACA GAA GGA GCT TCC ATT GAA GCT GGT GGC GGT CCA TTA GGC CTT      2443
   Asn Thr Glu Gly Ala Ser Ile Glu Ala Gly Gly Gly Pro Leu Gly Leu
   335                 340                 345                 350
   TCT TTT GGC GTG AGT GTT ACT TAT CAA CAC TCT GAA ACA GTT GCA CAA      2491
   Ser Phe Gly Val Ser Val Thr Tyr Gln His Ser Glu Thr Val Ala Gln
                   355                 360                 365
   GAA TGG GGA ACA TCT ACA GGA AAT ACT TCA CAA TTC AAT ACG GCT TCA      2539
   Glu Trp Gly Thr Ser Thr Gly Asn Thr Ser Gln Phe Asn Thr Ala Ser
               370                 375                 380
   GCG GGA TAT TTA AAT GCA AAT GTT CGG TAT AAC AAT GTA GGG ACT GGT      2587
   Ala Gly Tyr Leu Asn Ala Asn Val Arg Tyr Asn Asn Val Gly Thr Gly
           385                 390                 395
   GCC ATC TAT GAT GTA AAA CCT ACA ACA AGT TTT GTA TTA AAT AAC AAT      2635
   Ala Ile Tyr Asp Val Lys Pro Thr Thr Ser Phe Val Leu Asn Asn Asn
       400                 405                 410
   ACC ATC GCA ACG ATT ACA GCA AAA TCA AAT TCA ACA GCT TTA CGT ATA      2683
   Thr Ile Ala Thr Ile Thr Ala Lys Ser Asn Ser Thr Ala Leu Arg Ile
   415                 420                 425                 430
   TCT CCG GGG GAT AGT TAT CCA GAA ATA GGA GAA AAC GCT ATT GCG ATT      2731
   Ser Pro Gly Asp Ser Tyr Pro Glu Ile Gly Glu Asn Ala Ile Ala Ile
                   435                 440                 445
   ACA TCT ATG GAT GAT TTT AAT TCT CAT CCA ATT ACA TTA AAT AAA CAA      2779
   Thr Ser Met Asp Asp Phe Asn Ser His Pro Ile Thr Leu Asn Lys Gln
               450                 455                 460
   CAG GTA AAT CAA TTG ATA AAT AAT AAG CCA ATT ATG CTA GAG ACA GAC      2827
   Gln Val Asn Gln Leu Ile Asn Asn Lys Pro Ile Met Leu Glu Thr Asp
       465                 470                 475
   CAA ACA GAT GGT GTT TAT AAA ATA AGA GAT ACA CAT GGA AAT ATT GTA      2875
   Gln Thr Asp Gly Val Tyr Lys Ile Arg Asp Thr His Gly Asn Ile Val
       480                 485                 490
   ACT GGT GGA GAA TGG AAT GGT GTA ACA CAA CAA ATT AAA GCA AAA ACA      2923
   Thr Gly Gly Glu Trp Asn Gly Val Thr Gln Gln Ile Lys Ala Lys Thr
   495                 500                 505                 510
   GCG TCT ATT ATT GTG GAT GAC GGG AAA CAG GTA GCA GAA AAA CGT GTG      2971
   Ala Ser Ile Ile Val Asp Asp Gly Lys Gln Val Ala Glu Lys Arg Val
                   515                 520                 525
   GCG GCA AAA GAT TAT GGT CAT CCA GAA GAT AAA ACA CCA CCT TTA ACT      3019
   Ala Ala Lys Asp Tyr Gly His Pro Glu Asp Lys Thr Pro Pro Leu Thr
               530                 535                 540
   TTA AAA GAT ACC CTG AAG CTT TCA TAC CCA GAT GAA ATA AAA GAA ACT      3067
   Leu Lys Asp Thr Leu Lys Leu Ser Tyr Pro Asp Glu Ile Lys Glu Thr
           545                 550                 555
   AAT GGA TTG TTG TAC TAT GAT GAC AAA CCA ATC TAT GAA TCG AGT GTC      3115
   Asn Gly Leu Leu Tyr Tyr Asp Asp Lys Pro Ile Tyr Glu Ser Ser Val
       560                 565                 570
   ATG ACT TAT CTG GAT GAA AAT ACG GCA AAA GAA GTC AAA AAA CAA ATA      3163
   Met Thr Tyr Leu Asp Glu Asn Thr Ala Lys Glu Val Lys Lys Gln Ile
   575                 580                 585                 590
   AAT GAT ACA ACC GGA AAA TTT AAG GAT GTA AAT CAC TTA TAT GAT GTA      3211
   Asn Asp Thr Thr Gly Lys Phe Lys Asp Val Asn His Leu Tyr Asp Val
                   595                 600                 605
   AAA CTG ACT CCA AAA ATG AAT TTT ACG ATT AAA ATG GCT TCC TTG TAT      3259
   Lys Leu Thr Pro Lys Met Asn Phe Thr Ile Lys Met Ala Ser Leu Tyr
               610                 615                 620
   GAT GGG GCT GAA AAT AAT CAT AAC TCT TTA GGA ACC TGG TAT TTA ACA      3307
   Asp Gly Ala Glu Asn Asn His Asn Ser Leu Gly Thr Trp Tyr Leu Thr
           625                 630                 635
   TAT AAT GTT GCT GGT GGA AAT ACT GGG AAG AGA CAA TAT CGT TCA GCT      3355
   Tyr Asn Val Ala Gly Gly Asn Thr Gly Lys Arg Gln Tyr Arg Ser Ala
       640                 645                 650
   CAT TCT TGT GCA CAT GTA GCT CTA TCT TCA GAA GCG AAA AAG AAA CTA      3403
   His Ser Cys Ala His Val Ala Leu Ser Ser Glu Ala Lys Lys Lys Leu
   655                 660                 665                 670
   AAT CAA AAT GCG AAT TAC TAT CTT AGC ATG TAT ATG AAG GCT GAT TCT      3451
   Asn Gln Asn Ala Asn Tyr Tyr Leu Ser Met Tyr Met Lys Ala Asp Ser
                   675                 680                 685
   ACT ACG GAA CCT ACA ATA GAA GTA GCT GGG GAA AAA TCT GCA ATA ACA      3499
   Thr Thr Glu Pro Thr Ile Glu Val Ala Gly Glu Lys Ser Ala Ile Thr
               690                     695                     700
   AGT AAA AAA GTA AAA TTA AAT AAT CAA AAT TAT CAA AGA GTT GAT ATT        3547
   Ser Lys Lys Val Lys Leu Asn Asn Gln Asn Tyr Gln Arg Val Asp Ile
           705                 710                     715
   TTA GTG AAA AAT TCT GAA AGA AAT CCA ATG GAT AAA ATA TAT ATA AGA        3595
   Leu Val Lys Asn Ser Glu Arg Asn Pro Met Asp Lys Ile Tyr Ile Arg
       720                     725                     730
   GGA AAT GGC ACG ACA AAT GTT TAT GGG GAT GAT GTT ACT ATC CCA GAG        3643
   Gly Asn Gly Thr Thr Asn Val Tyr Gly Asp Asp Val Thr Ile Pro Glu
   735                     740                     745                 750
   GTA TCA GCT ATA AAT CCG GCT AGT CTA TCA GAT GAA GAA ATT CAA GAA        3691
   Val Ser Ala Ile Asn Pro Ala Ser Leu Ser Asp Glu Glu Ile Gln Glu
                       755                     760                 765
   ATA TTT AAA GAC TCA ACT ATT GAA TAT GGA AAT CCT AGT TTC GTT GCT        3739
   Ile Phe Lys Asp Ser Thr Ile Glu Tyr Gly Asn Pro Ser Phe Val Ala
               770                     775                     780
   GAT GCC GTA ACA TTT AAA AAT ATA AAA CCT TTA CAA AAT TAT GTA AAG        3787
   Asp Ala Val Thr Phe Lys Asn Ile Lys Pro Leu Gln Asn Tyr Val Lys
           785                 790                     795
   GAA TAT GAA ATA TAT CAT AAA TCT CAT CGA TAT GAA AAG AAA ACG GTC        3835
   Glu Tyr Glu Ile Tyr His Lys Ser His Arg Tyr Glu Lys Lys Thr Val
       800                     805                     810
   TTT GAT ATC ATG GGT GTT CAT TAT GAG TAT AGT ATA GCT AGG GAA CAA        3883
   Phe Asp Ile Met Gly Val His Tyr Glu Tyr Ser Ile Ala Arg Glu Gln
   815                     820                     825                 830
   AAG AAA GCC GCA TAATTTTAAA AATAAAACTC GTTAGAGTTT ATTTAGCATG            3935
   Lys Lys Ala Ala
   GTATTTTTAA GAATAATCAA TATGTTGAAC CGTTTGTAGC TGTTTTGGAA GGGAATTTCA      3995
   TTTTATTTGG TCTCTTAAGT TGATGGGCAT GGGATATGTT CAGCATCCAA GCGTTTNGGG      4055
   GGTTANAAAA TCCAATTTT                                                   4074
(2) INFORMATION FOR SEQ ID NO:20:
   (i) SEQUENCE CHARACTERISTICS:
      (A) LENGTH: 462 amino acids
      (B) TYPE: amino acid
      (D) TOPOLOGY: linear
   (ii) MOLECULE TYPE: protein
   (xi) SEQUENCE DESCRIPTION: SEQ ID NO:20:
   Met Gln Arg Met Glu Gly Lys Leu Phe Val Val Ser Lys Thr Leu Gln
   1 5 10 15
   Val Val Thr Arg Thr Val Leu Leu Ser Thr Val Tyr Ser Ile Thr Leu
   20 25 30
   Leu Asn Asn Val Val Ile Lys Ala Asp Gln Leu Asn Ile Asn Ser Gln
   35 40 45
   Ser Lys Tyr Thr Asn Leu Gln Asn Leu Lys Ile Pro Asp Asn Ala Glu
   50 55 60
   Asp Phe Lys Glu Asp Lys Gly Lys Ala Lys Glu Trp Gly Lys Glu Lys
   65 70 75 80
   Gly Glu Glu Trp Arg Pro Pro Ala Thr Glu Lys Gly Glu Met Asn Asn
   85 90 95
   Phe Leu Asp Asn Lys Asn Asp Ile Lys Thr Asn Tyr Lys Glu Ile Thr
   100 105 110
   Phe Ser Met Ala Gly Ser Cys Glu Asp Glu Ile Lys Asp Leu Glu Glu
   115 120 125
   Ile Asp Lys Ile Phe Asp Lys Ala Asn Leu Ser Ser Ser Ile Ile Thr
   130 135 140
   Tyr Lys Asn Val Glu Pro Ala Thr Ile Gly Phe Asn Lys Ser Leu Thr
   145 150 155 160
   Glu Gly Asn Thr Ile Asn Ser Asp Ala Met Ala Gln Phe Lys Glu Gln
   165 170 175
   Phe Leu Gly Lys Asp Met Lys Phe Asp Ser Tyr Leu Asp Thr His Leu
   180 185 190
   Thr Ala Gln Gln Val Ser Ser Lys Lys Arg Val Ile Leu Lys Val Thr
   195 200 205
   Val Pro Ser Gly Lys Gly Ser Thr Thr Pro Thr Lys Ala Gly Val Ile
   210 215 220
   Leu Asn Asn Asn Glu Tyr Lys Met Leu Ile Asp Asn Gly Tyr Val Leu
   225 230 235 240
   His Val Asp Lys Val Ser Lys Val Val Lys Lys Gly Met Glu Cys Leu
   245 250 255
   Gln Val Glu Gly Thr Leu Lys Lys Ser Leu Asp Phe Lys Asn Asp Ile
   260 265 270
   Asn Ala Glu Ala His Ser Trp Gly Met Lys Ile Tyr Glu Asp Trp Ala
   275 280 285
   Lys Asn Leu Thr Ala Ser Gln Arg Glu Ala Leu Asp Gly Tyr Ala Arg
   290 295 300
   Gln Asp Tyr Lys Glu Ile Asn Asn Tyr Leu Arg Asn Gln Gly Gly Ser
   305 310 315 320
   Gly Asn Glu Lys Leu Asp Ala Gln Leu Lys Asn Ile Ser Asp Ala Leu
   325 330 335
   Gly Lys Lys Pro Ile Pro Glu Asn Ile Thr Val Tyr Arg Trp Cys Gly
   340 345 350
   Met Pro Glu Phe Gly Tyr Gln Ile Ser Asp Pro Leu Pro Ser Leu Lys
   355 360 365
   Asp Phe Glu Glu Gln Phe Leu Asn Thr Ile Lys Glu Asp Lys Gly Tyr
   370 375 380
   Met Ser Thr Ser Leu Ser Ser Glu Arg Leu Ala Ala Phe Gly Ser Arg
   385 390 395 400
   Lys Ile Ile Leu Arg Leu Gln Val Pro Lys Gly Ser Thr Gly Ala Tyr
   405 410 415
   Leu Ser Ala Ile Gly Gly Phe Ala Ser Glu Lys Glu Ile Leu Leu Asp
   420 425 430
   Lys Asp Ser Lys Tyr His Ile Asp Lys Ala Thr Glu Val Ile Ile Lys
   435 440 445
   Gly Val Lys Arg Tyr Val Val Asp Ala Thr Leu Leu Thr Asn
   450 455 460
(2) INFORMATION FOR SEQ ID NO:21:
   (i) SEQUENCE CHARACTERISTICS:
      (A) LENGTH: 834 amino acids
      (B) TYPE: amino acid
      (D) TOPOLOGY: linear
   (ii) MOLECULE TYPE: protein
   (xi) SEQUENCE DESCRIPTION: SEQ ID NO:21:
   Met Lys Asn Met Lys Lys Lys Leu Ala Ser Val Val Thr Cys Met Leu
   1 5 10 15
   Leu Ala Pro Met Phe Leu Asn Gly Asn Val Asn Ala Val Asn Ala Asp
   20 25 30
   Ser Lys Ile Asn Gln Ile Ser Thr Thr Gln Glu Asn Gln Gln Lys Glu
   35 40 45
   Met Asp Arg Lys Gly Leu Leu Gly Tyr Tyr Phe Lys Gly Lys Asp Phe
   50 55 60
   Asn Asn Leu Thr Met Phe Ala Pro Thr Arg Asp Asn Thr Leu Met Tyr
   65 70 75 80
   Asp Gln Gln Thr Ala Asn Ala Leu Leu Asp Lys Lys Gln Gln Glu Tyr
   85 90 95
   Gln Ser Ile Arg Trp Ile Gly Leu Ile Gln Arg Lys Glu Thr Gly Asp
   100 105 110
   Phe Thr Phe Asn Leu Ser Lys Asp Glu Gln Ala Ile Ile Glu Ile Asp
   115 120 125
   Gly Lys Ile Ile Ser Asn Lys Gly Lys Glu Lys Gln Val Val His Leu
   130 135 140
   Glu Lys Glu Lys Leu Val Pro Ile Lys Ile Glu Tyr Gln Ser Asp Thr
   145 150 155 160
   Lys Phe Asn Ile Asp Ser Lys Thr Phe Lys Glu Leu Lys Leu Phe Lys
   165 170 175
   Ile Asp Ser Gln Asn Gln Ser Gln Gln Val Gln Leu Arg Asn Pro Glu
   180 185 190
   Phe Asn Lys Lys Glu Ser Gln Glu Phe Leu Ala Lys Ala Ser Lys Thr
   195 200 205
   Asn Leu Phe Lys Gln Lys Met Lys Arg Asp Ile Asp Glu Asp Thr Asp
   210 215 220
   Thr Asp Gly Asp Ser Ile Pro Asp Leu Trp Glu Glu Asn Gly Tyr Thr
   225 230 235 240
   Ile Gln Asn Lys Val Ala Val Lys Trp Asp Asp Ser Leu Ala Ser Lys
   245 250 255
   Gly Tyr Thr Lys Phe Val Ser Asn Pro Leu Asp Ser His Thr Val Gly
   260 265 270
   Asp Pro Tyr Thr Asp Tyr Glu Lys Ala Ala Arg Asp Leu Asp Leu Ser
   275 280 285
   Asn Ala Lys Glu Thr Phe Asn Pro Leu Val Ala Ala Phe Pro Ser Val
   290 295 300
   Asn Val Ser Met Glu Lys Val Ile Leu Ser Pro Asn Glu Asn Leu Ser
   305 310 315 320
   Asn Ser Val Glu Ser His Ser Ser Thr Asn Trp Ser Tyr Thr Asn Thr
   325 330 335
   Glu Gly Ala Ser Ile Glu Ala Gly Gly Gly Pro Leu Gly Leu Ser Phe
   340 345 350
   Gly Val Ser Val Thr Tyr Gln His Ser Glu Thr Val Ala Gln Glu Trp
   355 360 365
   Gly Thr Ser Thr Gly Asn Thr Ser Gln Phe Asn Thr Ala Ser Ala Gly
   370 375 380
   Tyr Leu Asn Ala Asn Val Arg Tyr Asn Asn Val Gly Thr Gly Ala Ile
   385 390 395 400
   Tyr Asp Val Lys Pro Thr Thr Ser Phe Val Leu Asn Asn Asn Thr Ile
   405 410 415
   Ala Thr Ile Thr Ala Lys Ser Asn Ser Thr Ala Leu Arg Ile Ser Pro
   420 425 430
   Gly Asp Ser Tyr Pro Glu Ile Gly Glu Asn Ala Ile Ala Ile Thr Ser
   435 440 445
   Met Asp Asp Phe Asn Ser His Pro Ile Thr Leu Asn Lys Gln Gln Val
   450 455 460
   Asn Gln Leu Ile Asn Asn Lys Pro Ile Met Leu Glu Thr Asp Gln Thr
   465 470 475 480
   Asp Gly Val Tyr Lys Ile Arg Asp Thr His Gly Asn Ile Val Thr Gly
   485 490 495
   Gly Glu Trp Asn Gly Val Thr Gln Gln Ile Lys Ala Lys Thr Ala Ser
   500 505 510
   Ile Ile Val Asp Asp Gly Lys Gln Val Ala Glu Lys Arg Val Ala Ala
   515 520 525
   Lys Asp Tyr Gly His Pro Glu Asp Lys Thr Pro Pro Leu Thr Leu Lys
   530 535 540
   Asp Thr Leu Lys Leu Ser Tyr Pro Asp Glu Ile Lys Glu Thr Asn Gly
   545 550 555 560
   Leu Leu Tyr Tyr Asp Asp Lys Pro Ile Tyr Glu Ser Ser Val Met Thr
   565 570 575
   Tyr Leu Asp Glu Asn Thr Ala Lys Glu Val Lys Lys Gln Ile Asn Asp
   580 585 590
   Thr Thr Gly Lys Phe Lys Asp Val Asn His Leu Tyr Asp Val Lys Leu
   595 600 605
   Thr Pro Lys Met Asn Phe Thr Ile Lys Met Ala Ser Leu Tyr Asp Gly
   610 615 620
   Ala Glu Asn Asn His Asn Ser Leu Gly Thr Trp Tyr Leu Thr Tyr Asn
   625 630 635 640
   Val Ala Gly Gly Asn Thr Gly Lys Arg Gln Tyr Arg Ser Ala His Ser
   645 650 655
   Cys Ala His Val Ala Leu Ser Ser Glu Ala Lys Lys Lys Leu Asn Gln
   660 665 670
   Asn Ala Asn Tyr Tyr Leu Ser Met Tyr Met Lys Ala Asp Ser Thr Thr
   675 680 685
   Glu Pro Thr Ile Glu Val Ala Gly Glu Lys Ser Ala Ile Thr Ser Lys
   690 695 700
   Lys Val Lys Leu Asn Asn Gln Asn Tyr Gln Arg Val Asp Ile Leu Val
   705 710 715 720
   Lys Asn Ser Glu Arg Asn Pro Met Asp Lys Ile Tyr Ile Arg Gly Asn
   725 730 735
   Gly Thr Thr Asn Val Tyr Gly Asp Asp Val Thr Ile Pro Glu Val Ser
   740 745 750
   Ala Ile Asn Pro Ala Ser Leu Ser Asp Glu Glu Ile Gln Glu Ile Phe
   755 760 765
   Lys Asp Ser Thr Ile Glu Tyr Gly Asn Pro Ser Phe Val Ala Asp Ala
   770 775 780
   Val Thr Phe Lys Asn Ile Lys Pro Leu Gln Asn Tyr Val Lys Glu Tyr
   785 790 795 800
   Glu Ile Tyr His Lys Ser His Arg Tyr Glu Lys Lys Thr Val Phe Asp
   805 810 815
   Ile Met Gly Val His Tyr Glu Tyr Ser Ile Ala Arg Glu Gln Lys Lys
   820 825 830
   Ala Ala
(2) INFORMATION FOR SEQ ID NO:22:
   (i) SEQUENCE CHARACTERISTICS:
      (A) LENGTH: 4041 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..4038
      (D) OTHER INFORMATION: /product= "VIP1A(a)/VIP2A(a) fusion product"
   (xi) SEQUENCE DESCRIPTION: SEQ ID NO:22:
   ATG AAA AGA ATG GAG GGA AAG TTG TTT ATG GTG TCA AAA AAA TTA CAA      48
   Met Lys Arg Met Glu Gly Lys Leu Phe Met Val Ser Lys Lys Leu Gln
   835                 840                 845                 850
   GTA GTT ACT AAA ACT GTA TTG CTT AGT ACA GTT TTC TCT ATA TCT TTA      96
   Val Val Thr Lys Thr Val Leu Leu Ser Thr Val Phe Ser Ile Ser Leu
                   855                 860                 865
   TTA AAT AAT GAA GTG ATA AAA GCT GAA CAA TTA AAT ATA AAT TCT CAA     144
   Leu Asn Asn Glu Val Ile Lys Ala Glu Gln Leu Asn Ile Asn Ser Gln
               870                 875                 880
   AGT AAA TAT ACT AAC TTG CAA AAT CTA AAA ATC ACT GAC AAG GTA GAG     192
   Ser Lys Tyr Thr Asn Leu Gln Asn Leu Lys Ile Thr Asp Lys Val Glu
           885                 890                 895
   GAT TTT AAA GAA GAT AAG GAA AAA GCG AAA GAA TGG GGG AAA GAA AAA     240
   Asp Phe Lys Glu Asp Lys Glu Lys Ala Lys Glu Trp Gly Lys Glu Lys
       900                 905                 910
   GAA AAA GAG TGG AAA CTA ACT GCT ACT GAA AAA GGA AAA ATG AAT AAT     288
   Glu Lys Glu Trp Lys Leu Thr Ala Thr Glu Lys Gly Lys Met Asn Asn
   915                 920                 925                 930
   TTT TTA GAT AAT AAA AAT GAT ATA AAG ACA AAT TAT AAA GAA ATT ACT     336
   Phe Leu Asp Asn Lys Asn Asp Ile Lys Thr Asn Tyr Lys Glu Ile Thr
                   935                 940                 945
   TTT TCT ATG GCA GGC TCA TTT GAA GAT GAA ATA AAA GAT TTA AAA GAA     384
   Phe Ser Met Ala Gly Ser Phe Glu Asp Glu Ile Lys Asp Leu Lys Glu
               950                 955                 960
   ATT GAT AAG ATG TTT GAT AAA ACC AAT CTA TCA AAT TCT ATT ATC ACC     432
   Ile Asp Lys Met Phe Asp Lys Thr Asn Leu Ser Asn Ser Ile Ile Thr
           965                 970                 975
   TAT AAA AAT GTG GAA CCG ACA ACA ATT GGA TTT AAT AAA TCT TTA ACA     480
   Tyr Lys Asn Val Glu Pro Thr Thr Ile Gly Phe Asn Lys Ser Leu Thr
       980                 985                 990
   GAA GGT AAT ACG ATT AAT TCT GAT GCA ATG GCA CAG TTT AAA GAA CAA     528
   Glu Gly Asn Thr Ile Asn Ser Asp Ala Met Ala Gln Phe Lys Glu Gln
   995                1000                1005                1010
   TTT TTA GAT AGG GAT ATT AAG TTT GAT AGT TAT CTA GAT ACG CAT TTA     576
   Phe Leu Asp Arg Asp Ile Lys Phe Asp Ser Tyr Leu Asp Thr His Leu
                  1015                1020                1025
   ACT GCT CAA CAA GTT TCC AGT AAA GAA AGA GTT ATT TTG AAG GTT ACG     624
   Thr Ala Gln Gln Val Ser Ser Lys Glu Arg Val Ile Leu Lys Val Thr
   1030 1035 1040
   GTT CCG AGT GGG AAA GGT TCT ACT ACT CCA ACA AAA GCA GGT GTC ATT 672
   Val Pro Ser Gly Lys Gly Ser Thr Thr Pro Thr Lys Ala Gly Val Ile
   1045 1050 1055
   TTA AAT AAT AGT GAA TAC AAA ATG CTC ATT GAT AAT GGG TAT ATG GTC 720
   Leu Asn Asn Ser Glu Tyr Lys Met Leu Ile Asp Asn Gly Tyr Met Val
   1060 1065 1070
   CAT GTA GAT AAG GTA TCA AAA GTG GTG AAA AAA GGG GTG GAG TGC TTA 768
   His Val Asp Lys Val Ser Lys Val Val Lys Lys Gly Val Glu Cys Leu
   1075 1080 1085 1090
   CAA ATT GAA GGG ACT TTA AAA AAG AGT CTT GAC TTT AAA AAT GAT ATA 816
   Gln Ile Glu Gly Thr Leu Lys Lys Ser Leu Asp Phe Lys Asn Asp Ile
   1095 1100 1105
   AAT GCT GAA GCG CAT AGC TGG GGT ATG AAG AAT TAT GAA GAG TGG GCT 864
   Asn Ala Glu Ala His Ser Trp Gly Met Lys Asn Tyr Glu Glu Trp Ala
   1110 1115 1120
   AAA GAT TTA ACC GAT TCG CAA AGG GAA GCT TTA GAT GGG TAT GCT AGG 912
   Lys Asp Leu Thr Asp Ser Gln Arg Glu Ala Leu Asp Gly Tyr Ala Arg
   1125 1130 1135
   CAA GAT TAT AAA GAA ATC AAT AAT TAT TTA AGA AAT CAA GGC GGA AGT 960
   Gln Asp Tyr Lys Glu Ile Asn Asn Tyr Leu Arg Asn Gln Gly Gly Ser
   1140 1145 1150
   GGA AAT GAA AAA CTA GAT GCT CAA ATA AAA AAT ATT TCT GAT GCT TTA 1008
   Gly Asn Glu Lys Leu Asp Ala Gln Ile Lys Asn Ile Ser Asp Ala Leu
   1155 1160 1165 1170
   GGG AAG AAA CCA ATA CCG GAA AAT ATT ACT GTG TAT AGA TGG TGT GGC 1056
   Gly Lys Lys Pro Ile Pro Glu Asn Ile Thr Val Tyr Arg Trp Cys Gly
   1175 1180 1185
   ATG CCG GAA TTT GGT TAT CAA ATT AGT GAT CCG TTA CCT TCT TTA AAA 1104
   Met Pro Glu Phe Gly Tyr Gln Ile Ser Asp Pro Leu Pro Ser Leu Lys
   1190 1195 1200
   GAT TTT GAA GAA CAA TTT TTA AAT ACA ATC AAA GAA GAC AAA GGA TAT 1152
   Asp Phe Glu Glu Gln Phe Leu Asn Thr Ile Lys Glu Asp Lys Gly Tyr
   1205 1210 1215
   ATG AGT ACA AGC TTA TCG AGT GAA CGT CTT GCA GCT TTT GGA TCT AGA 1200
   Met Ser Thr Ser Leu Ser Ser Glu Arg Leu Ala Ala Phe Gly Ser Arg
   1220 1225 1230
   AAA ATT ATA TTA CGA TTA CAA GTT CCG AAA GGA AGT ACG GGT GCG TAT 1248
   Lys Ile Ile Leu Arg Leu Gln Val Pro Lys Gly Ser Thr Gly Ala Tyr
   1235 1240 1245 1250
   TTA AGT GCC ATT GGT GGA TTT GCA AGT GAA AAA GAG ATC CTA CTT GAT 1296
   Leu Ser Ala Ile Gly Gly Phe Ala Ser Glu Lys Glu Ile Leu Leu Asp
                   1255                1260                1265
   AAA GAT AGT AAA TAT CAT ATT GAT AAA GTA ACA GAG GTA ATT ATT AAA      1344
   Lys Asp Ser Lys Tyr His Ile Asp Lys Val Thr Glu Val Ile Ile Lys
               1270                1275                1280
   GGT GTT AAG CGA TAT GTA GTG GAT GCA ACA TTA TTA ACA AAT ATG AAA      1392
   Gly Val Lys Arg Tyr Val Val Asp Ala Thr Leu Leu Thr Asn Met Lys
           1285                1290                1295
   AAT ATG AAG AAA AAG TTA GCA AGT GTT GTA ACG TGT ACG TTA TTA GCT      1440
   Asn Met Lys Lys Lys Leu Ala Ser Val Val Thr Cys Thr Leu Leu Ala
       1300                1305                1310
   CCT ATG TTT TTG AAT GGA AAT GTG AAT GCT GTT TAC GCA GAC AGC AAA      1488
   Pro Met Phe Leu Asn Gly Asn Val Asn Ala Val Tyr Ala Asp Ser Lys
   1315                1320                1325                1330
   ACA AAT CAA ATT TCT ACA ACA CAG AAA AAT CAA CAG AAA GAG ATG GAC      1536
   Thr Asn Gln Ile Ser Thr Thr Gln Lys Asn Gln Gln Lys Glu Met Asp
                   1335                1340                1345
   CGA AAA GGA TTA CTT GGG TAT TAT TTC AAA GGA AAA GAT TTT AGT AAT      1584
   Arg Lys Gly Leu Leu Gly Tyr Tyr Phe Lys Gly Lys Asp Phe Ser Asn
               1350                1355                1360
   CTT ACT ATG TTT GCA CCG ACA CGT GAT AGT ACT CTT ATT TAT GAT CAA      1632
   Leu Thr Met Phe Ala Pro Thr Arg Asp Ser Thr Leu Ile Tyr Asp Gln
           1365                1370                1375
   CAA ACA GCA AAT AAA CTA TTA GAT AAA AAA CAA CAA GAA TAT CAG TCT      1680
   Gln Thr Ala Asn Lys Leu Leu Asp Lys Lys Gln Gln Glu Tyr Gln Ser
       1380                1385                1390
   ATT CGT TGG ATT GGT TTG ATT CAG AGT AAA GAA ACG GGA GAT TTC ACA      1728
   Ile Arg Trp Ile Gly Leu Ile Gln Ser Lys Glu Thr Gly Asp Phe Thr
   1395                1400                1405                1410
   TTT AAC TTA TCT GAG GAT GAA CAG GCA ATT ATA GAA ATC AAT GGG AAA      1776
   Phe Asn Leu Ser Glu Asp Glu Gln Ala Ile Ile Glu Ile Asn Gly Lys
                   1415                1420                1425
   ATT ATT TCT AAT AAA GGG AAA GAA AAG CAA GTT GTC CAT TTA GAA AAA      1824
   Ile Ile Ser Asn Lys Gly Lys Glu Lys Gln Val Val His Leu Glu Lys
               1430                1435                1440
   GGA AAA TTA GTT CCA ATC AAA ATA GAG TAT CAA TCA GAT ACA AAA TTT      1872
   Gly Lys Leu Val Pro Ile Lys Ile Glu Tyr Gln Ser Asp Thr Lys Phe
           1445                1450                1455
   AAT ATT GAC AGT AAA ACA TTT AAA GAA CTT AAA TTA TTT AAA ATA GAT      1920
   Asn Ile Asp Ser Lys Thr Phe Lys Glu Leu Lys Leu Phe Lys Ile Asp
       1460                1465                1470
   AGT CAA AAC CAA CCC CAG CAA GTC CAG CAA GAT GAA CTG AGA AAT CCT 1968
   Ser Gln Asn Gln Pro Gln Gln Val Gln Gln Asp Glu Leu Arg Asn Pro
   1475 1480 1485 1490
   GAA TTT AAC AAG AAA GAA TCA CAG GAA TTC TTA GCG AAA CCA TCG AAA 2016
   Glu Phe Asn Lys Lys Glu Ser Gln Glu Phe Leu Ala Lys Pro Ser Lys
   1495 1500 1505
   ATA AAT CTT TTC ACT CAA AAA ATG AAA AGG GAA ATT GAT GAA GAC ACG 2064
   Ile Asn Leu Phe Thr Gln Lys Met Lys Arg Glu Ile Asp Glu Asp Thr
   1510 1515 1520
   GAT ACG GAT GGG GAC TCT ATT CCT GAC CTT TGG GAA GAA AAT GGG TAT 2112
   Asp Thr Asp Gly Asp Ser Ile Pro Asp Leu Trp Glu Glu Asn Gly Tyr
   1525 1530 1535
   ACG ATT CAA AAT AGA ATC GCT GTA AAG TGG GAC GAT TCT CTA GCA AGT 2160
   Thr Ile Gln Asn Arg Ile Ala Val Lys Trp Asp Asp Ser Leu Ala Ser
   1540 1545 1550
   AAA GGG TAT ACG AAA TTT GTT TCA AAT CCA CTA GAA AGT CAC ACA GTT 2208
   Lys Gly Tyr Thr Lys Phe Val Ser Asn Pro Leu Glu Ser His Thr Val
   1555 1560 1565 1570
   GGT GAT CCT TAT ACA GAT TAT GAA AAG GCA GCA AGA GAT CTA GAT TTG 2256
   Gly Asp Pro Tyr Thr Asp Tyr Glu Lys Ala Ala Arg Asp Leu Asp Leu
   1575 1580 1585
   TCA AAT GCA AAG GAA ACG TTT AAC CCA TTG GTA GCT GCT TTT CCA AGT 2304
   Ser Asn Ala Lys Glu Thr Phe Asn Pro Leu Val Ala Ala Phe Pro Ser
   1590 1595 1600
   GTG AAT GTT AGT ATG GAA AAG GTG ATA TTA TCA CCA AAT GAA AAT TTA 2352
   Val Asn Val Ser Met Glu Lys Val Ile Leu Ser Pro Asn Glu Asn Leu
   1605 1610 1615
   TCC AAT AGT GTA GAG TCT CAT TCA TCC ACG AAT TGG TCT TAT ACA AAT 2400
   Ser Asn Ser Val Glu Ser His Ser Ser Thr Asn Trp Ser Tyr Thr Asn
   1620 1625 1630
   ACA GAA GGT GCT TCT GTT GAA GCG GGG ATT GGA CCA AAA GGT ATT TCG 2448
   Thr Glu Gly Ala Ser Val Glu Ala Gly Ile Gly Pro Lys Gly Ile Ser
   1635 1640 1645 1650
   TTC GGA GTT AGC GTA AAC TAT CAA CAC TCT GAA ACA GTT GCA CAA GAA 2496
   Phe Gly Val Ser Val Asn Tyr Gln His Ser Glu Thr Val Ala Gln Glu
   1655 1660 1665
   TGG GGA ACA TCT ACA GGA AAT ACT TCG CAA TTC AAT ACG GCT TCA GCG 2544
   Trp Gly Thr Ser Thr Gly Asn Thr Ser Gln Phe Asn Thr Ala Ser Ala
   1670 1675 1680
   GGA TAT TTA AAT GCA AAT GTT CGA TAT AAC AAT GTA GGA ACT GGT GCC 2592
   Gly Tyr Leu Asn Ala Asn Val Arg Tyr Asn Asn Val Gly Thr Gly Ala
   1685 1690 1695
   ATC TAC GAT GTA AAA CCT ACA ACA AGT TTT GTA TTA AAT AAC GAT ACT      2640
   Ile Tyr Asp Val Lys Pro Thr Thr Ser Phe Val Leu Asn Asn Asp Thr
       1700                1705                1710
   ATC GCA ACT ATT ACG GCG AAA TCT AAT TCT ACA GCC TTA AAT ATA TCT      2688
   Ile Ala Thr Ile Thr Ala Lys Ser Asn Ser Thr Ala Leu Asn Ile Ser
   1715                1720                1725                1730
   CCT GGA GAA AGT TAC CCG AAA AAA GGA CAA AAT GGA ATC GCA ATA ACA      2736
   Pro Gly Glu Ser Tyr Pro Lys Lys Gly Gln Asn Gly Ile Ala Ile Thr
                   1735                1740                1745
   TCA ATG GAT GAT TTT AAT TCC CAT CCG ATT ACA TTA AAT AAA AAA CAA      2784
   Ser Met Asp Asp Phe Asn Ser His Pro Ile Thr Leu Asn Lys Lys Gln
               1750                1755                1760
   GTA GAT AAT CTG CTA AAT AAT AAA CCT ATG ATG TTG GAA ACA AAC CAA      2832
   Val Asp Asn Leu Leu Asn Asn Lys Pro Met Met Leu Glu Thr Asn Gln
           1765                1770                1775
   ACA GAT GGT GTT TAT AAG ATA AAA GAT ACA CAT GGA AAT ATA GTA ACT      2880
   Thr Asp Gly Val Tyr Lys Ile Lys Asp Thr His Gly Asn Ile Val Thr
       1780                1785                1790
   GGC GGA GAA TGG AAT GGT GTC ATA CAA CAA ATC AAG GCT AAA ACA GCG      2928
   Gly Gly Glu Trp Asn Gly Val Ile Gln Gln Ile Lys Ala Lys Thr Ala
   1795                1800                1805                1810
   TCT ATT ATT GTG GAT GAT GGG GAA CGT GTA GCA GAA AAA CGT GTA GCG      2976
   Ser Ile Ile Val Asp Asp Gly Glu Arg Val Ala Glu Lys Arg Val Ala
                   1815                1820                1825
   GCA AAA GAT TAT GAA AAT CCA GAA GAT AAA ACA CCG TCT TTA ACT TTA      3024
   Ala Lys Asp Tyr Glu Asn Pro Glu Asp Lys Thr Pro Ser Leu Thr Leu
               1830                1835                1840
   AAA GAT GCC CTG AAG CTT TCA TAT CCA GAT GAA ATA AAA GAA ATA GAG      3072
   Lys Asp Ala Leu Lys Leu Ser Tyr Pro Asp Glu Ile Lys Glu Ile Glu
           1845                1850                1855
   GGA TTA TTA TAT TAT AAA AAC AAA CCG ATA TAC GAA TCG AGC GTT ATG      3120
   Gly Leu Leu Tyr Tyr Lys Asn Lys Pro Ile Tyr Glu Ser Ser Val Met
       1860                1865                1870
   ACT TAC TTA GAT GAA AAT ACA GCA AAA GAA GTG ACC AAA CAA TTA AAT      3168
   Thr Tyr Leu Asp Glu Asn Thr Ala Lys Glu Val Thr Lys Gln Leu Asn
   1875                1880                1885                1890
   GAT ACC ACT GGG AAA TTT AAA GAT GTA AGT CAT TTA TAT GAT GTA AAA      3216
   Asp Thr Thr Gly Lys Phe Lys Asp Val Ser His Leu Tyr Asp Val Lys
                   1895                1900                1905
   CTG ACT CCA AAA ATG AAT GTT ACA ATC AAA TTG TCT ATA CTT TAT GAT      3264
   Leu Thr Pro Lys Met Asn Val Thr Ile Lys Leu Ser Ile Leu Tyr Asp
               1910                 1915                 1920
   AAT GCT GAG TCT AAT GAT AAC TCA ATT GGT AAA TGG ACA AAC ACA AAT          3312
   Asn Ala Glu Ser Asn Asp Asn Ser Ile Gly Lys Trp Thr Asn Thr Asn
           1925                1930                1935
   ATT GTT TCA GGT GGA AAT AAC GGA AAA AAA CAA TAT TCT TCT AAT AAT          3360
   Ile Val Ser Gly Gly Asn Asn Gly Lys Lys Gln Tyr Ser Ser Asn Asn
       1940                1945                1950
   CCG GAT GCT AAT TTG ACA TTA AAT ACA GAT GCT CAA GAA AAA TTA AAT          3408
   Pro Asp Ala Asn Leu Thr Leu Asn Thr Asp Ala Gln Glu Lys Leu Asn
   1955                1960                1965                1970
   AAA AAT CGT GAC TAT TAT ATA AGT TTA TAT ATG AAG TCA GAA AAA AAC          3456
   Lys Asn Arg Asp Tyr Tyr Ile Ser Leu Tyr Met Lys Ser Glu Lys Asn
                   1975                1980                1985
   ACA CAA TGT GAG ATT ACT ATA GAT GGG GAG ATT TAT CCG ATC ACT ACA          3504
   Thr Gln Cys Glu Ile Thr Ile Asp Gly Glu Ile Tyr Pro Ile Thr Thr
               1990                1995                2000
   AAA ACA GTG AAT GTG AAT AAA GAC AAT TAC AAA AGA TTA GAT ATT ATA          3552
   Lys Thr Val Asn Val Asn Lys Asp Asn Tyr Lys Arg Leu Asp Ile Ile
           2005                2010                2015
   GCT CAT AAT ATA AAA AGT AAT CCA ATT TCT TCA CTT CAT ATT AAA ACG          3600
   Ala His Asn Ile Lys Ser Asn Pro Ile Ser Ser Leu His Ile Lys Thr
       2020                2025                2030
   AAT GAT GAA ATA ACT TTA TTT TGG GAT GAT ATT TCT ATA ACA GAT GTA          3648
   Asn Asp Glu Ile Thr Leu Phe Trp Asp Asp Ile Ser Ile Thr Asp Val
   2035                2040                2045                2050
   GCA TCA ATA AAA CCG GAA AAT TTA ACA GAT TCA GAA ATT AAA CAG ATT          3696
   Ala Ser Ile Lys Pro Glu Asn Leu Thr Asp Ser Glu Ile Lys Gln Ile
                   2055                2060                2065
   TAT AGT AGG TAT GGT ATT AAG TTA GAA GAT GGA ATC CTT ATT GAT AAA          3744
   Tyr Ser Arg Tyr Gly Ile Lys Leu Glu Asp Gly Ile Leu Ile Asp Lys
               2070                2075                2080
   AAA GGT GGG ATT CAT TAT GGT GAA TTT ATT AAT GAA GCT AGT TTT AAT          3792
   Lys Gly Gly Ile His Tyr Gly Glu Phe Ile Asn Glu Ala Ser Phe Asn
           2085                2090                2095
   ATT GAA CCA TTG CAA AAT TAT GTG ACC AAA TAT GAA GTT ACT TAT AGT          3840
   Ile Glu Pro Leu Gln Asn Tyr Val Thr Lys Tyr Glu Val Thr Tyr Ser
       2100                2105                2110
   AGT GAG TTA GGA CCA AAC GTG AGT GAC ACA CTT GAA AGT GAT AAA ATT          3888
   Ser Glu Leu Gly Pro Asn Val Ser Asp Thr Leu Glu Ser Asp Lys Ile
   2115                2120                2125                2130
   TAC AAG GAT GGG ACA ATT AAA TTT GAT TTT ACC AAA TAT AGT AAA AAT          3936
   Tyr Lys Asp Gly Thr Ile Lys Phe Asp Phe Thr Lys Tyr Ser Lys Asn
   2135 2140 2145
   GAA CAA GGA TTA TTT TAT GAC AGT GGA TTA AAT TGG GAC TTT AAA ATT 3984
   Glu Gln Gly Leu Phe Tyr Asp Ser Gly Leu Asn Trp Asp Phe Lys Ile
   2150 2155 2160
   AAT GCT ATT ACT TAT GAT GGT AAA GAG ATG AAT GTT TTT CAT AGA TAT 4032
   Asn Ala Ile Thr Tyr Asp Gly Lys Glu Met Asn Val Phe His Arg Tyr
   2165 2170 2175
   AAT AAA TAG 4041
   Asn Lys
   2180
(2) INFORMATION FOR SEQ ID NO: 23:
   (i) SEQUENCE CHARACTERISTICS:
      (A) LENGTH: 1346 amino acids
      (B) TYPE: amino acid
      (D) TOPOLOGY: linear
   (ii) MOLECULE TYPE: protein
   (xi) SEQUENCE DESCRIPTION: SEQ ID NO:23:
   Met Lys Arg Met Glu Gly Lys Leu Phe Met Val Ser Lys Lys Leu Gln
   1 5 10 15
   Val Val Thr Lys Thr Val Leu Leu Ser Thr Val Phe Ser Ile Ser Leu
   20 25 30
   Leu Asn Asn Glu Val Ile Lys Ala Glu Gln Leu Asn Ile Asn Ser Gln
   35 40 45
   Ser Lys Tyr Thr Asn Leu Gln Asn Leu Lys Ile Thr Asp Lys Val Glu
   50 55 60
   Asp Phe Lys Glu Asp Lys Glu Lys Ala Lys Glu Trp Gly Lys Glu Lys
   65 70 75 80
   Glu Lys Glu Trp Lys Leu Thr Ala Thr Glu Lys Gly Lys Met Asn Asn
   85 90 95
   Phe Leu Asp Asn Lys Asn Asp Ile Lys Thr Asn Tyr Lys Glu Ile Thr
   100 105 110
   Phe Ser Met Ala Gly Ser Phe Glu Asp Glu Ile Lys Asp Leu Lys Glu
   115 120 125
   Ile Asp Lys Met Phe Asp Lys Thr Asn Leu Ser Asn Ser Ile Ile Thr
   130 135 140
   Tyr Lys Asn Val Glu Pro Thr Thr Ile Gly Phe Asn Lys Ser Leu Thr
   145 150 155 160
   Glu Gly Asn Thr Ile Asn Ser Asp Ala Met Ala Gln Phe Lys Glu Gln
   165 170 175
   Phe Leu Asp Arg Asp Ile Lys Phe Asp Ser Tyr Leu Asp Thr His Leu
   180 185 190
   Thr Ala Gln Gln Val Ser Ser Lys Glu Arg Val Ile Leu Lys Val Thr
   195 200 205
   Val Pro Ser Gly Lys Gly Ser Thr Thr Pro Thr Lys Ala Gly Val Ile
   210 215 220
   Leu Asn Asn Ser Glu Tyr Lys Met Leu Ile Asp Asn Gly Tyr Met Val
   225 230 235 240
   His Val Asp Lys Val Ser Lys Val Val Lys Lys Gly Val Glu Cys Leu
   245 250 255
   Gln Ile Glu Gly Thr Leu Lys Lys Ser Leu Asp Phe Lys Asn Asp Ile
   260 265 270
   Asn Ala Glu Ala His Ser Trp Gly Met Lys Asn Tyr Glu Glu Trp Ala
   275 280 285
   Lys Asp Leu Thr Asp Ser Gln Arg Glu Ala Leu Asp Gly Tyr Ala Arg
   290 295 300
   Gln Asp Tyr Lys Glu Ile Asn Asn Tyr Leu Arg Asn Gln Gly Gly Ser
   305 310 315 320
   Gly Asn Glu Lys Leu Asp Ala Gln Ile Lys Asn Ile Ser Asp Ala Leu
   325 330 335
   Gly Lys Lys Pro Ile Pro Glu Asn Ile Thr Val Tyr Arg Trp Cys Gly
   340 345 350
   Met Pro Glu Phe Gly Tyr Gln Ile Ser Asp Pro Leu Pro Ser Leu Lys
   355 360 365
   Asp Phe Glu Glu Gln Phe Leu Asn Thr Ile Lys Glu Asp Lys Gly Tyr
   370 375 380
   Met Ser Thr Ser Leu Ser Ser Glu Arg Leu Ala Ala Phe Gly Ser Arg
   385 390 395 400
   Lys Ile Ile Leu Arg Leu Gln Val Pro Lys Gly Ser Thr Gly Ala Tyr
   405 410 415
   Leu Ser Ala Ile Gly Gly Phe Ala Ser Glu Lys Glu Ile Leu Leu Asp
   420 425 430
   Lys Asp Ser Lys Tyr His Ile Asp Lys Val Thr Glu Val Ile Ile Lys
   435 440 445
   Gly Val Lys Arg Tyr Val Val Asp Ala Thr Leu Leu Thr Asn Met Lys
   450 455 460
   Asn Met Lys Lys Lys Leu Ala Ser Val Val Thr Cys Thr Leu Leu Ala
   465 470 475 480
   Pro Met Phe Leu Asn Gly Asn Val Asn Ala Val Tyr Ala Asp Ser Lys
   485 490 495
   Thr Asn Gln Ile Ser Thr Thr Gln Lys Asn Gln Gln Lys Glu Met Asp
   500 505 510
   Arg Lys Gly Leu Leu Gly Tyr Tyr Phe Lys Gly Lys Asp Phe Ser Asn
   515 520 525
   Leu Thr Met Phe Ala Pro Thr Arg Asp Ser Thr Leu Ile Tyr Asp Gln
   530 535 540
   Gln Thr Ala Asn Lys Leu Leu Asp Lys Lys Gln Gln Glu Tyr Gln Ser
   545 550 555 560
   Ile Arg Trp Ile Gly Leu Ile Gln Ser Lys Glu Thr Gly Asp Phe Thr
   565 570 575
   Phe Asn Leu Ser Glu Asp Glu Gln Ala Ile Ile Glu Ile Asn Gly Lys
   580 585 590
   Ile Ile Ser Asn Lys Gly Lys Glu Lys Gln Val Val His Leu Glu Lys
   595 600 605
   Gly Lys Leu Val Pro Ile Lys Ile Glu Tyr Gln Ser Asp Thr Lys Phe
   610 615 620
   Asn Ile Asp Ser Lys Thr Phe Lys Glu Leu Lys Leu Phe Lys Ile Asp
   625 630 635 640
   Ser Gln Asn Gln Pro Gln Gln Val Gln Gln Asp Glu Leu Arg Asn Pro
   645 650 655
   Glu Phe Asn Lys Lys Glu Ser Gln Glu Phe Leu Ala Lys Pro Ser Lys
   660 665 670
   Ile Asn Leu Phe Thr Gln Lys Met Lys Arg Glu Ile Asp Glu Asp Thr
   675 680 685
   Asp Thr Asp Gly Asp Ser Ile Pro Asp Leu Trp Glu Glu Asn Gly Tyr
   690 695 700
   Thr Ile Gln Asn Arg Ile Ala Val Lys Trp Asp Asp Ser Leu Ala Ser
   705 710 715 720
   Lys Gly Tyr Thr Lys Phe Val Ser Asn Pro Leu Glu Ser His Thr Val
   725 730 735
   Gly Asp Pro Tyr Thr Asp Tyr Glu Lys Ala Ala Arg Asp Leu Asp Leu
   740 745 750
   Ser Asn Ala Lys Glu Thr Phe Asn Pro Leu Val Ala Ala Phe Pro Ser
   755 760 765
   Val Asn Val Ser Met Glu Lys Val Ile Leu Ser Pro Asn Glu Asn Leu
   770 775 780
   Ser Asn Ser Val Glu Ser His Ser Ser Thr Asn Trp Ser Tyr Thr Asn
   785 790 795 800
   Thr Glu Gly Ala Ser Val Glu Ala Gly Ile Gly Pro Lys Gly Ile Ser
   805 810 815
   Phe Gly Val Ser Val Asn Tyr Gln His Ser Glu Thr Val Ala Gln Glu
   820 825 830
   Trp Gly Thr Ser Thr Gly Asn Thr Ser Gln Phe Asn Thr Ala Ser Ala
   835 840 845
   Gly Tyr Leu Asn Ala Asn Val Arg Tyr Asn Asn Val Gly Thr Gly Ala
   850 855 860
   Ile Tyr Asp Val Lys Pro Thr Thr Ser Phe Val Leu Asn Asn Asp Thr
   865 870 875 880
   Ile Ala Thr Ile Thr Ala Lys Ser Asn Ser Thr Ala Leu Asn Ile Ser
   885 890 895
   Pro Gly Glu Ser Tyr Pro Lys Lys Gly Gln Asn Gly Ile Ala Ile Thr
   900 905 910
   Ser Met Asp Asp Phe Asn Ser His Pro Ile Thr Leu Asn Lys Lys Gln
   915 920 925
   Val Asp Asn Leu Leu Asn Asn Lys Pro Met Met Leu Glu Thr Asn Gln
   930 935 940
   Thr Asp Gly Val Tyr Lys Ile Lys Asp Thr His Gly Asn Ile Val Thr
   945 950 955 960
   Gly Gly Glu Trp Asn Gly Val Ile Gln Gln Ile Lys Ala Lys Thr Ala
   965 970 975
   Ser Ile Ile Val Asp Asp Gly Glu Arg Val Ala Glu Lys Arg Val Ala
   980 985 990
   Ala Lys Asp Tyr Glu Asn Pro Glu Asp Lys Thr Pro Ser Leu Thr Leu
   995 1000 1005
   Lys Asp Ala Leu Lys Leu Ser Tyr Pro Asp Glu Ile Lys Glu Ile Glu
   1010 1015 1020
   Gly Leu Leu Tyr Tyr Lys Asn Lys Pro Ile Tyr Glu Ser Ser Val Met
   1025 1030 1035 1040
   Thr Tyr Leu Asp Glu Asn Thr Ala Lys Glu Val Thr Lys Gln Leu Asn
   1045 1050 1055
   Asp Thr Thr Gly Lys Phe Lys Asp Val Ser His Leu Tyr Asp Val Lys
   1060 1065 1070
   Leu Thr Pro Lys Met Asn Val Thr Ile Lys Leu Ser Ile Leu Tyr Asp
   1075 1080 1085
   Asn Ala Glu Ser Asn Asp Asn Ser Ile Gly Lys Trp Thr Asn Thr Asn
   1090 1095 1100
   Ile Val Ser Gly Gly Asn Asn Gly Lys Lys Gln Tyr Ser Ser Asn Asn
   1105 1110 1115 1120
   Pro Asp Ala Asn Leu Thr Leu Asn Thr Asp Ala Gln Glu Lys Leu Asn
   1125 1130 1135
   Lys Asn Arg Asp Tyr Tyr Ile Ser Leu Tyr Met Lys Ser Glu Lys Asn
   1140 1145 1150
   Thr Gln Cys Glu Ile Thr Ile Asp Gly Glu Ile Tyr Pro Ile Thr Thr
   1155 1160 1165
   Lys Thr Val Asn Val Asn Lys Asp Asn Tyr Lys Arg Leu Asp Ile Ile
   1170 1175 1180
   Ala His Asn Ile Lys Ser Asn Pro Ile Ser Ser Leu His Ile Lys Thr
   1185 1190 1195 1200
   Asn Asp Glu Ile Thr Leu Phe Trp Asp Asp Ile Ser Ile Thr Asp Val
   1205 1210 1215
   Ala Ser Ile Lys Pro Glu Asn Leu Thr Asp Ser Glu Ile Lys Gln Ile
   1220 1225 1230
   Tyr Ser Arg Tyr Gly Ile Lys Leu Glu Asp Gly Ile Leu Ile Asp Lys
   1235 1240 1245
   Lys Gly Gly Ile His Tyr Gly Glu Phe Ile Asn Glu Ala Ser Phe Asn
   1250 1255 1260
   Ile Glu Pro Leu Gln Asn Tyr Val Thr Lys Tyr Glu Val Thr Tyr Ser
   1265 1270 1275 1280
   Ser Glu Leu Gly Pro Asn Val Ser Asp Thr Leu Glu Ser Asp Lys Ile
   1285 1290 1295
   Tyr Lys Asp Gly Thr Ile Lys Phe Asp Phe Thr Lys Tyr Ser Lys Asn
   1300 1305 1310
   Glu Gln Gly Leu Phe Tyr Asp Ser Gly Leu Asn Trp Asp Phe Lys Ile
   1315 1320 1325
   Asn Ala Ile Thr Tyr Asp Gly Lys Glu Met Asn Val Phe His Arg Tyr
       1330                    1335                    1340
   Asn Lys
   1345
(2) INFORMATION FOR SEQ ID NO:24:
   (i) SEQUENCE CHARACTERISTICS:
      (A) LENGTH: 1399 base pairs
      (B) TYPE: nucleic acid
      (C) STRANDEENESS: single
      (D) TOPOLOGY: linear
   (ii) MOLECULE TYPE: DNA (genomic)
   (ix) FEATURE:
      (A) NAME/KEY: misc_feature
      (B) LOCATION: 1..1386
      (D) OTHER INFORMATION: /note= "Maize optimized DNA sequence for VIP2A(a) protein from AB78"
   (xi) SEQUENCE DESCRIPTION: SEQ ID NO:24:
   ATGAAGCGCA TGGAGGGCAA GCTGTTCATG GTGAGCAAGA AGCTCCAGGT GGTGACCAAG    60
   ACCGTGCTGC TGAGCACCGT GTTCAGCATC AGCCTGCTGA ACAACGAGGT GATCAAGGCC   120
   GAGCAGCTGA ACATCAACAG CCAGAGCAAG TACACCAACC TCCAGAACCT GAAGATCACC   180
   GACAAGGTGG AGGACTTCAA GGAGGACAAG GAGAAGGCCA AGGAGTGGGG CAAGGAGAAG   240
   GAGAAGGAGT GGAAGCTTAC CGCCACCGAG AAGGGCAAGA TGAACAACTT CCTGGACAAC   300
   AAGAACGACA TCAAGACCAA CTACAAGGAG ATCACCTTCA GCATGGCCGG CAGCTTCGAG   360
   GACGAGATCA AGGACCTGAA GGAGATCGAC AAGATGTTCG ACAAGACCAA CCTGAGCAAC   420
   AGCATCATCA CCTACAAGAA CGTGGAGCCC ACCACCATCG GCTTCAACAA GAGCCTGACC   480
   GAGGGCAACA CCATCAACAG CGACGCCATG GCCCAGTTCA AGGAGCAGTT CCTGGACCGC   540
   GACATCAAGT TCGACAGCTA CCTGGACACC CACCTGACCG CCCAGCAGGT GAGCAGCAAG   600
   GAGCGCGTGA TCCTGAAGGT GACCGTCCCC AGCGGCAAGG GCAGCACCAC CCCCACCAAG   660
   GCCGGCGTGA TCCTGAACAA CAGCGAGTAC AAGATGCTGA TCGACAACGG CTACATGGTG   720
   CACGTGGACA AGGTGAGCAA GGTGGTGAAG AAGGGCGTGG AGTGCCTCCA GATCGAGGGC   780
   ACCCTGAAGA AGAGTCTAGA CTTCAAGAAC GACATCAACG CCGAGGCCCA CAGCTGGGGC   840
   ATGAAGAACT ACGAGGAGTG GGCCAAGGAC CTGACCGACA GCCAGCGCGA GGCCCTGGAC   900
   GGCTACGCCC GCCAGGACTA CAAGGAGATC AACAACTACC TGCGCAACCA GGGCGGCAGC   960
   GGCAACGAGA AGCTGGACGC CCAGATCAAG AACATCAGCG ACGCCCTGGG CAAGAAGCCC  1020
   ATCCCCGAGA ACATCACCGT GTACCGCTGG TGCGGCATGC CCGAGTTCGG CTACCAGATC  1080
   AGCGACCCCC TGCCCAGCCT GAAGGACTTC GAGGAGCAGT TCCTGAACAC CATCAAGGAG  1140
   GACAAGGGCT ACATGAGCAC CAGCCTGAGC AGCGAGCGCC TGGCCGCCTT CGGCAGCCGC  1200
   AAGATCATCC TGCGCCTGCA GGTGCCCAAG GGCAGCACCG GCGCCTACCT GAGCGCCATC  1260
   GGCGGCTTCG CCAGCGAGAA GGAGATCCTG CTGGACAAGG ACAGCAAGTA CCACATCGAC  1320
   AAGGTGACCG AGGTGATCAT CAAGGGCGTG AAGCGCTACG TGGTGGACGC CACCCTGCTG  1380
   ACCAACTAGA TCTGAGCTC                                               1399
(2) INFORMATION FOR SEQ ID NO:25:
   (i) SEQUENCE CHARACTERISTICS:
      (A) LENGTH: 19 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Peptide
      (B) LOCATION: 1..19
      (D) OTHER INFORMATION: /note= "Secretion signal peptide to secrete VIP2 out of a cell"
   (xi) SEQUENCE DESCRIPTION: SEQ ID NO:25:
   Gly Trp Ser Trp Ile Phe Leu Phe Leu Leu Ser Gly Ala Ala Gly Val
   1               5                   10                  15
   His Cys Leu
(2) INFORMATION FOR SEQ ID NO:26:
   (i) SEQUENCE CHARACTERISTICS:
      (A) LENGTH: 2655 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Synthetic DNA"
   (iii) HYPOTHETICAL: NO
   (ix) FEATURE:
      (A) NAME/KEY: misc_feature
      (B) LOCATION: 1..2655
      (D) OTHER INFORMATION: /note= "maize optimized DNA sequence encoding VIP1A(a)"
   (xi) SEQUENCE DESCRIPTION: SEQ ID NO:26:
   ATGAAGAACA TGAAGAAGAA GCTGGCCAGC GTGGTGACCT GCACCCTGCT GGCCCCCATG     60
   TTCCTGAACG GCAACGTGAA CGCCGTGTAC GCCGACAGCA AGACCAACCA GATCAGCACC    120
   ACCCAGAAGA ACCAGCAGAA GGAGATGGAC CGCAAGGGCC TGCTGGGCTA CTACTTCAAG    180
   GGCAAGGACT TCAGCAACCT GACCATGTTC GCCCCCACGC GTGACAGCAC CCTGATCTAC    240
   GACCAGCAGA CCGCCAACAA GCTGCTGGAC AAGAAGCAGC AGGAGTACCA GAGCATCCGC    300
   TGGATCGGCC TGATCCAGAG CAAGGAGACC GGCGACTTCA CCTTCAACCT GAGCGAGGAC    360
   GAGCAGGCCA TCATCGAGAT CAACGGCAAG ATCATCAGCA ACAAGGGCAA GGAGAAGCAG    420
   GTGGTGCACC TGGAGAAGGG CAAGCTGGTG CCCATCAAGA TCGAGTACCA GAGCGACACC    480
   AAGTTCAACA TCGACAGCAA GACCTTCAAG GAGCTGAAGC TTTTCAAGAT CGACAGCCAG    540
   AACCAGCCCC AGCAGGTGCA GCAGGACGAG CTGCGCAACC CCGAGTTCAA CAAGAAGGAG    600
   AGCCAGGAGT TCCTGGCCAA GCCCAGCAAG ATCAACCTGT TCACCCAGCA GATGAAGCGC    660
   GAGATCGACG AGGACACCGA CACCGACGGC GACAGCATCC CCGACCTGTG GGAGGAGAAC    720
   GGCTACACCA TCCAGAACCG CATCGCCGTG AAGTGGGACG ACAGCCTGGC TAGCAAGGGC    780
   TACACCAAGT TCGTGAGCAA CCCCCTGGAG AGCCACACCG TGGGCGACCC CTACACCGAC    840
   TACGAGAAGG CCGCCCGCGA CCTGGACCTG AGCAACGCCA AGGAGACCTT CAACCCCCTG    900
   GTGGCCGCCT TCCCCAGCGT GAACGTGAGC ATGGAGAAGG TGATCCTGAG CCCCAACGAG    960
   AACCTGAGCA ACAGCGTGGA GAGCCACTCG AGCACCAACT GGAGCTACAC CAACACCGAG   1020
   GGCGCCAGCG TGGAGGCCGG CATCGGTCCC AAGGGCATCA GCTTCGGCGT GAGCGTGAAC   1080
   TACCAGCACA GCGAGACCGT GGCCCAGGAG TGGGGCACCA GCACCGGCAA CACCAGCCAG   1140
   TTCAACACCG CCAGCGCCGG CTACCTGAAC GCCAACGTGC GCTACAACAA CGTGGGCACC   1200
   GGCGCCATCT ACGACGTGAA GCCCACCACC AGCTTCGTGC TGAACAACGA CACCATCGCC   1260
   ACCATCACCG CCAAGTCGAA TTCCACCGCC CTGAACATCA GCCCCGGCGA GAGCTACCCC 1320
   AAGAAGGGCC AGAACGGCAT CGCCATCACC AGCATGGACG ACTTCAACAG CCACCCCATC 1380
   ACCCTGAACA AGAAGCAGGT GGACAACCTG CTGAACAACA AGCCCATGAT GCTGGAGACC 1440
   AACCAGACCG ACGGCGTCTA CAAGATCAAG GACACCCACG GCAACATCGT GACGGGCGGC 1500
   GAGTGGAACG GCGTGATCCA GCAGATCAAG GCCAAGACCG CCAGCATCAT CGTCGACGAC 1560
   GGCGAGCGCG TGGCCGAGAA GCGCGTGGCC GCCAAGGACT ACGAGAACCC CGAGGACAAG 1620
   ACCCCCAGCC TGACCCTGAA GGACGCCCTG AAGCTGAGCT ACCCCGACGA GATCAAGGAG 1680
   ATCGAGGGCT TGCTGTACTA CAAGAACAAG CCCATCTACG AGAGCAGCGT GATGACCTAT 1740
   CTAGACGAGA ACACCGCCAA GGAGGTGACC AAGCAGCTGA ACGACACCAC CGGCAAGTTC 1800
   AAGGACGTGA GCCACCTGTA CGACGTGAAG CTGACCCCCA AGATGAACGT GACCATCAAG 1860
   CTGAGCATCC TGTACGACAA CGCCGAGAGC AACGACAACA GCATCGGCAA GTGGACCAAC 1920
   ACCAACATCG TGAGCGGCGG CAACAACGGC AAGAAGCAGT ACAGCAGCAA CAACCCCGAC 1980
   GCCAACCTGA CCCTGAACAC CGACGCCCAG GAGAAGCTGA ACAAGAACCG CGACTACTAC 2040
   ATCAGCCTGT ACATGAAGAG CGAGAAGAAC ACCCAGTGCG AGATCACCAT CGACGGCGAG 2100
   ATATACCCCA TCACCACCAA GACCGTGAAC GTGAACAAGG ACAACTACAA GCGCCTGGAC 2160
   ATCATCGCCC ACAACATCAA GAGCAACCCC ATCAGCAGCC TGCACATCAA GACCAACGAC 2220
   GAGATCACCC TGTTCTGGGA CGACATATCG ATTACCGACG TCGCCAGCAT CAAGCCCGAG 2280
   AACCTGACCG ACAGCGAGAT CAAGCAGATA TACAGTCGCT ACGGCATCAA GCTGGAGGAC 2340
   GGCATCCTGA TCGACAAGAA AGGCGGCATC CACTACGGCG AGTTCATCAA CGAGGCCAGC 2400
   TTCAACATCG AGCCCCTGCA GAACTACGTG ACCAAGTACG AGGTGACCTA CAGCAGCGAG 2460
   CTGGGCCCCA ACGTGAGCGA CACCCTGGAG AGCGACAAGA TTTACAAGGA CGGCACCATC 2520
   AAGTTCGACT TCACCAAGTA CAGCAAGAAC GAGCAGGGCC TGTTCTACGA CAGCGGCCTG 2580
   AACTGGGACT TCAAGATCAA CGCCATCACC TACGACGGCA AGGAGATGAA CGTGTTCCAC 2640
   CGCTACAACA AGTAG 2655
(2) INFORMATION FOR SEQ ID NO:27:
   (i) SEQUENCE CHARACTERISTICS:
      (A) LENGTH: 1389 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Synthetic DNA"
   (iii) HYPOTHETICAL: NO
   (ix) FEATURE:
      (A) NAME/KEY: misc_feature
      (B) LOCATION: 1..1389
      (D) OTHER INFORMATION: /note= "maize optimized DNA sequence encoding VIP2A(a)"
   (xi) SEQUENCE DESCRIPTION: SEQ ID NO:27:
   ATGAAGCGCA TGGAGGGCAA GCTGTTCATG GTGAGCAAGA AGCTCCAGGT GGTGACCAAG 60
   ACCGTGCTGC TGAGCACCGT GTTCAGCATC AGCCTGCTGA ACAACGAGGT GATCAAGGCC 120
   GAGCAGCTGA ACATCAACAG CCAGAGCAAG TACACCAACC TCCAGAACCT GAAGATCACC 180
   GACAAGGTGG AGGACTTCAA GGAGGACAAG GAGAAGGCCA AGGAGTGGGG CAAGGAGAAG 240
   GAGAAGGAGT GGAAGCTTAC CGCCACCGAG AAGGGCAAGA TGAACAACTT CCTGGACAAC 300
   AAGAACGACA TCAAGACCAA CTACAAGGAG ATCACCTTCA GCATAGCCGG CAGCTTCGAG 360
   GACGAGATCA AGGACCTGAA GGAGATCGAC AAGATGTTCG ACAAGACCAA CCTGAGCAAC 420
   AGCATCATCA CCTACAAGAA CGTGGAGCCC ACCACCATCG GCTTCAACAA GAGCCTGACC 480
   GAGGGCAACA CCATCAACAG CGACGCCATG GCCCAGTTCA AGGAGCAGTT CCTGGACCGC 540
   GACATCAAGT TCGACAGCTA CCTGGACACC CACCTGACCG CCCAGCAGGT GAGCAGCAAG 600
   GAGCGCGTGA TCCTGAAGGT GACCGTCCCC AGCGGCAAGG GCAGCACCAC CCCCACCAAG 660
   GCCGGCGTGA TCCTGAACAA CAGCGAGTAC AAGATGCTGA TCGACAACGG CTACATGGTG 720
   CACGTGGACA AGGTGAGCAA GGTGGTGAAG AAGGGCGTGG AGTGCCTCCA GATCGAGGGC 780
   ACCCTGAAGA AGAGTCTAGA CTTCAAGAAC GACATCAACG CCGAGGCCCA CAGCTGGGGC 840
   ATGAAGAACT ACGAGGAGTG GGCCAAGGAC CTGACCGACA GCCAGCGCGA GGCCCTGGAC 900
   GGCTACGCCC GCCAGGACTA CAAGGAGATC AACAACTACC TGCGCAACCA GGGCGGCAGC 960
   GGCAACGAGA AGCTGGACGC CCAGATCAAG AACATCAGCG ACGCCCTGGG CAAGAAGCCC 1020
   ATCCCCGAGA ACATCACCGT GTACCGCTGG TGCGGCATGC CCGAGTTCGG CTACCAGATC 1080
   AGCGACCCCC TGCCCAGCCT GAAGGACTTC GAGGAGCAGT TCCTGAACAC CATCAAGGAG 1140
   GACAAGGGCT ACATGAGCAC CAGCCTGAGC AGCGAGCGCC TGGCCGCCTT CGGCAGCCGC 1200
   AAGATCATCC TGCGCCTGCA GGTGCCCAAG GGCAGCACTG GTGCCTACCT GAGCGCCATC 1260
   GGCGGCTTCG CCAGCGAGAA GGAGATCCTG CTGGATAAGG ACAGCAAGTA CCACATCGAC 1320
   AAGGTGACCG AGGTGATCAT CAAGGGCGTG AAGCGCTACG TGGTGGACGC CACCCTGCTG 1380
   ACCAACTAG 1389
(2) INFORMATION FOR SEQ ID NO:28:
   (i) SEQUENCE CHARACTERISTICS:
      (A) LENGTH: 2378 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (ix) FEATURE:
      (A) NAME/KEY: CDS
      (B) LOCATION: 9..2375
      (D) OTHER INFORMATION: /note= "Native DNA sequence encoding VIP3A(a) protein from AB88 as contained in pCIB7104"
   (xi) SEQUENCE DESCRIPTION: SEQ ID NO:28:
   AGATGAAC ATG AAC AAG AAT AAT ACT AAA TTA AGC ACA AGA GCC TTA CCA      50
            Met Asn Lys Asn Asn Thr Lys Leu Ser Thr Arg Ala Leu Pro
             1               5                  10
   AGT TTT ATT GAT TAT TTT AAT GGC ATT TAT GGA TTT GCC ACT GGT ATC       98
   Ser Phe Ile Asp Tyr Phe Asn Gly Ile Tyr Gly Phe Ala Thr Gly Ile
    15                  20                  25                  30
   AAA GAC ATT ATG AAC ATG ATT TTT AAA ACG GAT ACA GGT GGT GAT CTA      146
   Lys Asp Ile Met Asn Met Ile Phe Lys Thr Asp Thr Gly Gly Asp Leu
                    35                  40                  45
   ACC CTA GAC GAA ATT TTA AAG AAT CAG CAG TTA CTA AAT GAT ATT TCT      194
   Thr Leu Asp Glu Ile Leu Lys Asn Gln Gln Leu Leu Asn Asp Ile Ser
                50                  55                  60
   GGT AAA TTG GAT GGG GTG AAT GGA AGC TTA AAT GAT CTT ATC GCA CAG      242
   Gly Lys Leu Asp Gly Val Asn Gly Ser Leu Asn Asp Leu Ile Ala Gln
            65                  70                  75
   GGA AAC TTA AAT ACA GAA TTA TCT AAG GAA ATA TTA AAA ATT GCA AAT      290
   Gly Asn Leu Asn Thr Glu Leu Ser Lys Glu Ile Leu Lys Ile Ala Asn
        80                  85                  90
   GAA CAA AAT CAA GTT TTA AAT GAT GTT AAT AAC AAA CTC GAT GCG ATA 338
   Glu Gln Asn Gln Val Leu Asn Asp Val Asn Asn Lys Leu Asp Ala Ile
   95 100 105 110
   AAT ACG ATG CTT CGG GTA TAT CTA CCT AAA ATT ACC TCT ATG TTG AGT 386
   Asn Thr Met Leu Arg Val Tyr Leu Pro Lys Ile Thr Ser Met Leu Ser
   115 120 125
   GAT GTA ATG AAA CAA AAT TAT GCG CTA AGT CTG CAA ATA GAA TAC TTA 434
   Asp Val Met Lys Gln Asn Tyr Ala Leu Ser Leu Gln Ile Glu Tyr Leu
   130 135 140
   AGT AAA CAA TTG CAA GAG ATT TCT GAT AAG TTG GAT ATT ATT AAT GTA 482
   Ser Lys Gln Leu Gln Glu Ile Ser Asp Lys Leu Asp Ile Ile Asn Val
   145 150 155
   AAT GTA CTT ATT AAC TCT ACA CTT ACT GAA ATT ACA CCT GCG TAT CAA 530
   Asn Val Leu Ile Asn Ser Thr Leu Thr Glu Ile Thr Pro Ala Tyr Gln
   160 165 170
   AGG ATT AAA TAT GTG AAC GAA AAA TTT GAG GAA TTA ACT TTT GCT ACA 578
   Arg Ile Lys Tyr Val Asn Glu Lys Phe Glu Glu Leu Thr Phe Ala Thr
   175 180 185 190
   GAA ACT AGT TCA AAA GTA AAA AAG GAT GGC TCT CCT GCA GAT ATT CTT 626
   Glu Thr Ser Ser Lys Val Lys Lys Asp Gly Ser Pro Ala Asp Ile Leu
   195 200 205
   GAT GAG TTA ACT GAG TTA ACT GAA CTA GCG AAA AGT GTA ACA AAA AAT 674
   Asp Glu Leu Thr Glu Leu Thr Glu Leu Ala Lys Ser Val Thr Lys Asn
   210 215 220
   GAT GTG GAT GGT TTT GAA TTT TAC CTT AAT ACA TTC CAC GAT GTA ATG 722
   Asp Val Asp Gly Phe Glu Phe Tyr Leu Asn Thr Phe His Asp Val Met
   225 230 235
   GTA GGA AAT AAT TTA TTC GGG CGT TCA GCT TTA AAA ACT GCA TCG GAA 770
   Val Gly Asn Asn Leu Phe Gly Arg Ser Ala Leu Lys Thr Ala Ser Glu
   240 245 250
   TTA ATT ACT AAA GAA AAT GTG AAA ACA AGT GGC AGT GAG GTC GGA AAT 818
   Leu Ile Thr Lys Glu Asn Val Lys Thr Ser Gly Ser Glu Val Gly Asn
   255 260 265 270
   GTT TAT AAC TTC TTA ATT GTA TTA ACA GCT CTG CAA GCC CAA GCT TTT 866
   Val Tyr Asn Phe Leu Ile Val Leu Thr Ala Leu Gln Ala Gln Ala Phe
   275 280 285
   CTT ACT TTA ACA ACA TGC CGA AAA TTA TTA GGC TTA GCA GAT ATT GAT 914
   Leu Thr Leu Thr Thr Cys Arg Lys Leu Leu Gly Leu Ala Asp Ile Asp
   290 295 300
   TAT ACT TCT ATT ATG AAT GAA CAT TTA AAT AAG GAA AAA GAG GAA TTT 962
   Tyr Thr Ser Ile Met Asn Glu His Leu Asn Lys Glu Lys Glu Glu Phe
   305 310 315
   AGA GTA AAC ATC CTC CCT ACA CTT TCT AAT ACT TTT TCT AAT CCT AAT 1010
   Arg Val Asn Ile Leu Pro Thr Leu Ser Asn Thr Phe Ser Asn Pro Asn
   320 325 330
   TAT GCA AAA GTT AAA GGA AGT GAT GAA GAT GCA AAG ATG ATT GTG GAA 1058
   Tyr Ala Lys Val Lys Gly Ser Asp Glu Asp Ala Lys Met Ile Val Glu
   335 340 345 350
   GCT AAA CCA GGA CAT GCA TTG ATT GGG TTT GAA ATT AGT AAT GAT TCA 1106
   Ala Lys Pro Gly His Ala Leu Ile Gly Phe Glu Ile Ser Asn Asp Ser
   355 360 365
   ATT ACA GTA TTA AAA GTA TAT GAG GCT AAG CTA AAA CAA AAT TAT CAA 1154
   Ile Thr Val Leu Lys Val Tyr Glu Ala Lys Leu Lys Gln Asn Tyr Gln
   370 375 380
   GTC GAT AAG GAT TCC TTA TCG GAA GTT ATT TAT GGT GAT ATG GAT AAA 1202
   Val Asp Lys Asp Ser Leu Ser Glu Val Ile Tyr Gly Asp Met Asp Lys
   385 390 395
   TTA TTG TGC CCA GAT CAA TCT GAA CAA ATC TAT TAT ACA AAT AAC ATA 1250
   Leu Leu Cys Pro Asp Gln Ser Glu Gln Ile Tyr Tyr Thr Asn Asn Ile
   400 405 410
   GTA TTT CCA AAT GAA TAT GTA ATT ACT AAA ATT GAT TTC ACT AAA AAA 1298
   Val Phe Pro Asn Glu Tyr Val Ile Thr Lys Ile Asp Phe Thr Lys Lys
   415 420 425 430
   ATG AAA ACT TTA AGA TAT GAG GTA ACA GCG AAT TTT TAT GAT TCT TCT 1346
   Met Lys Thr Leu Arg Tyr Glu Val Thr Ala Asn Phe Tyr Asp Ser Ser
   435 440 445
   ACA GGA GAA ATT GAC TTA AAT AAG AAA AAA GTA GAA TCA AGT GAA GCG 1394
   Thr Gly Glu Ile Asp Leu Asn Lys Lys Lys Val Glu Ser Ser Glu Ala
   450 455 460
   GAG TAT AGA ACG TTA AGT GCT AAT GAT GAT GGG GTG TAT ATG CCG TTA 1442
   Glu Tyr Arg Thr Leu Ser Ala Asn Asp Asp Gly Val Tyr Met Pro Leu
   465 470 475
   GGT GTC ATC AGT GAA ACA TTT TTG ACT CCG ATT AAT GGG TTT GGC CTC 1490
   Gly Val Ile Ser Glu Thr Phe Leu Thr Pro Ile Asn Gly Phe Gly Leu
   480 485 490
   CAA GCT GAT GAA AAT TCA AGA TTA ATT ACT TTA ACA TGT AAA TCA TAT 1538
   Gln Ala Asp Glu Asn Ser Arg Leu Ile Thr Leu Thr Cys Lys Ser Tyr
   495 500 505 510
   TTA AGA GAA CTA CTG CTA GCA ACA GAC TTA AGC AAT AAA GAA ACT AAA 1586
   Leu Arg Glu Leu Leu Leu Ala Thr Asp Leu Ser Asn Lys Glu Thr Lys
   515 520 525
   TTG ATC GTC CCG CCA AGT GGT TTT ATT AGC AAT ATT GTA GAG AAC GGG 1634
   Leu Ile Val Pro Pro Ser Gly Phe Ile Ser Asn Ile Val Glu Asn Gly
   530 535 540
   TCC ATA GAA GAG GAC AAT TTA GAG CCG TGG AAA GCA AAT AAT AAG AAT 1682
   Ser Ile Glu Glu Asp Asn Leu Glu Pro Trp Lys Ala Asn Asn Lys Asn
   545 550 555
   GCG TAT GTA GAT CAT ACA GGC GGA GTG AAT GGA ACT AAA GCT TTA TAT 1730
   Ala Tyr Val Asp His Thr Gly Gly Val Asn Gly Thr Lys Ala Leu Tyr
   560 565 570
   GTT CAT AAG GAC GGA GGA ATT TCA CAA TTT ATT GGA GAT AAG TTA AAA 1778
   Val His Lys Asp Gly Gly Ile Ser Gln Phe Ile Gly Asp Lys Leu Lys
   575 580 585 590
   CCG AAA ACT GAG TAT GTA ATC CAA TAT ACT GTT AAA GGA AAA CCT TCT 1826
   Pro Lys Thr Glu Tyr Val Ile Gln Tyr Thr Val Lys Gly Lys Pro Ser
   595 600 605
   ATT CAT TTA AAA GAT GAA AAT ACT GGA TAT ATT CAT TAT GAA GAT ACA 1874
   Ile His Leu Lys Asp Glu Asn Thr Gly Tyr Ile His Tyr Glu Asp Thr
   610 615 620
   AAT AAT AAT TTA GAA GAT TAT CAA ACT ATT AAT AAA CGT TTT ACT ACA 1922
   Asn Asn Asn Leu Glu Asp Tyr Gln Thr Ile Asn Lys Arg Phe Thr Thr
   625 630 635
   GGA ACT GAT TTA AAG GGA GTG TAT TTA ATT TTA AAA AGT CAA AAT GGA 1970
   Gly Thr Asp Leu Lys Gly Val Tyr Leu Ile Leu Lys Ser Gln Asn Gly
   640 645 650
   GAT GAA GCT TGG GGA GAT AAC TTT ATT ATT TTG GAA ATT AGT CCT TCT 2018
   Asp Glu Ala Trp Gly Asp Asn Phe Ile Ile Leu Glu Ile Ser Pro Ser
   655 660 665 670
   GAA AAG TTA TTA AGT CCA GAA TTA ATT AAT ACA AAT AAT TGG ACG AGT 2066
   Glu Lys Leu Leu Ser Pro Glu Leu Ile Asn Thr Asn Asn Trp Thr Ser
   675 680 685
   ACG GGA TCA ACT AAT ATT AGC GGT AAT ACA CTC ACT CTT TAT CAG GGA 2114
   Thr Gly Ser Thr Asn Ile Ser Gly Asn Thr Leu Thr Leu Tyr Gln Gly
   690 695 700
   GGA CGA GGG ATT CTA AAA CAA AAC CTT CAA TTA GAT AGT TTT TCA ACT 2162
   Gly Arg Gly Ile Leu Lys Gln Asn Leu Gln Leu Asp Ser Phe Ser Thr
   705 710 715
   TAT AGA GTG TAT TTT TCT GTG TCC GGA GAT GCT AAT GTA AGG ATT AGA 2210
   Tyr Arg Val Tyr Phe Ser Val Ser Gly Asp Ala Asn Val Arg Ile Arg
   720 725 730
   AAT TCT AGG GAA GTG TTA TTT GAA AAA AGA TAT ATG AGC GGT GCT AAA 2258
   Asn Ser Arg Glu Val Leu Phe Glu Lys Arg Tyr Met Ser Gly Ala Lys
   735 740 745 750
   GAT GTT TCT GAA ATG TTC ACT ACA AAA TTT GAG AAA GAT AAC TTT TAT      2306
   Asp Val Ser Glu Met Phe Thr Thr Lys Phe Glu Lys Asp Asn Phe Tyr
                   755                 760                 765
   ATA GAG CTT TCT CAA GGG AAT AAT TTA TAT GGT GGT CCT ATT GTA CAT      2354
   Ile Glu Leu Ser Gln Gly Asn Asn Leu Tyr Gly Gly Pro Ile Val His
               770                 775                 780
   TTT TAC GAT GTC TCT ATT AAG TAA                                      2378
   Phe Tyr Asp Val Ser Ile Lys
           785
(2) INFORMATION FOR SEQ ID NO:29:
   (i) SEQUENCE CHARACTERISTICS:
      (A) LENGTH: 789 amino acids
      (B) TYPE: amino acid
      (D) TOPOLOGY: linear
   (ii) MOLECULE TYPE: protein
   (xi) SEQUENCE DESCRIPTION: SEQ ID NO:29:
   Met Asn Lys Asn Asn Thr Lys Leu Ser Thr Arg Ala Leu Pro Ser Phe
   1 5 10 15
   Ile Asp Tyr Phe Asn Gly Ile Tyr Gly Phe Ala Thr Gly Ile Lys Asp
   20 25 30
   Ile Met Asn Met Ile Phe Lys Thr Asp Thr Gly Gly Asp Leu Thr Leu
   35 40 45
   Asp Glu Ile Leu Lys Asn Gln Gln Leu Leu Asn Asp Ile Ser Gly Lys
   50 55 60
   Leu Asp Gly Val Asn Gly Ser Leu Asn Asp Leu Ile Ala Gln Gly Asn
   65 70 75 80
   Leu Asn Thr Glu Leu Ser Lys Glu Ile Leu Lys Ile Ala Asn Glu Gln
   85 90 95
   Asn Gln Val Leu Asn Asp Val Asn Asn Lys Leu Asp Ala Ile Asn Thr
   100 105 110
   Met Leu Arg Val Tyr Leu Pro Lys Ile Thr Ser Met Leu Ser Asp Val
   115 120 125
   Met Lys Gln Asn Tyr Ala Leu Ser Leu Gln Ile Glu Tyr Leu Ser Lys
   130 135 140
   Gln Leu Gln Glu Ile Ser Asp Lys Leu Asp Ile Ile Asn Val Asn Val
   145 150 155 160
   Leu Ile Asn Ser Thr Leu Thr Glu Ile Thr Pro Ala Tyr Gln Arg Ile
   165 170 175
   Lys Tyr Val Asn Glu Lys Phe Glu Glu Leu Thr Phe Ala Thr Glu Thr
   180 185 190
   Ser Ser Lys Val Lys Lys Asp Gly Ser Pro Ala Asp Ile Leu Asp Glu
   195 200 205
   Leu Thr Glu Leu Thr Glu Leu Ala Lys Ser Val Thr Lys Asn Asp Val
   210 215 220
   Asp Gly Phe Glu Phe Tyr Leu Asn Thr Phe His Asp Val Met Val Gly
   225 230 235 240
   Asn Asn Leu Phe Gly Arg Ser Ala Leu Lys Thr Ala Ser Glu Leu Ile
   245 250 255
   Thr Lys Glu Asn Val Lys Thr Ser Gly Ser Glu Val Gly Asn Val Tyr
   260 265 270
   Asn Phe Leu Ile Val Leu Thr Ala Leu Gln Ala Gln Ala Phe Leu Thr
   275 280 285
   Leu Thr Thr Cys Arg Lys Leu Leu Gly Leu Ala Asp Ile Asp Tyr Thr
   290 295 300
   Ser Ile Met Asn Glu His Leu Asn Lys Glu Lys Glu Glu Phe Arg Val
   305 310 315 320
   Asn Ile Leu Pro Thr Leu Ser Asn Thr Phe Ser Asn Pro Asn Tyr Ala
   325 330 335
   Lys Val Lys Gly Ser Asp Glu Asp Ala Lys Met Ile Val Glu Ala Lys
   340 345 350
   Pro Gly His Ala Leu Ile Gly Phe Glu Ile Ser Asn Asp Ser Ile Thr
   355 360 365
   Val Leu Lys Val Tyr Glu Ala Lys Leu Lys Gln Asn Tyr Gln Val Asp
   370 375 380
   Lys Asp Ser Leu Ser Glu Val Ile Tyr Gly Asp Met Asp Lys Leu Leu
   385 390 395 400
   Cys Pro Asp Gln Ser Glu Gln Ile Tyr Tyr Thr Asn Asn Ile Val Phe
   405 410 415
   Pro Asn Glu Tyr Val Ile Thr Lys Ile Asp Phe Thr Lys Lys Met Lys
   420 425 430
   Thr Leu Arg Tyr Glu Val Thr Ala Asn Phe Tyr Asp Ser Ser Thr Gly
   435 440 445
   Glu Ile Asp Leu Asn Lys Lys Lys Val Glu Ser Ser Glu Ala Glu Tyr
   450 455 460
   Arg Thr Leu Ser Ala Asn Asp Asp Gly Val Tyr Met Pro Leu Gly Val
   465 470 475 480
   Ile Ser Glu Thr Phe Leu Thr Pro Ile Asn Gly Phe Gly Leu Gln Ala
   485 490 495
   Asp Glu Asn Ser Arg Leu Ile Thr Leu Thr Cys Lys Ser Tyr Leu Arg
   500 505 510
   Glu Leu Leu Leu Ala Thr Asp Leu Ser Asn Lys Glu Thr Lys Leu Ile
   515 520 525
   Val Pro Pro Ser Gly Phe Ile Ser Asn Ile Val Glu Asn Gly Ser Ile
   530 535 540
   Glu Glu Asp Asn Leu Glu Pro Trp Lys Ala Asn Asn Lys Asn Ala Tyr
   545 550 555 560
   Val Asp His Thr Gly Gly Val Asn Gly Thr Lys Ala Leu Tyr Val His
   565 570 575
   Lys Asp Gly Gly Ile Ser Gln Phe Ile Gly Asp Lys Leu Lys Pro Lys
   580 585 590
   Thr Glu Tyr Val Ile Gln Tyr Thr Val Lys Gly Lys Pro Ser Ile His
   595 600 605
   Leu Lys Asp Glu Asn Thr Gly Tyr Ile His Tyr Glu Asp Thr Asn Asn
   610 615 620
   Asn Leu Glu Asp Tyr Gln Thr Ile Asn Lys Arg Phe Thr Thr Gly Thr
   625 630 635 640
   Asp Leu Lys Gly Val Tyr Leu Ile Leu Lys Ser Gln Asn Gly Asp Glu
   645 650 655
   Ala Trp Gly Asp Asn Phe Ile Ile Leu Glu Ile Ser Pro Ser Glu Lys
   660 665 670
   Leu Leu Ser Pro Glu Leu Ile Asn Thr Asn Asn Trp Thr Ser Thr Gly
   675 680 685
   Ser Thr Asn Ile Ser Gly Asn Thr Leu Thr Leu Tyr Gln Gly Gly Arg
   690 695 700
   Gly Ile Leu Lys Gln Asn Leu Gln Leu Asp Ser Phe Ser Thr Tyr Arg
   705 710 715 720
   Val Tyr Phe Ser Val Ser Gly Asp Ala Asn Val Arg Ile Arg Asn Ser
   725 730 735
   Arg Glu Val Leu Phe Glu Lys Arg Tyr Met Ser Gly Ala Lys Asp Val
   740 745 750
   Ser Glu Met Phe Thr Thr Lys Phe Glu Lys Asp Asn Phe Tyr Ile Glu
   755 760 765
   Leu Ser Gln Gly Asn Asn Leu Tyr Gly Gly Pro Ile Val His Phe Tyr
   770 775 780
   Asp Val Ser Ile Lys
   785
(2) INFORMATION FOR SEQ ID NO:30:
   (i) SEQUENCE CHARACTERISTICS:
      (A) LENGTH: 2403 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Synthetic DNA"
   (iii) HYPOTHETICAL: NO
   (ix) FEATURE:
      (A) NAME/KEY: misc_feature
      (B) LOCATION: 11..2389
      (D) OTHER INFORMATION: /note= "maize optimized DNA sequence encoding VIP3A(a)"
   (xi) SEQUENCE DESCRIPTION: SEQ ID NO:30:
   GGATCCACCA ATGAACATGA ACAAGAACAA CACCAAGCTG AGCACCCGCG CCCTGCCGAG     60
   CTTCATCGAC TACTTCAACG GCATCTACGG CTTCGCCACC GGCATCAAGG ACATCATGAA    120
   CATGATCTTC AAGACCGACA CCGGCGGCGA CCTGACCCTG GACGAGATCC TGAAGAACCA    180
   GCAGCTGCTG AACGACATCA GCGGCAAGCT GGACGGCGTG AACGGCAGCC TGAACGACCT    240
   GATCGCCCAG GGCAACCTGA ACACCGAGCT GAGCAAGGAG ATCCTTAAGA TCGCCAACGA    300
   GCAGAACCAG GTGCTGAACG ACGTGAACAA CAAGCTGGAC GCCATCAACA CCATGCTGCG    360
   CGTGTACCTG CCGAAGATCA CCAGCATGCT GAGCGACGTG ATGAAGCAGA ACTACGCCCT    420
   GAGCCTGCAG ATCGAGTACC TGAGCAAGCA GCTGCAGGAG ATCAGCGACA AGCTGGACAT    480
   CATCAACGTG AACGTCCTGA TCAACAGCAC CCTGACCGAG ATCACCCCGG CCTACCAGCG    540
   CATCAAGTAC GTGAACGAGA AGTTCGAAGA GCTGACCTTC GCCACCGAGA CCAGCAGCAA    600
   GGTGAAGAAG GACGGCAGCC CGGCCGACAT CCTGGACGAG CTGACCGAGC TGACCGAGCT    660
   GGCCAAGAGC GTGACCAAGA ACGACGTGGA CGGCTTCGAG TTCTACCTGA ACACCTTCCA    720
   CGACGTGATG GTGGGCAACA ACCTGTTCGG CCGCAGCGCC CTGAAGACCG CCAGCGAGCT 780
   GATCACCAAG GAGAACGTGA AGACCAGCGG CAGCGAGGTG GGCAACGTGT ACAACTTCCT 840
   GATCGTGCTG ACCGCCCTGC AGGCCCAGGC CTTCCTGACC CTGACCACCT GTCGCAAGCT 900
   GCTGGGCCTG GCCGACATCG ACTACACCAG CATCATGAAC GAGCACTTGA ACAAGGAGAA 960
   GGAGGAGTTC CGCGTGAACA TCCTGCCGAC CCTGAGCAAC ACCTTCAGCA ACCCGAACTA 1020
   CGCCAAGGTG AAGGGCAGCG ACGAGGACGC CAAGATGATC GTGGAGGCTA AGCCGGGCCA 1080
   CGCGTTGATC GGCTTCGAGA TCAGCAACGA CAGCATCACC GTGCTGAAGG TGTACGAGGC 1140
   CAAGCTGAAG CAGAACTACC AGGTGGACAA GGACAGCTTG AGCGAGGTGA TCTACGGCGA 1200
   CATGGACAAG CTGCTGTGTC CGGACCAGAG CGAGCAAATC TACTACACCA ACAACATCGT 1260
   GTTCCCGAAC GAGTACGTGA TCACCAAGAT CGACTTCACC AAGAAGATGA AGACCCTGCG 1320
   CTACGAGGTG ACCGCCAACT TCTACGACAG CAGCACCGGC GAGATCGACC TGAACAAGAA 1380
   GAAGGTGGAG AGCAGCGAGG CCGAGTACCG CACCCTGAGC GCGAACGACG ACGGCGTCTA 1440
   CATGCCACTG GGCGTGATCA GCGAGACCTT CCTGACCCCG ATCAACGGCT TTGGCCTGCA 1500
   GGCCGACGAG AACAGCCGCC TGATCACCCT GACCTGTAAG AGCTACCTGC GCGAGCTGCT 1560
   GCTAGCCACC GACCTGAGCA ACAAGGAGAC CAAGCTGATC GTGCCACCGA GCGGCTTCAT 1620
   CAGCAACATC GTGGAGAACG GCAGCATCGA GGAGGACAAC CTGGAGCCGT GGAAGGCCAA 1680
   CAACAAGAAC GCCTACGTGG ACCACACCGG CGGCGTGAAC GGCACCAAGG CCCTGTACGT 1740
   GCACAAGGAC GGCGGCATCA GCCAGTTCAT CGGCGACAAG CTGAAGCCGA AGACCGAGTA 1800
   CGTGATCCAG TACACCGTGA AGGGCAAGCC ATCGATTCAC CTGAAGGACG AGAACACCGG 1860
   CTACATCCAC TACGAGGACA CCAACAACAA CCTGGAGGAC TACCAGACCA TCAACAAGCG 1920
   CTTCACCACC GGCACCGACC TGAAGGGCGT GTACCTGATC CTGAAGAGCC AGAACGGCGA 1980
   CGAGGCCTGG GGCGACAACT TCATCATCCT GGAGATCAGC CCGAGCGAGA AGCTGCTGAG 2040
   CCCGGAGCTG ATCAACACCA ACAACTGGAC CAGCACCGGC AGCACCAACA TCAGCGGCAA 2100
   CACCCTGACC CTGTACCAGG GCGGCCGCGG CATCCTGAAG CAGAACCTGC AGCTGGACAG 2160
   CTTCAGCACC TACCGCGTGT ACTTCAGCGT GAGCGGCGAC GCCAACGTGC GCATCCGCAA 2220
   CAGCCGCGAG GTGCTGTTCG AGAAGAGGTA CATGAGCGGC GCCAAGGACG TGAGCGAGAT 2280
   GTTCACCACC AAGTTCGAGA AGGACAACTT CTACATCGAG CTGAGCCAGG GCAACAACCT 2340
   GTACGGCGGC CCGATCGTGC ACTTCTACGA CGTGAGCATC AAGTTAACGT AGAGCTCAGA 2400
   TCT 2403
(2) INFORMATION FOR SEQ ID NO:31:
   (i) SEQUENCE CHARACTERISTICS:
      (A) LENGTH: 2612 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (ix) FEATURE:
      (A) NAME/KEY: CDS
      (B) LOCATION: 118..2484
      (D) OTHER INFORMATION: /note= "Native DNA sequence encoding VIP3A(b) from AB424"
   (xi) SEQUENCE DESCRIPTION: SEQ ID NO:31:
   ATTGAAATTG ATAAAAAGTT ATGAGTGTTT AATAATCAGT AATTACCAAT AAAGAATTAA      60
   GAATACAAGT TTACAAGAAA TAAGTGTTAC AAAAAATAGC TGAAAAGGAA GATGAAC        117
   ATG AAC AAG AAT AAT ACT AAA TTA AGC ACA AGA GCC TTA CCA AGT TTT       165
   Met Asn Lys Asn Asn Thr Lys Leu Ser Thr Arg Ala Leu Pro Ser Phe
   790                 795                 800                 805
   ATT GAT TAT TTC AAT GGC ATT TAT GGA TTT GCC ACT GGT ATC AAA GAC       213
   Ile Asp Tyr Phe Asn Gly Ile Tyr Gly Phe Ala Thr Gly Ile Lys Asp
                   810                 815                 820
   ATT ATG AAC ATG ATT TTT AAA ACG GAT ACA GGT GGT GAT CTA ACC CTA       261
   Ile Met Asn Met Ile Phe Lys Thr Asp Thr Gly Gly Asp Leu Thr Leu
               825                 830                 835
   GAC GAA ATT TTA AAG AAT CAG CAG CTA CTA AAT GAT ATT TCT GGT AAA       309
   Asp Glu Ile Leu Lys Asn Gln Gln Leu Leu Asn Asp Ile Ser Gly Lys
           840                 845                 850
   TTG GAT GGG GTG AAT GGA AGC TTA AAT GAT CTT ATC GCA CAG GGA AAC       357
   Leu Asp Gly Val Asn Gly Ser Leu Asn Asp Leu Ile Ala Gln Gly Asn
       855                 860                 865
   TTA AAT ACA GAA TTA TCT AAG GAA ATA TTA AAA ATT GCA AAT GAA CAA       405
   Leu Asn Thr Glu Leu Ser Lys Glu Ile Leu Lys Ile Ala Asn Glu Gln
   870                 875                 880                 885
   AAT CAA GTT TTA AAT GAT GTT AAT AAC AAA CTC GAT GCG ATA AAT ACG       453
   Asn Gln Val Leu Asn Asp Val Asn Asn Lys Leu Asp Ala Ile Asn Thr
   890 895 900
   ATG CTT CGG GTA TAT CTA CCT AAA ATT ACC TCT ATG TTG AGT GAT GTA 501
   Met Leu Arg Val Tyr Leu Pro Lys Ile Thr Ser Met Leu Ser Asp Val
   905 910 915
   ATG AAA CAA AAT TAT GCG CTA AGT CTG CAA ATA GAA TAC TTA AGT AAA 549
   Met Lys Gln Asn Tyr Ala Leu Ser Leu Gln Ile Glu Tyr Leu Ser Lys
   920 925 930
   CAA TTG CAA GAG ATT TCT GAT AAG TTG GAT ATT ATT AAT GTA AAT GTA 597
   Gln Leu Gln Glu Ile Ser Asp Lys Leu Asp Ile Ile Asn Val Asn Val
   935 940 945
   CTT ATT AAC TCT ACA CTT ACT GAA ATT ACA CCT GCG TAT CAA AGG ATT 645
   Leu Ile Asn Ser Thr Leu Thr Glu Ile Thr Pro Ala Tyr Gln Arg Ile
   950 955 960 965
   AAA TAT GTG AAC GAA AAA TTT GAG GAA TTA ACT TTT GCT ACA GAA ACT 693
   Lys Tyr Val Asn Glu Lys Phe Glu Glu Leu Thr Phe Ala Thr Glu Thr
   970 975 980
   AGT TCA AAA GTA AAA AAG GAT GGC TCT CCT GCA GAT ATT CGT GAT GAG 741
   Ser Ser Lys Val Lys Lys Asp Gly Ser Pro Ala Asp Ile Arg Asp Glu
   985 990 995
   TTA ACT GAG TTA ACT GAA CTA GCG AAA AGT GTA ACA AAA AAT GAT GTG 789
   Leu Thr Glu Leu Thr Glu Leu Ala Lys Ser Val Thr Lys Asn Asp Val
   1000 1005 1010
   GAT GGT TTT GAA TTT TAC CTT AAT ACA TTC CAC GAT GTA ATG GTA GGA 837
   Asp Gly Phe Glu Phe Tyr Leu Asn Thr Phe His Asp Val Met Val Gly
   1015 1020 1025
   AAT AAT TTA TTC GGG CGT TCA GCT TTA AAA ACT GCA TCG GAA TTA ATT 885
   Asn Asn Leu Phe Gly Arg Ser Ala Leu Lys Thr Ala Ser Glu Leu Ile
   1030 1035 1040 1045
   ACT AAA GAA AAT GTG AAA ACA AGT GGC AGT GAG GTC GGA AAT GTT TAT 933
   Thr Lys Glu Asn Val Lys Thr Ser Gly Ser Glu Val Gly Asn Val Tyr
   1050 1055 1060
   AAC TTC CTA ATT GTA TTA ACA GCT CTG CAA GCA AAA GCT TTT CTT ACT 981
   Asn Phe Leu Ile Val Leu Thr Ala Leu Gln Ala Lys Ala Phe Leu Thr
   1065 1070 1075
   TTA ACA CCA TGC CGA AAA TTA TTA GGC TTA GCA GAT ATT GAT TAT ACT 1029
   Leu Thr Pro Cys Arg Lys Leu Leu Gly Leu Ala Asp Ile Asp Tyr Thr
   1080 1085 1090
   TCT ATT ATG AAT GAA CAT TTA AAT AAG GAA AAA GAG GAA TTT AGA GTA 1077
   Ser Ile Met Asn Glu His Leu Asn Lys Glu Lys Glu Glu Phe Arg Val
   1095 1100 1105
   AAC ATC CTC CCT ACA CTT TCT AAT ACT TTT TCT AAT CCT AAT TAT GCA      1125
   Asn Ile Leu Pro Thr Leu Ser Asn Thr Phe Ser Asn Pro Asn Tyr Ala
   1110                1115                1120                1125
   AAA GTT AAA GGA AGT GAT GAA GAT GCA AAG ATG ATT GTG GAA GCT AAA      1173
   Lys Val Lys Gly Ser Asp Glu Asp Ala Lys Met Ile Val Glu Ala Lys
                   1130                1135                1140
   CCA GGA CAT GCA TTG ATT GGG TTT GAA ATT AGT AAT GAT TCA ATT ACA      1221
   Pro Gly His Ala Leu Ile Gly Phe Glu Ile Ser Asn Asp Ser Ile Thr
               1145                1150                1155
   GTA TTA AAA GTA TAT GAG GCT AAG CTA AAA CAA AAT TAT CAA GTC GAT      1269
   Val Leu Lys Val Tyr Glu Ala Lys Leu Lys Gln Asn Tyr Gln Val Asp
           1160                1165                1170
   AAG GAT TCC TTA TCG GAA GTT ATT TAT GGC GAT ATG GAT AAA TTA TTG      1317
   Lys Asp Ser Leu Ser Glu Val Ile Tyr Gly Asp Met Asp Lys Leu Leu
       1175                1180                1185
   TGC CCA GAT CAA TCT GGA CAA ATC TAT TAT ACA AAT AAC ATA GTA TTT      1365
   Cys Pro Asp Gln Ser Gly Gln Ile Tyr Tyr Thr Asn Asn Ile Val Phe
   1190                1195                1200                1205
   CCA AAT GAA TAT GTA ATT ACT AAA ATT GAT TTC ACT AAA AAA ATG AAA      1413
   Pro Asn Glu Tyr Val Ile Thr Lys Ile Asp Phe Thr Lys Lys Met Lys
                   1210                1215                1220
   ACT TTA AGA TAT GAG GTA ACA GCG AAT TTT TAT GAT TCT TCT ACA GGA      1461
   Thr Leu Arg Tyr Glu Val Thr Ala Asn Phe Tyr Asp Ser Ser Thr Gly
               1225                1230                1235
   GAA ATT GAC TTA AAT AAG AAA AAA GTA GAA TCA AGT GAA GCG GAG TAT      1509
   Glu Ile Asp Leu Asn Lys Lys Lys Val Glu Ser Ser Glu Ala Glu Tyr
           1240                1245                1250
   AGA ACG TTA AGT GCT AAT GAT GAT GGG GTG TAT ATG CCG TTA GGT GTC      1557
   Arg Thr Leu Ser Ala Asn Asp Asp Gly Val Tyr Met Pro Leu Gly Val
       1255                1260                1265
   ATC AGT GAA ACA TTT TTG ACT CCG ATT AAT GGG TTT GGC CTC CAA GCT      1605
   Ile Ser Glu Thr Phe Leu Thr Pro Ile Asn Gly Phe Gly Leu Gln Ala
   1270                1275                1280                1285
   GAT GAA AAT TCA AGA TTA ATT ACT TTA ACA TGT AAA TCA TAT TTA AGA      1653
   Asp Glu Asn Ser Arg Leu Ile Thr Leu Thr Cys Lys Ser Tyr Leu Arg
                   1290                1295                1300
   GAA CTA CTG CTA GCA ACA GAC TTA AGC AAT AAA GAA ACT AAA TTG ATC      1701
   Glu Leu Leu Leu Ala Thr Asp Leu Ser Asn Lys Glu Thr Lys Leu Ile
               1305                1310                1315
   GTC CCG CCA AGT GGT TTT ATT AGC AAT ATT GTA GAG AAC GGG TCC ATA      1749
   Val Pro Pro Ser Gly Phe Ile Ser Asn Ile Val Glu Asn Gly Ser Ile
           1320                1325                1330
   GAA GAG GAC AAT TTA GAG CCG TGG AAA GCA AAT AAT AAG AAT GCG TAT        1797
   Glu Glu Asp Asn Leu Glu Pro Trp Lys Ala Asn Asn Lys Asn Ala Tyr
       1335                1340                1345
   GTA GAT CAT ACA GGC GGA GTG AAT GGA ACT AAA GCT TTA TAT GTT CAT        1845
   Val Asp His Thr Gly Gly Val Asn Gly Thr Lys Ala Leu Tyr Val His
   1350                1355                1360                1365
   AAG GAC GGA GGA ATT TCA CAA TTT ATT GGA GAT AAG TTA AAA CCG AAA        1893
   Lys Asp Gly Gly Ile Ser Gln Phe Ile Gly Asp Lys Leu Lys Pro Lys
                   1370                1375                1380
   ACT GAG TAT GTA ATC CAA TAT ACT GTT AAA GGA AAA CCT TCT ATT CAT        1941
   Thr Glu Tyr Val Ile Gln Tyr Thr Val Lys Gly Lys Pro Ser Ile His
               1385                1390                1395
   TTA AAA GAT GAA AAT ACT GGA TAT ATT CAT TAT GAA GAT ACA AAT AAT        1989
   Leu Lys Asp Glu Asn Thr Gly Tyr Ile His Tyr Glu Asp Thr Asn Asn
           1400                1405                1410
   AAT TTA GAA GAT TAT CAA ACT ATT AAT AAA CGT TTT ACT ACA GGA ACT        2037
   Asn Leu Glu Asp Tyr Gln Thr Ile Asn Lys Arg Phe Thr Thr Gly Thr
       1415                1420                1425
   GAT TTA AAG GGA GTG TAT TTA ATT TTA AAA AGT CAA AAT GGA GAT GAA        2085
   Asp Leu Lys Gly Val Tyr Leu Ile Leu Lys Ser Gln Asn Gly Asp Glu
   1430                1435                1440                1445
   GCT TGG GGA GAT AAC TTT ATT ATT TTG GAA ATT AGT CCT TCT GAA AAG        2133
   Ala Trp Gly Asp Asn Phe Ile Ile Leu Glu Ile Ser Pro Ser Glu Lys
                   1450                1455                1460
   TTA TTA AGT CCA GAA TTA ATT AAT ACA AAT AAT TGG ACG AGT ACG GGA        2181
   Leu Leu Ser Pro Glu Leu Ile Asn Thr Asn Asn Trp Thr Ser Thr Gly
               1465                1470                1475
   TCA ACT AAT ATT AGC GGT AAT ACA CTC ACT CTT TAT CAG GGA GGA CGA        2229
   Ser Thr Asn Ile Ser Gly Asn Thr Leu Thr Leu Tyr Gln Gly Gly Arg
           1480                1485                1490
   GGG ATT CTA AAA CAA AAC CTT CAA TTA GAT AGT TTT TCA ACT TAT AGA        2277
   Gly Ile Leu Lys Gln Asn Leu Gln Leu Asp Ser Phe Ser Thr Tyr Arg
       1495                1500                1505
   GTG TAT TTC TCT GTG TCC GGA GAT GCT AAT GTA AGG ATT AGA AAT TCT        2325
   Val Tyr Phe Ser Val Ser Gly Asp Ala Asn Val Arg Ile Arg Asn Ser
   1510                1515                1520                1525
   AGG GAA GTG TTA TTT GAA AAA AGA TAT ATG AGC GGT GCT AAA GAT GTT        2373
   Arg Glu Val Leu Phe Glu Lys Arg Tyr Met Ser Gly Ala Lys Asp Val
                   1530                1535                1540
   TCT GAA ATG TTC ACT ACA AAA TTT GAG AAA GAT AAC TTC TAT ATA GAG        2421
   Ser Glu Met Phe Thr Thr Lys Phe Glu Lys Asp Asn Phe Tyr Ile Glu
   1545 1550 1555
   CTT TCT CAA GGG AAT AAT TTA TAT GGT GGT CCT ATT GTA CAT TTT TAC 2469
   Leu Ser Gln Gly Asn Asn Leu Tyr Gly Gly Pro Ile Val His Phe Tyr
   1560 1565 1570
   GAT GTC TCT ATT AAG TAAGATCGGG ATCTAATATT AACAGTTTTT AGAAGCTAAT 2524
   Asp Val Ser Ile Lys
   1575
   TCTTGTATAA TGTCCTTGAT TATGGAAAAA CACAATTTTG TTTGCTAAGA TGTATATATA 2584
   GCTCACTCAT TAAAAGGCAA TCAAGCTT 2612
(2) INFORMATION FOR SEQ ID NO:32:
   (i) SEQUENCE CHARACTERISTICS:
      (A) LENGTH: 789 amino acids
      (B) TYPE: amino acid
      (D) TOPOLOGY: linear
   (ii) MOLECULE TYPE: protein
   (xi) SEQUENCE DESCRIPTION: SEQ ID NO:32:
   Met Asn Lys Asn Asn Thr Lys Leu Ser Thr Arg Ala Leu Pro Ser Phe
   1 5 10 15
   Ile Asp Tyr Phe Asn Gly Ile Tyr Gly Phe Ala Thr Gly Ile Lys Asp
   20 25 30
   Ile Met Asn Met Ile Phe Lys Thr Asp Thr Gly Gly Asp Leu Thr Leu
   35 40 45
   Asp Glu Ile Leu Lys Asn Gln Gln Leu Leu Asn Asp Ile Ser Gly Lys
   50 55 60
   Leu Asp Gly Val Asn Gly Ser Leu Asn Asp Leu Ile Ala Gln Gly Asn
   65 70 75 80
   Leu Asn Thr Glu Leu Ser Lys Glu Ile Leu Lys Ile Ala Asn Glu Gln
   85 90 95
   Asn Gln Val Leu Asn Asp Val Asn Asn Lys Leu Asp Ala Ile Asn Thr
   100 105 110
   Met Leu Arg Val Tyr Leu Pro Lys Ile Thr Ser Met Leu Ser Asp Val
   115 120 125
   Met Lys Gln Asn Tyr Ala Leu Ser Leu Gln Ile Glu Tyr Leu Ser Lys
   130 135 140
   Gln Leu Gln Glu Ile Ser Asp Lys Leu Asp Ile Ile Asn Val Asn Val
   145 150 155 160
   Leu Ile Asn Ser Thr Leu Thr Glu Ile Thr Pro Ala Tyr Gln Arg Ile
                   165                 170                 175
   Lys Tyr Val Asn Glu Lys Phe Glu Glu Leu Thr Phe Ala Thr Glu Thr
               180                 185                 190
   Ser Ser Lys Val Lys Lys Asp Gly Ser Pro Ala Asp Ile Arg Asp Glu
           195                 200                 205
   Leu Thr Glu Leu Thr Glu Leu Ala Lys Ser Val Thr Lys Asn Asp Val
       210                 215                 220
   Asp Gly Phe Glu Phe Tyr Leu Asn Thr Phe His Asp Val Met Val Gly
   225                 230                 235                 240
   Asn Asn Leu Phe Gly Arg Ser Ala Leu Lys Thr Ala Ser Glu Leu Ile
                   245                 250                 255
   Thr Lys Glu Asn Val Lys Thr Ser Gly Ser Glu Val Gly Asn Val Tyr
               260                 265                 270
   Asn Phe Leu Ile Val Leu Thr Ala Leu Gln Ala Lys Ala Phe Leu Thr
           275                 280                 285
   Leu Thr Pro Cys Arg Lys Leu Leu Gly Leu Ala Asp Ile Asp Tyr Thr
       290                 295                 300
   Ser Ile Met Asn Glu His Leu Asn Lys Glu Lys Glu Glu Phe Arg Val
   305                 310                 315                 320
   Asn Ile Leu Pro Thr Leu Ser Asn Thr Phe Ser Asn Pro Asn Tyr Ala
                   325                 330                 335
   Lys Val Lys Gly Ser Asp Glu Asp Ala Lys Met Ile Val Glu Ala Lys
               340                 345                 350
   Pro Gly His Ala Leu Ile Gly Phe Glu Ile Ser Asn Asp Ser Ile Thr
           355                 360                 365
   Val Leu Lys Val Tyr Glu Ala Lys Leu Lys Gln Asn Tyr Gln Val Asp
       370                 375                 380
   Lys Asp Ser Leu Ser Glu Val Ile Tyr Gly Asp Met Asp Lys Leu Leu
   385                 390                 395                 400
   Cys Pro Asp Gln Ser Gly Gln Ile Tyr Tyr Thr Asn Asn Ile Val Phe
                   405                 410                 415
   Pro Asn Glu Tyr Val Ile Thr Lys Ile Asp Phe Thr Lys Lys Met Lys
               420                 425                 430
   Thr Leu Arg Tyr Glu Val Thr Ala Asn Phe Tyr Asp Ser Ser Thr Gly
           435                 440                 445
   Glu Ile Asp Leu Asn Lys Lys Lys Val Glu Ser Ser Glu Ala Glu Tyr
   450 455 460
   Arg Thr Leu Ser Ala Asn Asp Asp Gly Val Tyr Met Pro Leu Gly Val
   465 470 475 480
   Ile Ser Glu Thr Phe Leu Thr Pro Ile Asn Gly Phe Gly Leu Gln Ala
   485 490 495
   Asp Glu Asn Ser Arg Leu Ile Thr Leu Thr Cys Lys Ser Tyr Leu Arg
   500 505 510
   Glu Leu Leu Leu Ala Thr Asp Leu Ser Asn Lys Glu Thr Lys Leu Ile
   515 520 525
   Val Pro Pro Ser Gly Phe Ile Ser Asn Ile Val Glu Asn Gly Ser Ile
   530 535 540
   Glu Glu Asp Asn Leu Glu Pro Trp Lys Ala Asn Asn Lys Asn Ala Tyr
   545 550 555 560
   Val Asp His Thr Gly Gly Val Asn Gly Thr Lys Ala Leu Tyr Val His
   565 570 575
   Lys Asp Gly Gly Ile Ser Gln Phe Ile Gly Asp Lys Leu Lys Pro Lys
   580 585 590
   Thr Glu Tyr Val Ile Gln Tyr Thr Val Lys Gly Lys Pro Ser Ile His
   595 600 605
   Leu Lys Asp Glu Asn Thr Gly Tyr Ile His Tyr Glu Asp Thr Asn Asn
   610 615 620
   Asn Leu Glu Asp Tyr Gln Thr Ile Asn Lys Arg Phe Thr Thr Gly Thr
   625 630 635 640
   Asp Leu Lys Gly Val Tyr Leu Ile Leu Lys Ser Gln Asn Gly Asp Glu
   645 650 655
   Ala Trp Gly Asp Asn Phe Ile Ile Leu Glu Ile Ser Pro Ser Glu Lys
   660 665 670
   Leu Leu Ser Pro Glu Leu Ile Asn Thr Asn Asn Trp Thr Ser Thr Gly
   675 680 685
   Ser Thr Asn Ile Ser Gly Asn Thr Leu Thr Leu Tyr Gln Gly Gly Arg
   690 695 700
   Gly Ile Leu Lys Gln Asn Leu Gln Leu Asp Ser Phe Ser Thr Tyr Arg
   705 710 715 720
   Val Tyr Phe Ser Val Ser Gly Asp Ala Asn Val Arg Ile Arg Asn Ser
   725 730 735
   Arg Glu Val Leu Phe Glu Lys Arg Tyr Met Ser Gly Ala Lys Asp Val
   740 745 750
   Ser Glu Met Phe Thr Thr Lys Phe Glu Lys Asp Asn Phe Tyr Ile Glu
   755 760 765
   Leu Ser Gln Gly Asn Asn Leu Tyr Gly Gly Pro Ile Val His Phe Tyr
   770 775 780
   Asp Val Ser Ile Lys
   785
(2) INFORMATION FOR SEQ ID NO:33:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "forward primer used to make pCIB5526"
   (iii) HYPOTHETICAL: NO
   (xi) SEQUENCE DESCRIPTION: SEQ ID NO:33:
   GGATCCACCA TGAAGACCAA CCAGATCAGC 30
(2) INFORMATION FOR SEQ ID NO:34:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "reverse primer used to make pCIB5526"
   (iii) HYPOTHETICAL: NO
   (xi) SEQUENCE DESCRIPTION: SEQ ID NO:34:
   AAGCTTCAGC TCCTT 15
(2) INFORMATION FOR SEQ ID NO:35:
   (i) SEQUENCE CHARACTERISTICS:
      (A) LENGTH: 2576 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Synthetic DNA"
   (iii) HYPOTHETICAL: NO
   (ix) FEATURE:
      (A) NAME/KEY: CDS
      (B) LOCATICN: 9..2564
      (D) OTHER INFORMATION: /note= "Maize optimized sequence encoding VIP1A(a) with the Bacillus secretion signal removed as contained in pCIB5526"
   (xi) SEQUENCE DESCRIPTION: SEQ ID NO:35:
   GATCCACC ATG AAG ACC AAC CAG ATC AGC ACC ACC CAG AAG AAC CAG CAG      50
            Met Lys Thr Asn Gln Ile Ser Thr Thr Gln Lys Asn Gln Gln
                        825                 830                 835
   AAG GAG ATG GAC CGC AAG GGC CTG CTG GGC TAC TAC TTC AAG GGC AAG       98
   Lys Glu Met Asp Arg Lys Gly Leu Leu Gly Tyr Tyr Phe Lys Gly Lys
                   840                 845                 850
   GAC TTC AGC AAC CTG ACC ATG TTC GCC CCC ACG CGT GAC AGC ACC CTG      146
   Asp Phe Ser Asn Leu Thr Met Phe Ala Pro Thr Arg Asp Ser Thr Leu
               855                 860                 865
   ATC TAC GAC CAG CAG ACC GCC AAC AAG CTG CTG GAC AAG AAG CAG CAG      194
   Ile Tyr Asp Gln Gln Thr Ala Asn Lys Leu Leu Asp Lys Lys Gln Gln
           870                 875                 880
   GAG TAC CAG AGC ATC CGC TGG ATC GGC CTG ATC CAG AGC AAG GAG ACC      242
   Glu Tyr Gln Ser Ile Arg Trp Ile Gly Leu Ile Gln Ser Lys Glu Thr
       885                 890                 895
   GGC GAC TTC ACC TTC AAC CTG AGC GAG GAC GAG CAG GCC ATC ATC GAG      290
   Gly Asp Phe Thr Phe Asn Leu Ser Glu Asp Glu Gln Ala Ile Ile Glu
   900                 905                 910                 915
   ATC AAC GGC AAG ATC ATC AGC AAC AAG GGC AAG GAG AAG CAG GTG GTG      338
   Ile Asn Gly Lys Ile Ile Ser Asn Lys Gly Lys Glu Lys Gln Val Val
                   920                 925                 930
   CAC CTG GAG AAG GGC AAG CTG GTG CCC ATC AAG ATC GAG TAC CAG AGC      386
   His Leu Glu Lys Gly Lys Leu Val Pro Ile Lys Ile Glu Tyr Gln Ser
               935                 940                 945
   GAC ACC AAG TTC AAC ATC GAC AGC AAG ACC TTC AAG GAG CTG AAG CTT      434
   Asp Thr Lys Phe Asn Ile Asp Ser Lys Thr Phe Lys Glu Leu Lys Leu
           950                 955                 960
   TTC AAG ATC GAC AGC CAG AAC CAG CCC CAG CAG GTG CAG CAG GAC GAG          482
   Phe Lys Ile Asp Ser Gln Asn Gln Pro Gln Gln Val Gln Gln Asp Glu
       965                 970                 975
   CTG CGC AAC CCC GAG TTC AAC AAG AAG GAG AGC CAG GAG TTC CTG GCC          530
   Leu Arg Asn Pro Glu Phe Asn Lys Lys Glu Ser Gln Glu Phe Leu Ala
   980                 985                 990                 995
   AAG CCC AGC AAG ATC AAC CTG TTC ACC CAG CAG ATG AAG CGC GAG ATC          578
   Lys Pro Ser Lys Ile Asn Leu Phe Thr Gln Gln Met Lys Arg Glu Ile
                   1000                1005                1010
   GAC GAG GAC ACC GAC ACC GAC GGC GAC AGC ATC CCC GAC CTG TGG GAG          626
   Asp Glu Asp Thr Asp Thr Asp Gly Asp Ser Ile Pro Asp Leu Trp Glu
               1015                1020                1025
   GAG AAC GGC TAC ACC ATC CAG AAC CGC ATC GCC GTG AAG TGG GAC GAC          674
   Glu Asn Gly Tyr Thr Ile Gln Asn Arg Ile Ala Val Lys Trp Asp Asp
           1030                1035                1040
   AGC CTG GCT AGC AAG GGC TAC ACC AAG TTC GTG AGC AAC CCC CTG GAG          722
   Ser Leu Ala Ser Lys Gly Tyr Thr Lys Phe Val Ser Asn Pro Leu Glu
       1045                1050                1055
   AGC CAC ACC GTG GGC GAC CCC TAC ACC GAC TAC GAG AAG GCC GCC CGC          770
   Ser His Thr Val Gly Asp Pro Tyr Thr Asp Tyr Glu Lys Ala Ala Arg
   1060                1065                1070                1075
   GAC CTG GAC CTG AGC AAC GCC AAG GAG ACC TTC AAC CCC CTG GTG GCC          818
   Asp Leu Asp Leu Ser Asn Ala Lys Glu Thr Phe Asn Pro Leu Val Ala
                   1080                1085                1090
   GCC TTC CCC AGC GTG AAC GTG AGC ATG GAG AAG GTG ATC CTG AGC CCC          866
   Ala Phe Pro Ser Val Asn Val Ser Met Glu Lys Val Ile Leu Ser Pro
               1095                1100                1105
   AAC GAG AAC CTG AGC AAC AGC GTG GAG AGC CAC TCG AGC ACC AAC TGG          914
   Asn Glu Asn Leu Ser Asn Ser Val Glu Ser His Ser Ser Thr Asn Trp
           1110                1115                1120
   AGC TAC ACC AAC ACC GAG GGC GCC AGC GTG GAG GCC GGC ATC GGT CCC          962
   Ser Tyr Thr Asn Thr Glu Gly Ala Ser Val Glu Ala Gly Ile Gly Pro
       1125                1130                1135
   AAG GGC ATC AGC TTC GGC GTG AGC GTG AAC TAC CAG CAC AGC GAG ACC         1010
   Lys Gly Ile Ser Phe Gly Val Ser Val Asn Tyr Gln His Ser Glu Thr
   1140                1145                1150                1155
   GTG GCC CAG GAG TGG GGC ACC AGC ACC GGC AAC ACC AGC CAG TTC AAC         1058
   Val Ala Gln Glu Trp Gly Thr Ser Thr Gly Asn Thr Ser Gln Phe Asn
                   1160                1165                1170
   ACC GCC AGC GCC GGC TAC CTG AAC GCC AAC GTG CGC TAC AAC AAC GTG      1106
   Thr Ala Ser Ala Gly Tyr Leu Asn Ala Asn Val Arg Tyr Asn Asn Val
               1175                    1180                    1185
   GGC ACC GGC GCC ATC TAC GAC GTG AAG CCC ACC ACC AGC TTC GTG CTG      1154
   Gly Thr Gly Ala Ile Tyr Asp Val Lys Pro Thr Thr Ser Phe Val Leu
           1190                    1195                    1200
   AAC AAC GAC ACC ATC GCC ACC ATC ACC GCC AAG TCG AAT TCC ACC GCC      1202
   Asn Asn Asp Thr Ile Ala Thr Ile Thr Ala Lys Ser Asn Ser Thr Ala
       1205                    1210                    1215
   CTG AAC ATC AGC CCC GGC GAG AGC TAC CCC AAG AAG GGC CAG AAC GGC      1250
   Leu Asn Ile Ser Pro Gly Glu Ser Tyr Pro Lys Lys Gly Gln Asn Gly
   1220                    1225                    1230                    1235
   ATC GCC ATC ACC AGC ATG GAC GAC TTC AAC AGC CAC CCC ATC ACC CTG      1298
   Ile Ala Ile Thr Ser Met Asp Asp Phe Asn Ser His Pro Ile Thr Leu
                   1240                    1245                    1250
   AAC AAG AAG CAG GTG GAC AAC CTG CTG AAC AAC AAG CCC ATG ATG CTG      1346
   Asn Lys Lys Gln Val Asp Asn Leu Leu Asn Asn Lys Pro Met Met Leu
               1255                    1260                    1265
   GAG ACC AAC CAG ACC GAC GGC GTC TAC AAG ATC AAG GAC ACC CAC GGC      1394
   Glu Thr Asn Gln Thr Asp Gly Val Tyr Lys Ile Lys Asp Thr His Gly
           1270                    1275                    1280
   AAC ATC GTG ACG GGC GGC GAG TGG AAC GGC GTG ATC CAG CAG ATC AAG      1442
   Asn Ile Val Thr Gly Gly Glu Trp Asn Gly Val Ile Gln Gln Ile Lys
       1285                    1290                    1295
   GCC AAG ACC GCC AGC ATC ATC GTC GAC GAC GGC GAG CGC GTG GCC GAG      1490
   Ala Lys Thr Ala Ser Ile Ile Val Asp Asp Gly Glu Arg Val Ala Glu
   1300                    1305                    1310                    1315
   AAG CGC GTG GCC GCC AAG GAC TAC GAG AAC CCC GAG GAC AAG ACC CCC      1538
   Lys Arg Val Ala Ala Lys Asp Tyr Glu Asn Pro Glu Asp Lys Thr Pro
                   1320                    1325                    1330
   AGC CTG ACC CTG AAG GAC GCC CTG AAG CTG AGC TAC CCC GAC GAG ATC      1586
   Ser Leu Thr Leu Lys Asp Ala Leu Lys Leu Ser Tyr Pro Asp Glu Ile
               1335                    1340                    1345
   AAG GAG ATC GAG GGC TTG CTG TAC TAC AAG AAC AAG CCC ATC TAC GAG      1634
   Lys Glu Ile Glu Gly Leu Leu Tyr Tyr Lys Asn Lys Pro Ile Tyr Glu
           1350                    1355                    1360
   AGC AGC GTG ATG ACC TAT CTA GAC GAG AAC ACC GCC AAG GAG GTG ACC      1682
   Ser Ser Val Met Thr Tyr Leu Asp Glu Asn Thr Ala Lys Glu Val Thr
       1365                    1370                    1375
   AAG CAG CTG AAC GAC ACC ACC GGC AAG TTC AAG GAC GTG AGC CAC CTG      1730
   Lys Gln Leu Asn Asp Thr Thr Gly Lys Phe Lys Asp Val Ser His Leu
   1380                    1385                    1390                    1395
   TAC GAC GTG AAG CTG ACC CCC AAG ATG AAC GTG ACC ATC AAG CTG AGC 1778
   Tyr Asp Val Lys Leu Thr Pro Lys Met Asn Val Thr Ile Lys Leu Ser
   1400 1405 1410
   ATC CTG TAC GAC AAC GCC GAG AGC AAC GAC AAC AGC ATC GGC AAG TGG 1826
   Ile Leu Tyr Asp Asn Ala Glu Ser Asn Asp Asn Ser Ile Gly Lys Trp
   1415 1420 1425
   ACC AAC ACC AAC ATC GTG AGC GGC GGC AAC AAC GGC AAG AAG CAG TAC 1874
   Thr Asn Thr Asn Ile Val Ser Gly Gly Asn Asn Gly Lys Lys Gln Tyr
   1430 1435 1440
   AGC AGC AAC AAC CCC GAC GCC AAC CTG ACC CTG AAC ACC GAC GCC CAG 1922
   Ser Ser Asn Asn Pro Asp Ala Asn Leu Thr Leu Asn Thr Asp Ala Gln
   1445 1450 1455
   GAG AAG CTG AAC AAG AAC CGC GAC TAC TAC ATC AGC CTG TAC ATG AAG 1970
   Glu Lys Leu Asn Lys Asn Arg Asp Tyr Tyr Ile Ser Leu Tyr Met Lys
   1460 1465 1470 1475
   AGC GAG AAG AAC ACC CAG TGC GAG ATC ACC ATC GAC GGC GAG ATA TAC 2018
   Ser Glu Lys Asn Thr Gln Cys Glu Ile Thr Ile Asp Gly Glu Ile Tyr
   1480 1485 1490
   CCC ATC ACC ACC AAG ACC GTG AAC GTG AAC AAG GAC AAC TAC AAG CGC 2066
   Pro Ile Thr Thr Lys Thr Val Asn Val Asn Lys Asp Asn Tyr Lys Arg
   1495 1500 1505
   CTG GAC ATC ATC GCC CAC AAC ATC AAG AGC AAC CCC ATC AGC AGC CTG 2114
   Leu Asp Ile Ile Ala His Asn Ile Lys Ser Asn Pro Ile Ser Ser Leu
   1510 1515 1520
   CAC ATC AAG ACC AAC GAC GAG ATC ACC CTG TTC TGG GAC GAC ATA TCG 2162
   His Ile Lys Thr Asn Asp Glu Ile Thr Leu Phe Trp Asp Asp Ile Ser
   1525 1530 1535
   ATT ACC GAC GTC GCC AGC ATC AAG CCC GAG AAC CTG ACC GAC AGC GAG 2210
   Ile Thr Asp Val Ala Ser Ile Lys Pro Glu Asn Leu Thr Asp Ser Glu
   1540 1545 1550 1555
   ATC AAG CAG ATA TAC AGT CGC TAC GGC ATC AAG CTG GAG GAC GGC ATC 2258
   Ile Lys Gln Ile Tyr Ser Arg Tyr Gly Ile Lys Leu Glu Asp Gly Ile
   1560 1565 1570
   CTG ATC GAC AAG AAA GGC GGC ATC CAC TAC GGC GAG TTC ATC AAC GAG 2306
   Leu Ile Asp Lys Lys Gly Gly Ile His Tyr Gly Glu Phe Ile Asn Glu
   1575 1580 1585
   GCC AGC TTC AAC ATC GAG CCC CTG CAG AAC TAC GTG ACC AAG TAC GAG 2354
   Ala Ser Phe Asn Ile Glu Pro Leu Gln Asn Tyr Val Thr Lys Tyr Glu
   1590 1595 1600
   GTG ACC TAC AGC AGC GAG CTG GGC CCC AAC GTG AGC GAC ACC CTG GAG 2402
   Val Thr Tyr Ser Ser Glu Leu Gly Pro Asn Val Ser Asp Thr Leu Glu
       1605                1610                1615
   AGC GAC AAG ATT TAC AAG GAC GGC ACC ATC AAG TTC GAC TTC ACC AAG      2450
   Ser Asp Lys Ile Tyr Lys Asp Gly Thr Ile Lys Phe Asp Phe Thr Lys
   1620                1625                1630                1635
   TAC AGC AAG AAC GAG CAG GGC CTG TTC TAC GAC AGC GGC CTG AAC TGG      2498
   Tyr Ser Lys Asn Glu Gln Gly Leu Phe Tyr Asp Ser Gly Leu Asn Trp
                   1640                1645                1650
   GAC TTC AAG ATC AAC GCC ATC ACC TAC GAC GGC AAG GAG ATG AAC GTG      2546
   Asp Phe Lys Ile Asn Ala Ile Thr Tyr Asp Gly Lys Glu Met Asn Val
               1655                1660                1665
   TTC CAC CGC TAC AAC AAG TAGATCTGAG CT                                2576
   Phe His Arg Tyr Asn Lys
           1670
(2) INFORMATION FOR SEQ ID NO:36:
   (i) SEQUENCE CHARACTERISTICS:
      (A) LENGTH: 852 amino acids
      (B) TYPE: amino acid
      (D) TOPOLOGY: linear
   (ii) MOLECULE TYPE: protein
   (xi) SEQUENCE DESCRIPTION: SEQ ID NO:36:
   Met Lys Thr Asn Gln Ile Ser Thr Thr Gln Lys Asn Gln Gln Lys Glu
   1 5 10 15
   Met Asp Arg Lys Gly Leu Leu Gly Tyr Tyr Phe Lys Gly Lys Asp Phe
   20 25 30
   Ser Asn Leu Thr Met Phe Ala Pro Thr Arg Asp Ser Thr Leu Ile Tyr
   35 40 45
   Asp Gln Gln Thr Ala Asn Lys Leu Leu Asp Lys Lys Gln Gln Glu Tyr
   50 55 60
   Gln Ser Ile Arg Trp Ile Gly Leu Ile Gln Ser Lys Glu Thr Gly Asp
   65 70 75 80
   Phe Thr Phe Asn Leu Ser Glu Asp Glu Gln Ala Ile Ile Glu Ile Asn
   85 90 95
   Gly Lys Ile Ile Ser Asn Lys Gly Lys Glu Lys Gln Val Val His Leu
   100 105 110
   Glu Lys Gly Lys Leu Val Pro Ile Lys Ile Glu Tyr Gln Ser Asp Thr
   115 120 125
   Lys Phe Asn Ile Asp Ser Lys Thr Phe Lys Glu Leu Lys Leu Phe Lys
   130 135 140
   Ile Asp Ser Gln Asn Gln Pro Gln Gln Val Gln Gln Asp Glu Leu Arg
   145 150 155 160
   Asn Pro Glu Phe Asn Lys Lys Glu Ser Gln Glu Phe Leu Ala Lys Pro
   165 170 175
   Ser Lys Ile Asn Leu Phe Thr Gln Gln Met Lys Arg Glu Ile Asp Glu
   180 185 190
   Asp Thr Asp Thr Asp Gly Asp Ser Ile Pro Asp Leu Trp Glu Glu Asn
   195 200 205
   Gly Tyr Thr Ile Gln Asn Arg Ile Ala Val Lys Trp Asp Asp Ser Leu
   210 215 220
   Ala Ser Lys Gly Tyr Thr Lys Phe Val Ser Asn Pro Leu Glu Ser His
   225 230 235 240
   Thr Val Gly Asp Pro Tyr Thr Asp Tyr Glu Lys Ala Ala Arg Asp Leu
   245 250 255
   Asp Leu Ser Asn Ala Lys Glu Thr Phe Asn Pro Leu Val Ala Ala Phe
   260 265 270
   Pro Ser Val Asn Val Ser Met Glu Lys Val Ile Leu Ser Pro Asn Glu
   275 280 285
   Asn Leu Ser Asn Ser Val Glu Ser His Ser Ser Thr Asn Trp Ser Tyr
   290 295 300
   Thr Asn Thr Glu Gly Ala Ser Val Glu Ala Gly Ile Gly Pro Lys Gly
   305 310 315 320
   Ile Ser Phe Gly Val Ser Val Asn Tyr Gln His Ser Glu Thr Val Ala
   325 330 335
   Gln Glu Trp Gly Thr Ser Thr Gly Asn Thr Ser Gln Phe Asn Thr Ala
   340 345 350
   Ser Ala Gly Tyr Leu Asn Ala Asn Val Arg Tyr Asn Asn Val Gly Thr
   355 360 365
   Gly Ala Ile Tyr Asp Val Lys Pro Thr Thr Ser Phe Val Leu Asn Asn
   370 375 380
   Asp Thr Ile Ala Thr Ile Thr Ala Lys Ser Asn Ser Thr Ala Leu Asn
   385 390 395 400
   Ile Ser Pro Gly Glu Ser Tyr Pro Lys Lys Gly Gln Asn Gly Ile Ala
   405 410 415
   Ile Thr Ser Met Asp Asp Phe Asn Ser His Pro Ile Thr Leu Asn Lys
   420 425 430
   Lys Gln Val Asp Asn Leu Leu Asn Asn Lys Pro Met Met Leu Glu Thr
   435 440 445
   Asn Gln Thr Asp Gly Val Tyr Lys Ile Lys Asp Thr His Gly Asn Ile
   450 455 460
   Val Thr Gly Gly Glu Trp Asn Gly Val Ile Gln Gln Ile Lys Ala Lys
   465 470 475 480
   Thr Ala Ser Ile Ile Val Asp Asp Gly Glu Arg Val Ala Glu Lys Arg
   485 490 495
   Val Ala Ala Lys Asp Tyr Glu Asn Pro Glu Asp Lys Thr Pro Ser Leu
   500 505 510
   Thr Leu Lys Asp Ala Leu Lys Leu Ser Tyr Pro Asp Glu Ile Lys Glu
   515 520 525
   Ile Glu Gly Leu Leu Tyr Tyr Lys Asn Lys Pro Ile Tyr Glu Ser Ser
   530 535 540
   Val Met Thr Tyr Leu Asp Glu Asn Thr Ala Lys Glu Val Thr Lys Gln
   545 550 555 560
   Leu Asn Asp Thr Thr Gly Lys Phe Lys Asp Val Ser His Leu Tyr Asp
   565 570 575
   Val Lys Leu Thr Pro Lys Met Asn Val Thr Ile Lys Leu Ser Ile Leu
   580 585 590
   Tyr Asp Asn Ala Glu Ser Asn Asp Asn Ser Ile Gly Lys Trp Thr Asn
   595 600 605
   Thr Asn Ile Val Ser Gly Gly Asn Asn Gly Lys Lys Gln Tyr Ser Ser
   610 615 620
   Asn Asn Pro Asp Ala Asn Leu Thr Leu Asn Thr Asp Ala Gln Glu Lys
   625 630 635 640
   Leu Asn Lys Asn Arg Asp Tyr Tyr Ile Ser Leu Tyr Met Lys Ser Glu
   645 650 655
   Lys Asn Thr Gln Cys Glu Ile Thr Ile Asp Gly Glu Ile Tyr Pro Ile
   660 665 670
   Thr Thr Lys Thr Val Asn Val Asn Lys Asp Asn Tyr Lys Arg Leu Asp
   675 680 685
   Ile Ile Ala His Asn Ile Lys Ser Asn Pro Ile Ser Ser Leu His Ile
   690 695 700
   Lys Thr Asn Asp Glu Ile Thr Leu Phe Trp Asp Asp Ile Ser Ile Thr
   705 710 715 720
   Asp Val Ala Ser Ile Lys Pro Glu Asn Leu Thr Asp Ser Glu Ile Lys
   725 730 735
   Gln Ile Tyr Ser Arg Tyr Gly Ile Lys Leu Glu Asp Gly Ile Leu Ile
   740 745 750
   Asp Lys Lys Gly Gly Ile His Tyr Gly Glu Phe Ile Asn Glu Ala Ser
   755 760 765
   Phe Asn Ile Glu Pro Leu Gln Asn Tyr Val Thr Lys Tyr Glu Val Thr
   770 775 780
   Tyr Ser Ser Glu Leu Gly Pro Asn Val Ser Asp Thr Leu Glu Ser Asp
   785 790 795 800
   Lys Ile Tyr Lys Asp Gly Thr Ile Lys Phe Asp Phe Thr Lys Tyr Ser
   805 810 815
   Lys Asn Glu Gln Gly Leu Phe Tyr Asp Ser Gly Leu Asn Trp Asp Phe
   820 825 830
   Lys Ile Asn Ala Ile Thr Tyr Asp Gly Lys Glu Met Asn Val Phe His
   835 840 845
   Arg Tyr Asn Lys
   850
(2) INFORMATION FOR SEQ ID NO:37:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "forward primer used to make pCIB5527"
   (iii) HYPOTHETICAL: NO
   (xi) SEQUENCE DESCRIPTION: SEQ ID NO:37:
   GGATCCACCA TGCTGCAGAA CCTGAAGATC AC 32
(2) INFORMATION FOR SEQ ID NO:38:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "reverse primer used to make pCIB5527"
   (iii) HYPOTHETICAL: NO
   (xi) SEQUENCE DESCRIPTION: SEQ ID NO:38:
   AAGCTTCCAC TCCTTCTC                                        18
(2) INFORMATION FOR SEQ ID NO:39:
   (i) SEQUENCE CHARACTERISTICS:
      (A) LENGTH: 1241 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Synthetic DNA"
   (iii) HYPOTHETICAL: NO
   (ix) FEATURE:
      (A) NAME/KEY: CDS
      (B) LOCATION: 9..1238
      (D) OTHER INFORMATION: /note= "Maize optimized DNA sequence encoding VIP2A(a) with the Bacillus secretion signal removed as contained in pCIB5527"
   (xi) SEQUENCE DESCRIPTION: SEQ ID NO:39:
   GATCCACC ATG CTG CAG AAC CTG AAG ATC ACC GAC AAG GTG GAG GAC TTC     50
            Met Leu Gln Asn Leu Lys Ile Thr Asp Lys Val Glu Asp Phe
                        855                 860                 865
   AAG GAG GAC AAG GAG AAG GCC AAG GAG TGG GGC AAG GAG AAG GAG AAG      98
   Lys Glu Asp Lys Glu Lys Ala Lys Glu Trp Gly Lys Glu Lys Glu Lys
               870                 875                 880
   GAG TGG AAG CTT ACC GCC ACC GAG AAG GGC AAG ATG AAC AAC TTC CTG     146
   Glu Trp Lys Leu Thr Ala Thr Glu Lys Gly Lys Met Asn Asn Phe Leu
       885                 890                 895
   GAC AAC AAG AAC GAC ATC AAG ACC AAC TAC AAG GAG ATC ACC TTC AGC     194
   Asp Asn Lys Asn Asp Ile Lys Thr Asn Tyr Lys Glu Ile Thr Phe Ser
   900                 905                 910
   ATA GCC GGC AGC TTC GAG GAC GAG ATC AAG GAC CTG AAG GAG ATC GAC     242
   Ile Ala Gly Ser Phe Glu Asp Glu Ile Lys Asp Leu Lys Glu Ile Asp
   915 920 925 930
   AAG ATG TTC GAC AAG ACC AAC CTG AGC AAC AGC ATC ATC ACC TAC AAG 290
   Lys Met Phe Asp Lys Thr Asn Leu Ser Asn Ser Ile Ile Thr Tyr Lys
   935 940 945
   AAC GTG GAG CCC ACC ACC ATC GGC TTC AAC AAG AGC CTG ACC GAG GGC 338
   Asn Val Glu Pro Thr Thr Ile Gly Phe Asn Lys Ser Leu Thr Glu Gly
   950 955 960
   AAC ACC ATC AAC AGC GAC GCC ATG GCC CAG TTC AAG GAG CAG TTC CTG 386
   Asn Thr Ile Asn Ser Asp Ala Met Ala Gln Phe Lys Glu Gln Phe Leu
   965 970 975
   GAC CGC GAC ATC AAG TTC GAC AGC TAC CTG GAC ACC CAC CTG ACC GCC 434
   Asp Arg Asp Ile Lys Phe Asp Ser Tyr Leu Asp Thr His Leu Thr Ala
   980 985 990
   CAG CAG GTG AGC AGC AAG GAG CGC GTG ATC CTG AAG GTG ACC GTC CCC 482
   Gln Gln Val Ser Ser Lys Glu Arg Val Ile Leu Lys Val Thr Val Pro
   995 1000 1005 1010
   AGC GGC AAG GGC AGC ACC ACC CCC ACC AAG GCC GGC GTG ATC CTG AAC 530
   Ser Gly Lys Gly Ser Thr Thr Pro Thr Lys Ala Gly Val Ile Leu Asn
   1015 1020 1025
   AAC AGC GAG TAC AAG ATG CTG ATC GAC AAC GGC TAC ATG GTG CAC GTG 578
   Asn Ser Glu Tyr Lys Met Leu Ile Asp Asn Gly Tyr Met Val His Val
   1030 1035 1040
   GAC AAG GTG AGC AAG GTG GTG AAG AAG GGC GTG GAG TGC CTC CAG ATC 626
   Asp Lys Val Ser Lys Val Val Lys Lys Gly Val Glu Cys Leu Gln Ile
   1045 1050 1055
   GAG GGC ACC CTG AAG AAG AGT CTA GAC TTC AAG AAC GAC ATC AAC GCC 674
   Glu Gly Thr Leu Lys Lys Ser Leu Asp Phe Lys Asn Asp Ile Asn Ala
   1060 1065 1070
   GAG GCC CAC AGC TGG GGC ATG AAG AAC TAC GAG GAG TGG GCC AAG GAC 722
   Glu Ala His Ser Trp Gly Met Lys Asn Tyr Glu Glu Trp Ala Lys Asp
   1075 1080 1085 1090
   CTG ACC GAC AGC CAG CGC GAG GCC CTG GAC GGC TAC GCC CGC CAG GAC 770
   Leu Thr Asp Ser Gln Arg Glu Ala Leu Asp Gly Tyr Ala Arg Gln Asp
   1095 1100 1105
   TAC AAG GAG ATC AAC AAC TAC CTG CGC AAC CAG GGC GGC AGC GGC AAC 818
   Tyr Lys Glu Ile Asn Asn Tyr Leu Arg Asn Gln Gly Gly Ser Gly Asn
   1110 1115 1120
   GAG AAG CTG GAC GCC CAG ATC AAG AAC ATC AGC GAC GCC CTG GGC AAG 866
   Glu Lys Leu Asp Ala Gln Ile Lys Asn Ile Ser Asp Ala Leu Gly Lys
   1125 1130 1135
   AAG CCC ATC CCC GAG AAC ATC ACC GTG TAC CGC TGG TGC GGC ATG CCC       914
   Lys Pro Ile Pro Glu Asn Ile Thr Val Tyr Arg Trp Cys Gly Met Pro
       1140                1145                1150
   GAG TTC GGC TAC CAG ATC AGC GAC CCC CTG CCC AGC CTG AAG GAC TTC       962
   Glu Phe Gly Tyr Gln Ile Ser Asp Pro Leu Pro Ser Leu Lys Asp Phe
   1155                1160                1165                1170
   GAG GAG CAG TTC CTG AAC ACC ATC AAG GAG GAC AAG GGC TAC ATG AGC      1010
   Glu Glu Gln Phe Leu Asn Thr Ile Lys Glu Asp Lys Gly Tyr Met Ser
                   1175                1180                1185
   ACC AGC CTG AGC AGC GAG CGC CTG GCC GCC TTC GGC AGC CGC AAG ATC      1058
   Thr Ser Leu Ser Ser Glu Arg Leu Ala Ala Phe Gly Ser Arg Lys Ile
               1190                1195                1200
   ATC CTG CGC CTG CAG GTG CCC AAG GGC AGC ACT GGT GCC TAC CTG AGC      1106
   Ile Leu Arg Leu Gln Val Pro Lys Gly Ser Thr Gly Ala Tyr Leu Ser
           1205                1210                1215
   GCC ATC GGC GGC TTC GCC AGC GAG AAG GAG ATC CTG CTG GAT AAG GAC      1154
   Ala Ile Gly Gly Phe Ala Ser Glu Lys Glu Ile Leu Leu Asp Lys Asp
       1220                1225                1230
   AGC AAG TAC CAC ATC GAC AAG GTG ACC GAG GTG ATC ATC AAG GGC GTG      1202
   Ser Lys Tyr His Ile Asp Lys Val Thr Glu Val Ile Ile Lys Gly Val
   1235                1240                1245                1250
   AAG CGC TAC GTG GTG GAC GCC ACC CTG CTG ACC AAC TAG                  1241
   Lys Arg Tyr Val Val Asp Ala Thr Leu Leu Thr Asn
                   1255                1260
(2) INFORMATION FOR SEQ ID NO:40:
   (i) SEQUENCE CHARACTERISTICS:
      (A) LENGTH: 410 amino acids
      (B) TYPE: amino acid
      (D) TOPOLOGY: linear
   (ii) MOLECULE TYPE: protein
   (xi) SEQUENCE DESCRIPTION: SEQ ID NO:40:
   Met Leu Gln Asn Leu Lys Ile Thr Asp Lys Val Glu Asp Phe Lys Glu
   1 5 10 15
   Asp Lys Glu Lys Ala Lys Glu Trp Gly Lys Glu Lys Glu Lys Glu Trp
   20 25 30
   Lys Leu Thr Ala Thr Glu Lys Gly Lys Met Asn Asn Phe Leu Asp Asn
   35 40 45
   Lys Asn Asp Ile Lys Thr Asn Tyr Lys Glu Ile Thr Phe Ser Ile Ala
   50 55 60
   Gly Ser Phe Glu Asp Glu Ile Lys Asp Leu Lys Glu Ile Asp Lys Met
   65 70 75 80
   Phe Asp Lys Thr Asn Leu Ser Asn Ser Ile Ile Thr Tyr Lys Asn Val
   85 90 95
   Glu Pro Thr Thr Ile Gly Phe Asn Lys Ser Leu Thr Glu Gly Asn Thr
   100 105 110
   Ile Asn Ser Asp Ala Met Ala Gln Phe Lys Glu Gln Phe Leu Asp Arg
   115 120 125
   Asp Ile Lys Phe Asp Ser Tyr Leu Asp Thr His Leu Thr Ala Gln Gln
   130 135 140
   Val Ser Ser Lys Glu Arg Val Ile Leu Lys Val Thr Val Pro Ser Gly
   145 150 155 160
   Lys Gly Ser Thr Thr Pro Thr Lys Ala Gly Val Ile Leu Asn Asn Ser
   165 170 175
   Glu Tyr Lys Met Leu Ile Asp Asn Gly Tyr Met Val His Val Asp Lys
   180 185 190
   Val Ser Lys Val Val Lys Lys Gly Val Glu Cys Leu Gln Ile Glu Gly
   195 200 205
   Thr Leu Lys Lys Ser Leu Asp Phe Lys Asn Asp Ile Asn Ala Glu Ala
   210 215 220
   His Ser Trp Gly Met Lys Asn Tyr Glu Glu Trp Ala Lys Asp Leu Thr
   225 230 235 240
   Asp Ser Gln Arg Glu Ala Leu Asp Gly Tyr Ala Arg Gln Asp Tyr Lys
   245 250 255
   Glu Ile Asn Asn Tyr Leu Arg Asn Gln Gly Gly Ser Gly Asn Glu Lys
   260 265 270
   Leu Asp Ala Gln Ile Lys Asn Ile Ser Asp Ala Leu Gly Lys Lys Pro
   275 280 285
   Ile Pro Glu Asn Ile Thr Val Tyr Arg Trp Cys Gly Met Pro Glu Phe
   290 295 300
   Gly Tyr Gln Ile Ser Asp Pro Leu Pro Ser Leu Lys Asp Phe Glu Glu
   305 310 315 320
   Gln Phe Leu Asn Thr Ile Lys Glu Asp Lys Gly Tyr Met Ser Thr Ser
   325 330 335
   Leu Ser Ser Glu Arg Leu Ala Ala Phe Gly Ser Arg Lys Ile Ile Leu
   340 345 350
   Arg Leu Gln Val Pro Lys Gly Ser Thr Gly Ala Tyr Leu Ser Ala Ile
   355 360 365
   Gly Gly Phe Ala Ser Glu Lys Glu Ile Leu Leu Asp Lys Asp Ser Lys
   370 375 380
   Tyr His Ile Asp Lys Val Thr Glu Val Ile Ile Lys Gly Val Lys Arg
   385 390 395 400
   Tyr Val Val Asp Ala Thr Leu Leu Thr Asn
   405 410
(2) INFORMATION FOR SEQ ID NO:41:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = ''oligonucleotide encoding eukaryotic secretion signal used to construct pCIB5527"
   (iii) HYPOTHETICAL: NO
   (xi) SEQUENCE DESCRIPTION: SEQ ID NO:41:
   GGATCCACCA TGGGCTGGAG CTGGATCTTC CTGTTCCTGC TGAGCGGCGC CGCGGGCGTG 60
   CACTGCCTGC AG 72
(2) INFORMATION FOR SEQ ID NO:42:
   (i) SEQUENCE CHARACTERISTICS:
      (A) LENGTH: 1241 base pairs
      (B) TYPE: nucleic acid
      (C) STRANDEDNESS: single
      (D) TOPOLOSY: linear
   (ii), MOLECULE TYPE: other nucleic acid
      (A) DESCRIPTION: /desc = "Synthetic DNA"
   (iii) HYPOTHETICAL: NO
   (ix) FEATURE:
      (A) NAME/KEY: CDS
      (B) LOCATION: 9..1238
      (D) OTHER INFORMATION: /note= "Maize optimized DNA sequence encoding VIP2A(a) with the Bacillus secretion signal removed and the eukaryotic secretion signal inserted as contained in pCIB5528"
   (xi) SEQUENCE DESCRIPTION: SEQ ID NO:42:
   GATCCACC ATG CTG CAG AAC CTG AAG ATC ACC GAC AAG GTG GAG GAC TTC      50
            Met Leu Gln Asn Leu Lys Ile Thr Asp Lys Val Glu Asp Phe
                            415                 420
   AAG GAG GAC AAG GAG AAG GCC AAG GAG TGG GGC AAG GAG AAG GAG AAG       98
   Lys Glu Asp Lys Glu Lys Ala Lys Glu Trp Gly Lys Glu Lys Glu Lys
   425                 430                 435                 440
   GAG TGG AAG CTT ACC GCC ACC GAG AAG GGC AAG ATG AAC AAC TTC CTG      146
   Glu Trp Lys Leu Thr Ala Thr Glu Lys Gly Lys Met Asn Asn Phe Leu
                   445                 450                 455
   GAC AAC AAG AAC GAC ATC AAG ACC AAC TAC AAG GAG ATC ACC TTC AGC      194
   Asp Asn Lys Asn Asp Ile Lys Thr Asn Tyr Lys Glu Ile Thr Phe Ser
               460                 465                 470
   ATA GCC GGC AGC TTC GAG GAC GAG ATC AAG GAC CTG AAG GAG ATC GAC      242
   Ile Ala Gly Ser Phe Glu Asp Glu Ile Lys Asp Leu Lys Glu Ile Asp
           475                 480                 485
   AAG ATG TTC GAC AAG ACC AAC CTG AGC AAC AGC ATC ATC ACC TAC AAG      290
   Lys Met Phe Asp Lys Thr Asn Leu Ser Asn Ser Ile Ile Thr Tyr Lys
       490                 495                 500
   AAC GTG GAG CCC ACC ACC ATC GGC TTC AAC AAG AGC CTG ACC GAG GGC      338
   Asn Val Glu Pro Thr Thr Ile Gly Phe Asn Lys Ser Leu Thr Glu Gly
   505                 510                 515                 520
   AAC ACC ATC AAC AGC GAC GCC ATG GCC CAG TTC AAG GAG CAG TTC CTG      386
   Asn Thr Ile Asn Ser Asp Ala Met Ala Gln Phe Lys Glu Gln Phe Leu
                   525                 530                 535
   GAC CGC GAC ATC AAG TTC GAC AGC TAC CTG GAC ACC CAC CTG ACC GCC      434
   Asp Arg Asp Ile Lys Phe Asp Ser Tyr Leu Asp Thr His Leu Thr Ala
               540                 545                 550
   CAG CAG GTG AGC AGC AAG GAG CGC GTG ATC CTG AAG GTG ACC GTC CCC      482
   Gln Gln Val Ser Ser Lys Glu Arg Val Ile Leu Lys Val Thr Val Pro
           555                 560                 565
   AGC GGC AAG GGC AGC ACC ACC CCC ACC AAG GCC GGC GTG ATC CTG AAC      530
   Ser Gly Lys Gly Ser Thr Thr Pro Thr Lys Ala Gly Val Ile Leu Asn
       570                 575                 580
   AAC AGC GAG TAC AAG ATG CTG ATC GAC AAC GGC TAC ATG GTG CAC GTG      578
   Asn Ser Glu Tyr Lys Met Leu Ile Asp Asn Gly Tyr Met Val His Val
   585                 590                 595                 600
   GAC AAG GTG AGC AAG GTG GTG AAG AAG GGC GTG GAG TGC CTC CAG ATC      626
   Asp Lys Val Ser Lys Val Val Lys Lys Gly Val Glu Cys Leu Gln Ile
                     605                 610                 615
   GAG GGC ACC CTG AAG AAG AGT CTA GAC TTC AAG AAC GAC ATC AAC GCC       674
   Glu Gly Thr Leu Lys Lys Ser Leu Asp Phe Lys Asn Asp Ile Asn Ala
               620                 625                 630
   GAG GCC CAC AGC TGG GGC ATG AAG AAC TAC GAG GAG TGG GCC AAG GAC       722
   Glu Ala His Ser Trp Gly Met Lys Asn Tyr Glu Glu Trp Ala Lys Asp
           635                 640                 645
   CTG ACC GAC AGC CAG CGC GAG GCC CTG GAC GGC TAC GCC CGC CAG GAC       770
   Leu Thr Asp Ser Gln Arg Glu Ala Leu Asp Gly Tyr Ala Arg Gln Asp
       650                 655                 660
   TAC AAG GAG ATC AAC AAC TAC CTG CGC AAC CAG GGC GGC AGC GGC AAC       818
   Tyr Lys Glu Ile Asn Asn Tyr Leu Arg Asn Gln Gly Gly Ser Gly Asn
   665                 670                 675                 680
   GAG AAG CTG GAC GCC CAG ATC AAG AAC ATC AGC GAC GCC CTG GGC AAG       866
   Glu Lys Leu Asp Ala Gln Ile Lys Asn Ile Ser Asp Ala Leu Gly Lys
                   685                 690                 695
   AAG CCC ATC CCC GAG AAC ATC ACC GTG TAC CGC TGG TGC GGC ATG CCC       914
   Lys Pro Ile Pro Glu Asn Ile Thr Val Tyr Arg Trp Cys Gly Met Pro
               700                 705                 710
   GAG TTC GGC TAC CAG ATC AGC GAC CCC CTG CCC AGC CTG AAG GAC TTC       962
   Glu Phe Gly Tyr Gln Ile Ser Asp Pro Leu Pro Ser Leu Lys Asp Phe
           715                 720                 725
   GAG GAG CAG TTC CTG AAC ACC ATC AAG GAG GAC AAG GGC TAC ATG AGC      1010
   Glu Glu Gln Phe Leu Asn Thr Ile Lys Glu Asp Lys Gly Tyr Met Ser
       730                 735                 740
   ACC AGC CTG AGC AGC GAG CGC CTG GCC GCC TTC GGC AGC CGC AAG ATC      1058
   Thr Ser Leu Ser Ser Glu Arg Leu Ala Ala Phe Gly Ser Arg Lys Ile
   745                 750                 755                 760
   ATC CTG CGC CTG CAG GTG CCC AAG GGC AGC ACT GGT GCC TAC CTG AGC      1106
   Ile Leu Arg Leu Gln Val Pro Lys Gly Ser Thr Gly Ala Tyr Leu Ser
                   765                 770                 775
   GCC ATC GGC GGC TTC GCC AGC GAG AAG GAG ATC CTG CTG GAT AAG GAC      1154
   Ala Ile Gly Gly Phe Ala Ser Glu Lys Glu Ile Leu Leu Asp Lys Asp
               780                 785                 790
   AGC AAG TAC CAC ATC GAC AAG GTG ACC GAG GTG ATC ATC AAG GGC GTG      1202
   Ser Lys Tyr His Ile Asp Lys Val Thr Glu Val Ile Ile Lys Gly Val
           795                 800                 805
   AAG CGC TAC GTG GTG GAC GCC ACC CTG CTG ACC AAC TAG                  1241
   Lys Arg Tyr Val Val Asp Ala Thr Leu Leu Thr Asn
       810                 815                 820
(2) INFORMATION FOR SEQ ID NO:43:
   (i) SEQUENCE CHARACTERISTICS:
      (A) LENGTH: 410 amino acids
      (B) TYPE: amino acid
      (D) TOPOLOGY: linear
   (ii) MOLECULE TYPE: protein
   (xi) SEQUENCE DESCRIPTION: SEQ ID NO:43:
   Met Leu Gln Asn Leu Lys Ile Thr Asp Lys Val Glu Asp Phe Lys Glu
    1               5                  10                  15
   Asp Lys Glu Lys Ala Lys Glu Trp Gly Lys Glu Lys Glu Lys Glu Trp
               20                  25                  30
   Lys Leu Thr Ala Thr Glu Lys Gly Lys Met Asn Asn Phe Leu Asp Asn
           35                  40                  45
   Lys Asn Asp Ile Lys Thr Asn Tyr Lys Glu Ile Thr Phe Ser Ile Ala
       50                  55                  60
   Gly Ser Phe Glu Asp Glu Ile Lys Asp Leu Lys Glu Ile Asp Lys Met
   65                  70                  75                  80
   Phe Asp Lys Thr Asn Leu Ser Asn Ser Ile Ile Thr Tyr Lys Asn Val
                   85                  90                  95
   Glu Pro Thr Thr Ile Gly Phe Asn Lys Ser Leu Thr Glu Gly Asn Thr
               100                 105                 110
   Ile Asn Ser Asp Ala Met Ala Gln Phe Lys Glu Gln Phe Leu Asp Arg
           115                 120                 125
   Asp Ile Lys Phe Asp Ser Tyr Leu Asp Thr His Leu Thr Ala Gln Gln
       130                 135                 140
   Val Ser Ser Lys Glu Arg Val Ile Leu Lys Val Thr Val Pro Ser Gly
   145                 150                 155                 160
   Lys Gly Ser Thr Thr Pro Thr Lys Ala Gly Val Ile Leu Asn Asn Ser
                   165                 170                 175
   Glu Tyr Lys Met Leu Ile Asp Asn Gly Tyr Met Val His Val Asp Lys
               180                 185                 190
   Val Ser Lys Val Val Lys Lys Gly Val Glu Cys Leu Gln Ile Glu Gly
           195                 200                 205
   Thr Leu Lys Lys Ser Leu Asp Phe Lys Asn Asp Ile Asn Ala Glu Ala
       210                 215                 220
   His Ser Trp Gly Met Lys Asn Tyr Glu Glu Trp Ala Lys Asp Leu Thr
   225                 230                 235                 240
   Asp Ser Gln Arg Glu Ala Leu Asp Gly Tyr Ala Arg Gln Asp Tyr Lys
                   245                 250                 255
   Glu Ile Asn Asn Tyr Leu Arg Asn Gln Gly Gly Ser Gly Asn Glu Lys
               260                 265                 270
   Leu Asp Ala Gln Ile Lys Asn Ile Ser Asp Ala Leu Gly Lys Lys Pro
           275                 280                 285
   Ile Pro Glu Asn Ile Thr Val Tyr Arg Trp Cys Gly Met Pro Glu Phe
       290                 295                 300
   Gly Tyr Gln Ile Ser Asp Pro Leu Pro Ser Leu Lys Asp Phe Glu Glu
   305                 310                 315                 320
   Gln Phe Leu Asn Thr Ile Lys Glu Asp Lys Gly Tyr Met Ser Thr Ser
                   325                 330                 335
   Leu Ser Ser Glu Arg Leu Ala Ala Phe Gly Ser Arg Lys Ile Ile Leu
               340                 345                 350
   Arg Leu Gln Val Pro Lys Gly Ser Thr Gly Ala Tyr Leu Ser Ala Ile
           355                 360                 365
   Gly Gly Phe Ala Ser Glu Lys Glu Ile Leu Leu Asp Lys Asp Ser Lys
       370                 375                 380
   Tyr His Ile Asp Lys Val Thr Glu Val Ile Ile Lys Gly Val Lys Arg
   385                 390                 395                 400
   Tyr Val Val Asp Ala Thr Leu Leu Thr Asn
                   405                 410
(2) INFORMATION FOR SEQ ID NO:44:
   (i) SEQUENCE CHARACTERISTICS:
      (A) LENGTH: 86 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide encoding vacuolar targetting peptide used to construct pCIB5533"
   (iii) HYPOTHETICAL: NO
   (xi) SEQUENCE DESCRIPTICN: SEQ ID NO:44:
   CCGCGGGCGT GCACTGCCTC AGCAGCAGCA GCTTCGCCGA CAGCAACCCC ATCCGCGTGA        60
   CCGACCGCGC CGCCAGCACC CTGCAG 86
(2) INFORMATION FOR SEQ ID NO:45:
   (i) SEQUENCE CHARACTERISTICS:
      (A) LENGTH: 1358 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Synthetic DNA"
   (iii) HYPOTHETICAL: NO
   (ix) FEATURE:
      (A) NAME/KEY: CDS
      (B) LOCATION: 9..1355
      (D) OTHER INFORMATION: /note= "Maize optimized VIP2A(a) with the Bacillus secretion signal removed and the vacuolar targetting signal inserted as contained in pCIB5533"
   (xi) SEQUENCE DESCRIPTION: SEQ ID NO:45:
   GATCCACC ATG GGC TGG AGC TGG ATC TTC CTG TTC CTG CTG AGC GGC GCC      50
            Met Gly Trp Ser Trp Ile Phe Leu Phe Leu Leu Ser Gly Ala
                            415                 420
   GCG GGC GTG CAC TGC CTC AGC AGC AGC AGC TTC GCC GAC AGC AAC CCC       98
   Ala Gly Val His Cys Leu Ser Ser Ser Ser Phe Ala Asp Ser Asn Pro
   425                 430                 435                 440
   ATC CGC GTG ACC GAC CGC GCC GCC AGC ACC CTG CAG AAC CTG AAG ATC      146
   Ile Arg Val Thr Asp Arg Ala Ala Ser Thr Leu Gln Asn Leu Lys Ile
                   445                 450                 455
   ACC GAC AAG GTG GAG GAC TTC AAG GAG GAC AAG GAG AAG GCC AAG GAG      194
   Thr Asp Lys Val Glu Asp Phe Lys Glu Asp Lys Glu Lys Ala Lys Glu
               460                 465                 470
   TGG GGC AAG GAG AAG GAG AAG GAG TGG AAG CTT ACC GCC ACC GAG AAG      242
   Trp Gly Lys Glu Lys Glu Lys Glu Trp Lys Leu Thr Ala Thr Glu Lys
           475                 480                 485
   GGC AAG ATG AAC AAC TTC CTG GAC AAC AAG AAC GAC ATC AAG ACC AAC      290
   Gly Lys Met Asn Asn Phe Leu Asp Asn Lys Asn Asp Ile Lys Thr Asn
       490                 495                 500
   TAC AAG GAG ATC ACC TTC AGC ATA GCC GGC AGC TTC GAG GAC GAG ATC      338
   Tyr Lys Glu Ile Thr Phe Ser Ile Ala Gly Ser Phe Glu Asp Glu Ile
   505                 510                 515                 520
   AAG GAC CTG AAG GAG ATC GAC AAG ATG TTC GAC AAG ACC AAC CTG AGC      386
   Lys Asp Leu Lys Glu Ile Asp Lys Met Phe Asp Lys Thr Asn Leu Ser
   525 530 535
   AAC AGC ATC ATC ACC TAC AAG AAC GTG GAG CCC ACC ACC ATC GGC TTC 434
   Asn Ser Ile Ile Thr Tyr Lys Asn Val Glu Pro Thr Thr Ile Gly Phe
   540 545 550
   AAC AAG AGC CTG ACC GAG GGC AAC ACC ATC AAC AGC GAC GCC ATG GCC 482
   Asn Lys Ser Leu Thr Glu Gly Asn Thr Ile Asn Ser Asp Ala Met Ala
   555 560 565
   CAG TTC AAG GAG CAG TTC CTG GAC CGC GAC ATC AAG TTC GAC AGC TAC 530
   Gln Phe Lys Glu Gln Phe Leu Asp Arg Asp Ile Lys Phe Asp Ser Tyr
   570 575 580
   CTG GAC ACC CAC CTG ACC GCC CAG CAG GTG AGC AGC AAG GAG CGC GTG 578
   Leu Asp Thr His Leu Thr Ala Gln Gln Val Ser Ser Lys Glu Arg Val
   585 590 595 600
   ATC CTG AAG GTG ACC GTC CCC AGC GGC AAG GGC AGC ACC ACC CCC ACC 626
   Ile Leu Lys Val Thr Val Pro Ser Gly Lys Gly Ser Thr Thr Pro Thr
   605 610 615
   AAG GCC GGC GTG ATC CTG AAC AAC AGC GAG TAC AAG ATG CTG ATC GAC 674
   Lys Ala Gly Val Ile Leu Asn Asn Ser Glu Tyr Lys Met Leu Ile Asp
   620 625 630
   AAC GGC TAC ATG GTG CAC GTG GAC AAG GTG AGC AAG GTG GTG AAG AAG 722
   Asn Gly Tyr Met Val His Val Asp Lys Val Ser Lys Val Val Lys Lys
   635 640 645
   GGC GTG GAG TGC CTC CAG ATC GAG GGC ACC CTG AAG AAG AGT CTA GAC 770
   Gly Val Glu Cys Leu Gln Ile Glu Gly Thr Leu Lys Lys Ser Leu Asp
   650 655 660
   TTC AAG AAC GAC ATC AAC GCC GAG GCC CAC AGC TGG GGC ATG AAG AAC 818
   Phe Lys Asn Asp Ile Asn Ala Glu Ala His Ser Trp Gly Met Lys Asn
   665 670 675 680
   TAC GAG GAG TGG GCC AAG GAC CTG ACC GAC AGC CAG CGC GAG GCC CTG 866
   Tyr Glu Glu Trp Ala Lys Asp Leu Thr Asp Ser Gln Arg Glu Ala Leu
   685 690 695
   GAC GGC TAC GCC CGC CAG GAC TAC AAG GAG ATC AAC AAC TAC CTG CGC 914
   Asp Gly Tyr Ala Arg Gln Asp Tyr Lys Glu Ile Asn Asn Tyr Leu Arg
   700 705 710
   AAC CAG GGC GGC AGC GGC AAC GAG AAG CTG GAC GCC CAG ATC AAG AAC 962
   Asn Gln Gly Gly Ser Gly Asn Glu Lys Leu Asp Ala Gln Ile Lys Asn
   715 720 725
   ATC AGC GAC GCC CTG GGC AAG AAG CCC ATC CCC GAG AAC ATC ACC GTG 1010
   Ile Ser Asp Ala Leu Gly Lys Lys Pro Ile Pro Glu Asn Ile Thr Val
   730 735 740
   TAC CGC TGG TGC GGC ATG CCC GAG TTC GGC TAC CAG ATC AGC GAC CCC      1058
   Tyr Arg Trp Cys Gly Met Pro Glu Phe Gly Tyr Gln Ile Ser Asp Pro
   745                 750                 755                 760
   CTG CCC AGC CTG AAG GAC TTC GAG GAG CAG TTC CTG AAC ACC ATC AAG      1106
   Leu Pro Ser Leu Lys Asp Phe Glu Glu Gln Phe Leu Asn Thr Ile Lys
                   765                 770                 775
   GAG GAC AAG GGC TAC ATG AGC ACC AGC CTG AGC AGC GAG CGC CTG GCC      1154
   Glu Asp Lys Gly Tyr Met Ser Thr Ser Leu Ser Ser Glu Arg Leu Ala
               780                 785                 790
   GCC TTC GGC AGC CGC AAG ATC ATC CTG CGC CTG CAG GTG CCC AAG GGC      1202
   Ala Phe Gly Ser Arg Lys Ile Ile Leu Arg Leu Gln Val Pro Lys Gly
           795                 800                 805
   AGC ACT GGT GCC TAC CTG AGC GCC ATC GGC GGC TTC GCC AGC GAG AAG      1250
   Ser Thr Gly Ala Tyr Leu Ser Ala Ile Gly Gly Phe Ala Ser Glu Lys
       810                 815                 820
   GAG ATC CTG CTG GAT AAG GAC AGC AAG TAC CAC ATC GAC AAG GTG ACC      1298
   Glu Ile Leu Leu Asp Lys Asp Ser Lys Tyr His Ile Asp Lys Val Thr
   825                 830                 835                 840
   GAG GTG ATC ATC AAG GGC GTG AAG CGC TAC GTG GTG GAC GCC ACC CTG      1346
   Glu Val Ile Ile Lys Gly Val Lys Arg Tyr Val Val Asp Ala Thr Leu
                   845                 850                 855
   CTG ACC AAC TAG                                                      1358
   Leu Thr Asn
(2) INFORMATION FOR SEQ ID NO:46:
   (i) SEQUENCE CHARACTERISTICS:
      (A) LENGTH: 449 amino acids
      (B) TYPE: amino acid
      (D) TOPOLOGY: linear
   (ii) MOLECULE TYPE: protein
   (xi) SEQUENCE DESCRIPTION: SEQ ID NO:46:
   Met Gly Trp Ser Trp Ile Phe Leu Phe Leu Leu Ser Gly Ala Ala Gly
    1               5                  10                  15
   Val His Cys Leu Ser Ser Ser Ser Phe Ala Asp Ser Asn Pro Ile Arg
               20                  25                  30
   Val Thr Asp Arg Ala Ala Ser Thr Leu Gln Asn Leu Lys Ile Thr Asp
           35                  40                  45
   Lys Val Glu Asp Phe Lys Glu Asp Lys Glu Lys Ala Lys Glu Trp Gly
       50                  55                  60
   Lys Glu Lys Glu Lys Glu Trp Lys Leu Thr Ala Thr Glu Lys Gly Lys
   65 70 75 80
   Met Asn Asn Phe Leu Asp Asn Lys Asn Asp Ile Lys Thr Asn Tyr Lys
   85 90 95
   Glu Ile Thr Phe Ser Ile Ala Gly Ser Phe Glu Asp Glu Ile Lys Asp
   100 105 110
   Leu Lys Glu Ile Asp Lys Met Phe Asp Lys Thr Asn Leu Ser Asn Ser
   115 120 125
   Ile Ile Thr Tyr Lys Asn Val Glu Pro Thr Thr Ile Gly Phe Asn Lys
   130 135 140
   Ser Leu Thr Glu Gly Asn Thr Ile Asn Ser Asp Ala Met Ala Gln Phe
   145 150 155 160
   Lys Glu Gln Phe Leu Asp Arg Asp Ile Lys Phe Asp Ser Tyr Leu Asp
   165 170 175
   Thr His Leu Thr Ala Gln Gln Val Ser Ser Lys Glu Arg Val Ile Leu
   180 185 190
   Lys Val Thr Val Pro Ser Gly Lys Gly Ser Thr Thr Pro Thr Lys Ala
   195 200 205
   Gly Val Ile Leu Asn Asn Ser Glu Tyr Lys Met Leu Ile Asp Asn Gly
   210 215 220
   Tyr Met Val His Val Asp Lys Val Ser Lys Val Val Lys Lys Gly Val
   225 230 235 240
   Glu Cys Leu Gln Ile Glu Gly Thr Leu Lys Lys Ser Leu Asp Phe Lys
   245 250 255
   Asn Asp Ile Asn Ala Glu Ala His Ser Trp Gly Met Lys Asn Tyr Glu
   260 265 270
   Glu Trp Ala Lys Asp Leu Thr Asp Ser Gln Arg Glu Ala Leu Asp Gly
   275 280 285
   Tyr Ala Arg Gln Asp Tyr Lys Glu Ile Asn Asn Tyr Leu Arg Asn Gln
   290 295 300
   Gly Gly Ser Gly Asn Glu Lys Leu Asp Ala Gln Ile Lys Asn Ile Ser
   305 310 315 320
   Asp Ala Leu Gly Lys Lys Pro Ile Pro Glu Asn Ile Thr Val Tyr Arg
   325 330 335
   Trp Cys Gly Met Pro Glu Phe Gly Tyr Gln Ile Ser Asp Pro Leu Pro
   340 345 350
   Ser Leu Lys Asp Phe Glu Glu Gln Phe Leu Asn Thr Ile Lys Glu Asp
   355 360 365
   Lys Gly Tyr Met Ser Thr Ser Leu Ser Ser Glu Arg Leu Ala Ala Phe
   370 375 380
   Gly Ser Arg Lys Ile Ile Leu Arg Leu Gln Val Pro Lys Gly Ser Thr
   385 390 395 400
   Gly Ala Tyr Leu Ser Ala Ile Gly Gly Phe Ala Ser Glu Lys Glu Ile
   405 410 415
   Leu Leu Asp Lys Asp Ser Lys Tyr His Ile Asp Lys Val Thr Glu Val
   420 425 430
   Ile Ile Lys Gly Val Lys Arg Tyr Val Val Asp Ala Thr Leu Leu Thr
   435 440 445
   Asn
(2) INFORMATION FOR SEQ ID NO:47:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (ix) FEATURE:
      (A) NAME/KEY: Peptide
      (B) LOCATION: 1..16
      (D) OTHER INFORMATION: /note= "linker peptide for fusion of VIP1A(a) and VIP2A(a) used to construct pCIB5533"
   (xi) SEQUENCE DESCRIPTION: SEQ ID NO:47:
   Pro Ser Thr Pro Pro Thr Pro Ser Pro Ser Thr Pro Pro Thr Pro Ser
   1 5 10 15
(2) INFORMATION FOR SEQ ID NO:48:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "DNA encoding linker peptide used to construct pCIB5533"
   (iii) HYPOTHETICAL: NO
   (xi) SEQUENCE DESCRIPTION: SEQ ID NO:48:
   CCCGGGCCTT CTACTCCCCC AACTCCCTCT CCTAGCACGC CTCCGACACC TAGCGATATC    60
   GGATCC                                                               66
(2) INFORMATION FOR SEQ ID NO:49:
   (i) SEQUENCE CHARACTERISTICS:
      (A) LENGTH: 4031 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Synthetic DNA"
   (iii) HYPOTHETICAL: NO
   (ix) FEATURE:
      (A) NAME/KEY: CDS
      (B) LOCATION: 6..4019
      (D) OTHER INFORMATION: /note= "Maize optimized DNA sequence encoding a VIP2A(a) - VIP1A(a) fusion protein as contained in pCIB5531"
   (xi) SEQUENCE DESCRIPTION: SEQ ID NO:49:
   GATCC ATG AAG CGC ATG GAG GGC AAG CTG TTC ATG GTG AGC AAG AAG          47
         Met Lys Arg Met Glu Gly Lys Leu Phe Met Val Ser Lys Lys
         450                 455                 460
   CTC CAG GTG GTG ACC AAG ACC GTG CTG CTG AGC ACC GTG TTC AGC ATC        95
   Leu Gln Val Val Thr Lys Thr Val Leu Leu Ser Thr Val Phe Ser Ile
       465                 470                 475
   AGC CTG CTG AAC AAC GAG GTG ATC AAG GCC GAG CAG CTG AAC ATC AAC       143
   Ser Leu Leu Asn Asn Glu Val Ile Lys Ala Glu Gln Leu Asn Ile Asn
   480                 485                 490                 495
   AGC CAG AGC AAG TAC ACC AAC CTC CAG AAC CTG AAG ATC ACC GAC AAG       191
   Ser Gln Ser Lys Tyr Thr Asn Leu Gln Asn Leu Lys Ile Thr Asp Lys
                   500                 505                 510
   GTG GAG GAC TTC AAG GAG GAC AAG GAG AAG GCC AAG GAG TGG GGC AAG       239
   Val Glu Asp Phe Lys Glu Asp Lys Glu Lys Ala Lys Glu Trp Gly Lys
   515 520 525
   GAG AAG GAG AAG GAG TGG AAG CTT ACC GCC ACC GAG AAG GGC AAG ATG 287
   Glu Lys Glu Lys Glu Trp Lys Leu Thr Ala Thr Glu Lys Gly Lys Met
   530 535 540
   AAC AAC TTC CTG GAC AAC AAG AAC GAC ATC AAG ACC AAC TAC AAG GAG 335
   Asn Asn Phe Leu Asp Asn Lys Asn Asp Ile Lys Thr Asn Tyr Lys Glu
   545 550 555
   ATC ACC TTC AGC ATA GCC GGC AGC TTC GAG GAC GAG ATC AAG GAC CTG 383
   Ile Thr Phe Ser Ile Ala Gly Ser Phe Glu Asp Glu Ile Lys Asp Leu
   560 565 570 575
   AAG GAG ATC GAC AAG ATG TTC GAC AAG ACC AAC CTG AGC AAC AGC ATC 431
   Lys Glu Ile Asp Lys Met Phe Asp Lys Thr Asn Leu Ser Asn Ser Ile
   580 585 590
   ATC ACC TAC AAG AAC GTG GAG CCC ACC ACC ATC GGC TTC AAC AAG AGC 479
   Ile Thr Tyr Lys Asn Val Glu Pro Thr Thr Ile Gly Phe Asn Lys Ser
   595 600 605
   CTG ACC GAG GGC AAC ACC ATC AAC AGC GAC GCC ATG GCC CAG TTC AAG 527
   Leu Thr Glu Gly Asn Thr Ile Asn Ser Asp Ala Met Ala Gln Phe Lys
   610 615 620
   GAG CAG TTC CTG GAC CGC GAC ATC AAG TTC GAC AGC TAC CTG GAC ACC 575
   Glu Gln Phe Leu Asp Arg Asp Ile Lys Phe Asp Ser Tyr Leu Asp Thr
   625 630 635
   CAC CTG ACC GCC CAG CAG GTG AGC AGC AAG GAG CGC GTG ATC CTG AAG 623
   His Leu Thr Ala Gln Gln Val Ser Ser Lys Glu Arg Val Ile Leu Lys
   640 645 650 655
   GTG ACC GTC CCC AGC GGC AAG GGC AGC ACC ACC CCC ACC AAG GCC GGC 671
   Val Thr Val Pro Ser Gly Lys Gly Ser Thr Thr Pro Thr Lys Ala Gly
   660 665 670
   GTG ATC CTG AAC AAC AGC GAG TAC AAG ATG CTG ATC GAC AAC GGC TAC 719
   Val Ile Leu Asn Asn Ser Glu Tyr Lys Met Leu Ile Asp Asn Gly Tyr
   675 680 685
   ATG GTG CAC GTG GAC AAG GTG AGC AAG GTG GTG AAG AAG GGC GTG GAG 767
   Met Val His Val Asp Lys Val Ser Lys Val Val Lys Lys Gly Val Glu
   690 695 700
   TGC CTC CAG ATC GAG GGC ACC CTG AAG AAG AGT CTA GAC TTC AAG AAC 815
   Cys Leu Gln Ile Glu Gly Thr Leu Lys Lys Ser Leu Asp Phe Lys Asn
   705 710 715
   GAC ATC AAC GCC GAG GCC CAC AGC TGG GGC ATG AAG AAC TAC GAG GAG 863
   Asp Ile Asn Ala Glu Ala His Ser Trp Gly Met Lys Asn Tyr Glu Glu
   720 725 730 735
   TGG GCC AAG GAC CTG ACC GAC AGC CAG CGC GAG GCC CTG GAC GGC TAC    911
   Trp Ala Lys Asp Leu Thr Asp Ser Gln Arg Glu Ala Leu Asp Gly Tyr
                   740                 745                 750
   GCC CGC CAG GAC TAC AAG GAG ATC AAC AAC TAC CTG CGC AAC CAG GGC    959
   Ala Arg Gln Asp Tyr Lys Glu Ile Asn Asn Tyr Leu Arg Asn Gln Gly
               755                 760                 765
   GGC AGC GGC AAC GAG AAG CTG GAC GCC CAG ATC AAG AAC ATC AGC GAC   1007
   Gly Ser Gly Asn Glu Lys Leu Asp Ala Gln Ile Lys Asn Ile Ser Asp
           770                 775                 780
   GCC CTG GGC AAG AAG CCC ATC CCC GAG AAC ATC ACC GTG TAC CGC TGG   1055
   Ala Leu Gly Lys Lys Pro Ile Pro Glu Asn Ile Thr Val Tyr Arg Trp
       785                 790                 795
   TGC GGC ATG CCC GAG TTC GGC TAC CAG ATC AGC GAC CCC CTG CCC AGC   1103
   Cys Gly Met Pro Glu Phe Gly Tyr Gln Ile Ser Asp Pro Leu Pro Ser
   800                 805                 810                 815
   CTG AAG GAC TTC GAG GAG CAG TTC CTG AAC ACC ATC AAG GAG GAC AAG   1151
   Leu Lys Asp Phe Glu Glu Gln Phe Leu Asn Thr Ile Lys Glu Asp Lys
                   820                 825                 830
   GGC TAC ATG AGC ACC AGC CTG AGC AGC GAG CGC CTG GCC GCC TTC GGC   1199
   Gly Tyr Met Ser Thr Ser Leu Ser Ser Glu Arg Leu Ala Ala Phe Gly
               835                 840                 845
   AGC CGC AAG ATC ATC CTG CGC CTG CAG GTG CCC AAG GGC AGC ACT GGT   1247
   Ser Arg Lys Ile Ile Leu Arg Leu Gln Val Pro Lys Gly Ser Thr Gly
           850                 855                 860
   GCC TAC CTG AGC GCC ATC GGC GGC TTC GCC AGC GAG AAG GAG ATC CTG   1295
   Ala Tyr Leu Ser Ala Ile Gly Gly Phe Ala Ser Glu Lys Glu Ile Leu
       865                 870                 875
   CTG GAT AAG GAC AGC AAG TAC CAC ATC GAC AAG GTG ACC GAG GTG ATC   1343
   Leu Asp Lys Asp Ser Lys Tyr His Ile Asp Lys Val Thr Glu Val Ile
   880                 885                 890                 895
   ATC AAG GGC GTG AAG CGC TAC GTG GTG GAC GCC ACC CTG CTG ACC AAC   1391
   Ile Lys Gly Val Lys Arg Tyr Val Val Asp Ala Thr Leu Leu Thr Asn
                   900                 905                 910
   TCC CGG GGG CCT TCT ACT CCC CCA ACT CCC TCT CCT AGC ACG CCT CCG   1439
   Ser Arg Gly Pro Ser Thr Pro Pro Thr Pro Ser Pro Ser Thr Pro Pro
               915                 920                 925
   ACA CCT AGC GAT ATC GGA TCC ACC ATG AAG ACC AAC CAG ATC AGC ACC   1487
   Thr Pro Ser Asp Ile Gly Ser Thr Met Lys Thr Asn Gln Ile Ser Thr
           930                 935                 940
   ACC CAG AAG AAC CAG CAG AAG GAG ATG GAC CGC AAG GGC CTG CTG GGC   1535
   Thr Gln Lys Asn Gln Gln Lys Glu Met Asp Arg Lys Gly Leu Leu Gly
       945                 950                 955
   TAC TAC TTC AAG GGC AAG GAC TTC AGC AAC CTG ACC ATG TTC GCC CCC 1583
   Tyr Tyr Phe Lys Gly Lys Asp Phe Ser Asn Leu Thr Met Phe Ala Pro
   960 965 970 975
   ACG CGT GAC AGC ACC CTG ATC TAC GAC CAG CAG ACC GCC AAC AAG CTG 1631
   Thr Arg Asp Ser Thr Leu Ile Tyr Asp Gln Gln Thr Ala Asn Lys Leu
   980 985 990
   CTG GAC AAG AAG CAG CAG GAG TAC CAG AGC ATC CGC TGG ATC GGC CTG 1679
   Leu Asp Lys Lys Gln Gln Glu Tyr Gln Ser Ile Arg Trp Ile Gly Leu
   995 1000 1005
   ATC CAG AGC AAG GAG ACC GGC GAC TTC ACC TTC AAC CTG AGC GAG GAC 1727
   Ile Gln Ser Lys Glu Thr Gly Asp Phe Thr Phe Asn Leu Ser Glu Asp
   1010 1015 1020
   GAG CAG GCC ATC ATC GAG ATC AAC GGC AAG ATC ATC AGC AAC AAG GGC 1775
   Glu Gln Ala Ile Ile Glu Ile Asn Gly Lys Ile Ile Ser Asn Lys Gly
   1025 1030 1035
   AAG GAG AAG CAG GTG GTG CAC CTG GAG AAG GGC AAG CTG GTG CCC ATC 1823
   Lys Glu Lys Gln Val Val His Leu Glu Lys Gly Lys Leu Val Pro Ile
   1040 1045 1050 1055
   AAG ATC GAG TAC CAG AGC GAC ACC AAG TTC AAC ATC GAC AGC AAG ACC 1871
   Lys Ile Glu Tyr Gln Ser Asp Thr Lys Phe Asn Ile Asp Ser Lys Thr
   1060 1065 1070
   TTC AAG GAG CTG AAG CTT TTC AAG ATC GAC AGC CAG AAC CAG CCC CAG 1919
   Phe Lys Glu Leu Lys Leu Phe Lys Ile Asp Ser Gln Asn Gln Pro Gln
   1075 1080 1085
   CAG GTG CAG CAG GAC GAG CTG CGC AAC CCC GAG TTC AAC AAG AAG GAG 1967
   Gln Val Gln Gln Asp Glu Leu Arg Asn Pro Glu Phe Asn Lys Lys Glu
   1090 1095 1100
   AGC CAG GAG TTC CTG GCC AAG CCC AGC AAG ATC AAC CTG TTC ACC CAG 2015
   Ser Gln Glu Phe Leu Ala Lys Pro Ser Lys Ile Asn Leu Phe Thr Gln
   1105 1110 1115
   CAG ATG AAG CGC GAG ATC GAC GAG GAC ACC GAC ACC GAC GGC GAC AGC 2063
   Gln Met Lys Arg Glu Ile Asp Glu Asp Thr Asp Thr Asp Gly Asp Ser
   1120 1125 1130 1135
   ATC CCC GAC CTG TGG GAG GAG AAC GGC TAC ACC ATC CAG AAC CGC ATC 2111
   Ile Pro Asp Leu Trp Glu Glu Asn Gly Tyr Thr Ile Gln Asn Arg Ile
   1140 1145 1150
   GCC GTG AAG TGG GAC GAC AGC CTG GCT AGC AAG GGC TAC ACC AAG TTC 2159
   Ala Val Lys Trp Asp Asp Ser Leu Ala Ser Lys Gly Tyr Thr Lys Phe
   1155 1160 1165
   GTG AGC AAC CCC CTG GAG AGC CAC ACC GTG GGC GAC CCC TAC ACC GAC 2207
   Val Ser Asn Pro Leu Glu Ser His Thr Val Gly Asp Pro Tyr Thr Asp
   1170 1175 1180
   TAC GAG AAG GCC GCC CGC GAC CTG GAC CTG AGC AAC GCC AAG GAG ACC 2255
   Tyr Glu Lys Ala Ala Arg Asp Leu Asp Leu Ser Asn Ala Lys Glu Thr
   1185 1190 1195
   TTC AAC CCC CTG GTG GCC GCC TTC CCC AGC GTG AAC GTG AGC ATG GAG 2303
   Phe Asn Pro Leu Val Ala Ala Phe Pro Ser Val Asn Val Ser Met Glu
   1200 1205 1210 1215
   AAG GTG ATC CTG AGC CCC AAC GAG AAC CTG AGC AAC AGC GTG GAG AGC 2351
   Lys Val Ile Leu Ser Pro Asn Glu Asn Leu Ser Asn Ser Val Glu Ser
   1220 1225 1230
   CAC TCG AGC ACC AAC TGG AGC TAC ACC AAC ACC GAG GGC GCC AGC GTG 2399
   His Ser Ser Thr Asn Trp Ser Tyr Thr Asn Thr Glu Gly Ala Ser Val
   1235 1240 1245
   GAG GCC GGC ATC GGT CCC AAG GGC ATC AGC TTC GGC GTG AGC GTG AAC 2447
   Glu Ala Gly Ile Gly Pro Lys Gly Ile Ser Phe Gly Val Ser Val Asn
   1250 1255 1260
   TAC CAG CAC AGC GAG ACC GTG GCC CAG GAG TGG GGC ACC AGC ACC GGC 2495
   Tyr Gln His Ser Glu Thr Val Ala Gln Glu Trp Gly Thr Ser Thr Gly
   1265 1270 1275
   AAC ACC AGC CAG TTC AAC ACC GCC AGC GCC GGC TAC CTG AAC GCC AAC 2543
   Asn Thr Ser Gln Phe Asn Thr Ala Ser Ala Gly Tyr Leu Asn Ala Asn
   1280 1285 1290 1295
   GTG CGC TAC AAC AAC GTG GGC ACC GGC GCC ATC TAC GAC GTG AAG CCC 2591
   Val Arg Tyr Asn Asn Val Gly Thr Gly Ala Ile Tyr Asp Val Lys Pro
   1300 1305 1310
   ACC ACC AGC TTC GTG CTG AAC AAC GAC ACC ATC GCC ACC ATC ACC GCC 2639
   Thr Thr Ser Phe Val Leu Asn Asn Asp Thr Ile Ala Thr Ile Thr Ala
   1315 1320 1325
   AAG TCG AAT TCC ACC GCC CTG AAC ATC AGC CCC GGC GAG AGC TAC CCC 2687
   Lys Ser Asn Ser Thr Ala Leu Asn Ile Ser Pro Gly Glu Ser Tyr Pro
   1330 1335 1340
   AAG AAG GGC CAG AAC GGC ATC GCC ATC ACC AGC ATG GAC GAC TTC AAC 2735
   Lys Lys Gly Gln Asn Gly Ile Ala Ile Thr Ser Met Asp Asp Phe Asn
   1345 1350 1355
   AGC CAC CCC ATC ACC CTG AAC AAG AAG CAG GTG GAC AAC CTG CTG AAC 2783
   Ser His Pro Ile Thr Leu Asn Lys Lys Gln Val Asp Asn Leu Leu Asn
   1360 1365 1370 1375
   AAC AAG CCC ATG ATG CTG GAG ACC AAC CAG ACC GAC GGC GTC TAC AAG 2831
   Asn Lys Pro Met Met Leu Glu Thr Asn Gln Thr Asp Gly Val Tyr Lys
   1380 1385 1390
   ATC AAG GAC ACC CAC GGC AAC ATC GTG ACG GGC GGC GAG TGG AAC GGC 2879
   Ile Lys Asp Thr His Gly Asn Ile Val Thr Gly Gly Glu Trp Asn Gly
   1395 1400 1405
   GTG ATC CAG CAG ATC AAG GCC AAG ACC GCC AGC ATC ATC GTC GAC GAC 2927
   Val Ile Gln Gln Ile Lys Ala Lys Thr Ala Ser Ile Ile Val Asp Asp
   1410 1415 1420
   GGC GAG CGC GTG GCC GAG AAG CGC GTG GCC GCC AAG GAC TAC GAG AAC 2975
   Gly Glu Arg Val Ala Glu Lys Arg Val Ala Ala Lys Asp Tyr Glu Asn
   1425 1430 1435
   CCC GAG GAC AAG ACC CCC AGC CTG ACC CTG AAG GAC GCC CTG AAG CTG 3023
   Pro Glu Asp Lys Thr Pro Ser Leu Thr Leu Lys Asp Ala Leu Lys Leu
   1440 1445 1450 1455
   AGC TAC CCC GAC GAG ATC AAG GAG ATC GAG GGC TTG CTG TAC TAC AAG 3071
   Ser Tyr Pro Asp Glu Ile Lys Glu Ile Glu Gly Leu Leu Tyr Tyr Lys
   1460 1465 1470
   AAC AAG CCC ATC TAC GAG AGC AGC GTG ATG ACC TAT CTA GAC GAG AAC 3119
   Asn Lys Pro Ile Tyr Glu Ser Ser Val Met Thr Tyr Leu Asp Glu Asn
   1475 1480 1485
   ACC GCC AAG GAG GTG ACC AAG CAG CTG AAC GAC ACC ACC GGC AAG TTC 3167
   Thr Ala Lys Glu Val Thr Lys Gln Leu Asn Asp Thr Thr Gly Lys Phe
   1490 1495 1500
   AAG GAC GTG AGC CAC CTG TAC GAC GTG AAG CTG ACC CCC AAG ATG AAC 3215
   Lys Asp Val Ser His Leu Tyr Asp Val Lys Leu Thr Pro Lys Met Asn
   1505 1510 1515
   GTG ACC ATC AAG CTG AGC ATC CTG TAC GAC AAC GCC GAG AGC AAC GAC 3263
   Val Thr Ile Lys Leu Ser Ile Leu Tyr Asp Asn Ala Glu Ser Asn Asp
   1520 1525 1530 1535
   AAC AGC ATC GGC AAG TGG ACC AAC ACC AAC ATC GTG AGC GGC GGC AAC 3311
   Asn Ser Ile Gly Lys Trp Thr Asn Thr Asn Ile Val Ser Gly Gly Asn
   1540 1545 1550
   AAC GGC AAG AAG CAG TAC AGC AGC AAC AAC CCC GAC GCC AAC CTG ACC 3359
   Asn Gly Lys Lys Gln Tyr Ser Ser Asn Asn Pro Asp Ala Asn Leu Thr
   1555 1560 1565
   CTG AAC ACC GAC GCC CAG GAG AAG CTG AAC AAG AAC CGC GAC TAC TAC 3407
   Leu Asn Thr Asp Ala Gln Glu Lys Leu Asn Lys Asn Arg Asp Tyr Tyr
   1570 1575 1580
   ATC AGC CTG TAC ATG AAG AGC GAG AAG AAC ACC CAG TGC GAG ATC ACC 3455
   Ile Ser Leu Tyr Met Lys Ser Glu Lys Asn Thr Gln Cys Glu Ile Thr
   1585 1590 1595
   ATC GAC GGC GAG ATA TAC CCC ATC ACC ACC AAG ACC GTG AAC GTG AAC 3503
   Ile Asp Gly Glu Ile Tyr Pro Ile Thr Thr Lys Thr Val Asn Val Asn
   1600 1605 1610 1615
   AAG GAC AAC TAC AAG CGC CTG GAC ATC ATC GCC CAC AAC ATC AAG AGC      3551
   Lys Asp Asn Tyr Lys Arg Leu Asp Ile Ile Ala His Asn Ile Lys Ser
                   1620                1625                1630
   AAC CCC ATC AGC AGC CTG CAC ATC AAG ACC AAC GAC GAG ATC ACC CTG      3599
   Asn Pro Ile Ser Ser Leu His Ile Lys Thr Asn Asp Glu Ile Thr Leu
               1635                1640                1645
   TTC TGG GAC GAC ATA TCG ATT ACC GAC GTC GCC AGC ATC AAG CCC GAG      3647
   Phe Trp Asp Asp Ile Ser Ile Thr Asp Val Ala Ser Ile Lys Pro Glu
           1650                1655                1660
   AAC CTG ACC GAC AGC GAG ATC AAG CAG ATA TAC AGT CGC TAC GGC ATC      3695
   Asn Leu Thr Asp Ser Glu Ile Lys Gln Ile Tyr Ser Arg Tyr Gly Ile
       1665                1670                1675
   AAG CTG GAG GAC GGC ATC CTG ATC GAC AAG AAA GGC GGC ATC CAC TAC      3743
   Lys Leu Glu Asp Gly Ile Leu Ile Asp Lys Lys Gly Gly Ile His Tyr
   1680                1685                1690                1695
   GGC GAG TTC ATC AAC GAG GCC AGC TTC AAC ATC GAG CCC CTG CAG AAC      3791
   Gly Glu Phe Ile Asn Glu Ala Ser Phe Asn Ile Glu Pro Leu Gln Asn
                   1700                1705                1710
   TAC GTG ACC AAG TAC GAG GTG ACC TAC AGC AGC GAG CTG GGC CCC AAC      3839
   Tyr Val Thr Lys Tyr Glu Val Thr Tyr Ser Ser Glu Leu Gly Pro Asn
               1715                1720                1725
   GTG AGC GAC ACC CTG GAG AGC GAC AAG ATT TAC AAG GAC GGC ACC ATC      3887
   Val Ser Asp Thr Leu Glu Ser Asp Lys Ile Tyr Lys Asp Gly Thr Ile
           1730                1735                1740
   AAG TTC GAC TTC ACC AAG TAC AGC AAG AAC GAG CAG GGC CTG TTC TAC      3935
   Lys Phe Asp Phe Thr Lys Tyr Ser Lys Asn Glu Gln Gly Leu Phe Tyr
       1745                1750                1755
   GAC AGC GGC CTG AAC TGG GAC TTC AAG ATC AAC GCC ATC ACC TAC GAC      3983
   Asp Ser Gly Leu Asn Trp Asp Phe Lys Ile Asn Ala Ile Thr Tyr Asp
   1760                1765                1770                1775
   GGC AAG GAG ATG AAC GTG TTC CAC CGC TAC AAC AAG TAGATCTGAG           4029
   Gly Lys Glu Met Asn Val Phe His Arg Tyr Asn Lys
                   1780                1785
   CT                                                                   4031
(2) INFORMATION FOR SEQ ID NO:50:
   (i) SEQUENCE CHARACTERISTICS:
      (A) LENGTH: 1338 amino acids
      (B) TYPE: amino acid
      (D) TOPOLOGY: linear
   (ii) MOLECULE TYPE: protein
   (xi) SEQUENCE DESCRIPTION: SEQ ID NO:50:
   Met Lys Arg Met Glu Gly Lys Leu Phe Met Val Ser Lys Lys Leu Gln
   1 5 10 15
   Val Val Thr Lys Thr Val Leu Leu Ser Thr Val Phe Ser Ile Ser Leu
   20 25 30
   Leu Asn Asn Glu Val Ile Lys Ala Glu Gln Leu Asn Ile Asn Ser Gln
   35 40 45
   Ser Lys Tyr Thr Asn Leu Gln Asn Leu Lys Ile Thr Asp Lys Val Glu
   50 55 60
   Asp Phe Lys Glu Asp Lys Glu Lys Ala Lys Glu Trp Gly Lys Glu Lys
   65 70 75 80
   Glu Lys Glu Trp Lys Leu Thr Ala Thr Glu Lys Gly Lys Met Asn Asn
   85 90 95
   Phe Leu Asp Asn Lys Asn Asp Ile Lys Thr Asn Tyr Lys Glu Ile Thr
   100 105 110
   Phe Ser Ile Ala Gly Ser Phe Glu Asp Glu Ile Lys Asp Leu Lys Glu
   115 120 125
   Ile Asp Lys Met Phe Asp Lys Thr Asn Leu Ser Asn Ser Ile Ile Thr
   130 135 140
   Tyr Lys Asn Val Glu Pro Thr Thr Ile Gly Phe Asn Lys Ser Leu Thr
   145 150 155 160
   Glu Gly Asn Thr Ile Asn Ser Asp Ala Met Ala Gln Phe Lys Glu Gln
   165 170 175
   Phe Leu Asp Arg Asp Ile Lys Phe Asp Ser Tyr Leu Asp Thr His Leu
   180 185 190
   Thr Ala Gln Gln Val Ser Ser Lys Glu Arg Val Ile Leu Lys Val Thr
   195 200 205
   Val Pro Ser Gly Lys Gly Ser Thr Thr Pro Thr Lys Ala Gly Val Ile
   210 215 220
   Leu Asn Asn Ser Glu Tyr Lys Met Leu Ile Asp Asn Gly Tyr Met Val
   225 230 235 240
   His Val Asp Lys Val Ser Lys Val Val Lys Lys Gly Val Glu Cys Leu
   245 250 255
   Gln Ile Glu Gly Thr Leu Lys Lys Ser Leu Asp Phe Lys Asn Asp Ile
   260 265 270
   Asn Ala Glu Ala His Ser Trp Gly Met Lys Asn Tyr Glu Glu Trp Ala
   275 280 285
   Lys Asp Leu Thr Asp Ser Gln Arg Glu Ala Leu Asp Gly Tyr Ala Arg
   290 295 300
   Gln Asp Tyr Lys Glu Ile Asn Asn Tyr Leu Arg Asn Gln Gly Gly Ser
   305 310 315 320
   Gly Asn Glu Lys Leu Asp Ala Gln Ile Lys Asn Ile Ser Asp Ala Leu
   325 330 335
   Gly Lys Lys Pro Ile Pro Glu Asn Ile Thr Val Tyr Arg Trp Cys Gly
   340 345 350
   Met Pro Glu Phe Gly Tyr Gln Ile Ser Asp Pro Leu Pro Ser Leu Lys
   355 360 365
   Asp Phe Glu Glu Gln Phe Leu Asn Thr Ile Lys Glu Asp Lys Gly Tyr
   370 375 380
   Met Ser Thr Ser Leu Ser Ser Glu Arg Leu Ala Ala Phe Gly Ser Arg
   385 390 395 400
   Lys Ile Ile Leu Arg Leu Gln Val Pro Lys Gly Ser Thr Gly Ala Tyr
   405 410 415
   Leu Ser Ala Ile Gly Gly Phe Ala Ser Glu Lys Glu Ile Leu Leu Asp
   420 425 430
   Lys Asp Ser Lys Tyr His Ile Asp Lys Val Thr Glu Val Ile Ile Lys
   435 440 445
   Gly Val Lys Arg Tyr Val Val Asp Ala Thr Leu Leu Thr Asn Ser Arg
   450 455 460
   Gly Pro Ser Thr Pro Pro Thr Pro Ser Pro Ser Thr Pro Pro Thr Pro
   465 470 475 480
   Ser Asp Ile Gly Ser Thr Met Lys Thr Asn Gln Ile Ser Thr Thr Gln
   485 490 495
   Lys Asn Gln Gln Lys Glu Met Asp Arg Lys Gly Leu Leu Gly Tyr Tyr
   500 505 510
   Phe Lys Gly Lys Asp Phe Ser Asn Leu Thr Met Phe Ala Pro Thr Arg
   515 520 525
   Asp Ser Thr Leu Ile Tyr Asp Gln Gln Thr Ala Asn Lys Leu Leu Asp
   530 535 540
   Lys Lys Gln Gln Glu Tyr Gln Ser Ile Arg Trp Ile Gly Leu Ile Gln
   545 550 555 560
   Ser Lys Glu Thr Gly Asp Phe Thr Phe Asn Leu Ser Glu Asp Glu Gln
   565 570 575
   Ala Ile Ile Glu Ile Asn Gly Lys Ile Ile Ser Asn Lys Gly Lys Glu
   580 585 590
   Lys Gln Val Val His Leu Glu Lys Gly Lys Leu Val Pro Ile Lys Ile
   595 600 605
   Glu Tyr Gln Ser Asp Thr Lys Phe Asn Ile Asp Ser Lys Thr Phe Lys
   610 615 620
   Glu Leu Lys Leu Phe Lys Ile Asp Ser Gln Asn Gln Pro Gln Gln Val
   625 630 635 640
   Gln Gln Asp Glu Leu Arg Asn Pro Glu Phe Asn Lys Lys Glu Ser Gln
   645 650 655
   Glu Phe Leu Ala Lys Pro Ser Lys Ile Asn Leu Phe Thr Gln Gln Met
   660 665 670
   Lys Arg Glu Ile Asp Glu Asp Thr Asp Thr Asp Gly Asp Ser Ile Pro
   675 680 685
   Asp Leu Trp Glu Glu Asn Gly Tyr Thr Ile Gln Asn Arg Ile Ala Val
   690 695 700
   Lys Trp Asp Asp Ser Leu Ala Ser Lys Gly Tyr Thr Lys Phe Val Ser
   705 710 715 720
   Asn Pro Leu Glu Ser His Thr Val Gly Asp Pro Tyr Thr Asp Tyr Glu
   725 730 735
   Lys Ala Ala Arg Asp Leu Asp Leu Ser Asn Ala Lys Glu Thr Phe Asn
   740 745 750
   Pro Leu Val Ala Ala Phe Pro Ser Val Asn Val Ser Met Glu Lys Val
   755 760 765
   Ile Leu Ser Pro Asn Glu Asn Leu Ser Asn Ser Val Glu Ser His Ser
   770 775 780
   Ser Thr Asn Trp Ser Tyr Thr Asn Thr Glu Gly Ala Ser Val Glu Ala
   785 790 795 800
   Gly Ile Gly Pro Lys Gly Ile Ser Phe Gly Val Ser Val Asn Tyr Gln
   805 810 815
   His Ser Glu Thr Val Ala Gln Glu Trp Gly Thr Ser Thr Gly Asn Thr
   820 825 830
   Ser Gln Phe Asn Thr Ala Ser Ala Gly Tyr Leu Asn Ala Asn Val Arg
   835 840 845
   Tyr Asn Asn Val Gly Thr Gly Ala Ile Tyr Asp Val Lys Pro Thr Thr
   850 855 860
   Ser Phe Val Leu Asn Asn Asp Thr Ile Ala Thr Ile Thr Ala Lys Ser
   865 870 875 880
   Asn Ser Thr Ala Leu Asn Ile Ser Pro Gly Glu Ser Tyr Pro Lys Lys
   885 890 895
   Gly Gln Asn Gly Ile Ala Ile Thr Ser Met Asp Asp Phe Asn Ser His
   900 905 910
   Pro Ile Thr Leu Asn Lys Lys Gln Val Asp Asn Leu Leu Asn Asn Lys
   915 920 925
   Pro Met Met Leu Glu Thr Asn Gln Thr Asp Gly Val Tyr Lys Ile Lys
   930 935 940
   Asp Thr His Gly Asn Ile Val Thr Gly Gly Glu Trp Asn Gly Val Ile
   945 950 955 960
   Gln Gln Ile Lys Ala Lys Thr Ala Ser Ile Ile Val Asp Asp Gly Glu
   965 970 975
   Arg Val Ala Glu Lys Arg Val Ala Ala Lys Asp Tyr Glu Asn Pro Glu
   980 985 990
   Asp Lys Thr Pro Ser Leu Thr Leu Lys Asp Ala Leu Lys Leu Ser Tyr
   995 1000 1005
   Pro Asp Glu Ile Lys Glu Ile Glu Gly Leu Leu Tyr Tyr Lys Asn Lys
   1010 1015 1020
   Pro Ile Tyr Glu Ser Ser Val Met Thr Tyr Leu Asp Glu Asn Thr Ala
   1025 1030 1035 1040
   Lys Glu Val Thr Lys Gln Leu Asn Asp Thr Thr Gly Lys Phe Lys Asp
   1045 1050 1055
   Val Ser His Leu Tyr Asp Val Lys Leu Thr Pro Lys Met Asn Val Thr
   1060 1065 1070
   Ile Lys Leu Ser Ile Leu Tyr Asp Asn Ala Glu Ser Asn Asp Asn Ser
   1075 1080 1085
   Ile Gly Lys Trp Thr Asn Thr Asn Ile Val Ser Gly Gly Asn Asn Gly
   1090 1095 1100
   Lys Lys Gln Tyr Ser Ser Asn Asn Pro Asp Ala Asn Leu Thr Leu Asn
   1105 1110 1115 1120
   Thr Asp Ala Gln Glu Lys Leu Asn Lys Asn Arg Asp Tyr Tyr Ile Ser
   1125 1130 1135
   Leu Tyr Met Lys Ser Glu Lys Asn Thr Gln Cys Glu Ile Thr Ile Asp
   1140 1145 1150
   Gly Glu Ile Tyr Pro Ile Thr Thr Lys Thr Val Asn Val Asn Lys Asp
   1155 1160 1165
   Asn Tyr Lys Arg Leu Asp Ile Ile Ala His Asn Ile Lys Ser Asn Pro
   1170 1175 1180
   Ile Ser Ser Leu His Ile Lys Thr Asn Asp Glu Ile Thr Leu Phe Trp
   1185 1190 1195 1200
   Asp Asp Ile Ser Ile Thr Asp Val Ala Ser Ile Lys Pro Glu Asn Leu
   1205 1210 1215
   Thr Asp Ser Glu Ile Lys Gln Ile Tyr Ser Arg Tyr Gly Ile Lys Leu
   1220 1225 1230
   Glu Asp Gly Ile Leu Ile Asp Lys Lys Gly Gly Ile His Tyr Gly Glu
   1235 1240 1245
   Phe Ile Asn Glu Ala Ser Phe Asn Ile Glu Pro Leu Gln Asn Tyr Val
   1250 1255 1260
   Thr Lys Tyr Glu Val Thr Tyr Ser Ser Glu Leu Gly Pro Asn Val Ser
   1265 1270 1275 1280
   Asp Thr Leu Glu Ser Asp Lys Ile Tyr Lys Asp Gly Thr Ile Lys Phe
   1285 1290 1295
   Asp Phe Thr Lys Tyr Ser Lys Asn Glu Gln Gly Leu Phe Tyr Asp Ser
   1300 1305 1310
   Gly Leu Asn Trp Asp Phe Lys Ile Asn Ala Ile Thr Tyr Asp Gly Lys
   1315 1320 1325
   Glu Met Asn Val Phe His Arg Tyr Asn Lys
   1330 1335
(2) INFORMATION FOR SEQ ID NO:51:
   (i) SEQUENCE CHARACTERISTICS:
      (A) LENGTH: 2444 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (ix) FEATURE:
      (A) NAME/KEY: CDS
      (B) LOCATION: 17..2444
      (D) OTHER INFORMATION: /product= "3A(a) synthetic:native fusion"
   (xi) SEQUENCE DESCRIPTION: SEQ ID NO:51:
   GGATCCACCA ATGAAC ATG AAC AAG AAC AAC ACC AAG CTG AGC ACC CGC      49
                     Met Asn Lys Asn Asn Thr Lys Leu Ser Thr Arg
                       1               5                  10
   GCC CTG CCG AGC TTC ATC GAC TAC TTC AAC GGC ATC TAC GGC TTC GCC    97
   Ala Leu Pro Ser Phe Ile Asp Tyr Phe Asn Gly Ile Tyr Gly Phe Ala
               15                  20                  25
   ACC GGC ATC AAG GAC ATC ATG AAC ATG ATC TTC AAG ACC GAC ACC GGC   145
   Thr Gly Ile Lys Asp Ile Met Asn Met Ile Phe Lys Thr Asp Thr Gly
           30                  35                  40
   GGC GAC CTG ACC CTG GAC GAG ATC CTG AAG AAC CAG CAG CTG CTG AAC   193
   Gly Asp Leu Thr Leu Asp Glu Ile Leu Lys Asn Gln Gln Leu Leu Asn
       45                  50                  55
   GAC ATC AGC GGC AAG CTG GAC GGC GTG AAC GGC AGC CTG AAC GAC CTG   241
   Asp Ile Ser Gly Lys Leu Asp Gly Val Asn Gly Ser Leu Asn Asp Leu
   60                  65                  70                  75
   ATC GCC CAG GGC AAC CTG AAC ACC GAG CTG AGC AAG GAG ATC CTT AAG   289
   Ile Ala Gln Gly Asn Leu Asn Thr Glu Leu Ser Lys Glu Ile Leu Lys
                   80                  85                  90
   ATC GCC AAC GAG CAG AAC CAG GTG CTG AAC GAC GTG AAC AAC AAG CTG   337
   Ile Ala Asn Glu Gln Asn Gln Val Leu Asn Asp Val Asn Asn Lys Leu
               95                 100                 105
   GAC GCC ATC AAC ACC ATG CTG CGC GTG TAC CTG CCG AAG ATC ACC AGC   385
   Asp Ala Ile Asn Thr Met Leu Arg Val Tyr Leu Pro Lys Ile Thr Ser
           110                 115                 120
   ATG CTG AGC GAC GTG ATG AAG CAG AAC TAC GCC CTG AGC CTG CAG ATC   433
   Met Leu Ser Asp Val Met Lys Gln Asn Tyr Ala Leu Ser Leu Gln Ile
       125                 130                 135
   GAG TAC CTG AGC AAG CAG CTG CAG GAG ATC AGC GAC AAG CTG GAC ATC   481
   Glu Tyr Leu Ser Lys Gln Leu Gln Glu Ile Ser Asp Lys Leu Asp Ile
   140                 145                 150                 155
   ATC AAC GTG AAC GTC CTG ATC AAC AGC ACC CTG ACC GAG ATC ACC CCG   529
   Ile Asn Val Asn Val Leu Ile Asn Ser Thr Leu Thr Glu Ile Thr Pro
                   160                 165                 170
   GCC TAC CAG CGC ATC AAG TAC GTG AAC GAG AAG TTC GAA GAG CTG ACC   577
   Ala Tyr Gln Arg Ile Lys Tyr Val Asn Glu Lys Phe Glu Glu Leu Thr
               175                 180                 185
   TTC GCC ACC GAG ACC AGC AGC AAG GTG AAG AAG GAC GGC AGC CCG GCC   625
   Phe Ala Thr Glu Thr Ser Ser Lys Val Lys Lys Asp Gly Ser Pro Ala
           190                 195                 200
   GAC ATC CTG GAC GAG CTG ACC GAG CTG ACC GAG CTG GCC AAG AGC GTG   673
   Asp Ile Leu Asp Glu Leu Thr Glu Leu Thr Glu Leu Ala Lys Ser Val
       205                 210                 215
   ACC AAG AAC GAC GTG GAC GGC TTC GAG TTC TAC CTG AAC ACC TTC CAC      721
   Thr Lys Asn Asp Val Asp Gly Phe Glu Phe Tyr Leu Asn Thr Phe His
   220                 225                 230                 235
   GAC GTG ATG GTG GGC AAC AAC CTG TTC GGC CGC AGC GCC CTG AAG ACC      769
   Asp Val Met Val Gly Asn Asn Leu Phe Gly Arg Ser Ala Leu Lys Thr
                   240                 245                 250
   GCC AGC GAG CTG ATC ACC AAG GAG AAC GTG AAG ACC AGC GGC AGC GAG      817
   Ala Ser Glu Leu Ile Thr Lys Glu Asn Val Lys Thr Ser Gly Ser Glu
               255                 260                 265
   GTG GGC AAC GTG TAC AAC TTC CTG ATC GTG CTG ACC GCC CTG CAG GCC      865
   Val Gly Asn Val Tyr Asn Phe Leu Ile Val Leu Thr Ala Leu Gln Ala
           270                 275                 280
   CAG GCC TTC CTG ACC CTG ACC ACC TGT CGC AAG CTG CTG GGC CTG GCC      913
   Gln Ala Phe Leu Thr Leu Thr Thr Cys Arg Lys Leu Leu Gly Leu Ala
       285                 290                 295
   GAC ATC GAC TAC ACC AGC ATC ATG AAC GAG CAC TTG AAC AAG GAG AAG      961
   Asp Ile Asp Tyr Thr Ser Ile Met Asn Glu His Leu Asn Lys Glu Lys
   300                 305                 310                 315
   GAG GAG TTC CGC GTG AAC ATC CTG CCG ACC CTG AGC AAC ACC TTC AGC     1009
   Glu Glu Phe Arg Val Asn Ile Leu Pro Thr Leu Ser Asn Thr Phe Ser
                   320                 325                 330
   AAC CCG AAC TAC GCC AAG GTG AAG GGC AGC GAC GAG GAC GCC AAG ATG     1057
   Asn Pro Asn Tyr Ala Lys Val Lys Gly Ser Asp Glu Asp Ala Lys Met
               335                 340                 345
   ATC GTG GAG GCT AAG CCG GGC CAC GCG TTG ATC GGC TTC GAG ATC AGC     1105
   Ile Val Glu Ala Lys Pro Gly His Ala Leu Ile Gly Phe Glu Ile Ser
           350                 355                 360
   AAC GAC AGC ATC ACC GTG CTG AAG GTG TAC GAG GCC AAG CTG AAG CAG     1153
   Asn Asp Ser Ile Thr Val Leu Lys Val Tyr Glu Ala Lys Leu Lys Gln
       365                 370                 375
   AAC TAC CAG GTG GAC AAG GAC AGC TTG AGC GAG GTG ATC TAC GGC GAC     1201
   Asn Tyr Gln Val Asp Lys Asp Ser Leu Ser Glu Val Ile Tyr Gly Asp
   380                 385                 390                 395
   ATG GAC AAG CTG CTG TGT CCG GAC CAG AGC GAG CAA ATC TAC TAC ACC     1249
   Met Asp Lys Leu Leu Cys Pro Asp Gln Ser Glu Gln Ile Tyr Tyr Thr
                   400                 405                 410
   AAC AAC ATC GTG TTC CCG AAC GAG TAC GTG ATC ACC AAG ATC GAC TTC     1297
   Asn Asn Ile Val Phe Pro Asn Glu Tyr Val Ile Thr Lys Ile Asp Phe
               415                 420                 425
   ACC AAG AAG ATG AAG ACC CTG CGC TAC GAG GTG ACC GCC AAC TTC TAC      1345
   Thr Lys Lys Met Lys Thr Leu Arg Tyr Glu Val Thr Ala Asn Phe Tyr
           430                 435                 440
   GAC AGC AGC ACC GGC GAG ATC GAC CTG AAC AAG AAG AAG GTG GAG AGC      1393
   Asp Ser Ser Thr Gly Glu Ile Asp Leu Asn Lys Lys Lys Val Glu Ser
       445                 450                 455
   AGC GAG GCC GAG TAC CGC ACC CTG AGC GCG AAC GAC GAC GGC GTC TAC      1441
   Ser Glu Ala Glu Tyr Arg Thr Leu Ser Ala Asn Asp Asp Gly Val Tyr
   460                 465                 470                 475
   ATG CCA CTG GGC GTG ATC AGC GAG ACC TTC CTG ACC CCG ATC AAC GGC      1489
   Met Pro Leu Gly Val Ile Ser Glu Thr Phe Leu Thr Pro Ile Asn Gly
                   480                 485                 490
   TTT GGC CTG CAG GCC GAC GAG AAC AGC CGC CTG ATC ACC CTG ACC TGT      1537
   Phe Gly Leu Gln Ala Asp Glu Asn Ser Arg Leu Ile Thr Leu Thr Cys
               495                 500                 505
   AAG AGC TAC CTG CGC GAG CTG CTG CTA GCC ACC GAC CTG AGC AAC AAG      1585
   Lys Ser Tyr Leu Arg Glu Leu Leu Leu Ala Thr Asp Leu Ser Asn Lys
           510                 515                 520
   GAG ACC AAG CTG ATC GTG CCA CCG AGC GGC TTC ATC AGC AAC ATC GTG      1633
   Glu Thr Lys Leu Ile Val Pro Pro Ser Gly Phe Ile Ser Asn Ile Val
       525                 530                 535
   GAG AAC GGC AGC ATC GAG GAG GAC AAC CTG GAG CCG TGG AAG GCC AAC      1681
   Glu Asn Gly Ser Ile Glu Glu Asp Asn Leu Glu Pro Trp Lys Ala Asn
   540                 545                 550                 555
   AAC AAG AAC GCC TAC GTG GAC CAC ACC GGC GGC GTG AAC GGC ACC AAG      1729
   Asn Lys Asn Ala Tyr Val Asp His Thr Gly Gly Val Asn Gly Thr Lys
                   560                 565                 570
   GCC CTG TAC GTG CAC AAG GAC GGC GGC ATC AGC CAG TTC ATC GGC GAC      1777
   Ala Leu Tyr Val His Lys Asp Gly Gly Ile Ser Gln Phe Ile Gly Asp
               575                 580                 585
   AAG CTG AAG CCG AAG ACC GAG TAC GTG ATC CAG TAC ACC GTG AAG GGC      1825
   Lys Leu Lys Pro Lys Thr Glu Tyr Val Ile Gln Tyr Thr Val Lys Gly
           590                 595                 600
   AAG CCA TCG ATT CAC CTG AAG GAC GAG AAC ACC GGC TAC ATC CAC TAC      1873
   Lys Pro Ser Ile His Leu Lys Asp Glu Asn Thr Gly Tyr Ile His Tyr
       605                 610                 615
   GAG GAC ACC AAC AAC AAC CTG GAG GAC TAC CAG ACC ATC AAC AAG CGC      1921
   Glu Asp Thr Asn Asn Asn Leu Glu Asp Tyr Gln Thr Ile Asn Lys Arg
   620                 625                 630                 635
   TTC ACC ACC GGC ACC GAC CTG AAG GGC GTG TAC CTG ATC CTG AAG AGC      1969
   Phe Thr Thr Gly Thr Asp Leu Lys Gly Val Tyr Leu Ile Leu Lys Ser
                   640                 645                 650
   CAG AAC GGC GAC GAG GCC TGG GGC GAC AAC TTC ATC ATC CTG GAG ATC        2017
   Gln Asn Gly Asp Glu Ala Trp Gly Asp Asn Phe Ile Ile Leu Glu Ile
               655                 660                 665
   AGC CCG AGC GAG AAG CTG CTG AGC CCG GAG CTG ATC AAC ACC AAC AAC        2065
   Ser Pro Ser Glu Lys Leu Leu Ser Pro Glu Leu Ile Asn Thr Asn Asn
           670                 675                 680
   TGG ACC AGC ACC GGC AGC ACC AAC ATC AGC GGC AAC ACC CTG ACC CTG        2113
   Trp Thr Ser Thr Gly Ser Thr Asn Ile Ser Gly Asn Thr Leu Thr Leu
       685                 690                 695
   TAC CAG GGC GGC CGG GGG ATT CTA AAA CAA AAC CTT CAA TTA GAT AGT        2161
   Tyr Gln Gly Gly Arg Gly Ile Leu Lys Gln Asn Leu Gln Leu Asp Ser
   700                 705                 710                 715
   TTT TCA ACT TAT AGA GTG TAT TTT TCT GTG TCC GGA GAT GCT AAT GTA        2209
   Phe Ser Thr Tyr Arg Val Tyr Phe Ser Val Ser Gly Asp Ala Asn Val
                   720                 725                 730
   AGG ATT AGA AAT TCT AGG GAA GTG TTA TTT GAA AAA AGA TAT ATG AGC        2257
   Arg Ile Arg Asn Ser Arg Glu Val Leu Phe Glu Lys Arg Tyr Met Ser
               735                 740                 745
   GGT GCT AAA GAT GTT TCT GAA ATG TTC ACT ACA AAA TTT GAG AAA GAT        2305
   Gly Ala Lys Asp Val Ser Glu Met Phe Thr Thr Lys Phe Glu Lys Asp
           750                 755                 760
   AAC TTT TAT ATA GAG CTT TCT CAA GGG AAT AAT TTA TAT GGT GGT CCT        2353
   Asn Phe Tyr Ile Glu Leu Ser Gln Gly Asn Asn Leu Tyr Gly Gly Pro
       765                 770                 775
   ATT GTA CAT TTT TAC GAT GTC TCT ATT AAG NAA GAT CGG GAT CTA ATA        2401
   Ile Val His Phe Tyr Asp Val Ser Ile Lys Xaa Asp Arg Asp Leu Ile
   780                 785                 790                 795
   TTA ACA GTT TTT AAA AGC NAA TTC TTG TAT AAT GTC CTT GAT  T             2444
   Leu Thr Val Phe Lys Ser Xaa Phe Leu Tyr Asn Val Leu Asp
                   800                 805
(2) INFORMATION FOR SEQ ID NO:52:
   (i) SEQUENCE CHARACTERISTICS:
      (A) LENGTH: 809 amino acids
      (B) TYPE: amino acid
      (D) TOPOLOGY: linear
   (ii) MOLECULE TYPE: protein
   (xi) SEQUENCE DESCRIPTION: SEQ ID NO:52:
   Met Asn Lys Asn Asn Thr Lys Leu Ser Thr Arg Ala Leu Pro Ser Phe
   1               5                   10                  15
   Ile Asp Tyr Phe Asn Gly Ile Tyr Gly Phe Ala Thr Gly Ile Lys Asp
   20 25 30
   Ile Met Asn Met Ile Phe Lys Thr Asp Thr Gly Gly Asp Leu Thr Leu
   35 40 45
   Asp Glu Ile Leu Lys Asn Gln Gln Leu Leu Asn Asp Ile Ser Gly Lys
   50 55 60
   Leu Asp Gly Val Asn Gly Ser Leu Asn Asp Leu Ile Ala Gln Gly Asn
   65 70 75 80
   Leu Asn Thr Glu Leu Ser Lys Glu Ile Leu Lys Ile Ala Asn Glu Gln
   85 90 95
   Asn Gln Val Leu Asn Asp Val Asn Asn Lys Leu Asp Ala Ile Asn Thr
   100 105 110
   Met Leu Arg Val Tyr Leu Pro Lys Ile Thr Ser Met Leu Ser Asp Val
   115 120 125
   Met Lys Gln Asn Tyr Ala Leu Ser Leu Gln Ile Glu Tyr Leu Ser Lys
   130 135 140
   Gln Leu Gln Glu Ile Ser Asp Lys Leu Asp Ile Ile Asn Val Asn Val
   145 150 155 160
   Leu Ile Asn Ser Thr Leu Thr Glu Ile Thr Pro Ala Tyr Gln Arg Ile
   165 170 175
   Lys Tyr Val Asn Glu Lys Phe Glu Glu Leu Thr Phe Ala Thr Glu Thr
   180 185 190
   Ser Ser Lys Val Lys Lys Asp Gly Ser Pro Ala Asp Ile Leu Asp Glu
   195 200 205
   Leu Thr Glu Leu Thr Glu Leu Ala Lys Ser Val Thr Lys Asn Asp Val
   210 215 220
   Asp Gly Phe Glu Phe Tyr Leu Asn Thr Phe His Asp Val Met Val Gly
   225 230 235 240
   Asn Asn Leu Phe Gly Arg Ser Ala Leu Lys Thr Ala Ser Glu Leu Ile
   245 250 255
   Thr Lys Glu Asn Val Lys Thr Ser Gly Ser Glu Val Gly Asn Val Tyr
   260 265 270
   Asn Phe Leu Ile Val Leu Thr Ala Leu Gln Ala Gln Ala Phe Leu Thr
   275 280 285
   Leu Thr Thr Cys Arg Lys Leu Leu Gly Leu Ala Asp Ile Asp Tyr Thr
   290 295 300
   Ser Ile Met Asn Glu His Leu Asn Lys Glu Lys Glu Glu Phe Arg Val
   305 310 315 320
   Asn Ile Leu Pro Thr Leu Ser Asn Thr Phe Ser Asn Pro Asn Tyr Ala
   325 330 335
   Lys Val Lys Gly Ser Asp Glu Asp Ala Lys Met Ile Val Glu Ala Lys
   340 345 350
   Pro Gly His Ala Leu Ile Gly Phe Glu Ile Ser Asn Asp Ser Ile Thr
   355 360 365
   Val Leu Lys Val Tyr Glu Ala Lys Leu Lys Gln Asn Tyr Gln Val Asp
   370 375 380
   Lys Asp Ser Leu Ser Glu Val Ile Tyr Gly Asp Met Asp Lys Leu Leu
   385 390 395 400
   Cys Pro Asp Gln Ser Glu Gln Ile Tyr Tyr Thr Asn Asn Ile Val Phe
   405 410 415
   Pro Asn Glu Tyr Val Ile Thr Lys Ile Asp Phe Thr Lys Lys Met Lys
   420 425 430
   Thr Leu Arg Tyr Glu Val Thr Ala Asn Phe Tyr Asp Ser Ser Thr Gly
   435 440 445
   Glu Ile Asp Leu Asn Lys Lys Lys Val Glu Ser Ser Glu Ala Glu Tyr
   450 455 460
   Arg Thr Leu Ser Ala Asn Asp Asp Gly Val Tyr Met Pro Leu Gly Val
   465 470 475 480
   Ile Ser Glu Thr Phe Leu Thr Pro Ile Asn Gly Phe Gly Leu Gln Ala
   485 490 495
   Asp Glu Asn Ser Arg Leu Ile Thr Leu Thr Cys Lys Ser Tyr Leu Arg
   500 505 510
   Glu Leu Leu Leu Ala Thr Asp Leu Ser Asn Lys Glu Thr Lys Leu Ile
   515 520 525
   Val Pro Pro Ser Gly Phe Ile Ser Asn Ile Val Glu Asn Gly Ser Ile
   530 535 540
   Glu Glu Asp Asn Leu Glu Pro Trp Lys Ala Asn Asn Lys Asn Ala Tyr
   545 550 555 560
   Val Asp His Thr Gly Gly Val Asn Gly Thr Lys Ala Leu Tyr Val His
   565 570 575
   Lys Asp Gly Gly Ile Ser Gln Phe Ile Gly Asp Lys Leu Lys Pro Lys
   580 585 590
   Thr Glu Tyr Val Ile Gln Tyr Thr Val Lys Gly Lys Pro Ser Ile His
   595 600 605
   Leu Lys Asp Glu Asn Thr Gly Tyr Ile His Tyr Glu Asp Thr Asn Asn
   610 615 620
   Asn Leu Glu Asp Tyr Gln Thr Ile Asn Lys Arg Phe Thr Thr Gly Thr
   625 630 635 640
   Asp Leu Lys Gly Val Tyr Leu Ile Leu Lys Ser Gln Asn Gly Asp Glu
   645 650 655
   Ala Trp Gly Asp Asn Phe Ile Ile Leu Glu Ile Ser Pro Ser Glu Lys
   660 665 670
   Leu Leu Ser Pro Glu Leu Ile Asn Thr Asn Asn Trp Thr Ser Thr Gly
   675 680 685
   Ser Thr Asn Ile Ser Gly Asn Thr Leu Thr Leu Tyr Gln Gly Gly Arg
   690 695 700
   Gly Ile Leu Lys Gln Asn Leu Gln Leu Asp Ser Phe Ser Thr Tyr Arg
   705 710 715 720
   Val Tyr Phe Ser Val Ser Gly Asp Ala Asn Val Arg Ile Arg Asn Ser
   725 730 735
   Arg Glu Val Leu Phe Glu Lys Arg Tyr Met Ser Gly Ala Lys Asp Val
   740 745 750
   Ser Glu Met Phe Thr Thr Lys Phe Glu Lys Asp Asn Phe Tyr Ile Glu
   755 760 765
   Leu Ser Gln Gly Asn Asn Leu Tyr Gly Gly Pro Ile Val His Phe Tyr
   770 775 780
   Asp Val Ser Ile Lys Xaa Asp Arg Asp Leu Ile Leu Thr Val Phe Lys
   785 790 795 800
   Ser Xaa Phe Leu Tyr Asn Val Leu Asp
   805
(2) INFORMATION FOR SEQ ID NO:53:
   (i) SEQUENCE CHARACTERISTICS:
      (A) LENGTH: 3474 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: YES
   (iv) ANTI-SENSE: NO
   (vi) ORIGINAL SOURCE:
      (A) ORGANISM: Pure maize optimized synthetic BT CryIA(b) gene
   (xi) SEQUENCE DESCRIPTION: SEQ ID NO:53:
   ATGGACAACA ACCCCAACAT CAACGAGTGC ATCCCCTACA ACTGCCTGAG CAACCCCGAG 60
   GTGGAGGTGC TGGGCGGCGA GCGCATCGAG ACCGGCTACA CCCCCATCGA CATCAGCCTG 120
   AGCCTGACCC AGTTCCTGCT GAGCGAGTTC GTGCCCGGCG CCGGCTTCGT GCTGGGCCTG 180
   GTGGACATCA TCTGGGGCAT CTTCGGCCCC AGCCAGTGGG ACGCCTTCCT GGTGCAGATC 240
   GAGCAGCTGA TCAACCAGCG CATCGAGGAG TTCGCCCGCA ACCAGGCCAT CAGCCGCCTG 300
   GAGGGCCTGA GCAACCTGTA CCAGATCTAC GCCGAGAGCT TCCGCGAGTG GGAGGCCGAC 360
   CCCACCAACC CCGCCCTGCG CGAGGAGATG CGCATCCAGT TCAACGACAT GAACAGCGCC
   420
   CTGACCACCG CCATCCCCCT GTTCGCCGTG CAGAACTACC AGGTGCCCCT GCTGAGCGTG
   480
   TACGTGCAGG CCGCCAACCT GCACCTGAGC GTGCTGCGCG ACGTGAGCGT GTTCGGCCAG
   540
   CGCTGGGGCT TCGACGCCGC CACCATCAAC AGCCGCTACA ACGACCTGAC CCGCCTGATC
   600
   GGCAACTACA CCGACCACGC CGTGCGCTGG TACAACACCG GCCTGGAGCG CGTGTGGGGC
   660
   CCCGACAGCC GCGACTGGAT CCGCTACAAC CAGTTCCGCC GCGAGCTGAC CCTGACCGTG
   720
   CTGGACATCG TGAGCCTGTT CCCCAACTAC GACAGCCGCA CCTACCCCAT CCGCACCGTG
   780
   AGCCAGCTGA CCCGCGAGAT CTACACCAAC CCCGTGCTGG AGAACTTCGA CGGCAGCTTC
   840
   CGCGGCAGCG CCCAGGGCAT CGAGGGCAGC ATCCGCAGCC CCCACCTGAT GGACATCCTG
   900
   AACAGCATCA CCATCTACAC CGACGCCCAC CGCGGCGAGT ACTACTGGAG CGGCCACCAG
   960
   ATCATGGCCA GCCCCGTGGG CTTCAGCGGC CCCGAGTTCA CCTTCCCCCT GTACGGCACC
   1020
   ATGGGCAACG CCGCCCCCCA GCAGCGCATC GTGGCCCAGC TGGGCCAGGG CGTGTACCGC
   1080
   ACCCTGAGCA GCACCCTGTA CCGCCGCCCC TTCAACATCG GCATCAACAA CCAGCAGCTG
   1140
   AGCGTGCTGG ACGGCACCGA GTTCGCCTAC GGCACCAGCA GCAACCTGCC CAGCGCCGTG
   1200
   TACCGCAAGA GCGGCACCGT GGACAGCCTG GACGAGATCC CCCCCCAGAA CAACAACGTG
   1260
   CCCCCCCGCC AGGGCTTCAG CCACCGCCTG AGCCACGTGA GCATGTTCCG CAGCGGCTTC
   1320
   AGCAACAGCA GCGTGAGCAT CATCCGCGCC CCCATGTTCA GCTGGATCCA CCGCAGCGCC
   1380
   GAGTTCAACA ACATCATCCC CAGCAGCCAG ATCACCCAGA TCCCCCTGAC CAAGAGCACC
   1440
   AACCTGGGCA GCGGCACCAG CGTGGTGAAG GGCCCCGGCT TCACCGGCGG CGACATCCTG
   1500
   CGCCGCACCA GCCCCGGCCA GATCAGCACC CTGCGCGTGA ACATCACCGC CCCCCTGAGC
   1560
   CAGCGCTACC GCGTGCGCAT CCGCTACGCC AGCACCACCA ACCTGCAGTT CCACACCAGC
   1620
   ATCGACGGCC GCCCCATCAA CCAGGGCAAC TTCAGCGCCA CCATGAGCAG CGGCAGCAAC
   1680
   CTGCAGAGCG GCAGCTTCCG CACCGTGGGC TTCACCACCC CCTTCAACTT CAGCAACGGC
   1740
   AGCAGCGTGT TCACCCTGAG CGCCCACGTG TTCAACAGCG GCAACGAGGT GTACATCGAC
   1800
   CGCATCGAGT TCGTGCCCGC CGAGGTGACC TTCGAGGCCG AGTACGACCT GGAGCGCGCC
   1860
   CAGAAGGCCG TGAACGAGCT GTTCACCAGC AGCAACCAGA TCGGCCTGAA GACCGACGTG
   1920
   ACCGACTACC ACATCGACCA GGTGAGCAAC CTGGTGGAGT GCCTGAGCGA CGAGTTCTGC
   1980
   CTGGACGAGA AGAAGGAGCT GAGCGAGAAG GTGAAGCACG CCAAGCGCCT GAGCGACGAG
   2040
   CGCAACCTGC TGCAGGACCC CAACTTCCGC GGCATCAACC GCCAGCTGGA CCGCGGCTGG
   2100
   CGCGGCAGCA CCGACATCAC CATCCAGGGC GGCGACGACG TGTTCAAGGA GAACTACGTG
   2160
   ACCCTGCTGG GCACCTTCGA CGAGTGCTAC CCCACCTACC TGTACCAGAA GATCGACGAG
   2220
   AGCAAGCTGA AGGCCTACAC CCGCTACCAG CTGCGCGGCT ACATCGAGGA CAGCCAGGAC
   2280
   CTGGAGATCT ACCTGATCCG CTACAACGCC AAGCACGAGA CCGTGAACGT GCCCGGCACC
   2340
   GGCAGCCTGT GGCCCCTGAG CGCCCCCAGC CCCATCGGCA AGTGCGCCCA CCACAGCCAC
   2400
   CACTTCAGCC TGGACATCGA CGTGGGCTGC ACCGACCTGA ACGAGGACCT GGGCGTGTGG
   2460
   GTGATCTTCA AGATCAAGAC CCAGGACGGC CACGCCCGCC TGGGCAACCT GGAGTTCCTG
   2520
   GAGGAGAAGC CCCTGGTGGG CGAGGCCCTG GCCCGCGTGA AGCGCGCCGA GAAGAAGTGG
   2580
   CGCGACAAGC GCGAGAAGCT GGAGTGGGAG ACCAACATCG TGTACAAGGA GGCCAAGGAG
   2640
   AGCGTGGACG CCCTGTTCGT GAACAGCCAG TACGACCGCC TGCAGGCCGA CACCAACATC
   2700
   GCCATGATCC ACGCCGCCGA CAAGCGCGTG CACAGCATCC GCGAGGCCTA CCTGCCCGAG
   2760
   CTGAGCGTGA TCCCCGGCGT GAACGCCGCC ATCTTCGAGG AGCTGGAGGG CCGCATCTTC
   2820
   ACCGCCTTCA GCCTGTACGA CGCCCGCAAC GTGATCAAGA ACGGCGACTT CAACAACGGC
   2880
   CTGAGCTGCT GGAACGTGAA GGGCCACGTG GACGTGGAGG AGCAGAACAA CCACCGCAGC
   2940
   GTGCTGGTGG TGCCCGAGTG GGAGGCCGAG GTGAGCCAGG AGGTGCGCGT GTGCCCCGGC
   3000
   CGCGGCTACA TCCTGCGCGT GACCGCCTAC AAGGAGGGCT ACGGCGAGGG CTGCGTGACC
   3060
   ATCCACGAGA TCGAGAACAA CACCGACGAG CTGAAGTTCA GCAACTGCGT GGAGGAGGAG
   3120
   GTGTACCCCA ACAACACCGT GACCTGCAAC GACTACACCG CCACCCAGGA GGAGTACGAG
   3180
   GGCACCTACA CCAGCCGCAA CCGCGGCTAC GACGGCGCCT ACGAGAGCAA CAGCAGCGTG
   3240
   CCCGCCGACT ACGCCAGCGC CTACGAGGAG AAGGCCTACA CCGACGGCCG CCGCGACAAC 3300
   CCCTGCGAGA GCAACCGCGG CTACGGCGAC TACACCCCCC TGCCCGCCGG CTACGTGACC 3360
   AAGGAGCTGG AGTACTTCCC CGAGACCGAC AAGGTGTGGA TCGAGATCGG CGAGACCGAG 3420
   GGCACCTTCA TCGTGGACAG CGTGGAGCTG CTGCTGATGG AGGAGTAGTA CATG 3474
(2) INFORMATION FOR SEQ ID NO:54:
   (i) SEQUENCE CHARACTERISTICS:
      (A) LENGTH: 3508 base pairs
      (B) TYPE: nucleic acid
      (C) STRANDEDNESS: single
      (D) TOPOLOGY: linear
   (ii) MOLECULE TYPE: DNA
   (iv) ANTI-SENSE: NO
   (vi) ORIGINAL SOURCE:
      (A) ORGANISM: Full length synthetic maize optimized BT CryIA(b) gene
   (xi) SEQUENCE DESCRIPTION: SEQ ID NO:54:
   GATCCAACAA TGGACAACAA CCCCAACATC AACGAGTGCA TCCCCTACAA CTGCCTGAGC 60
   AACCCCGAGG TGGAGGTGCT GGGCGGCGAG CGCATCGAGA CCGGCTACAC CCCCATCGAC 120
   ATCAGCCTGA GCCTGACCCA GTTCCTGCTG AGCGAGTTCG TGCCCGGCGC CGGCTTCGTG 180
   CTGGGCCTGG TGGACATCAT CTGGGGCATC TTCGGCCCCA GCCAGTGGGA CGCCTTCCTG 240
   GTGCAGATCG AGCAGCTGAT CAACCAGCGC ATCGAGGAGT TCGCCCGCAA CCAGGCCATC 300
   AGCCGCCTGG AGGGCCTGAG CAACCTGTAC CAAATCTACG CCGAGAGCTT CCGCGAGTGG 360
   GAGGCCGACC CCACCAACCC CGCCCTGCGC GAGGAGATGC GCATCCAGTT CAACGACATG 420
   AACAGCGCCC TGACCACCGC CATCCCCCTG TTCGCCGTGC AGAACTACCA GGTGCCCCTG 480
   CTGAGCGTGT ACGTGCAGGC CGCCAACCTG CACCTGAGCG TGCTGCGCGA CGTCAGCGTG 540
   TTCGGCCAGC GCTGGGGCTT CGACGCCGCC ACCATCAACA GCCGCTACAA CGACCTGACC 600
   CGCCTGATCG GCAACTACAC CGACCACGCC GTGCGCTGGT ACAACACCGG CCTGGAGCGC 660
   GTGTGGGGTC CCGACAGCCG CGACTGGATC AGGTACAACC AGTTCCGCCG CGAGCTGACC 720
   CTGACCGTGC TGGACATCGT GAGCCTGTTC CCCAACTACG ACAGCCGCAC CTACCCCATC 780
   CGCACCGTGA GCCAGCTGAC CCGCGAGATT TACACCAACC CCGTGCTGGA GAACTTCGAC 840
   GGCAGCTTCC GCGGCAGCGC CCAGGGCATC GAGGGCAGCA TCCGCAGCCC CCACCTGATG 900
   GACATCCTGA ACAGCATCAC CATCTACACC GACGCCCACC GCGGCGAGTA CTACTGGAGC
   960
   GGCCACCAGA TCATGGCCAG CCCCGTCGGC TTCAGCGGCC CCGAGTTCAC CTTCCCCCTG
   1020
   TACGGCACCA TGGGCAACGC TGCACCTCAG CAGCGCATCG TGGCACAGCT GGGCCAGGGA
   1080
   GTGTACCGCA CCCTGAGCAG CACCCTGTAC CGTCGACCTT TCAACATCGG CATCAACAAC
   1140
   CAGCAGCTGA GCGTGCTGGA CGGCACCGAG TTCGCCTACG GCACCAGCAG CAACCTGCCC
   1200
   AGCGCCGTGT ACCGCAAGAG CGGCACCGTG GACAGCCTGG ACGAGATCCC CCCTCAGAAC
   1260
   AACAACGTGC CACCTCGACA GGGCTTCAGC CACCGTCTGA GCCACGTGAG CATGTTCCGC
   1320
   AGTGGCTTCA GCAACAGCAG CGTGAGCATC ATCCGTGCAC CTATGTTCAG CTGGATTCAC
   1380
   CGCAGTGCCG AGTTCAACAA CATCATCCCC AGCAGCCAGA TCACCCAGAT CCCCCTGACC
   1440
   AAGAGCACCA ACCTGGGCAG CGGCACCAGC GTGGTGAAGG GCCCCGGCTT CACCGGCGGC
   1500
   GACATCCTGC GCCGCACCAG CCCCGGCCAG ATCAGCACCC TGCGCGTGAA CATCACCGCC
   1560
   CCCCTGAGCC AGCGCTACCG CGTCCGCATC CGCTACGCCA GCACCACCAA CCTGCAGTTC
   1620
   CACACCAGCA TCGACGGCCG CCCCATCAAC CAGGGCAACT TCAGCGCCAC CATGAGCAGC
   1680
   GGCAGCAACC TGCAGAGCGG CAGCTTCCGC ACCGTGGGCT TCACCACCCC CTTCAACTTC
   1740
   AGCAACGGCA GCAGCGTGTT CACCCTGAGC GCCCACGTGT TCAACAGCGG CAACGAGGTG
   1800
   TACATCGACC GCATCGAGTT CGTGCCCGCC GAGGTGACCT TCGAGGCCGA GTACGACCTG
   1860
   GAGAGGGCTC AGAAGGCCGT GAACGAGCTG TTCACCAGCA GCAACCAGAT CGGCCTGAAG
   1920
   ACCGACGTGA CCGACTACCA CATCGATCAG GTGAGCAACC TGGTGGAGTG CCTGAGCGAC
   1980
   GAGTTCTGCC TGGACGAGAA GAAGGAGCTG AGCGAGAAGG TGAAGCACGC CAAGCGCCTG
   2040
   AGCGACGAGC GCAACCTGCT GCAGGACCCC AACTTCCGCG GCATCAACCG CCAGCTGGAC
   2100
   CGCGGCTGGC GCGGCAGCAC CGACATCACC ATCCAGGGCG GCGACGACGT GTTCAAGGAG
   2160
   AACTACGTGA CCCTGCTGGG CACCTTCGAC GAGTGCTACC CCACCTACCT GTACCAGAAG
   2220
   ATCGACGAGA GCAAGCTGAA GGCCTACACC CGCTACCAGC TGCGCGGCTA CATCGAGGAC
   2280
   AGCCAGGACC TGGAGATCTA CCTGATCCGC TACAACGCCA AGCACGAGAC CGTGAACGTG
   2340
   CCCGGCACCG GCAGCCTGTG GCCCCTGAGC GCCCCCAGCC CCATCGGCAA GTGCGCCCAC 2400
   CACAGCCACC ACTTCAGCCT GGACATCGAC GTGGGCTGCA CCGACCTGAA CGAGGACCTG 2460
   GGCGTGTGGG TGATCTTCAA GATCAAGACC CAGGACGGCC ACGCCCGCCT GGGCAACCTG 2520
   GAGTTCCTGG AGGAGAAGCC CCTGGTGGGC GAGGCCCTGG CCCGCGTGAA GCGCGCCGAG 2580
   AAGAAGTGGC GCGACAAGCG CGAGAAGCTG GAGTGGGAGA CCAACATCGT GTACAAGGAG 2640
   GCCAAGGAGA GCGTGGACGC CCTGTTCGTG AACAGCCAGT ACGACCGCCT GCAGGCCGAC 2700
   ACCAACATCG CCATGATCCA CGCCGCCGAC AAGCGCGTGC ACAGCATTCG CGAGGCCTAC 2760
   CTGCCCGAGC TGAGCGTGAT CCCCGGCGTG AACGCCGCCA TCTTCGAGGA GCTGGAGGGC 2820
   CGCATCTTCA CCGCCTTCAG CCTGTACGAC GCCCGCAACG TGATCAAGAA CGGCGACTTC 2880
   AACAACGGCC TGAGCTGCTG GAACGTGAAG GGCCACGTGG ACGTGGAGGA GCAGAACAAC 2940
   CACCGCAGCG TGCTGGTGGT GCCCGAGTGG GAGGCCGAGG TGAGCCAGGA GGTGCGCGTG 3000
   TGCCCCGGCC GCGGCTACAT CCTGCGCGTG ACCGCCTACA AGGAGGGCTA CGGCGAGGGC 3060
   TGCGTGACCA TCCACGAGAT CGAGAACAAC ACCGACGAGC TCAAGTTCAG CAACTGCGTG 3120
   GAGGAGGAGG TGTACCCCAA CAACACCGTG ACCTGCAACG ACTACACCGC CACCCAGGAG 3180
   GAGTACGAGG GCACCTACAC CAGCCGCAAC CGCGGCTACG ACGGCGCCTA CGAGAGCAAC 3240
   AGCAGCGTGC CCGCCGACTA CGCCAGCGCC TACGAGGAGA AGGCCTACAC CGACGGCCGC 3300
   CGCGACAACC CCTGCGAGAG CAACCGCGGC TACGGCGACT ACACCCCCCT GCCCGCCGGC 3360
   TACGTGACCA AGGAGCTGGA GTACTTCCCC GAGACCGACA AGGTGTGGAT CGAGATCGGC 3420
   GAGACCGAGG GCACCTTCAT CGTGGACAGC GTGGAGCTGC TGCTGATGGA GGAGTAGTAC 3480
   ATGTGATAGT ACGTAAGCTC GAGGATCT 3508
(2) INFORMATION FOR SEQ ID NO:55:
   (i) SEQUENCE CHARACTERISTICS:
      (A) LENGTH: 1961 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (A) ORGANISM: Truncated synthetic maize optimized BT CryIA(b) gene
   (xi) SEQUENCE DESCRIPTION: SEQ ID NO:55:
   GATCCAACAA TGGACAACAA CCCCAACATC AACGAGTGCA TCCCCTACAA CTGCCTGAGC 60
   AACCCCGAGG TGGAGGTGCT GGGCGGCGAG CGCATCGAGA CCGGCTACAC CCCCATCGAC 120
   ATCAGCCTGA GCCTGACCCA GTTCCTGCTG AGCGAGTTCG TGCCCGGCGC CGGCTTCGTG 180
   CTGGGCCTGG TGGACATCAT CTGGGGCATC TTCGGCCCCA GCCAGTGGGA CGCCTTCCTG 240
   GTGCAGATCG AGCAGCTGAT CAACCAGCGC ATCGAGGAGT TCGCCCGCAA CCAGGCCATC 300
   AGCCGCCTGG AGGGCCTGAG CAACCTGTAC CAAATCTACG CCGAGAGCTT CCGCGAGTGG 360
   GAGGCCGACC CCACCAACCC CGCCCTGCGC GAGGAGATGC GCATCCAGTT CAACGACATG 420
   AACAGCGCCC TGACCACCGC CATCCCCCTG TTCGCCGTGC AGAACTACCA GGTGCCCCTG 480
   CTGAGCGTGT ACGTGCAGGC CGCCAACCTG CACCTGAGCG TGCTGCGCGA CGTCAGCGTG 540
   TTCGGCCAGC GCTGGGGCTT CGACGCCGCC ACCATCAACA GCCGCTACAA CGACCTGACC 600
   CGCCTGATCG GCAACTACAC CGACCACGCC GTGCGCTGGT ACAACACCGG CCTGGAGCGC 660
   GTGTGGGGTC CCGACAGCCG CGACTGGATC AGGTACAACC AGTTCCGCCG CGAGCTGACC 720
   CTGACCGTGC TGGACATCGT GAGCCTGTTC CCCAACTACG ACAGCCGCAC CTACCCCATC 780
   CGCACCGTGA GCCAGCTGAC CCGCGAGATT TACACCAACC CCGTGCTGGA GAACTTCGAC 840
   GGCAGCTTCC GCGGCAGCGC CCAGGGCATC GAGGGCAGCA TCCGCAGCCC CCACCTGATG 900
   GACATCCTGA ACAGCATCAC CATCTACACC GACGCCCACC GCGGCGAGTA CTACTGGAGC 960
   GGCCACCAGA TCATGGCCAG CCCCGTCGGC TTCAGCGGCC CCGAGTTCAC CTTCCCCCTG 1020
   TACGGCACCA TGGGCAACGC TGCACCTCAG CAGCGCATCG TGGCACAGCT GGGCCAGGGA 1080
   GTGTACCGCA CCCTGAGCAG CACCCTGTAC CGTCGACCTT TCAACATCGG CATCAACAAC 1140
   CAGCAGCTGA GCGTGCTGGA CGGCACCGAG TTCGCCTACG GCACCAGCAG CAACCTGCCC 1200
   AGCGCCGTGT ACCGCAAGAG CGGCACCGTG GACAGCCTGG ACGAGATCCC CCCTCAGAAC 1260
   AACAACGTGC CACCTCGACA GGGCTTCAGC CACCGTCTGA GCCACGTGAG CATGTTCCGC 1320
   AGTGGCTTCA GCAACAGCAG CGTGAGCATC ATCCGTGCAC CTATGTTCAG CTGGATTCAC 1380
   CGCAGTGCCG AGTTCAACAA CATCATCCCC AGCAGCCAGA TCACCCAGAT CCCCCTGACC 1440
   AAGAGCACCA ACCTGGGCAG CGGCACCAGC GTGGTGAAGG GCCCCGGCTT CACCGGCGGC 1500
   GACATCCTGC GCCGCACCAG CCCCGGCCAG ATCAGCACCC TGCGCGTGAA CATCACCGCC 1560
   CCCCTGAGCC AGCGCTACCG CGTCCGCATC CGCTACGCCA GCACCACCAA CCTGCAGTTC 1620
   CACACCAGCA TCGACGGCCG CCCCATCAAC CAGGGCAACT TCAGCGCCAC CATGAGCAGC 1680
   GGCAGCAACC TGCAGAGCGG CAGCTTCCGC ACCGTGGGCT TCACCACCCC CTTCAACTTC 1740
   AGCAACGGCA GCAGCGTGTT CACCCTGAGC GCCCACGTGT TCAACAGCGG CAACGAGGTG 1800
   TACATCGACC GCATCGAGTT CGTGCCCGCC GAGGTGACCT TCGAGGCCGA GTACGACCTG 1860
   GAGAGGGCTC AGAAGGCCGT GAACGAGCTG TTCACCAGCA GCAACCAGAT CGGCCTGAAG 1920
   ACCGACGTGA CCGACTACCA CATCGATCAG GTGTAGGAGC T 1961

## Description

The present invention relates to a method of controlling *Sesamia* species in crop plants by use of toxin proteins obtainable from *Bacillus thuringiensis* and/or other *Bacillus* species.

*Bacillus thuringiensis* belongs to the large group of gram-positive, aerobic, endospore-forming bacteria. Unlike other very closely related species of *Bacillus* such as *B. cereus* or *B. anthracis,* the majority of the hitherto known *Bacillus thuringiensis* species produce in the course of their sporulation a parasporal inclusion body which, due to its crystalline structure, is generally referred to also as a crystalline body. This crystalline body is composed of insecticidally active crystalline protoxin proteins, the so-called δ-endotoxins.

These protein crystals are responsible for the toxicity to insects of *Bacillus thuringiensis.* The δ-endotoxin does not exhibit its insecticidal activity until after oral ingestion of the crystalline body, when the latter is dissolved in the intestinal juice of the target insects. In most cases the actual toxic component is released from the protoxin as a result of proteolytic cleavage caused by the action of proteases from the digestive tract of the insects.

The δ-endotoxins of the various *Bacillus thuringiensis* strains are characterized by high specificity toward certain target insects, especially with respect to various Lepidoptera, Coleoptera and Diptera larvae, and by a high degree of activity against such succeptible larvae. A further advantage in using δ-endotoxins of *Bacillus thuringiensis* resides in the fact that the toxins are harmless to humans, other mammals, birds and fish.

The various insecticidal crystal proteins from *Bacillus thuringiensis* have been classified based upon their spectrum of activity and sequence similarity. The classification put forth by Höfte and Whiteley, Microbiol. Rev. 53: 242-255 (1989) placed the then known insecticidal crystal proteins into four major classes. Generally, the major classes are defined by their spectrum of activity, with the Cryl proteins active against Lepidoptera, Cryll proteins active against both Lepidoptera and Diptera, Crylll proteins beeing active against Goleoptera, and CryIV proteins against Diptera.

Within each major class, the δ-endotoxins are grouped according to sequence similarity. The Cryl proteins are typically produced as 130-140 kDa protoxin proteins which are proteolytically cleaved to produce insecticidally active toxin proteins about 60-70 kDa in size. The active portion of the δ-endotoxin resides in the NH₂-terminal portion of the full-length molecule. Höfte and Whiteley, *supra,* classified the then known Cryl proteins into six groups, IA(a), IA(b), IA(c), IB, IC, and ID. Since then, proteins classified as CryIE, CryIF, CryIG, CryIH and CryIX have also been characterized.

The spectrum of insecticidal activity of an individual δ―endotoxin from *Bacillus thuringiensis* tends to be quite narrow, with a given δ-endotoxin being active against only a few insects. Specificity is the result of the efficiency of the various steps involved in producing an active toxin protein and its subsequent ability to interact with the epithelial cells in the insect digestive tract.

Up to now no such activity is reported for species of the lepidopteran genus *Sesamia*, which cause considerable damage in cereal crops, especially in the southern hemisphere including the mediterranean area. The yield losses caused by *Sesamia* species in maize are in a range of between 5% to 10%, but can reach up to 40% in severe cases. To the contrary, it was believed in the art that *Bt* toxins such as the Cryl-type toxins mentioned above are not active against *Sesamia* species and can not be suitably used for controlling *Seamia* pests in crop plants.

It is therefore one of the objects of this invention to provide a method of controlling *Sesamia* species in crop plants, but preferably in maize and other cereal plants, including, but not limited to, *Sesamia nonagrioides, S inferens, S calamistis, S cretica,* etc. This object could surprisingly be achieved within the scope of the invention by applying a δ-endotoxin protein of *Bacillus thuringiensis* to the crop plant to be protected. In particular, a class of toxin proteins obtainable from vegetative cultures of *Bacillus* species, the so-called VIPs including VIP1, VIP2 and VIP3 [EP-A 0 690 916; International Application no EP95/03826, the disclosure of which is incorporated herein by reference in its entirety], can be used in a method according to the invention for controlling *Sesamia* pests.

The present invention thus relates to a method for protecting plants against damage caused by *Sesamia* species comprising directly or indirectly administering to the plant or the plant seed or the growing area to be protected a toxin protein of *Bacillus spp,* preferably a VIP-type protein mentioned above, either purely or in form of an entomocidal composition comprising at least one of the said proteins or a microorganism, but especially a *Bacillus thuringiensis* and/or a *Bacillus cereus* strain, containing at least one toxin gene encoding the said toxin proteins. Said microorganisms used in a method according to the invention may either be naturally occurring strains or, in the alternative, a recombinant strain comprising the toxin encoding DNA in recombinant form.

In a further embodiment, a transgenic plant according to the invention may be used in a method of protecting plants against damage caused by *Sesamia* species comprising transforming the said plant with a toxin gene encoding a toxin protein from a *Bacillus species,* but especially a VIP-type protein or a VIP-type protein and a Cryl-type protein, and expressing the said toxin protein in an amount sufficient to provide control against *Sesamia* species upon planting the so transformed plant within an area where the said insect pest may occur.

CrylA type genes are known from various *Bacillus thuringiensis* species including, but not limited to, *Bt kurstaki* HD1 [cry IA(a); Schnepf *et al* (1985) J Biol Chem 260, 6264-6272], *Bt* subsp *berliner* [cry IA(b); Wabiko *et al* (1986) DNA 5, 305-314; Höfte *et al* (1986) Eur J Biochem 161, 273-280], *Bt* subsp *kurstaki* HD-73 [cry IA(c); Adang *et al* (1985) Gene 36, 289-300], *Bt* HD2 [cry IB; Brizzard *et al* (1988) Nucl Acids Res 16, 2723-2724], *Bt* subsp *entomocidus* [cryIC; Honee *et al* (1988) Nucl Acids Res 16, 6240], *Bt* subsp *aizawai* [cry IC, crylD; cryIF; crylX; Sanchis *et al* (1988), EP-A 0 295 156; Gawron-Burke *et al* (1991), WO 91/16434], *Bt* subsp *darmstadiensis* [cry IE; Hofte and Whiteley (1989), EP-A 0 358 557], and *Bt* subsp *kenyae* [cryIE; Visser *et al* (1990) J Bacteriol 172, 6783-6788; Kramer *et al* (1995, WO 95/34656]. Further cryl-type genes are disclosed in European Application 0 367 474, 0 401 979 and in PCT International Application WO 90/13651. Preferred for use within the scope of the present invention are the expression products of crylA-type genes, but especially of crylA(b) type genes such as those derived from *Bacillus thuringiensis* var *kurstaki* or of synthetic variants thereof. Especially preferred are the crylA(b) type genes shown in SEQ ID NOS: 53 to 55.

The entomocidal compositions to be used in the method according to the invention for protecting crop plants against *Sesamia* pests preferably comprise as an active ingredient at least one toxin protein from *Bacillus thuringiensis* or a microorganism containing at least one gene encoding the said toxin protein, but especially a *Bacillus thuringiensis* strain containing at least one gene encoding the said toxin protein, or a derivative or mutant thereof, together with an agricultural adjuvant such as a carrier, diluent, surfactant or application-promoting adjuvant. The active ingredient contained in the entomocidal composition may be a VIP-type toxin protein as disclosed in the European Application EP-A 0 690 916 and the PCT International Application no EP95/03826 or a microorganism containing at least one gene encoding the said VIP-type toxin protein or a combination of Cryl-type and VIP-type proteins either in an essentially pure form or as part of a microorganism. Included within the scope of this application are VIP-type toxin proteins as shown in SEQ ID NOS: 1, 2, 4-7, 17-24, 26-32, 35, 36, 39, 40, 42, 43, 45, 46, 49, 50, 51 or 52.

The composition may also contain a further biologically active compound. The said compound can be both a fertilizer or micronutrient donor or other preparations that influence plant growth. It can also be a selective herbicide, insecticide, fungicide, bactericide, nematicide, molluscide or mixtures of several of these preparations, if desired, together with further agriculturally acceptable carriers, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers

The composition may comprise from 0.1 to 99% by weight of the active ingredient, from 1 to 99.9% by weight of a solid or liquid adjuvant, and from 0 to 25% by weight of a surfactant. The active ingredient or the composition containing the said active ingredient, may be administered to the plants or crops to be protected together with certain other insecticides or chemicals (1993 Crop Protection Chemicals Reference, Chemical and Pharmaceutical Press, Canada) without loss of potency. It is compatible with most other commonly used agricultural spray materials but should not be used in extremely alkaline spray solutions if a Cryl-type toxin is involved. It may be administered as a dust, a suspension, a wettable powder or in any other material form suitable for agricultural application.

The active ingredient, that is preferably a VIP-type proteins mentioned previously, or the composition comprising the said active ingredient may be applied to (a) an environment in which the insect pest may occur, (b) a plant or plant part in order to protect said plant or plant part from damage caused by an insect pest, or (c) seed in order to protect a plant which develops from said seed from damage caused by an insect pest.

A preferred method of application in the area of plant protection is application to the foliage of the plants (foliar application), with the number of applications and the rate of application depending on the plant to be protected and the risk of infestation by the pest in question. However, the active ingredient may also penetrate the plants through the roots (systemic action) if the locus of the plants is impregnated with a liquid formulation or if the active ingredient is incorporated in solid form into the locus of the plants, for example into the soil, e.g. in granular form (soil application). in paddy rice crops, such granules may be applied in metered amounts to the flooded rice field.

The compositions to be used in a method according to the invention are also suitable for protecting plant propagating material, e.g. seed, such as fruit, tubers or grains, or plant cuttings, from insect pests. The propagation material can be treated with the formulation before planting: seed, for example, can be dressed before being sown. The active ingredient of the invention can also be applied to grains (coating), either by impregnating the grains with a liquid formulation or by coating them with a solid formulation. The formulation can also be applied to the planting site when the propagating material is being planted, for example to the seed furrow during sowing. The invention relates also to those methods of treating plant propagation material and to the plant propagation material thus treated.

Within the scope of the invention the compositions may be applied in any method known for treatment of seed or soil with bacterial strains. For example, see US Patent No.4,863,866. The strains are effective for biocontrol even if the microorganism is not living. Preferred is, however, the application of the living microorganism.

Target crops to be protected within the scope of the present invention are those that are host plants for *Sesamia* species including, but not limited to, the following species of plants: maize, wheat, barley, rye, oats, rice, sorghum, millet and related crops, forage grasses (orchardgrass, fescue, and the like), and sugar cane.

The active ingredient according to the invention may be used in unmodified form or together with any suitable agriculturally acceptable carrier. Such carriers are adjuvants conventionally employed in the art of agricultural formulation, and are therefore formulated in known manner to emulsifiable concentrates, coatable pastes, directly sprayable or dilutable solutions, dilute emulsions, wettable powders, soluble powders, dusts, granulates, and also encapsulations, for example, in polymer substances. Like the nature of the compositions, the methods of application, such as spraying, atomizing, dusting, scattering or pouring, are chosen in accordance with the intended objective and the prevailing circumstances. Advantageous rates of application are normally from about 50 g to about 5 kg of active ingredient (a.i.) per hectare ("ha", approximately 2.471 acres), preferably from about 100 g to about 2kg a.i./ha. Important rates of application are about 200 g to about 1 kg a.i./ha and 200g to 500g a.i./ha.

For seed dressing advantageous application rates are 0.5 g to 1000 g a.i.per 100 kg seed, preferably 3 g to 100 g a.i. per 100 kg seed or 10 g to 50 g a.i.per 100 kg seed.

Suitable carriers and adjuvants can be solid or liquid and correspond to the substances ordinarily employed in formulation technology, e.g. natural or regenerated mineral substances, solvents, dispersants, wetting agents, tackifiers, binders or fertilizers. The formulations, i.e. the entomocidal compositions, preparations or mixtures thereof with other active ingredients, and, where appropriate, a solid or liquid adjuvant, are prepared in known manner, e.g., by homogeneously mixing and/or grinding the active ingredients with extenders, e.g., solvents, solid carriers, and in some cases surface-active compounds (surfactants).

Suitable solvents are: aromatic hydrocarbons, preferably the fractions containing 8 to 12 carbon atoms, e.g. xylene mixtures or substituted naphthalenes, phthalates such as dibutyl phthalate or dioctyl phthalate, aliphatic hydrocarbons such as cyclohexane or paraffins, alcohols and glycols and their ethers and esters, such as ethanol, ethylene glycol monomethyl or monoethyl ether, ketones such as cyclohexanone, strongly polar solvents such as N-methyl-2-pyrrolidone, dimethylsulfoxide or dimethylformamide, as well as vegetable oils or epoxidised vegetable oils such as epoxidised coconut oil or soybean oil; or water.

The solid carriers used, e.g., for dusts and dispersible powders, are normally natural mineral fillers such as calcite, talcum, kaolin, montmorillonite or attapulgite. In order to improve the physical properties it is also possible to add highly dispersed silicic acid or highly dispersed absorbent polymers. Suitable granulated adsorptive carriers are porous types, for example pumice, broken brick, sepiolite or bentonite; and suitable nonsorbent carriers are materials such as calcite or sand. In addition, a great number of pregranulated materials of inorganic or organic nature can be used, e.g. especially dolomite or pulverized plant residues.

Depending on the nature of the active ingredients to be formulated, suitable surface-active compounds are non-ionic, cationic and/or anionic surfactants having good emulsifying, dispersing and wetting properties. The term "surfactants" will also be understood as comprising mixtures of surfactants. Suitable anionic surfactants can be both water-soluble soaps and water-soluble synthetic surface-active compounds. Suitable soaps are the alkali metal salts, alkaline earth metal salts or unsubstituted or substituted ammonium salts of higher fatty acids (C sub 10 -C sub 22), e.g. the sodium or potassium salts of oleic or stearic acid, or of natural fatty acid mixtures which can be obtained, e.g. from coconut oil or tallow oil. Further suitable surfactants are also the fatty acid methyltaurin salts as well as modified and unmodified phospholipids.

More frequently. however, so-called synthetic surfactants are used, especially fatty sulfonates, fatty sulfates, sulfonated benzimidazole derivatives or alkylarylsulfonates. The fatty sulfonates or sulfates are usually in the forms of alkali metal salts, alkaline earth metal salts or unsubstituted or substituted ammonium salts and generally contain a C sub 8 -C sub 22 alkyl radical which also includes the alkyl moiety of acyl radicals, e.g. the sodium or calcium salt of lignosulfonic acid, of dodecylsulfate, or of a mixture of fatty alcohol sulfates obtained from natural fatty acids. These compounds also comprise the salts of sulfuric acid esters and sulfonic acids of fatty alcohol/ethylene oxide adducts. The sulfonated benzimidazole derivatives preferably contain 2 sulfonic acid groups and one fatty acid radical containing about 8 to 22 carbon atoms. Examples of alkylarylsulfonates are the sodium, calcium or triethanolamine salts of dodecylbenzenesulfonic acid, dibutylnaphthalenesulfonic acid, or of a naphthalenesulfonic acid/formaldehyde condensation product. Also suitable are corresponding phosphates, e.g. salts of the phosphoric acid ester of an adduct of p-nonylphenol with 4 to 14 moles of ethylene oxide.
Non-ionic surfactant are preferably polyglycol ether derivatives of aliphatic or cycloaliphatic alcohols, or saturated or unsaturated fatty acids and alkylphenols, said derivatives containing 3 to 30 glycol ether groups and 8 to 20 carbon atoms in the (aliphatic) hydrocarbon moiety and 6 to 18 carbon atoms in the alkyl moiety of the alkylphenols.

Further suitable non-ionic surfactants are the water-soluble adducts of polyethylene oxide with polypropylene glycol, ethylenediaminopolypropylene glycol and alkylpolypropylene glycol containing 1 to 10 carbon atoms in the alkyl chain, which adducts contain 20 to 250 ethylene glycol ether groups and 10 to 100 propylene glycol ether groups. These compounds usually contain 1 to 5 ethylene glycol units per propylene glycol unit. Representative examples of non-ionic surfactants are nonylphenolpolyethoxyethanols, castor oil polyglycol ethers, polypropylene/polyethylene oxide adducts, tributylphenoxypolyethoxyethanol, polyethylene glycol and octylphenoxypolyethoxyethanol. Fatty acid esters of polyoxyethylene sorbitan, such as polyoxyethylene sorbitan trioleate, are also suitable non-ionic surfactants.

Cationic surfactants are preferably quaternary ammonium salts which contain, as N-substituent, at least one C sub 8 -C sub 22 alkyl radical and, as further substituents, lower unsubstituted or halogenated alkyl, benzyl or hydroxyl-lower alkyl radicals. The salts are preferably in the form of halides, methylsulfates or ethylsulfates, e.g., stearyltrimethylammonium chloride or benzyldi-(2-chloroethyl)ethylammonium bromide.

The surfactants customarily employed in the art of formulation are described, e.g., in "McCutcheon's Detergents and Emulsifiers Annual", MC Publishing Corp. Ridgewood, N.J., 1979; Dr. Helmut Stache, "Tensid Taschenbuch" (Handbook of Surfactants), Carl Hanser Verlag, Munich/Vienna.

Another particularly preferred characteristic of an entomocidal composition of the present invention is the persistence of the active ingredient when applied to plants and soil. Possible causes for loss of activity include inactivation by ultra-violet light, heat, leaf exudates and pH. For example, at high pH, particularly in the presence of reductant, δ-endotoxin crystals are solubilized and thus become more accessible to proteolytic inactivation. High leaf pH might also be important, particularly where the leaf surface can be in the range of pH 8-10. Formulation of an entomocidal composition to be used in a method according to the present invention can address these problems by either including additives to help prevent loss of the active ingredient or encapsulating the material in such a way that the active ingredient is protected from inactivation. Encapsulation can be accomplished chemically (McGuire and Shasha, J Econ Entomol 85: 1425-1433, 1992) or biologically (Barnes and Cummings, 1986; EP-A 0 192 319). Chemical encapsulation involves a process in which the active ingredient is coated with a polymer while biological encapsulation involves the expression of the δ-endotoxin genes in a microbe. For biological encapsulation, the intact microbe containing the toxin protein is used as the active ingredient in the formulation. The addition of UV protectants might effectively reduce irradiation damage. Inactivation due to heat could also be controlled by including an appropriate additive.

Preferred within the present application are formulations comprising living microorganisms as an active ingredient either in form of the vegetative cell or more preferable in form of spores, if available. Suitable formulations may consist, for example, of polymer gels which are crosslinked with polyvalent cations and comprise these microorganisms. This is described, for example, by D.R. Fravel et al. in Phytopathology, Vol. 75, No. 7, 774-777, 1985 for alginate as the polymer material. It is also known from this publication that carrier materials can be co-used. These formulations are as a rule prepared by mixing solutions of naturally occurring or synthetic get-forming polymers, for example alginates, and aqueous salt solutions of polyvalent metal ions such that individual droplets form, it being possible for the microorganisms to be suspended in one of the two or in both reaction solutions. Gel formation starts with the mixing in drop form. Subsequent drying of these gel particles is possible. This process is called ionotropic gelling. Depending on the degree of drying, compact and hard particles of polymers which are structurally crosslinked via polyvalent cations and comprise the microorganisms and a carrier present predominantly uniformly distributed are formed. The size of the particles can be up to 5 mm.

Compositions based on partly crosslinked polysaccharides which, in addition to a microorganism, for example, can also comprise finely divided silicic acid as the carrier material, crosslinking taking place, for example, via Ca⁺⁺ ions, are described in EP-A1-0 097 571. The compositions have a water activity of not more than 0.3. W.J. Cornick et al. describe in a review article [New Directions in Biological Control: Alternatives for Suppressing Agricultural Pests and Diseases, pages 345-372, Alan R. Liss, Inc. (1990)] various formulation systems, granules with vermiculite as the carrier and compact alginate beads prepared by the ionotropic gelling process being mentioned. Such compositions are also disclosed by D.R.Fravel in Pesticide Formulations and Application Systems: 11th Volume, ASTM STP 1112 American Society for Testing and Materials, Philadelphia, 1992, pages 173 to 179 and can be used to formulate the recombinant microorganisms according to the invention. Further methods for formulating living microorganism are described in. WO96/02638.

The entomocidal compositions to be used in a method according to the invention usually contain from about 0.1 to about 99%, preferably about 0.1 to about 95%, and most preferably from about 3 to about 90% of the active ingredient, from about 1 to about 99.9%, preferably from about 1 to about 99%, and most preferably from about 5 to about 95% of a solid or liquid adjuvant, and from about 0 to about 25%, preferably about 0.1 to about 25%, and most preferably from about 0.1 to about 20% of a surfactant.

Whereas commercial products are preferably formulated as concentrates, the end user will normally employ dilute formulations of substantially lower concentration. The entomocidal compositions may also contain further ingredients, such as stabilizers, antifoams, viscosity regulators, binders, tackifiers as well as fertilizers or other active ingredients in order to obtain special effects.

It has surprisingly been found that a quantitatively variable combination of two active ingredients, on the one hand a Cry-type or δ-endotoxin protein and on the other hand a VIP-type protein, exhibits a synergistic action that is advantageously suitable for controlling *Sesamia* species in crop plants and which extends the limits of the action of both toxin proteins from two points of view:
Firstly, the rates of application of the compound of the Cry-type toxin protein and of the VIP-type proteins are reduced while retaining an equally good action. Secondly, the combined mixture also achieves a high degree of pest control where both individual substances have become completely ineffective when unduly low rates are applied. This allows an increased safety upon use.

The compositions according to the invention are valuable for preventive and/or curative treatment in the field of pest control even at low rates of application while being well tolerated by and non-toxic to warm-blooded species, fish and plants and have a very favourable biocidal spectrum. The compositions according to the invention are active against all or individual development stages of *Sesamia* pests. The insecticidal action of the compounds according to the invention can become obvious either directly, i.e. by destroying the pests immediately or only after some time has elapsed.

Accordingly, the invention further relates to a insecticidal composition comprising as an active ingredient a Cry-type toxin protein and a VIP-type protein, preferably in a synergistically effective amount together with a agronomically acceptable carrier. Preferred is a composition comprising a combination of a Cryl-type protein and a VIP-type protein.

A synergistic effect is always present when the action of the combination of the active ingredient of the Cry-type toxin protein with the VIP-type proteins exceeds the total of the action of the active ingredients applied individually. However, not only quantitative means are to be applied in assessing a synergistic activity, but also qualitative means such as, for example, a widening of the acitivity spectrum .

The said composition can be provided in form of a chemical mixture comprising the toxin proteins in an essentially pure form or in form of a mixture comprising at least one of the toxin proteins as part of a microorganism or a transgenic plant.
In a specific embodiment of the invention, one of the active ingredients may be applied to the plant directly by, for example, leaf application as described herein previously, whereas the second active principle may be provided by the plant itself upon expression of a previously transformed gene encoding the said second principle.

A further object of the invention relates to the use of a recombinant microorganisms comprising a toxin gene encoding a toxin protein of *Bacillus thuringiensis* in a method of controlling crop plants against damages caused by *Sesamia* species, which recombinant organism can either be applied directly to the plant to be protected or the recombinantly produced toxin protein can first be isolated from the recombinant microorganism and formulated in the above described manner before being applied to the crop plant to be protected. The recombinant microorganism may contain a toxin gene encoding a VIP-type toxin protein as disclosed in the European Application EP-A 0 690 916 and the PCT International Application no EP95/03826, or a combination of genes encoding at least a Cry-type toxin and a VIP-type toxin, respectively.

For recombinant production of the toxin protein in a host organism, the coding sequence may be inserted into an expression cassette designed for the chosen host and introduced into the host where it is recombinantly produced. The choice of specific regulatory sequences such as promoter, signal sequence, 5' and 3' untranslated sequences, and enhancer appropriate for the chosen host is within the level of skill of the routineer in the art. The resultant molecule, containing the individual elements linked in proper reading frame, may be inserted into a vector capable of being transformed into the host cell. Suitable expression vectors and methods for recombinant production of proteins are well known for host organisms such as *E. coli* (see, *e.g.* Studier and Moffatt, *J. Mol. Biol. 189:* 113 (1986); Brosius, *DNA 8:* 759 (1989)), yeast (see, e.g., Schneider and Guarente, *Meth. Enzymol. 194:* 373 (1991)) and insect cells (see, *e.g.*, Luckow and Summers, *Bio*/*Technol. 6*: 47 (1988)). Specific examples include plasmids such as pBluescript (Stratagene, La Jolla, CA), pFLAG (International Biotechnologies, Inc., New Haven, CT), pTrcHis (Invitrogen, La Jolla, CA), and baculovirus expression vectors, e.g., those derived from the genome of *Autographica califomica* nuclear polyhedrosis virus (AcMNPV). A preferred baculovirus/insect system is pVI11392/Sf21 cells (lnvitrogen, La Jolla, CA).

The recombinantly produced toxin protein can be isolated and purified using a variety of standard techniques. The actual techniques which may be used will vary depending upon the host organism used, whether the toxin protein is designed for secretion, and other such factors familiar to the skilled artisan (see, *e.g.* chapter 16 of Ausubel, F. *et al.,* "Current Protocols in Molecular Biology", pub. by John Wiley & Sons, Inc. (1994).

A further object of the invention relates to the use of transgenic plants comprising and expressing a toxin gene encoding a toxin protein of *Bacillus thuringiensis* in an amount sufficient to provide control against *Sesamia* species, in a method of protecting crop plants against damages caused by *Sesamia* pests. Especially preferred are transgenic plants expressing a toxin gene encoding a VIP-type protein as described in EP-A 0 690 916 and the PCT International Application no. EP95/03826, herein incorporated by reference in its entirety. The invention also relates to the use of transgenic plants comprising and expressing a toxin gene encoding a toxin protein of *Bacillus thuringiensis,* but especially a Cry-type toxin protein, and also comprising and expressing a toxin gene encoding a VIP-type protein in an amount sufficient to provide control against *Sesamia* species. A host plant expressing the said toxin genes will have enhanced resistance to insect attack of *Sesamia* species and will be thus better equipped to withstand crop losses associated with such attack.

In one preferred embodiment, expression of one or more Bt δ-endotoxins in a transgenic plant is accompanied by the expression of one or more VIP-type proteins. This co-expression of more than one insecticidal principle in the same transgenic plant can be achieved by genetically engineering a plant to contain and express all the genes necessary. Alternatively, a plant, Parent 1, can be genetically engineered for the expression of VIP-type proteins. A second plant, Parent 2, can be genetically engineered for the expression of Bt δ-endotoxin. By crossing Parent 1 with Parent 2, progeny plants are obtained which express all the genes introduced into Parents 1 and 2. Particularly preferred Bt δ-endotoxins are those disclosed in EP-A 0618976, herein incorporated by reference.

Also comprised by the present invention is the use of recombinant microorganisms or transgenic plants comprising a gene encoding DNA molecules which hybridizes to a DNA molecule encoding a toxin protein of *Bacillus* species, but preferably to an oligonucleotide probe obtainable from said DNA molecule comprising a contiguous portion of the coding sequence for the said toxin protein at least 10 nucleotides in length, under moderately stringent conditions. The invention preferably comprises the use of recombinant microorganisms or transgenic plants comprising a gene encoding DNA molecules which hybridizes to a DNA molecule encoding a toxin protein of *Bacillus thuringiensis* or *B cereus* especially to a DNA molecule encoding a VIP-type toxin protein.

Factors that effect the stability of hybrids determine the stringency of the hybridization. One such factor is the melting temperature Tₘ which can be easily calculated according to the formula provided in DNA PROBES, George H. Keller and Mark M. Manak , Macmillan Publishers Ltd, 1993, Section one: Molecular Hybridization Technology; page 8 ff.

The preferred hybridization temperature is in the range of about 25°C below the calculated melting temperature Tₘ and preferably in the range of about 12-15°C below the calculated melting temperature Tₘ and in the case of oligonucleotides in the range of about 5-10°C below the melting temperature Tₘ.

The invention further relates to a commercial bag comprising seed of a transgenic plant comprising at least a toxin gene encoding a toxin protein of *Bacillus thuringiensis,* preferably a VIP type toxin protein, and expressing the said toxin protein in an amount sufficient to provide control against *Sesamia* species, together with labele instructions for the use thereof for control of *Sesamia* pests in crop plants. Preferred within this invention is a commercial bag comprising seed of a transgenic plant comprising as an active ingredient a gene encoding at least a Cry-type toxin protein and a VIP-type protein, preferably in a synergistically effective amount. Especially preferred is a combination of a CrylA(b) toxin protein with a VIP-type protein.

The further object of the invention is a commercial bag comprising an insecticidal composition according to the invention together with labele instructions for the use thereof for control of *Sesamia* pests in crop plants.

By plant is meant any plant species which can be genetically transformed by methods known in the art, but especially those plants that are host plants for *Sesamia* species including, but not limited to, the following species of plants: maize, wheat, barley, rye, oats, rice, sorghum, millet and related crops, forage grasses (orchardgrass, fescue, and the like), and sugar cane.

Methods known in the art for plant transformation are discussed below. Host plants include, but are not limited to, those species previously listed as target crops.

It has been discovered that the codon usage of a native *Bacillus thuringiensis* toxin gene is significantly different from that which is typical of a plant gene. In particular, the codon usage of a native *Bacillus thuringiensis* gene is very different from that of a maize gene. As a result, the mRNA from this gene may not be efficiently utilized. Codon usage might influence the expression of genes at the level of translation or transcription or mRNA processing. To optimize a toxin gene for expression in plants, for example in maize, the codon usage is optimized by using the codons which are most preferred in maize (maize preferred codons) in the synthesis of a synthetic gene which encodes the same protein as found for the native toxin gene sequence. The optimized maize preferred codon usage is effective for expression of high levels of the *Bt* insecticidal protein. Further details for constructing maize-optimized synthetic toxin genes can be found in WO 93/07278, herein incorporated by reference in its entirety.

Toxin genes derived from microorganisms may also differ from plant genes. Plant genes differ from genes found in microorganisms in that their transcribed RNA does not possess defined ribosome binding site sequence adjacent to the initiating methionine. Consequently, microbial genes can be enhanced by the inclusion of a eukaryotic consensus translation initiator at the ATG. Clontech (1993/1994 catalog, page 210) has suggested the sequence GTCGACCATGGTC as a consensus translation initiator for the expression of the *E. coli uidA* gene in plants. Further, Joshi (Nucl Acids Res 15: 6643-6653 (1987)) has compared many plant sequences adjacent to the ATG and suggests the consensus TAAACAATGGCT. In situations where difficulties are encountered in the expression of microbial ORFs in plants, inclusion of one of these sequences at the initiating ATG may improve translation. In such cases the last three nucleotides of the consensus may not be appropriate for inclusion in the modified sequence due to their modification of the second AA residue. Preferred sequences adjacent to the initiating methionine may differ between different plant species. By surveying the sequence of maize genes present in the GenBank/EMBL database it can be discerned which nucleotides adjacent to the ATG should be modified to enhance translation of the toxin gene introduced into maize.

In addition, it has been shown that removal of illegitimate splice sites can enhance expression and stability of introduced genes. Genes cloned from non-plant sources and not optimized for expression in plants may contain motifs which can be recognized in plants as 5' or 3' splice sites. Consequently, the transcription process can be prematurely terminated, generating truncated or deleted mRNA. The toxin genes can be engineered to remove these illegitimate splice sites using the techniques well known in the art.

It is well known that many δ-endotoxin proteins from *Bacillus thuringiensis* are actually expressed as protoxins. These protoxins are solubilized in the alkaline environment of the insect gut and are proteolytically converted by proteases into a toxic core fragment (Höfte and Whiteley, Microbiol. Rev. 53: 242-255 (1989)). For δ-endotoxin proteins of the Cryl class, the toxic core fragment is localized in the N-terminat half of the protoxin. It is within the scope of the present invention that genes encoding either the full-length protoxin form or the truncated toxic core fragment of the novel toxin protein can be used in plant transformation vectors to confer insecticidal properties upon the host plant.

The recombinant DNA molecules can be introduced into the plant cell in a number of art-recognized ways. Those skilled in the art will appreciate that the choice of method might depend on the type of plant, i.e. monocot or dicot, targeted for transformation. Suitable methods of transforming plant cells include microinjection (Crossway et al., BioTechniques 4:320-334 (1986)), electroporation (Riggs at al, Proc. Natl. Acad. Sci. USA 83:5602-5606 (1986), *Agrobacterium-*mediated transformation (Hinchee et al., Biotechnology 6:915-921 (1988)), direct gene transfer (Paszkowski et al., EMBO J. 3:2717-2722 (1984)), and ballistic particle acceleration using devices available from Agracetus, Inc., Madison, Wisconsin and Dupont, Inc., Wilmington, Delaware (see, for example, Sanford et al., U.S. Patent 4,945,050; and McCabe et al., Biotechnology 6:923-926 (1988)). See also, Weissinger et at., Annual Rev. Genet. 22:421-477 (1988); Sanford et al., Particulate Science and Technology 5:27-37 91987)(onion); Christou et at., Plant Physiol. 87:671-674 (1988)(soybean); McCabe et al., Bio/Technology 6:923-926 (1988)(soybean); Datta et al., Bio/Technology 8:736-740 (1990)(rice); Klein et at., Proc. Natl. Acad. Sci. USA, 85:4305-4309 (1988)(maize); Klein et al., Bio/Technology 6:559-563 (1988)(maize); Klein et al., Plant Physiol. 91:440-444 (1988)(maize); Fromm et at., Bio/Technology 8:833-839 (1990); and Gordon-Kamm et al., Plant Cell 2:603-618 (1990)(maize); Svab et at. Proc. Natl. Acad. Sci. USA 87: 8526-8530 (1990) (tobacco chloroplast); Koziel *et al.* (Biotechnology 11: 194-200 (1993)) (maize); Shimamoto *et al.* Nature 338: 274-277 (1989) (rice); Christou *et al.* Biotechnology 9: 957-962 (1991) (rice); European Patent Application EP 0 332 581 (orchardgrass and other *Pooideae);* Vasil *et al.* (Biotechnology 11: 1553-1558 (1993) (wheat); Weeks *et al.* (Plant Physio). 102: 1077-1084 (1993) (wheat); Wan *et al* (Plant Physio) 104: 37-48 (1994) (barley); Umbeck *et al,* (Bio/Technology 5: 263-266 (1987) (cotton).

One particularly preferred set of embodiments for the introduction of recombinant DNA molecules into maize by microprojectile bombardment can be found in WO 93/07278, herein incorporated by reference in its entirety. An additional preferred embodiment is the protoplast transformation method for maize as disclosed in European Patent Application EP 0 292 435, hereby incorporated by reference in its entirety.

The genetic properties engineered into the transgenic seeds and plants described above are passed on by sexual reproduction or vegetative growth and can thus be maintained and propagated in progeny plants. Generally said maintenance and propagation make use of known agricultural methods developed to fit specific purposes such as tilling, sowing or harvesting. Specialized processes such as hydroponics or greenhouse technologies can also be applied. As the growing crop is vulnerable to attack and damages caused by insects or infections as well as to competition by weed plants, measures are

undertaken to control weeds, plant diseases, insects, nematodes, and other adverse conditions to improve yield. These include mechanical measures such a tillage of the soil or removal of weeds and infected plants, as well as the application of agrochemicals such as herbicides, fungicides, gametocides, nematicides, growth regulants, ripening agents and insecticides.

Use of the advantageous genetic properties of the transgenic plants and seeds according to the invention can further be made in plant breeding which aims at the development of plants with improved properties such as tolerance of pests, herbicides, or stress, improved nutritional value, increased yield, or improved structure causing less loss from lodging or shattering. The various breeding steps are characterized by well-defined human intervention such as selecting the lines to be crossed, directing pollination of the parental lines, or selecting appropriate progeny plants. Depending on the desired properties different breeding measures are taken. The relevant techniques are well known in the art and include but are not limited to hybridization, inbreeding, backcross breeding, multiline breeding, variety blend, interspecific hybridization, aneuploid techniques, etc. Hybridization techniques also include the sterilization of plants to yield male or female sterile plants by mechanical, chemical or biochemical means. Cross pollination of a male sterile plant with pollen of a different line assures that the genome of the male sterile but female fertile plant will uniformly obtain properties of both parental lines. Thus, the transgenic seeds and plants according to the invention can be used for the breeding of improved plant lines which for example increase the effectiveness of conventional methods such as herbicide or pesticide treatment or allow to dispense with said methods due to their modified genetic properties. Alternatively new crops with improved stress tolerance can be obtained which, due to their optimized genetic "equipment", yield harvested product of better quality than products which were not able to tolerate comparable adverse developmental conditions.

In seeds production germination quality and uniformity of seeds are essential product characteristics, whereas germination quality and uniformity of seeds harvested and sold by the farmer is not important. As it is difficult to keep a crop free from other crop and weed seeds, to control seedborne diseases, and to produce seed with good germination, fairly extensive and well-defined seed production practices have been developed by seed producers, who are experienced in the art of growing, conditioning and marketing of pure seed. Thus, it is common practice for the farmer to buy certified seed meeting specific quality standards instead of using seed harvested from his own crop. Propagation material to be used as seeds is customarily treated with a protectant coating comprising herbicides, insecticides, fungicides, bactericides, nematicides, molluscicides or mixtures thereof. Customarily used protectant coatings comprise compounds such as captan, carboxin, thiram (TMTD®), methalaxyl (Apron®), and pirimiphos-methyl (Actellic®). If desired these compounds are formulated together with further carriers, surfactants or application-promoting adjuvants customarily employed in the art of formulation to provide protection against damage caused by bacterial, fungal or animal pests. The protectant coatings may be applied by impregnating propagation material with a liquid formulation or by coating with a combined wet or dry formulation. Other methods of application are also possible such as treatment directed at the buds or the fruit.

It is a further aspect of the present invention to provide new agricultural methods such as the methods examplified above which are characterized by the use of transgenic plants, transgenic plant material, or transgenic seed according to the present invention to provide control against Sesamia species.

To breed progeny from plants transformed according to the method of the present invention, a method such as that which follows may be used: maize plants produced as described in the examples set forth below are grown in pots in a greenhouse or in soil, as is known in the art, and permitted to flower. Pollen is obtained from the mature tassel and used to pollinate the ears of the same plant, sibling plants, or any desirable maize plant. Similarly. the ear developing on the transformed plant may be pollinated by pollen obtained from the same plant, sibling plants, or any desirable maize plant. Transformed progeny obtained by this method may be distinguished from non-transformed progeny by the presence of the introduced gene(s) and/or accompanying DNA (genotype), or the phenotype conferred. The transformed progeny may similarly be selfed or crossed to other plants, as is normally done with any plant carrying a desirable trait. Similarly, tobacco or other transformed plants produced by this method may be selfed or crossed as is known in the art in order to produce progeny with desired characteristics. Similarly, other transgenic organisms produced by a combination of the methods known in the art and this invention may be bred as is known in the art in order to produce progeny with desired characteristics.

The so produced seeds and plants can be introduced into a plant cultivation scheme desinged to protect crop plants against damage cause by *Sesamia* species.

It is long knwon that control of the second generation of *Sesamia species* is difficult to achieve by application of insecticides because the larvae are usually located at the bottom of the full-grown plant and are thus difficult to reach by insecticide spraying. It is therefore advisable to control the diapause population in winter and the first generation of the *Sesamia species* in spring and thus to reduce the population of the second generation of insects. In winter, the diapause population can be controlled by turn of soil and simultaneous grinding of the remaining plant residues (plant stump, roots) after harvest. In spring, the first generation can be controlled by application of insecticides. As a result the second generation is reduced and can be controlled with less effort.

It is thus a further aspect of the present invention to provide an agricultural method for controlling the first and second generation of *Sesamia* insects, which method combines state of the art methods with those developed within the scope of this invention. In particular, the agricultural method according to the invention comprises combining mechanical, chemical and genetic engineering means in order to achieve a full control of *Sesamia* in crop plants.

The agricultural method for fully controlling *Sesamia* species in crop plants according to the invention comprises the use of seeds or plants to which a toxin protein of a *Bacillus* species, but preferably a VIP-type toxin protein or a VIP-type protein and a Cryl-type protein, has been applied directly or indirectly as an active ingredient. Direct application of the said toxin proteins to the seed or plant may be accomplished by treating the said seed or plants with a composition according to the invention as described hereinbefore. Alternatively, the active ingredients can be applied to the seed or plant to be protected indirectly, that is by expressing one or more genes encoding at least VIP-type protein or a VIP-type protein and a Cry-type protein within the said seed or plant, preferably in a synergistically effective amount.

Direct and indirect application of the active ingredients according to the invention may be combined such that either a VIP-type or a Cry-type protein is expressed within the plant while the other protein is applied to the plant externally.

The transgenic expression of a VIP-type a VIP-type and a Cry-type protein within the plant may also be combined with a chemical treatment of said plant using any one of the commonly applied insecticides.

The invention is thus further directed to an agricultural method for protecting plants against damage caused by *Sesamia* species comprising a combination of steps (2) and (3) with any one of steps (1) or (4):
(1) control of diapause population by mechanical grinding of the plant residues after harvest
(2) control of the first generation of Sesamia species by one of the methods according to the invention
(3) control of the second generation of Sesamia species by one of the methods according to the invention.
(4) control of the diapause population, the first and/or the second generation by treating them with a commonly applied insecticide.

A preferred embodiment of the present invention is the use of plants transformed according to the invention in an agricultural method for controlling *Sesamia* species by reducing the population of the first generation and, preferably also the population of the second generation of *Sesamia* species. Also preferred is the combination of transgenic plants with the application of compositions comprising a biological compound as defined above or a commonly applied insecticide.

### EXAMPLES

The following examples further describe the materials and methods used in carrying out the invention. They are offered by way of illustration, and not by way of limitation.

### EXAMPLE 1: General Methods

DNA manipulations were done using procedures that are standard in the art. These procedures can often be modified and/or substituted without substantively changing the result. Except where other references are identified, most of these procedures are described in Sambrook et at., Molecular Cloning: A Laboratory Manual, Cold Spring Harbor Laboratory Press, second edition, 1989.

### EXAMPLE 2: Plant Transformation

### 2.1 Transformation Vector

Plant transformation is accomplished using plant transformation vectors pCIB 4431 and pCIB 3064, the construction of which is described in WO 93/07278 and in Koziel *et* al (1993) [Biotechnology Vol 11, 194-200], the disclosure of which are incorporated herein by reference.

pCIB4431 is a vector designed to transform maize. it contains two chimeric synthetic *Bt crylA(b)* endotoxin genes expressible in maize. These genes are the PEP carboxylase promoter/synthetic-*crylA(b)* and a pollen promoter/synthetic-*crylA(b)*.

pCIB 4431 containing the synthetic crylA(b) gene provided as SEQ ID NO: 1 was deposited September 21, 1992 with the Agricultural Research Service, Patent Culture Collection (NRRL), Northern Regional Research Center, 1815 North University Street, Peoria, Illinois 61604, U.S.A. under accession no NRRL #B-18998.

pCIB 3064 contains a plant expressible bar gene (615 bp), which was originally cloned from *Streptomyces hygroscopicus* [Thompson et *al* (1987) EMBO J 6, 2519-2523]. It encodes a phosphinotricin acetyltransferase (PAT), conferring tolerance to phosphinotricin. The *bar* gene is under the control of the CaMV 35S promoter and terminator [OW *et al* (1987) Proc Natl Acad Sci USA 84, 4870-4874] to provide resistance to phosphinotricin.

### EXAMPLE 3: Production of transgenic maize plants containing the synthetic maize CrylA(b) gene

The example below utilizes a Biolistic device to introduce DNA coated particles into maize cells, from which transformed plants are generated.

### 3.1 Tissue

Immature maize embryos, approximately 1.5-2.5 mm in length, were excised from an ear of genotype 6N615 14-15 days after pollination. The mother plant was grown in the greenhouse. Before excision, the ear was surface sterilized with 20% Clorox for 20 minutes and rinse 3 times with sterile water. Individual embryos were plated scutellum side up in a 2 cm square area, 36 embryos to a plate, on the callus initiation medium, 2DG4 + 5 chloramben medium (N6 major salts, B5 minor salts, MS iron, 2% sucrose, with 5 mg/l chloramben, 20 mg/l glucose, and 10 ml G4 additions (Table 1) added after autoclaving.

**TABLE 1 -**

| G4 Additions | |
|---|---|
| Ingredient | per liter medium |
| Casein hydrolysate | 0.5 g |
| Proline | 1.38 g |
| Nicotinic acid | 0.2 mg |
| Pyridoxine-HCI | 0.2 mg |
| Thiamine-HCI | 0.5 mg |
| Choline-HCl | 0.1 mg |
| Riboflavin | 0.05 mg |
| Biotin | 0.1 mg |
| Folic acid | 0.05 mg |
| Ca pantothenate | 0.1 mg |
| p-aminobenzoic acid | 0.05 mg |
| B12 | 0.136 µg |

### 3.2 Preparation of DNA for delivery

The microcarrier was prepared essentially according to the instructions supplied with the Biolistic device. While vortexing 50 µl 1.0 µm gold microcarrier, 5 µl of pCIB4431 (1.23 µg/µl) [#898] + 2 µl pCIB3064 (0.895 µg/µl) [#456] was added followed by 50 µl 2.5 M CaCl₂, then 20 µl 0.1 M spermidine (free base, TC grade). The resulting mixture was vortexed 3 minutes and microfuged for 10 sec. The supernatant was removed and the microcarriers washed 2 times with 250 µl of 100% EtOH (HPLC grade) by vortexing briefly, centrifuging and removing the supernatant. The microcarriers are resuspended in 65 µl 100% EtOH.

### 3.3 Bombardment

Tissue was bombarded using the PDS-1000He Biolistics device. The tissue was placed on the shelf 8 cm below the stopping screen shelf. The tissue was shot one time with the DNA/gold microcarrier solution, 10 µl dried onto the macrocarrier. The stopping screen used was hand punched using 10x10 stainless steel mesh screen. Rupture discs of 1550 psi value were used. After bombardment, the embryos were cultured in the dark at 25° C.

### 3.4 Callus formation

Embryos were transferred to callus initiation medium with 3 mg/l PPT 1 day after bombardment. Embryos were scored for callus initiation at 2 and 3 weeks after bombardment. Any responses were transferred to callus maintenance medium, 2DG4 + 0.5 2,4-D medium with 3 mg/L PPT. Callus maintenance medium is N6 major salts, B5 minor salts, MS iron, 2% sucrose, with 0.5 mg/l 2,4-D, 20 mg/l glucose, and 10 ml G4 additions added after autoclaving. Embryogenic callus was subcultured every 2 weeks to fresh maintenance medium containing 3 mg/L PPT. All callus was incubated in the dark at 25°C. The Type I callus formation response was 15%. Every embryo which produced callus was cultured as an individual event giving rise to an individual line.

### 3.5 Regeneration

After 12 weeks on selection, the tissue was removed from callus maintenance medium with PPT and was placed on regeneration medium. Regeneration medium is 0.25MS3S5BA (0.25 mg/l 2,4 D, 5 mg/l BAP, MS salts, 3% sucrose) for 2 weeks followed by subculture to MS3S medium for regeneration of plants. After 4 to 10 weeks, plants were removed and put into GA 7's.

### 3.6 Backcrossing

Regenerated transgenic plants are back-crossed into a different genetic background [2N217AF; 6Y021] resulting in Events AB01 [2N217AF(Bt)] and AB10 [6Y021 (Bt)], which are hemizygous for the *Bt* trait. Non-transforemd Inbred lines 2N217AF [CG01] and 6Y021 [CG10] are used as controls in the *Sesamia* Assay described below in Example 4.

### EXAMPLE 4: Sesamia Assay

Parameters recorded: 24/48 hours old larvae of *Sesamia nonagrioides* [MCB] were allowed to feed on maize leaves. Larvae had not previously been feeding. 72 and 120/152 hours later the number of larvae alive and development instar of each one were recorded. The kind of feeding injuries on leaf plant was observed in each case.

Treatments: Four different maize "events" were used (AB01, AB10, CG01, CG10). Leaves were exised and used in the feeding experiments as described below.

Number of larvae tested: Groups of 5 larvae were put inside translucent plastic containers with several pieces of maize leaves. Containers were cylindrical, diameter 5,4 cm and 3 cm high. Sixteen groups of 5 larvae each (80 larvae) were used for each "event". So, a total of 320 MCB larvae were used for all the evaluation. This was carried out in 2 steps. In the first step 24 hours old larvae were used; in the second step 48 hours old larvae.

### Experimental conditions: Temperature 25±1°C, Photoperiod: 16L/8D

### Summary of results:

Percentage of mortality after 72 hours (mean ± standard error):
AB01: 84±8% (n=15, n is the number of containers with 5 larvae)
AB10: 87±7% (n=15, n is the number of containers with 5 larvae)
CG10: 2,5±1,7% (n=16, n is the number of containers with 5 larvae)
CG01: 3,8±2,7% (n=16, n is the number of containers with 5 larvae)

Percentage of mortality after 120/152 hours (mean ± standard error):
ABOL: 100% (n=15, n is the number of containers with 5 larvae)
AB10: 100% (n=15, n is the number of containers with 5 larvae)
CG10: 14±3% (n=16, n is the number of containers with 5 larvae)
CG01: 14±5% (n=16, n is the number of containers with 5 larvae)

Development: Since most of larvae on AB01 and AB10 died before first observation (72h.), no host effect on larval development could be observed. However, there were some indications of larval development delay in AB01 and AB10.

Injuries on excised maize leaves: Two kind of injuries were clearly distinguished in maize leaves. Leaf pieces coded as AB01 and AB10 showed small holes in the upper epidermis whereas those coded as CG10 and CG01 showed wide areas without upper epidermis. Larvae on AB01 and AB10 scarcely ate and no excrements were observed in rearing containers. On the contrary, larvae on CG01 and CG10 ate big areas of upper epidermis and abundant excrements in rearing container was observed.

Conclusion: The results of the *Seamia* assay show a strong effect of AB01 and AB10 on larval mortality and larval feeding. With the methodology used in the present trials no differences between AB01 and AB10 were detected. Injuries in AB01 and AB10 were much lower than in CG01 and CG10.

### EXAMPLE 5: EFFECTS OF Vip3A ON NEONATE Sesamia nonagrioides LARVAE.

| **plant material** | **Expe- riment** | **number initial larvae** | **larvae alive after 72 h** | **mortality after 72h(%)** | **larvae alive after 120 h** | **mortality after 120 h (%)** | **leaves bitten** |
|---|---|---|---|---|---|---|---|
| **CG00526** **(control)** | 1 | 5 | 5 | | 5 | | Yes |
| | 2 | 5 | 5 | | 5 | | Yes |
| | 3 | 5 | 5 | | 5 | | Yes |
| | 4 | 5 | 5 | | 5 | | Yes |
| **Total** | | 20 | 20 | 0 | 20 | 0 | |
| **CG00526 x 891** **(negative segregants)** | 1 | 5 | 5 | 0 | 4 | 20 | Yes |
| **CG00526 x 891** **(positive segregants)** | 1 | 5 | 1 | | 0 | | No |
| | 2 | 5 | 0 | | 0 | | No |
| | 3 | 5 | 0 | | 0 | | No |
| **Total** | | 15 | 1 | 94 | 0 | 100 | |
| **CG00526 x 892** **(negative segregants)** | 1 | 5 | 4 | 20 | 4 | 20 | Yes |
| **CG00526 x 891** **(positive segregants)** | 1 | 5 | 0 | | 0 | | No |
| | 2 | 5 | 0 | | 0 | | No |
| | 3 | 5 | 1 | | 0 | | No |
| **Total** | | 15 | 1 | 94 | 0 | 100 | |

Effects of Vip3A on neonate larvae of the Mediterranean Corn Borer *Sesamia nonagrioides*. CG00526 is the maize genotype used as negative control. It is an inbred line, which has been described by Koziel et al. (1993), Bio/Technology 11, pp. 194-200.
The gene encoding the Vip3A protein was transformed into the CG00526 genotype by biolistics. The crosses CG00526 x 891 and 892 refer to crosses of transformation events (891 and 892) with the parental line CG00526. Two of the events obtained expressing the Vip3A protein have been used for the insect bioassay (positive segregants). Positive segregants are plants that inherited the *vip3A* gene. Negative segregants are T1 plants that did not inherit the *vip3A* gene, therefore they do not express the Vip3A protein.

## Claims

1. A method for protecting plants including the progeny thereof against damage caused by *Sesamia* species, comprising directly or indirectly applying to the plant or the plant seed or to the growing area of the plant a toxin protein of a *Bacillus* species as the active ingredient, wherein the toxin protein is a VIP3-type protein.

2. A method according to claim 1, wherein the VIP3-type protein is a VIP3A(a) protein or a VIP3A(b) protein.

3. A method according to claims 1 or 2, wherein the toxin protein is a VIP3-type protein according to SEQ ID Nos. 28-32, 51 or 52.

4. A method according to anyone of claims 1-3, wherein the toxin protein is applied to the plant in form of an entomocidal composition.

5. A method according to claim 4, wherein the entomocidal composition comprises a microorganism, but especially a *Bacillus thuringiensis* and/or a *Bacillus cereus* strain, containing at least one toxin gene encoding the said toxin protein together with a suitable carrier.

6. A method according to claim 5, wherein the microorganism contains at least one VIP3-type protein gene such as a VIP3A(a) protein or a VIP3A(b) protein gene encoding the said toxin protein.

7. A method according to claims 5 or 6, wherein the toxin protein is a VIP3-type protein according to SEQ ID Nos. 28-32, 51 or 52.

8. A method according to anyone of claims 4-7, wherein said composition comprises a further toxin protein, wherein said further protein is a Cry-type protein, preferably a Cryl-type protein, more preferably a CrylA-type protein, and most preferably a CrylA(b) protein or a microorganism expressing said toxin protein.

9. A method according to claims 6 or 7, wherein the microorganism is a recombinant-organism.

10. A method according to any one of claims 1 to 4, wherein the toxin protein is indirectly applied to the plant, by expressing within the said plant and the progeny thereof a toxin gene encoding a toxin protein of *Bacillus* species to produce the said toxin protein in an amount sufficient to provide control against *Sesamia* species upon planting the so transformed plant within an area where the said insect pest may occur, wherein the toxin gene encodes a VIP3-type toxin protein.

11. A method according to claim 10, wherein the VIP3-type protein is a VIP3A(a) protein or a VIP3A(b) protein.

12. A method according to claims 10 or 11, wherein the toxin gene is a synthetic gene the codon usage of which is optimized by using the codons which are most preferred in plants.

13. A method according to any one of claims 10-12, wherein the toxin gene encodes a VIP3-type protein according to SEO ID Nos. 28-32, 51 or 52.

14. A method according to any one of claims 1 to 13, wherein the plant to be protected is a cereal plant.

15. A method according to claim 14, wherein the plant to be protected is a maize plant.

16. A method according to any one of claims 10-15, wherein at least a VIP3-type toxin protein is expressed in the plant to be protected in combination with a Cry-type toxin protein in an amount sufficient to provide control against *Sesamia* pests.

17. A composition comprising as an active ingredient at least a VIP3-type protein and a Cry-type toxin protein in an insecticidally effective amount together with an agronomically acceptable carrier.

18. Use of an active ingredient as defined in the previous claims, for controlling *Sesamia* pests in crop plants.

19. Use of a composition according to claim 17 for controlling *Sesamia* pests in crop plants.

20. Use of a transgenic plant as defined in claim 10 for controlling *Sesamia* pests in crop plants.

21. Use of recombinant microorganisms or transgenic plants as defined in the previous claims comprising a DNA molecule which hybridizes to the complement of a VIP3-type gene encoding the respective toxin protein under moderate stringent conditions for controlling *Sesamia* pests in crop plants.

22. A commercial bag comprising seed of a transgenic plant or a microorganism, but especially a *Bacillus thuringiensis* and/or a *Bacillus cereus* strain, comprising at least a toxin gene encoding a toxin protein as defined in the previous claims, and expressing the said toxin protein in an amount sufficient to provide control against *Sesamia* species, together with label instructions for the use thereof for control of *Sesamia* pests in crop plants.

23. A commercial bag comprising seed of a transgenic plant or a microorganism, but especially a *Bacillus thuringiensis* and/or a *Bacillus cereus* strain, containing at least a VIP3-type protein or a VIP3-type protein and a Cryl-type toxin protein as defined herein before together with a suitable carrier in an amount sufficient to provide control against *Sesamia* species, together with label instructions for the use thereof for control of *Sesamia* pests in crop plants.

24. A commercial bag comprising an insecticidal composition according to claim 17 together with label instructions for the use thereof for control of *Sesamia* pests in crop plants.

25. An agricultural method, wherein a transgenic plant or the progeny thereof is used comprising a toxin gene encoding a toxin protein of a *Bacillus species* and expressing the said toxin protein in an amount sufficient to provide control against *Sesamia* species or transgenic seed thereof, as defined herein before.

26. A transgenic plant expressing at least a VIP3-type protein in combination with at least a Cryl-type toxin protein as defined herein before in an amount sufficient to provide control against *Sesamia* pests.

27. A transgenic plant according to claim 26, wherein the Cry-type protein is a CrylA(b) toxin protein.

28. A transgenic plant according to claims 26 or 27 which is a maize plant.

## Patentansprüche

1. Verfahren zum Schutz von Pflanzen, einschliesslich deren Nachkommenschaft, gegen Schädigung, die durch Sesamia-Arten verursacht wird, umfassend das direkte oder indirekte Ausbringen eines Toxinproteins einer Bacillus-Art als aktiver Bestandteil auf die Pflanze oder den Pflanzensamen oder auf das Anbaugebiet der Pflanze, worin das Toxinprotein ein Protein vom VIP3-Typ ist.

2. Verfahren gemäss Anspruch 1, worin das Protein vom VIP3-Typ ein VIP3A(a)-Protein oder ein VIP3A(b)-Protein ist.

3. Verfahren gemäss Anspruch 1 oder 2, worin das Toxinprotein ein Protein vom VIP3-Typ gemäss SEQ ID NOS: 28-32, 51 oder 52 ist.

4. Verfahren gemäss einem der Ansprüche 1 bis 3, worin das Toxinprotein auf die Pflanze in Form einer insektiziden Zusammensetzung ausgebracht wird.

5. Verfahren gemäss Anspruch 4, worin die insektizide Zusammensetzung einen Mikroorganismus, aber speziell einen Bacillus thuringiensis- und/oder einen Bacillus cereus-Stamm, der wenigstens ein Toxingen enthält, das das Toxinprotein codiert, zusammen mit einem geeigneten Träger umfasst.

6. Verfahren gemäss Anspruch 5, worin der Mikroorganismus wenigstens ein Gen für ein Protein vom VIP3-Typ enthält, wie ein Gen für ein VIP3A(a)-Protein oder ein VIP3A(b)-Protein, das das Toxinprotein codiert.

7. Verfahren gemäss Anspruch 5 oder 6, worin das Toxinprotein ein Protein von VIP3-Typ gemäss SEQ ID NOS: 28-32, 51 oder 52 ist.

8. Verfahren gemäss einem der Ansprüche 4 bis 7, worin die Zusammensetzung ein weiteres Toxinprotein, worin das weitere Protein ein Protein vom Cry-Typ ist, bevorzugt ein Protein vom CryI-Typ, besonders bevorzugt ein Protein vom CryIA-Typ und am meisten bevorzugt ein CryIA(b)-Protein ist, oder einen Mikroorganismus, der das Toxinprotein exprimiert, umfasst.

9. Verfahren gemäss Anspruch 6 oder 7, worin der Mikroorganismus ein rekombinanter Organismus ist.

10. Verfahren gemäss einem der Ansprüche 1 bis 4, worin das Toxinprotein indirekt auf die Pflanze ausgebracht wird, indem innerhalb der Pflanze und ihrer Nachkommenschaft ein Toxingen exprimiert wird, das ein Toxinprotein einer Bacillus-Art codiert, um das Toxinprotein in einer ausreichenden Menge zu erzeugen, um eine Bekämpfung gegen Sesamia-Arten bei Pflanzung der so transformierten Pflanze innerhalb eines Gebiets bereitzustellen, in dem der Insektenschädling auftreten kann, worin das Toxingen ein Toxinprotein vom VIP3-Typ codiert.

11. Verfahren gemäss Anspruch 10, worin das Protein vom VIP3-Typ ein VIP3A(a)-Protein oder ein VIP3A(b)-Protein ist.

12. Verfahren gemäss Anspruch 10 oder 11, worin das Toxingen ein synthetisches Gen ist, dessen Codonverwendung durch Verwendung der Codonen optimiert ist, die in Pflanzen am meisten bevorzugt sind.

13. Verfahren gemäss einem der Ansprüche 10 bis 12, worin das Toxingen ein Protein vom VIP3-Typ gemäss SEQ ID NOS: 28-32, 51 oder 52 codiert.

14. Verfahren gemäss einem der Ansprüche 1 bis 13, worin die zu schützende Pflanze eine Getreidepflanze ist.

15. Verfahren gemäss Anspruch 14, worin die zu schützende Pflanze eine Maispflanze ist.

16. Verfahren gemäss einem der Ansprüche 10 bis 15, worin wenigstens ein Toxinprotein vom VIP3-Typ in der zu schützenden Pflanze in Kombination mit einem Toxinprotein vom Cry-Typ in einer ausreichenden Menge zur Bereitstellung einer Bekämpfung gegen Sesamia-Schädlinge exprimiert wird.

17. Zusammensetzung, die als Wirkstoff wenigstens ein Protein vom VIP3-Typ und ein Toxinprotein vom Cry-Typ. in einer insektizid wirksamen Menge zusammen mit einem agronomisch akzeptablen Träger umfasst.

18. Verwendung eines Wirkstoffs wie in den vorhergehenden Ansprüchen definiert zur Bekämpfung von Sesamia-Schädlingen in Anbaupflanzen.

19. Verwendung einer Zusammensetzung gemäss Anspruch 17 zur Bekämpfung von Sesamia-Schädlingen in Anbaupflanzen.

20. Verwendung einer transgenen Pflanze wie in Anspruch 10 definiert zur Bekämpfung von Sesamia-Schädlingen in Anbaupflanzen.

21. Verwendung von rekombinanten Mikroorganismen oder transgenen Pflanzen wie in den vorhergehenden Ansprüchen definiert, die ein DNA-Molekül umfassen, das mit dem Komplement eines Gens vom VIP3-Typ, das das entsprechende Toxinprotein codiert, unter moderaten stringenten Bedingungen hybridisiert, zur Bekämpfung von Sesamia-Schädlingen in Anbaupflanzen.

22. Gewerbliche Tüte, die Saatgut einer transgenen Pflanze oder einen Mikroorganismus umfasst, aber speziell einen Bacillus thuringiensis- und/oder einen Bacillus cereus-Stamm, umfassend wenigstens ein Toxingen, das ein Toxinprotein wie in den vorhergehenden Ansprüchen definiert codiert, und das das Toxinprotein in einer ausreichenden Menge exprimiert, um eine Bekämpfung gegen Sesamia-Arten bereitzustellen, zusammen mit Etikettanweisungen für dessen Verwendung zur Bekämpfung von Sesamia-Schädlingen in Anbaupflanzen.

23. Gewerbliche Tüte, die Saatgut einer transgenen Pflanze oder einen Mikroorganismus umfasst, aber speziell einen Bacillus thuringiensis- und/oder einen Bacillus cereus-Stamm, enthaltend wenigstens ein Protein vom VIP3-Typ oder ein Protein vom VIP3-Typ und ein Toxinprotein vom CryI-Typ wie hier zuvor definiert zusammen mit einem geeigneten Träger in einer ausreichenden Menge, um eine Bekämpfung gegen Sesamia-Arten bereitzustellen, zusammen mit Etikettanweisungen für dessen Verwendung zur Bekämpfung von Sesamia-Schädlingen in Anbaupflanzen.

24. Gewerbliche Tüte, die eine insektizide Zusammensetzung gemäss Anspruch 17 zusammen mit Etikettanweisungen zu ihrer Verwendung zur Bekämpfung von Sesamia-Schädlingen in Anbaupflanzen umfasst.

25. Landwirtschaftliches Verfahren, worin eine transgene Pflanze oder deren Nachkommenschaft verwendet wird, umfassend ein Toxingen, das ein Toxinprotein einer Bacillus-Art codiert, und Exprimieren des Toxinproteins in einer ausreichenden Menge, um eine Bekämpfung gegen Sesamia-Arten bereitzustellen, oder transgenes Saatgut davon wie hier zuvor definiert.

26. Transgene Pflanze, die wenigstens ein Protein vom VIP3-Typ in Kombination mit wenigstens einem Toxinprotein vom CryI-Typ wie hier zuvor definiert in einer ausreichenden Menge exprimiert, um eine Bekämpfung gegen Sesamia-Schädlinge bereitzustellen.

27. Transgene Pflanze gemäss Anspruch 26, worin das Protein vom Cry-Typ ein CryIA(b)-Toxinprotein ist.

28. Transgene Pflanze gemäss Anspruch 26 oder 27, die eine Maispflanze ist.

## Revendications

1. Procédé pour protéger des plantes y compris leur descendance contre les dégâts causés par une espèce de *Sesamia,* comprenant l'application directe ou indirecte à la plante ou aux graines de la plante ou à la zone de croissance de la plante d'une protéine toxine d'une espèce de *Bacillus* comme ingrédient actif, où la protéine toxine est une protéine de type VIP3.

2. Procédé selon la revendication 1, où la protéine de type VIP3 est une protéine VIP3A(a) ou une protéine VIP3A(b).

3. Procédé selon la revendication 1 ou 2, où la protéine toxine est une protéine de type VIP3 selon SEQ ID NO. 28-32, 51 ou 52.

4. Procédé selon l'une quelconque des revendications 1 à 3, où la protéine toxine est appliquée à la plante sous forme d'une composition entomocide.

5. Procédé selon la revendication 4, où la composition entomocide comprend un microorganisme, mais en particulier une souche de *Bacillus thuringiensis* et/ou de *Bacillus cereus,* contenant au moins un gène de toxine codant ladite protéine toxine en même temps qu'un support approprié.

6. Procédé selon la revendication 5, où le microorganisme contient au moins un gène de protéine de type VIP3 comme un gène de protéine VIP3A(a) ou un gène de protéine VIP3A(b) codant ladite protéine toxine.

7. Procédé selon les revendications 5 ou 6, où la protéine toxine est une protéine de type VIP3 selon SEQ ID NO. 28-32, 51 ou 52.

8. Procédé selon l'une quelconque des revendications 4 à 7, où ladite composition comprend une autre protéine toxine, où ladite autre protéine est une protéine de type Cry, de préférence de type CryI, de w préférence encore une protéine de type CryIA, et de manière particulièrement préférable une protéine CryIA(b) ou un microorganisme exprimant ladite protéine toxine.

9. Procédé selon les revendications 6 ou 7, où le microorganisme est un organisme recombiné.

10. Procédé selon l'une quelconque des revendications 1 à 4, où la protéine toxine est appliquée indirectement à la plante, par expression dans ladite plante et sa descendance d'un gène de toxine codant une protéine toxine d'une espèce de *Bacillus* pour produire ladite protéine toxine en une quantité suffisante pour permettre une lutte contre une espèce de *Sesamia* lors de la plantation de la plante ainsi transformée dans une zone où ledit insecte nuisible peut survenir, où le gène de toxine code une protéine toxine de type VIP3.

11. Procédé selon la revendication 10, où la protéine de type VIP3 est une protéine VIP3A(a) ou une protéine VIP3(b).

12. Procédé seion les revendications 10 ou 11, où le gène de toxine est un gène synthétique dont l'usage des codons est optimisé par l'utilisation des codons qui sont particulièrement préférés dans les plantes.

13. Procédé selon l'une quelconque des revendications 10 à 12, où le gène de toxine code une protéine de type VIP3 selon SEQ ID NO.28-32, 51 ou 52.

14. Procédé selon l'une quelconque des revendications 1 à 13, où la plante à protéger est une plante de type céréale.

15. Procédé selon la revendication 14, où la plante à protéger est une plante de type maïs.

16. Procédé selon l'une quelconque des revendications 10 à 15, où au moins une protéine toxine de type VIP3 est exprimée dans la plante à protéger en combinaison avec une protéine toxine de type Cry en une quantité suffisante pour permettre une lutte contre les nuisibles *Sesamia.*

17. Composition comprenant comme ingrédient actif au moins une protéine de type VIP3 et une protéine toxine de type Cry en une quantité efficace du point de vue insecticide en même temps qu'un support agronomiquement acceptable.

18. Utilisation d'un ingrédient actif selon les revendications précédentes pour la lutte contre les nuisibles *Sesamia* dans des plantes cultivées.

19. Utilisation d'une composition selon la revendication 17, pour la lutte contre les nuisibles *Sesamia* dans des plantes cultivées.

20. Utilisation d'une plante transgénique selon la revendication 10, pour la lutte contre les nuisibles *Sesamia* dans des plantes cultivées.

21. Utilisation de microorganismes recombinés ou de plantes transgéniques selon la revendication précédente, comprenant une molécule d'ADN qui s'hybride au complément d'un gène de type *VIP3* codant la protéine toxine respective dans des conditions modérément astringentes pour la lutte contre des nuisibles *Sesamia* dans des plantes cultivées.

22. Sac du commerce comprenant des graines d'une plante transgénique ou un microorganisme, mais en particulier une souche de *Bacillus thuringiensis* et/ou de *Bacillus cereus,* comprenant au moins un gène de toxine codant une protéine toxine selon les revendications précédentes, et exprimant ladite protéine toxine en une quantité suffisante pour permettre une lutte contre des espèces de *Sesamia,* en même temps que des instructions étiquetées pour leur utilisation pour la lutte contre des nuisibles *Sesamia* dans des plantes cultivées.

23. Sac du commerce comprenant des graines d'une plante transgénique ou un microorganisme, mais en particulier une souche de *Bacillus thuringiensis* et/ou de *Bacillus cereus,* contenant au moins une protéine de type de VIP3 ou une protéine de VIP3 et une protéine toxine de type CryI définies précédemment en même temps qu'un support approprié en une quantité suffisante pour permettre une lutte contre des espèces de *Sesamia,* en même temps que des instructions étiquetées pour leur utilisation pour la lutte contre des nuisibles *Sesamia* dans des plantes cultivées.

24. Sac du commerce comprenant une composition insecticide selon la revendication 17 en même temps que des instructions étiquetées pour son utilisation pour la lutte contre des nuisibles *Sesamia* dans des plantes cultivées.

25. Procédé agricole, où une plante transgénique ou sa descendance est utilisée comprenant un gène de toxine codant une protéine toxine d'une espèce de *Bacillus* et exprimant ladite protéine toxine en une quantité suffisante pour permettre une lutte contre des espèces de *Sesamia* ou leurs graines transgéniques, comme défini précédemment.

26. Plante transgénique exprimant au moins une protéine de type VIP3 en combinaison avec au moins une protéine toxine de type CryI telle que définie précédemment en une quantité suffisante pour permettre une lutte contre des nuisibles *Sesamia.*

27. Plante transgénique selon la revendication 26, où la protéine de type Cry est une protéine toxine CryIA(b).

28. Plante transgénique selon les revendications 26 ou 27, qui est une plante de type maïs.
